(19) **Europäisches Patentamt / European Patent Office / Office européen des brevets**

(11) **EP 4 772 193 A1**

(12) **EUROPEAN PATENT APPLICATION**
published in accordance with Art. 153(4) EPC

(43) Date of publication:
**08.07.2026 Bulletin 2026/28**

(21) Application number: **24859867.4**

(22) Date of filing: **29.08.2024**

(51) International Patent Classification (IPC):
*A61K 39/395* (2006.01)     *A61P 3/00* (2006.01)
*A61P 3/04* (2006.01)     *A61P 3/10* (2006.01)
*A61P 9/00* (2006.01)     *A61P 9/10* (2006.01)
*A61P 21/00* (2006.01)     *A61P 25/02* (2006.01)
*A61P 25/04* (2006.01)     *A61P 29/00* (2006.01)
*A61P 35/00* (2006.01)     *A61P 37/06* (2006.01)
*A61P 43/00* (2006.01)     *C07K 16/28* (2006.01)
*C12N 15/13* (2006.01)     *C12N 15/63* (2006.01)
*C12N 15/85* (2006.01)     *C12P 21/08* (2006.01)

(52) Cooperative Patent Classification (CPC):
**A61K 39/395; A61P 3/00; A61P 3/04; A61P 3/10;
A61P 9/00; A61P 9/10; A61P 21/00; A61P 25/02;
A61P 25/04; A61P 29/00; A61P 35/00; A61P 37/06;
A61P 43/00; C07K 16/00; C07K 16/28;** (Cont.)

(86) International application number:
**PCT/JP2024/030861**

(87) International publication number:
**WO 2025/047838 (06.03.2025 Gazette 2025/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.08.2023 JP 2023140294**

(71) Applicant: **Teijin Pharma Limited
Tokyo 100-0013 (JP)**

(72) Inventors:
• **EGUCHI, Hiroshi**
  **Tokyo 1000013 (JP)**
• **GOTOU, Kaoru**
  **Tokyo 1000013 (JP)**
• **SHIMOYAMA, Hiroshi**
  **Tokyo 1000013 (JP)**
• **YAZAWA, Kana**
  **Tokyo 1000013 (JP)**

(74) Representative: **Lavoix
Bayerstraße 83
80335 München (DE)**

Remarks:
•The applicant has filed a text with which it is intended to bring the translation into conformity with the application as filed (Art. 14(2) EPC).
•The complete document including Reference Table(s) and the Sequence Listing(s) can be downloaded from the EPO website

(54) **PHARMACEUTICAL AND ANTI-gpNMB ANTIBODY FOR REMOVING DISEASE-ASSOCIATED CELLS**

(57)    A new means is provided that can effectively remove disease-related cells that induce or enhance the progression of various diseases. Specifically, a pharmaceutical composition for removing disease-related cells is provided that contains an anti-gpNMB antibody or its fragment or a derivative thereof that binds specifically to at least one site in a region from V78 to a PKD domain of human gpNMB.

Figure 2

**(Cont. next page)**

(52) Cooperative Patent Classification (CPC): (Cont.)
**C12N 15/63; C12N 15/85**

**Description**

**TECHNICAL FIELD**

The present invention relates to a novel pharmaceutical for removing disease-related cells and a novel anti-gpNMB antibody used therefor.

**BACKGROUND ART**

<Immunoglobulin (antibody)>

Human immunoglobulins (antibodies) can be classified based on the number of Y-shaped units and the type of heavy chains into five classes: IgG, IgM, IgA, IgD, and IgE. The heavy chains of IgG, IgM, IgA, IgD, and IgE are referred to as $\gamma$, $\mu$, $\alpha$, $\delta$, and $\varepsilon$, respectively. IgG have four subclasses: IgG1, IgG2, IgG3, and IgG4. The light chains of antibodies can be classified as either kappa ($\kappa$) or lambda ($\lambda$) type, depending on slight differences in polypeptide structure, while the subclasses of antibodies are determined by their heavy chains.

<Phagocytes>

Phagocytes include macrophages, microglia, dendritic cells, monocytes, neutrophils, etc. Tissue resident phagocytes include Kupffer cells, Langerhans cells, and osteoclasts. Other macrophages include cancer-infiltrating macrophages, aging macrophages, fat-accumulating macrophages, sphingomyelin-accumulating macrophages, foam cells, alveolar macrophages, peritoneal macrophages, splenic macrophages, and thymic macrophages. The microglia include cancer-infiltrating microglia, aging microglia, fat-accumulating microglia, and sphingomyelin-accumulating microglia. Dendritic cells include subtypes such as conventional type 1 dendritic cells (cDC1), conventional type 2 dendritic cells (cDC2), monocyte-derived dendritic cells (MoDC), and plasmacytoid dendritic cells (pDC).

<Macrophages>

There are various types of macrophages, which can be broadly classified based on their origin into two types: resident macrophages, which are endemic to tissues, and infiltrating macrophages, which are differentiated from monocytes and other cells migrated from the blood in response to changes in the tissue environment. Resident macrophages in the liver are called Kupffer cells, those in the brain are called microglia, those in bone tissue are called osteoclasts, those in the skin are called Langerhans cells, those in the lungs are called alveolar macrophages, and so on.

In terms of properties, they were largely divided into M1 macrophages, which induce inflammation and tissue repair, and M2 macrophages, which are anti-inflammatory. Recently, however, scRNAseq analysis and other methods have revealed a variety of macrophage subtypes and the presence of disease-specific macrophages in various diseases. As mentioned above, macrophages differentiate into different types depending on the environment. And each subtype has been shown to be involved in healing damage or, conversely, to induce disease or accelerate its progression.

<Senescent cells>

Senescent cells represent cells in which DNA damage, activation of oncogenes, or stress induces a sustained arrest of cell proliferation. Recently, senescent cells have been shown to acquire a pro-inflammatory phenotype called SASP (senescence-associated secretory phenotype), which has been implicated in many diseases (Non-Patent Literature 1). While cell senescence is thought to be a mechanism that prevents damaged cells from undergoing malignant transformation and becoming cancerous, a number of findings have also implicated cell senescence in various aging-related pathologies (e.g., tissue degeneration, chronic inflammation, and cancer progression).

Thus, the biological role of cell senescence is very complex and both beneficial and detrimental effects have been reported and depend on the physiological situation. On the other hand, several clinical trials are underway for the treatment of intractable diseases with senescent cell-depleting agents.

<Refractory cancer>

Since the development of molecularly targeted drugs, a certain level of therapeutic efficacy has been achieved in the treatment of cancers that were previously difficult to treat. In addition, with the advent of immune checkpoint inhibitors, the understanding of cancer immunotherapy has advanced and the therapeutic efficacy of molecularly targeted drugs has been further enhanced (Non-Patent Literature 2). However, there are many cancer patients with refractory cancers (melanoma, TNBC, glioblastoma, multiple myeloma, etc.) that have not yet been adequately treated, so there is a need to develop more innovative drugs with high therapeutic efficacy. In particular, it remains challenging to develop drugs for glioblastoma, which requires drug delivery to the brain and has a wide variety of antigenic properties.

In recent years, therapeutic methods have been developed to further enhance the cancer-cell-killing effects of T cells. Examples include: bispecific antibodies with dual specificity of anti-T cell antibodies and anti-cancer antigen antibodies, which are intended for T cell engagement effects (Non-Patent Literature 3), and CAR-T cell therapy, which delivers T cells directly to cancer cells (Non-Patent Literature 4). These methods have achieved higher therapeutic efficacy. However, there is a high unmet need for these therapies, as many patients do not exhibit sufficient T-cell activity or become resistant to these treatments. As such, NK cell engaging antibodies (Non-Patent Literature 5), CAR-NK to deliver NK cells (Non-Patent Literature 6), macrophage engaging antibodies (Non-Patent Literature 7) and CAR macrophages (chimeric antigen receptor macrophages) to deliver macrophages have also been devised (Non-Patent Literature 8, Non-Patent Literature 9). The combination of tumor-soluble viruses and T-cell engagement has also been investigated (Non-Patent Literature 10).

One of the causes of difficulties in cancer treatment is the formation of the cancer microenvironment. In the cancer microenvironment, cytokines produced by cancer cells themselves, macrophages and microglia infiltrating cancer tissue secrete or directly interact with humoral factors to create an immunosuppressive environment. It has also been reported that senescent cells create an immunosuppressive and chronic inflammatory environment that supports cancer invasion and proliferation (Non-Patent Literature 11).

The cancer microenvironment allows cancer cells to escape attack by host immunity and supports cancer cell proliferation and stem cell transformation. It has been reported that the cancer immunosuppressive environment is involved in refractory tumors and tumors with poor prognosis, which become resistant to treatments such as immune checkpoint inhibitors. Therefore, disrupting the cancer microenvironment is important in cancer therapy (Non-Patent Literature 12).

Several attempts to remove cancer-infiltrating macrophages have been reported. Examples include non-specific removal of macrophages by CSF1R inhibitors (Non-Patent Literature 13) and removal of cancer-infiltrating macrophages by anti-TREM2 antibodies

(Non-Patent Literature 14). Furthermore, focusing on aging macrophages, it has been reported that removal of cancer-infiltrating senescent macrophages with senescent cell-depleting drugs suppresses cancer growth and improves survival and survival time in a mouse model of lung cancer carcinoma (Non-Patent Literature 15).

<Refractory cancer and gpNMB>

It has been reported that gpNMB levels in the blood of cancer patients with poor response to treatment with immune checkpoint inhibitors were found to be elevated, and that a combination of an immune checkpoint inhibitor and an anti-gpNMB antibody was effective in a melanoma-transplanted mouse model (Non-Patent Literature 16).

With regard to glioblastomas, it has been shown that microglia and macrophages infiltrating glioblastomas have a different profile from M1 and M2 and express high levels of gpNMB and Spp1, and that high expression of gpNMB and Spp1 in glioblastoma patients is associated with a poor prognosis (Non-Patent Literature 17).

These reports suggest that gpNMBs are involved in the immunosuppressive environment that forms the cancer microenvironment.

<Fibrosis>

Fibrosis is a disease in which temporary or chronic damage to vital organs such as the lungs, liver, kidneys, heart, skin, and blood vessels causes excessive or abnormal accumulation of collagen and other fibers during the repair process, resulting in loss of elasticity and stiffening of the organs and inability to function normally. No fundamental therapeutic drug has been developed for this disease.

Idiopathic pulmonary fibrosis (IPF), a type of fibrosis of the lungs, is an incurable disease with no therapeutic drug. Clinical trials have been conducted on the therapeutic effect of using a combination therapy containing an anti-cancer drug, D+Q (dasatinib and quercetin), to eliminate senescent cells (Non-Patent Literature 18). However, the results have not yet shown sufficient efficacy, partly because the dosage is limited to avoid side effects. In addition, the results of a Phase III clinical trial of an anti-CTGF antibody (Non-Patent Literature 19) were recently reported, but no therapeutic effect was demonstrated.

Chronic kidney disease (CKD) is a condition in which the structure or function of the kidneys is abnormal for more than three months. There is no therapeutic drug for CKD, and its progression leads to end-stage renal disease, requiring dialysis therapy or kidney transplantation. Clinical trials of senescent cell depleting agents for diabetic nephropathy have been conducted, but no therapeutic effect has been demonstrated (Non-Patent Literature 20).

Nonalcoholic steatohepatitis (NASH), which involves inducement and progression of liver fibrosis, is a refractory disease with no therapeutic drug. It has been reported that fibrosis is induced by fat-storing macrophages (Non-Patent Literature 21). On the other hand, it has been reported that removal of senescent cells improves fatty liver pathology (Non-Patent Literature 22).

Vasculitis is an autoimmune vascular inflammation of unknown cause that causes destruction of the vessel wall and induces fibrosis. The involvement of monocytes and macrophages in the pathogenesis of Takayasu vasculitis and giant cell arteritis (GCA), which stimulate fibroblasts to induce fibrosis, has been reported (Non-Patent Literature 23).

Duchenne muscular dystrophy (DMD), a progressive muscular atrophy, has been reported to induce fibrosis in skeletal muscle and to involve certain types of macrophages (Non-Patent Literature 24). It has also been reported that a senescent cell depleting drug improved pathology and muscle strength in a DMD rat model (Non-Patent Literature 25).

<u><Fibrosis and gpNMB></u>

There are several reports that gpNMB is involved in fibrosis. However, there are conflicting reports as to whether gpNMB inhibits or promotes fibrosis.

In silicosis, a disease in which the lungs become fibrotic, scRNAseq analysis in a mouse model showed that gpNMB induces epithelial-mesenchymal transition in alveolar epithelial cells and increases fibrosis (Non-Patent Literature 26).

It has been reported that gpNMB is expressed in a subset of macrophages (scar-associated macrophages: SAM) that are thought to induce fibrosis in liver and lung fibrosis (Non-Patent Literature 27).

On the other hand, it has been reported that overexpression of gpNMB ameliorates fat accumulation and fibrosis of the liver in non-alcoholic fatty liver disease (NAFLD) induced by a high-fat, high-sucrose (HFHS) diet (Non-Patent Literature 28).

It has also been reported that repair of skin damage was enhanced by macrophage-derived gpNMBs (Non-Patent Literature 29).

It has been reported that vascular wall fibrosis is observed in Takayasu arteritis, a form of vascular fibrosis, in which macrophages produce TGF$\beta$ and gpNMB and activate fibroblasts (Non-Patent Literature 30).

It has been reported that a database analysis of expressed genes in skeletal muscle tissue of DMD patients revealed DMD-specific genes, which included gpNMB (Non-Patent Literature 31).

<u><Metabolic diseases></u>

Metabolic diseases include diabetes, hypertension, dyslipidemia (high cholesterol, high neutral fats, etc.), atherosclerosis, fatty liver disease, hyperuricemia (gout), metabolic syndrome, obesity, etc. Many of these diseases are thought to be caused by chronic inflammation.

Various diseases are induced by hyperglycemia in diabetes mellitus (Non-Patent Literature 32). These include diabetic retinopathy, diabetic nephropathy and diabetic neuropathy. Diabetic retinopathy (DR) is a microvascular complication characterized by abnormal angiogenesis and is the leading cause of irreversible blindness. Diabetic nephropathy occurs when the microvasculature lining the kidneys is damaged by sustained high blood glucose levels caused by diabetes mellitus. As the disease worsens, kidney failure can occur, requiring hemodialysis. In diabetic neuropathy, prolonged high blood sugar not only damages the blood vessels around nerves, but also changes the nature of the nerves themselves, causing various disorders of nerve function.

Dyslipidemia includes abnormal blood levels of LDL cholesterols (so-called bad cholesterols), HDL cholesterols (so-called good cholesterols), and triglycerides (neutral fats), all of which have been reported to be associated with accelerated atherosclerosis. In atherosclerosis, macrophages phagocytose oxidized LDL in the vascular endothelium and change into fat- accumulatimg macrophages called foam cells (Non-Patent Literature 33), which create a lipid core that becomes the center of atherosclerotic foci (plaque) and narrow the lumen of the blood vessel.

Fatty liver is caused by an abnormal accumulation of fats in the liver. Non-alcoholic fatty liver disease (NAFLD) is a general term for fatty liver diseases that are not caused by alcohol or viruses. It develops as a phenotype in the liver of lifestyle-related diseases such as obesity, diabetes, and metabolic syndrome, and the liver disease progresses like alcoholic liver disease even in people who do not drink much alcohol. An additional 20 to 30% of these patients will progress to non-alcoholic steatohepatitis (NASH), which involves inflammation and fibrosis and has a risk of progression to cirrhosis and hepatocarcinoma. Studies of macrophage subsets involved in these and other progressions are ongoing (Non-Patent Literature 34).

<u><Metabolic diseases and gpNMB ></u>

It has been reported that blood gpNMB can be used as a biomarker that correlates with disease in diabetes and diabetic complications (Non-Patent Literature 35).

It is important to improve insulin resistance in diabetes. In a mouse model of diabetes induced by a high-fat diet, liver-produced soluble gpNMB increased lipid synthesis in white adipocytes, while administration of a polyclonal anti-gpNMB antibody improved obesity and insulin resistance (Non-Patent Literature 36).

In human retinal microvascular endothelial cells (HRMECs), high glucose treatment increased gpNMB and integrin β1, suggesting that gpNMB functions as an angiogenic factor through integrin β1/VEGFA, and knockdown of gpNMB by shRNA-expressing lentivirus inhibited cell viability, migration, and tube formation in HRMECs, suggesting the involvement of gpNMB in the pathogenesis of diabetic retinopathy (Non-Patent Literature 37).

It has been reported that macrophages involved in atherosclerosis in ApoE KO mice fed a high-fat diet and other diets were positive for gpNMB (Non-Patent Literature 38). It has also been reported that gpNMB is expressed in lipid-laden macrophages found in atherosclerosis, called foam cells (Non-Patent Literature 39).

< Cardiovascular diseases>

Cardiovascular diseases are diseases in which the heart and blood vessels, the organs that circulate blood throughout the body, fail to function normally, and are classified as hypertension, heart disease (ischemic heart disease such as acute myocardial infarction and heart failure), cerebrovascular disease (cerebral infarction, cerebral hemorrhage, subarachnoid hemorrhage), aneurysm, etc.

In heart disease, the infiltration of inflammatory cells such as monocytes and lymphocytes increases after myocardial infarction, causing chronic inflammation and myocardial remodeling leading to heart failure. It has been reported that control of inflammatory cell infiltration, increased expression of inflammatory cytokines and cardiac macrophages is important to inhibit progression (Non-Patent Literature 40). Studies analyzing common macrophage subsets associated with heart disease have also been reported (Non-Patent Literature 41).

< Cardiovascular diseases and gpNMB>

Myocardial remodeling after myocardial infarction has suggested the involvement of macrophages, and the expression of gpNMB in these macrophages has been reported (Non-Patent Literature 42).

<Lysosomal stress-associated diseases>

Various diseases are caused when lysosomes become dysfunctional due to various stresses and toxic proteins accumulate in the body. Lysosomal disease is a genetic disorder induced by mucopolysaccharides and lipids accumulating in lysosomes due to genetic deficiency or reduced activity of degradative enzymes in lysosomes and causing lysosomal and cellular dysfunction. Lysosomal diseases include Gaucher disease, Pompe disease, and Niemann-Pick disease types A, B, and C.

<lysosomal stress-associated diseases and gpNMB>

gpNMB is an important protein in the degradation of proteins and lipids in lysosomes, and its expression increases when lysosomal stress occurs due to the accumulation of substances that need to be degraded. Therefore, gpNMB can be an indicator of cells undergoing excessive lysosomal stress. High expression of gpNMB is also observed in dysfunctional senescent cells.

Fat-accumulating gpNMB-high expressing cells have been reported in lysosomal diseases such as Gaucher disease and Niemann-Pick disease. In Gaucher disease, lipid-laden macrophages, called Gaucher cells, are observed, and in Niemann-Pick disease types A and B (acid sphingomyelinase deficiency (ASMD)), sphingomyelin-accumulating macrophages accumulate in the spleen and liver (Non-Patent Literature 43).

<Neuropathic pain-associated diseases>

Neuropathic pain is caused by direct damage to the brain or nerves (after stroke, spinal cord injury, surgery, or compression of nerves due to cancer infiltration, etc.). Neuropathic pain is difficult to treat due to the lack of efficacy of antipyretic analgesics, and the likelihood of chronic disease is high, so a fundamental treatment is desired. Fibromyalgia pain is classified as neuropathic pain and is thought to be caused by inflammation of nerves in the brain induced by some cause.

<Neuropathic pain-associated diseases and gpNMB >

It was reported that in a neuropathic pain rat model, pain was reduced by injecting, into the subarachnoid space, an siRNA that suppresses the expression of gpNMB (Non-Patent Literature 97).

<u><gpNMB ></u>

gpNMB (Glycoprotein nonmetastatic melanoma protein B) (also known as Osteoactivin, DC-HIL, Non-Patent Literature 44) is a single transmembrane glycoprotein and has as many as 12 glycosylation sites in humans. gpNMB is normally expressed in intracellular organelles (endoplasmic reticulum, lysosomes, Golgi apparatus, melanosomes), etc. (Non-Patent Literature 45). However, gpNMB has also been reported to be expressed on the plasma membrane when overexpressed (Non-Patent Literature 46). It has also been reported that gpNMB is expressed in cancer cells (Non-Patent Literature 47), and that KLD (kringle-like domain) is important for cell growth and that intracellular ITIM motifs induce signaling (Non-Patent Literature 48).

gpNMB has a region homologous to the CAF (Core Amyloid Fragment) region of the family protein PMEL (Premelanosome Protein) (Non-Patent Literature 49) (hereinafter referred to as the amyloid core domain) and PKD (Polycystic Kidney Disease homologous) domain. The PKD domain has a β-sandwich structure formed by a β-sheet consisting of three strands and a β-sheet consisting of four strands that fold together. It was named the PKD domain because it is a characteristic structure possessed by polycystin-1, the gene responsible for polycystic kidney disease (Non-Patent Literature 50).

Molecules that have been reported to interact with gpNMBs include syndecan-4 (SD4, SDC4), CD44, integrins, Na+/K+-ATPase, and EGFR (Non-Patent Literature 98). SD4 has been reported to interact with the PKD domain of gpNMB, and gpNMB regulates T cell activity via SD4 on T cells. CD44 is expressed on mesenchymal stem cells, osteoclasts, astrocytes (stellate cells) and adipocytes, and gpNMB has been reported to interact with CD44 to regulate cell proliferation, adhesion, migration and activity. In addition, gpNMBs have RGD sequences on their N-terminal side and regulate migration and adhesion of various cells by interacting with integrins, which are adhesion molecules, and soluble gpNMBs also modify various physiological functions and pathological conditions by binding to cells via RGD sequences. It has also been reported that gpNMB interacts with EGFR in its intracellular domain to regulate cell proliferation. It has also been reported that gpNMB interacts with the α-subunit of Na+/K+-ATPase and exhibits cytoprotective effects.

gpNMB is important for the function of phagosomes and lysosomes (Non-Patent Literature 51), as it has been reported that lysosome function is impaired in gpNMB-deficient mice (Non-Patent Literature 52). Abnormalities of the iris and the development of glaucoma have been reported in mice lacking gpNMB (Non-Patent Literature 53), and humans lacking gpNMB have been reported to have cutaneous amyloidosis (Non-Patent Literature 54).

In addition, recent genome-wide association studies (GWAS) analysis has reported gpNMB as a risk gene for Parkinson's disease (Non-Patent Literature 55), and its involvement in the propagation of α-synuclein, a causative agent of Parkinson's disease, has also been reported (Non-Patent Literature 56). On the other hand, another report has indicated that loss of gpNMB does not alter synuclein-associated pathology (Non-Patent Literature 57).

It is known that gpNMB is cleaved by membrane proteases such as ADAM10 and released as soluble gpNMB (Non-Patent Literature 58). Soluble gpNMB has been reported to be involved in a variety of physiological and pharmacological actions, including cytoprotection (Non-Patent Literature 59), neuroprotection (Non-Patent Literature 60), anti-inflammation (Non-Patent Literature 45, Non-Patent Literature 61, Non-Patent Literature 62) and cell proliferation (Non-Patent Literature 63). It has also been reported that gpNMB plays a protective role against metabolic abnormalities induced by obesity (Non-Patent Literature 64). It has also been reported that neural function was improved in transgenic mice overexpressing soluble gpNMB (extracellular region gpNMB) (Non-Patent Literature 65), and cognitive function was increased in an Alzheimer's disease model mice (APP/PS1) when autophagy was enhanced by using lentiviral vectors to express gpNMB (Non-Patent Literature 66).

On the other hand, it has been reported that administration of a mouse gpNMB-specific vaccine peptide with some amino acid sequences of mouse gpNMB reduces aged vascular endothelial cells and fibroblasts, improves insulin resistance and atherosclerosis in a high-fat diet load model mice, and extends survival in mice with accelerated aging. It has also been reported that antibody-dependent cellular cytotoxicity (ADCC) activity is important in this process, and gpNMB partial peptide sequences that are effective in removing senescent cells have been disclosed (Patent Literature 1, Non-Patent Literature 67). Likewise, it has been reported that administration of a polyclonal antibody against mouse gpNMB in a high-fat diet-loaded mouse model improved obesity and insulin resistance by inhibiting soluble gpNMB produced by the liver (Non-Patent Literature 68).

Soluble gpNMB has also been studied extensively as a biomarker. Blood gpNMB levels have been reported to correlate with the progression of diabetes-related diseases (Non-Patent Literature 35).

It has been reported that the soluble gpNMB concentration in cerebrospinal fluid is associated with Parkinson's disease (Non-Patent Literature 69) and with ALS (Non-Patent Literature 70). There is also a report on gpNMB as a biomarker for cerebral adrenoleukodystrophy (Non-Patent Literature 71). On the other hand, regarding the association with Alzheimer's disease, some reports indicate that the gpNMB concentration is increased in patients with Alzheimer's disease (Non-Patent Literature 72), while others indicate that there is no association between gpNMB and AD (Non-Patent Literature 73).

<Anti-gpNMB antibodies>

Glembatumumab (Patent Literature 2), an anti-human gpNMB-specific human antibody, is known as an anti-gpNMB antibody. Glembatumumab Vedotin, a conjugate of Glembatumumab and an anticancer drug, advanced to Phase 11 trials as a potential cancer drug, but its development was discontinued (Non-Patent Literature 74). The antibody has also been attempted for use in diagnostic imaging (Non-Patent Literature 75). Another anti-gpNMB antibody that binds to gpNMB expressed on the surface of cancer cells and treats cancer with immunotoxins has also been disclosed (Patent Literature 3). There is also a report on the acquisition of another anti-human gpNMB-specific antibody, which describes diagnostic and therapeutic applications against human-derived cancer cells (Non-Patent Literature 76). Anti-rat gpNMB-specific antibodies specific for activated rat microglia have also been reported (Non-Patent Literature 77). It has also been reported that antimouse DC-HIL specific antibodies put signals into the cells (Non-Patent Literature 78).

<gpNMB expressing cells>

Since gpNMB is highly expressed in a number of cancer tissues, including breast cancer (Non-Patent Literature 79), lung cancer (Non-Patent Literature 80), hepatocellular carcinoma (Non-Patent Literature 81), glioblastoma multiforme (Non-Patent Literature 82), and mesothelioma (Non-Patent Literature 83), clinical development using anti-gpNMB antibodies to target gpNMB is therefore underway, clinical development of ADCs (antibody-drug conjugates) targeting gpNMBs and delivering anticancer drugs through anti-gpNMB antibodies has progressed to Phase II clinical trials (Non-Patent Literature 74) in several refractory cancers. It has also been reported that gpNMB expression is high in cancers with poor prognosis (Non-Patent Literature 84), in cancer stem cells (Non-Patent Literature 85), and in infiltrating macrophages and microglia, myeloid-derived suppressor cells (MDSCs) and dendritic cells (DCs) that form the cancer microenvironment (Non-Patent Literature 86). It has further been reported that the interaction of syndecan-4 (SD4, SDC4) on T cells with gpNMB-expressing cells (such as infiltrating macrophages and microglia that form the cancer microenvironment, myeloid-derived suppressor cells (MDSCs) and dendritic cells (DCs) interact with each other to suppress T cell activity (Non-Patent Literature 87).

It has also been reported that gpNMB can be used as a marker of senescent cells, and that administration of a partial peptide of gpNMB to senescence-promoting mouse models ameliorated atherosclerosis and diabetes by eliminating senescent vascular endothelial cells and senescent fibroblasts that express gpNMB (Non-Patent Literature 71). Furthermore, gpNMB expression has been observed in white adipose tissue macrophages with crown-like structures in obesity (Non-Patent Literature 88).

It has been reported that gpNMB is expressed in a subset of macrophages (SAM: scar-associated macrophages), which is thought to be a fibrosis inducer in fibrosis in the liver and lung (Non-Patent Literature 27).

It has been reported that macrophages involved in atherosclerosis in ApoE-KO mice fed a high-fat diet are positive for gpNMB (Non-Patent Literature 38). It has also been reported that gpNMB is expressed in lipid-laden macrophages, called foam cells, which are found in atherosclerosis (Non-Patent Literature 39). Similar lipid-laden cells expressing a high level of gpNMB have also been reported to be observed in lysosomal diseases such as Gaucher disease and Niemann-Pick disease. In Gaucher disease, lipid-laden macrophages called Gaucher cells are observed, and in Niemann-Pick disease type A and B (acid sphingomyelinase deficiency (ASMD)), sphingomyelin-accumulating macrophages accumulate in the spleen and liver (Non-Patent Literature 43). Furthermore, it has been reported that gpNMB is highly expressed in lipid-rich myelin-laden macrophages (foam macrophages) in multiple sclerosis (Non-Patent Literature 96).

**LIST OF CITATIONS**

**Patent Literature**

[Patent Literature 1] WO2021/020047 A
[Patent Literature 2] JP6334496 A
[Patent Literature 3] WO2007/053718 A

**Non-Patent Literature**

[Non-Patent Literature 1] Zhang et al., J. Clin. Invest., 2022, 132[15]:e158450

[Non-Patent Literature 2] Kurt et al., J. Clin. Invest., 2023, 133[13]:e170762

[Non-Patent Literature 3] Wei et al., Front. Immunol., 2022, 13:1035276

[Non-Patent Literature 4] Geng et al., Front. Cell Dev. Biol., 2023, 11:1158539

[Non-Patent Literature 5] Pinto et al., Trends Immunol., 2022, 43[11]:932-946

[Non-Patent Literature 6] Klaihmon et al., Int. J. Mol. Sci., 2023, 24[13]:10508

[Non-Patent Literature 7] Kaiuchi-Kiyota et al., Leukemia, 2022, 36:1006-1014

[Non-Patent Literature 8] Gatto et al., Immunotherapy, 2021, 13[11]:879-883

[Non-Patent Literature 9] Unver et al., Clin. Exp. Med., 2023, doi 10.100_s10238-023-01106-0

[Non-Patent Literature 10] Huang et al., Am. J. Cancer Res., 2021, 11[6]:2430-2455

[Non-Patent Literature 11] Malayaperumal et al., Clin. Pract., 2023, 13:838-852

[Non-Patent Literature 12] Zhao et al., Front. Immunol., 2023, 14:1157537

[Non-Patent Literature 13] Jahchan et al., Front. Immunol., 2019, 10:1611

[Non-Patent Literature 14] Binnewies et al., Cell Reports, 2021, 37:109844

[Non-Patent Literature 15] Haston et al., Cancer Cell, 2023, 41:1242-1260

[Non-Patent Literature 16] Chung et al., Clin. Cancer Res., 2020, 26[6]:1449-1459

[Non-Patent Literature 17] Szulzewsky et al., PLoS ONE, 2015, 10[2}:e0116644

[Non-Patent Literature 18] Nambiar et al., eBioMedicine, 2023, 90:104481

[Non-Patent Literature 19] Leask et al., J. Cell Commun. and Signaling, 2019, 13:441-442

[Non-Patent Literature 20] Hickson et al., EBioMedicine, 2019, 47:446-456

[Non-Patent Literature 21] Shimizu et al., Am. J. Pathol., 2022, 192:31-42

[Non-Patent Literature 22] Ogrodnik et al., Nat. Commun., 2017, 8:15691

[Non-Patent Literature 23] Watanabe et al., Front. Immunol., 2022, 13:859502

[Non-Patent Literature 24] Coulis et al., Sci. Adv., 2023, 9:eadd9984

[Non-Patent Literature 25] Sugihara et al., Sci. Rep., 2020, 10:16385

[Non-Patent Literature 26] Yang et al., Part. Fibre Toxicol., 2023, 20:29

[Non-Patent Literature 27] Fabre et al., Sci. Immunol., 2023, 8:eadd8945

[Non-Patent Literature 28] Katayama et al., Sci. Rep., 2015, 5:16920

[Non-Patent Literature 29] Silva et al., Exp. Dermatol., 2018, 27:630-635

[Non-Patent Literature 30] Dai et al., Transl. Res., 2023, 255:128-139

[Non-Patent Literature 31] An et al., J. Zheijang Univ-Sci. G., 2013, 14[11]:973-982

[Non-Patent Literature 32] Reynolds et al., Front. Immunol., 2023, 14:1219598

[Non-Patent Literature 33] Cervantes et al., Front. Cardiovasc. Med., 2023, 10:1213177

[Non-Patent Literature 34] He et al., J. Trans. Med., 2023, 21:448

[Non-Patent Literature 35] Huo et al., Front. Endocrinol., 2023, 14:1110337

[Non-Patent Literature 36] Gong et al., Nat. Metab., 2019, 1[5]:570-583

[Non-Patent Literature 37] Quin et al., Mol. Cell Biochem., 2023, 478:697-706

[Non-Patent Literature 38] Haerdtner et al., Atherosclerosis, 2023, 371:1-13

[Non-Patent Literature 39] van Eijk et al., Int. J. Mol. Sci., 2021, 22:4039

[Non-Patent Literature 40] Chen et al., Front. Immunol., 2023, 14:1207100

[Non-Patent Literature 41] Li et al., J. Leukoc. Biol., 2023, 113:139-148

[Non-Patent Literature 42] Jarve et al., FASEB J., 2017, 31:556-568

[Non-Patent Literature 43] Eskes et al., Mol. Genet. Metab., 2023, 139:107631

[Non-Patent Literature 44] Shikano et al., J. Biol. Chem., (2001), 276[11]:8125-8134

[Non-Patent Literature 45] Ripoll et al., J. Immunol., (2007), 178:6557-6566

[Non-Patent Literature 46] Tse et al., Clin. Cancer Res., (2006), 12[4]:1373-1382

[Non-Patent Literature 47] Maric et al., OncoTargets Ther., (2013), 6:839-852

[Non-Patent Literature 48] Xie et al., Cancer Sci., (2019), 110[7]:2237-2246

[Non-Patent Literature 49] Hee et al., Scientific Reports, (2017), 7:44064

[Non-Patent Literature 50] Bycroft et al., EMBO J., (1999), 18[2]:297-305

[Non-Patent Literature 51] Li et al., FASEB J., (2010), 24[12]:4767-4781

[Non-Patent Literature 52] Robinet et al., Scientific Reports, (2021), 11:10249

[Non-Patent Literature 53] Anderson et al., Nat. Genet., (2002), 30:81-85

[Non-Patent Literature 54] Yang et al., Am. J. Hum. Genet., (2018), 102[2]:219-232

[Non-Patent Literature 55] Murthy et al., Neurogenetics, 2017, 18:121-133

[Non-Patent Literature 56] Diaz-Ortiz et al., Science, 2022, 377:833

[Non-Patent Literature 57] Grendza et al., Neurobiol. Dis., 2021, 159:105494

[Non-Patent Literature 58] Rose et al., PLoS One, (2010), 5[8]:e12093

[Non-Patent Literature 59] Wang et al., Int. J. Mol. Sci., 2021, 22:10843

[Non-Patent Literature 60] Nakano et al., Neuroscience, (2014), 277:123-131

[Non-Patent Literature 61] Saade et al., Front. Immunol., 2021, 12:674739

[Non-Patent Literature 62] Neal et al., J. Neuroinflammation, 2018, 15:73

[Non-Patent Literature 63] Wang et al., Cancer Sci.. (2021), 112:4187-4197

[Non-Patent Literature 64] Prabata et al., J. Biol. Chem., 2021, 297[5]:101232

[Non-Patent Literature 65] Murata et al., J. Neurochem., (2015), 132:583-594

[Non-Patent Literature 66] Zhu et al., Neurosci. Lett., (2022), 767:136300

[Non-Patent Literature 67] Suda et al., Nat. Aging, (2021), 1:1117-1126

[Non-Patent Literature 68] Gong et al., Nat. Metab., (2019), 1[5]:570-583

[Non-Patent Literature 69] Hansson et al., EMBO Mol. Med., 2023, 15:e16359

[Non-Patent Literature 70] Oh et al., Biomedicines, 2023, 11:1250

[Non-Patent Literature 71] Taghizadeh et al., Sci. Rep., 2022, 12:7985

[Non-Patent Literature 72] Huettenrauch et al., Acta Neuropathol. Commun., 2018, 6:108

[Non-Patent Literature 73] Aichholzer et al., Alzheimers Res. Ther., (2021), 13:94

[Non-Patent Literature 74] Vahdat et al., npj Breast Cancer, 2021, 7:57

[Non-Patent Literature 75] Li et al., Cancers, 2023, 15:1589

[Non-Patent Literature 76] Zhang et al., Monoclon. Antibodies Immunodiagn. Immunother., (2013), .32[4]:265-269

[Non-Patent Literature 77] Kawahara et al., Glia, (2016), 64[11]:1938-1961

[Non-Patent Literature 78] Chung et al., J. Immunol., (2019), 183[5]:5190-5198

[Non-Patent Literature 79] Huang et al., Sci. Rep. 2021, 11:12171

[Non-Patent Literature 80] Han et al., Cancer Sci., 2021, 112:1911-1923

[Non-Patent Literature 81] Sakano et al., Cancer Sci., 2022, 113[5]:1625-1638

[Non-Patent Literature 82] Kuan et al., Clin. Cancer Res., 2006, 12[7]:1970-1982

[Non-Patent Literature 83] Wu et al., PNAS, 2023, 120[9]:e2210836120

[Non-Patent Literature 84] Feng., Oncol. Lett., 2020, 20[3]:2356-2368

[Non-Patent Literature 85] Okita et al., Oncotarget, 2018, 9[99]:37289-37290

[Non-Patent Literature 86] Lazaratos et al., Oncogene, 2022, 41:4573-4590

[Non-Patent Literature 87] Tomihari et al., Cancer Res., 2010, 70[14]:5778-5787

[Non-Patent Literature 88] Gabriel et al., Diabetes, 2014, 63:3310-3323

[Non-Patent Literature 89] Chrystal et al., Molecules, 2021, 26:3529

[Non-Patent Literature 90] Alexander et al., Pigment Cell Melanoma Res. 2013, 26[4]:470-486

[Non-Patent Literature 91] Strohl et al., Current Opinion in Biotechnology 2009, 20, pp685-691.

[Non-Patent Literature 92] Lee et al., Nat Commun. 2019 Nov 6;10(1), 5031.

[Non-Patent Literature 93] Kouhi et al., Int. J. Mol. Sci. 2019, 20, 3108

[Non-Patent Literature 94] C. Spiess et al., Molecular Immunology 2015, Vol.67, pp95-106.

[Non-Patent Literature 95] Chocarro et al., Biomedicines 2022, 10:3035.

[Non-Patent Literature 96] Hendrickx et al., Front Immunol. 2017, 8:1810

[Non-Patent Literature 97] Hou et al., J Mol Neurosci, 2015, 55:533-540

[Non-Patent Literature 98] Tsou et al., The FASEB Journal., (2020), 34:8810-8823

## SUMMARY OF INVENTION

### PROBLEM TO BE SOLVED BY THE INVENTION

Various attempts have been made to develop means to eliminate various cells that induce or accelerate the progression of various diseases (referred to as "disease-associated cells"). However, reports on methods to eliminate such disease-involved cells have been extremely limited so far. In particular, the development of a method that can specifically remove highly malignant disease-involved cells, which are the root cause of disease induction, has not been achieved to date.

The present invention has been made in view of these issues. Thus, an objective to be achieved by the present invention is to provide a new means with which various disease-associated cells can be effectively removed.

### MEANS TO SOLVE THE PROBLEM

The present inventors have studied intensively to develop a means of specifically eliminating highly malignant disease-associated cells, and have focused on gpNMB (glycoprotein nonmetastatic melanoma protein B) as a common antigen for disease-associated cells involved in various diseases. As a result, the present inventors have succeeded in obtaining novel anti-gpNMB antibodies that bind to a new epitope on gpNMB, which is different from previously known antibodies, through diligent investigation using various experimental means and methods under various constraints. Based on these findings, the inventors have completed the following inventions.

Specifically, aspects of the present invention include the following.

[Aspect 1] A pharmaceutical composition for removing disease-related cells, comprising an anti-gpNMB antibody or its fragment or a derivative thereof that binds specifically to at least one site in a region from V78 to a PKD domain of human gpNMB (glycoprotein nonmetastatic melanoma protein B) having the amino acid sequence of SEQ ID NO 209.

[Aspect 2] The pharmaceutical composition according to Aspect 1, wherein the anti-gpNMB antibody specifically binds to a region containing amino acid residue(s) D287 and/or H301 of human gpNMB having the amino acid sequence of SEQ ID NO 209.

[Aspect 3] The pharmaceutical composition according to Aspect 1 or 2, wherein the anti-gpNMB antibody specifically binds to a region containing amino acid residue(s) R214 and/or R215 of human gpNMB having the amino acid sequence of SEQ ID NO 209.

[Aspect 4] The pharmaceutical composition according to Aspect 2 or 3, wherein the anti-gpNMB antibody specifically binds to one or more regions selected from a region containing amino acid residue(s) K257 and/or D258, a region containing amino acid residue(s) H268 and/or D269, a region containing amino acid residue K282, a region containing amino acid residue K316, a region containing amino acid residue K186, and a region containing amino acid residue(s) H216 and/or R218 of human gpNMB having the amino acid sequence of SEQ ID NO 209.

[Aspect 5] The pharmaceutical composition according to Aspect 1, wherein the anti-gpNMB antibody binds specifically to at least one site in a region from V78 to S92 of human gpNMB having the amino acid sequence of SEQ ID NO 209.

[Aspect 6] The pharmaceutical composition according to any one of Aspects 1 to 5, wherein the anti-gpNMB antibody specifically binds to at least one site in a region from V78 to a PKD domain of mouse gpNMB having the amino acid sequence of SEQ ID NO 211.

[Aspect 7] The pharmaceutical composition according to any one of Aspects 1 to 6, wherein the anti-gpNMB antibody is a monoclonal antibody or its fragment or a derivative thereof.

[Aspect 8] The pharmaceutical composition according to any one of Aspects 1 to 7, wherein the anti-gpNMB antibody comprises at least a heavy chain variable region comprising:

(1) as a CDR-H1 sequence, the amino acid sequence defined in SEQ ID NO 1, or an amino acid sequence derived from SEQ ID NO 1 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 1,

as a CDR-H2 sequence, the amino acid sequence defined in SEQ ID NO 3, or an amino acid sequence derived from SEQ ID NO 3 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 3, and

as a CDR-H3 sequence, the amino acid sequence defined in SEQ ID NO 5, or an amino acid sequence derived from SEQ ID NO 5 via substitution, deletion, or insertion of any one, two, three, or four amino acid residues, or an amino acid sequence having a homology (preferably an identity) of 66.6 % or more, 75.0 % or more, 83.3 % or more, or 91.6 % or more to the amino acid sequence defined in SEQ ID NO 5, or

(2) as a CDR-H1 sequence, the amino acid sequence defined in SEQ ID NO 17, or an amino acid sequence derived from SEQ ID NO 17 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 17,

as a CDR-H2 sequence, the amino acid sequence defined in SEQ ID NO 19, or an amino acid sequence derived from SEQ ID NO 19 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 19, and

as a CDR-H3 sequence, the amino acid sequence defined in SEQ ID NO 21, or an amino acid sequence derived from SEQ ID NO 21 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 21, or

(3) as a CDR-H1 sequence, the amino acid sequence defined in SEQ ID NO 33, or an amino acid sequence derived from SEQ ID NO 33 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 33,

as a CDR-H2 sequence, the amino acid sequence defined in SEQ ID NO 35, or an amino acid sequence derived from SEQ ID NO 35 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 35, and

as a CDR-H3 sequence, the amino acid sequence defined in SEQ ID NO 37, or an amino acid sequence derived from SEQ ID NO 37 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 37, or

(4) as a CDR-H1 sequence, the amino acid sequence defined in SEQ ID NO 49, or an amino acid sequence derived from SEQ ID NO 49 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 49,

as a CDR-H2 sequence, the amino acid sequence defined in SEQ ID NO 51, or an amino acid sequence derived from SEQ ID NO 51 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 51, and

as a CDR-H3 sequence, the amino acid sequence defined in SEQ ID NO 53, or an amino acid sequence derived from SEQ ID NO 53 via substitution, deletion, or insertion of any one, two, three, four, five, or six amino acid residues, or an amino acid sequence having a homology (preferably an identity) of 62.5 % or more, 68.7 % or more, 75.0 % or more, 81.2 % or more, 87.5 % or more, or 93.7 % or more to the amino acid sequence defined in SEQ ID NO 53, or

(5) as a CDR-H1 sequence, the amino acid sequence defined in SEQ ID NO 65, or an amino acid sequence derived from SEQ ID NO 65 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 65,

as a CDR-H2 sequence, the amino acid sequence defined in SEQ ID NO 67, or an amino acid sequence derived from SEQ ID NO 67 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 67, and

as a CDR-H3 sequence, the amino acid sequence defined in SEQ ID NO 69, or an amino acid sequence derived from SEQ ID NO 69 via substitution, deletion, or insertion of any one, two, three, four, or five amino acid residues, or an amino acid sequence having a homology (preferably an identity) of 64.2 % or more, 71.4 % or more, 78.5 % or more, 85.7 % or more, or 92.8 % or more to the amino acid sequence defined in SEQ ID NO 69, or

(6) as a CDR-H1 sequence, the amino acid sequence defined in SEQ ID NO 81, or an amino acid sequence derived from SEQ ID NO 81 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 81,

as a CDR-H2 sequence, the amino acid sequence defined in SEQ ID NO 83, or an amino acid sequence derived from SEQ ID NO 83 via substitution, deletion, or insertion of any one or two amino acid residues, or an amino acid sequence having a homology (preferably an identity) of 71.4 % or more or 85.7 % or more to the amino acid sequence defined in SEQ ID NO 83, and

as a CDR-H3 sequence, the amino acid sequence defined in SEQ ID NO 85, or an amino acid sequence derived from SEQ ID NO 85 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 85, or

(7) as a CDR-H1 sequence, the amino acid sequence defined in SEQ ID NO 97, or an amino acid sequence derived from SEQ ID NO 97 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 97,

as a CDR-H2 sequence, the amino acid sequence defined in SEQ ID NO 99, or an amino acid sequence derived from SEQ ID NO 99 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 99, and
as a CDR-H3 sequence, the amino acid sequence defined in SEQ ID NO 101, or an amino acid sequence derived from SEQ ID NO 101 via substitution, deletion, or insertion of any one or two amino acid residues, or an amino acid sequence having a homology (preferably an identity) of 60.0 % or more or 80.0 % or more to the amino acid sequence defined in SEQ ID NO 101, or

(8) as a CDR-H1 sequence, the amino acid sequence defined in SEQ ID NO 113, or an amino acid sequence derived from SEQ ID NO 113 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 113,

as a CDR-H2 sequence, the amino acid sequence defined in SEQ ID NO 115, or an amino acid sequence derived from SEQ ID NO 115 via substitution, deletion, or insertion of any one or two amino acid residues, or an amino acid sequence having a homology (preferably an identity) of 71.4 % or more or 85.7 % or more to the amino acid sequence defined in SEQ ID NO 115, and
as a CDR-H3 sequence, the amino acid sequence defined in SEQ ID NO 117, or an amino acid sequence derived from SEQ ID NO 117 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 117, or

(9) as a CDR-H1 sequence, the amino acid sequence defined in SEQ ID NO 129, or an amino acid sequence derived from SEQ ID NO 129 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 129,

as a CDR-H2 sequence, the amino acid sequence defined in SEQ ID NO 131, or an amino acid sequence derived from SEQ ID NO 131 via substitution, deletion, or insertion of any one or two amino acid residues, or an amino acid sequence having a homology (preferably an identity) of 71.4 % or more or 85.7 % or more to the amino acid sequence defined in SEQ ID NO 131, and
as a CDR-H3 sequence, the amino acid sequence defined in SEQ ID NO 133, or an amino acid sequence derived from SEQ ID NO 133 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 133, or

(10) as a CDR-H1 sequence, the amino acid sequence defined in SEQ ID NO 145, or an amino acid sequence derived from SEQ ID NO 145 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 145,

as a CDR-H2 sequence, the amino acid sequence defined in SEQ ID NO 147, or an amino acid sequence derived from SEQ ID NO 147 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 147, and
as a CDR-H3 sequence, the amino acid sequence defined in SEQ ID NO 149, or an amino acid sequence derived from SEQ ID NO 149 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 149, or

(11) as a CDR-H1 sequence, the amino acid sequence defined in SEQ ID NO 161, or an amino acid sequence derived from SEQ ID NO 161 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 161,

as a CDR-H2 sequence, the amino acid sequence defined in SEQ ID NO 163, or an amino acid sequence derived from SEQ ID NO 163 via substitution, deletion, or insertion of any one or two amino acid residues, or an amino acid sequence having a homology (preferably an identity) of 71.4 % or more or 85.7 % or more to the amino acid sequence defined in SEQ ID NO 163, and

as a CDR-H3 sequence, the amino acid sequence defined in SEQ ID NO 165, or an amino acid sequence derived from SEQ ID NO 165 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 165, or

(12) as a CDR-H1 sequence, the amino acid sequence defined in SEQ ID NO 255, or an amino acid sequence derived from SEQ ID NO 255 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 255,

as a CDR-H2 sequence, the amino acid sequence defined in SEQ ID NO 257, or an amino acid sequence derived from SEQ ID NO 257 via substitution, deletion, or insertion of any one or two amino acid residues, or an amino acid sequence having a homology (preferably an identity) of 71.4 % or more or 85.7 % or more to the amino acid sequence defined in SEQ ID NO 257, and

as a CDR-H3 sequence, the amino acid sequence defined in SEQ ID NO 259, or an amino acid sequence derived from SEQ ID NO 259 via substitution, deletion, or insertion of any one or two amino acid residues, or an amino acid sequence having a homology (preferably an identity) of 66.6 % or more or 83.3 % or more to the amino acid sequence defined in SEQ ID NO 259, or

(13) as a CDR-H1 sequence, the amino acid sequence defined in SEQ ID NO 283, or an amino acid sequence derived from SEQ ID NO 283 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 283,

as a CDR-H2 sequence, the amino acid sequence defined in SEQ ID NO 285, or an amino acid sequence derived from SEQ ID NO 285 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 285,

as a CDR-H3 sequence, the amino acid sequence defined in SEQ ID NO 287, or an amino acid sequence derived from SEQ ID NO 287 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 287, or

(14) as a CDR-H1 sequence, the amino acid sequence defined in SEQ ID NO 311, or an amino acid sequence derived from SEQ ID NO 311 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 311,

as a CDR-H2 sequence, the amino acid sequence defined in SEQ ID NO 313, or an amino acid sequence derived from SEQ ID NO 313 via substitution, deletion, or insertion of any one or two amino acid residues, or an amino acid sequence having a homology (preferably an identity) of 71.4 % or more or 85.7 % or more to the amino acid sequence defined in SEQ ID NO 313,

as a CDR-H3 sequence, the amino acid sequence defined in SEQ ID NO 315, or an amino acid sequence derived from SEQ ID NO 315 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 315, or

(15) as a CDR grafting region H1 sequence, the amino acid sequence defined in SEQ ID NO 271, or an amino acid sequence derived from SEQ ID NO 271 via substitution, deletion, or insertion of any one, two, three, or four amino acid residues other than tyrosine at position 27, asparagine at position 32, and tryptophan at position 33,

as a CDR grafting region H2 sequence, the amino acid sequence defined in SEQ ID NO 273, or an amino acid sequence derived from SEQ ID NO 273 via substitution, deletion, or insertion of any one, two, three, four, five, or six amino acid residues other than aspartic acid at position 55, phenylalanine at position 57, threonine at position 58, asparagine at position 59, tyrosine at position 60, and asparagine at position 61,
as a CDR grafting region H3 sequence, the amino acid sequence defined in SEQ ID NO 275, or an amino acid sequence defined in SEQ ID NO 275 via substitution, deletion, or insertion of any one, two, three, four, five, or six amino acid residues other than arginine at position 98, glycine at position 100, and glycine at position 109, or

(16) as a CDR grafting region H1 sequence, the amino acid sequence defined in SEQ ID NO 299, or an amino acid sequence derived from SEQ ID NO 299 via substitution, deletion, or insertion of any one, two, three, or four amino acid residues,

as a CDR grafting region H2 sequence, the amino acid sequence defined in SEQ ID NO 301, or an amino acid sequence derived from SEQ ID NO 301 via substitution, deletion, or insertion of any one, two, three, four, five, or six amino acid residues other than aspartic acid at position 52, tryptophan at position 54, aspartic acid at position 58, and proline at position 63,
as a CDR grafting region H3 sequence, the amino acid sequence defined in SEQ ID NO 303, or an amino acid sequence derived from SEQ ID NO 303 via substitution, deletion, or insertion of any one, two, three, four, five, or six amino acid residues other than arginine at position 99, threonine at position 100, tyrosine at position 102, tyrosine at position 105, tyrosine at position 107, and methionine at position 110, or

(17) as a CDR-H1 sequence, the amino acid sequence defined in SEQ ID NO 177, or an amino acid sequence derived from SEQ ID NO 177 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 177,

as a CDR-H2 sequence, the amino acid sequence defined in SEQ ID NO 179, or an amino acid sequence derived from SEQ ID NO 179 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 179, and
as a CDR-H3 sequence, the amino acid sequence defined in SEQ ID NO 181, or an amino acid sequence derived from SEQ ID NO 181 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 181, or

(18) as a CDR-H1 sequence, the amino acid sequence defined in SEQ ID NO 330, or an amino acid sequence derived from SEQ ID NO 330 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 330,

as a CDR-H2 sequence, the amino acid sequence defined in SEQ ID NO 331, or an amino acid sequence derived from SEQ ID NO 331 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 331, and
as a CDR-H3 sequence, the amino acid sequence defined in SEQ ID NO 332, or an amino acid sequence derived from SEQ ID NO 332 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 332, or

(19) as a CDR-H1 sequence, the amino acid sequence defined in SEQ ID NO 337, or an amino acid sequence derived from SEQ ID NO 337 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 337,

as a CDR-H2 sequence, the amino acid sequence defined in SEQ ID NO 338, or an amino acid sequence derived from SEQ ID NO 338 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 338, and

as a CDR-H3 sequence, the amino acid sequence defined in SEQ ID NO 339, or an amino acid sequence derived from SEQ ID NO 339 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 339, or

(20) as a CDR-H1 sequence, the amino acid sequence defined in SEQ ID NO 344, or an amino acid sequence derived from SEQ ID NO 344 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 344,

as a CDR-H2 sequence, the amino acid sequence defined in SEQ ID NO 345, or an amino acid sequence derived from SEQ ID NO 345 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 345, and

as a CDR-H3 sequence, the amino acid sequence defined in SEQ ID NO 346, or an amino acid sequence derived from SEQ ID NO 346 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 346, or

(21) as a CDR grafting region H1 sequence, the amino acid sequence defined in SEQ ID NO 327, or an amino acid sequence derived from SEQ ID NO 327 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 327, as a CDR grafting region H2 sequence, the amino acid sequence defined in SEQ ID NO 328, or an amino acid sequence derived from SEQ ID NO 328 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 328,

as a CDR grafting region H3 sequence, the amino acid sequence defined in SEQ ID NO 329, or an amino acid sequence derived from SEQ ID NO 329 via substitution, deletion, or insertion of any one or two amino acid residues, or an amino acid sequence having a homology (preferably an identity) of 66.6 % or more or 83.3 % or more to the amino acid sequence defined in SEQ ID NO 329, or

(22) as a CDR grafting region H1 sequence, the amino acid sequence defined in SEQ ID NO 334, or an amino acid sequence derived from SEQ ID NO 334 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 334, as a CDR grafting region H2 sequence, the amino acid sequence defined in SEQ ID NO 335, or an amino acid sequence derived from SEQ ID NO 335 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 335,

as a CDR grafting region H3 sequence, the amino acid sequence defined in SEQ ID NO 336, or an amino acid sequence derived from SEQ ID NO 336 via substitution, deletion, or insertion of any one or two amino acid residues, or an amino acid sequence having a homology (preferably an identity) of 66.6 % or more or 83.3 % or more to the amino acid sequence defined in SEQ ID NO 336, or

(23) as a CDR grafting region H1 sequence, the amino acid sequence defined in SEQ ID NO 341, or an amino acid sequence derived from SEQ ID NO 341 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 341, as a CDR grafting region H2 sequence, the amino acid sequence defined in SEQ ID NO 342, or an amino acid sequence derived from SEQ ID NO 342 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 342,

as a CDR grafting region H3 sequence, the amino acid sequence defined in SEQ ID NO 343, or an amino acid sequence derived from SEQ ID NO 343 via substitution, deletion, or insertion of any one or two amino acid residues, or an amino acid sequence having a homology (preferably an identity) of 66.6 % or more or 83.3 % or more to the amino acid sequence defined in SEQ ID NO 343.

[Aspect 9] The pharmaceutical composition according to any one of Aspects 1 to 7, wherein the anti-gpNMB antibody comprises at least a heavy chain variable region comprising:

(1) the amino acid sequence defined in SEQ ID NO 13, or an amino acid sequence having a homology of 60 % or more to the amino acid sequence defined in SEQ ID NO 13, or
(2) the amino acid sequence defined in SEQ ID NO 29, or an amino acid sequence having a homology of 60 % or more to the amino acid sequence defined in SEQ ID NO 29, or
(3) the amino acid sequence defined in SEQ ID NO 45, or an amino acid sequence having a homology of 60 % or more to the amino acid sequence defined in SEQ ID NO 45, or
(4) the amino acid sequence defined in SEQ ID NO 61, or an amino acid sequence having a homology of 60 % or more to the amino acid sequence defined in SEQ ID NO 61, or
(5) the amino acid sequence defined in SEQ ID NO 77, or an amino acid sequence having a homology of 60 % or more to the amino acid sequence defined in SEQ ID NO 77, or
(6) the amino acid sequence defined in SEQ ID NO 93, or an amino acid sequence having a homology of 60 % or more to the amino acid sequence defined in SEQ ID NO 93, or
(7) the amino acid sequence defined in SEQ ID NO 109, or an amino acid sequence having a homology of 60 % or more to the amino acid sequence defined in SEQ ID NO 109, or
(8) the amino acid sequence defined in SEQ ID NO 125, or an amino acid sequence having a homology of 60 % or more to the amino acid sequence defined in SEQ ID NO 125, or
(9) the amino acid sequence defined in SEQ ID NO 141, or an amino acid sequence having a homology of 60 % or more to the amino acid sequence defined in SEQ ID NO 141, or
(10) the amino acid sequence defined in SEQ ID NO 157, or an amino acid sequence having a homology of 60 % or more to the amino acid sequence defined in SEQ ID NO 157, or
(11) the amino acid sequence defined in SEQ ID NO 173, or an amino acid sequence having a homology of 60 % or more to the amino acid sequence defined in SEQ ID NO 173, or
(12) the amino acid sequence defined in SEQ ID NO 267, or an amino acid sequence having a homology of 60 % or more to the amino acid sequence defined in SEQ ID NO 267, or
(13) the amino acid sequence defined in SEQ ID NO 295, or an amino acid sequence having a homology of 60 % or more to the amino acid sequence defined in SEQ ID NO 295, or
(14) the amino acid sequence defined in SEQ ID NO 323, or an amino acid sequence having a homology of 60 % or more to the amino acid sequence defined in SEQ ID NO 323, or
(17) the amino acid sequence defined in SEQ ID NO 189, or an amino acid sequence having a homology of 60 % or more to the amino acid sequence defined in SEQ ID NO 189, or
(18) the amino acid sequence defined in SEQ ID NO 333, or an amino acid sequence having a homology of 60 % or more to the amino acid sequence defined in SEQ ID NO 333, or
(19) the amino acid sequence defined in SEQ ID NO 340, or an amino acid sequence having a homology of 60 % or more to the amino acid sequence defined in SEQ ID NO 340, or
(20) the amino acid sequence defined in SEQ ID NO 347, or an amino acid sequence having a homology of 60 % or more to the amino acid sequence defined in SEQ ID NO 347.

[Aspect 10] The pharmaceutical composition according to Aspect 8 or 9, wherein the heavy chain variable region comprises, as a framework sequence, a framework sequence of a class of human immunoglobulin.
[Aspect 11] The pharmaceutical composition according to any one of Aspects 8 to 10, wherein the anti-gpNMB antibody further comprises a heavy chain constant region having an amino acid sequence of a heavy chain constant region in a class of human immunoglobulin.
[Aspect 12] The pharmaceutical composition according to any one of Aspects 1 to 11, wherein the anti-gpNMB antibody comprises at least a light chain variable region comprising:

(1) as a CDR-L1 sequence, the amino acid sequence defined in SEQ ID NO 7, or an amino acid sequence derived from SEQ ID NO 7 via substitution, deletion, or insertion of any one or two amino acid residues, or an amino acid sequence having a homology (preferably an identity) of 66.6 % or more or 83.3 % or more to the amino acid sequence defined in SEQ ID NO 7,

as a CDR-L2 sequence, the amino acid sequence defined in SEQ ID NO 9, or an amino acid sequence derived from SEQ ID NO 9 via substitution, deletion, or insertion of any one amino acid residue, or an amino acid sequence having a homology (preferably an identity) of 66.6 % or more to the amino acid sequence defined in SEQ ID NO 9, and

as a CDR-L3 sequence, the amino acid sequence defined in SEQ ID NO 11, or an amino acid sequence derived from SEQ ID NO 11 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 11, or

(2) as a CDR-L1 sequence, the amino acid sequence defined in SEQ ID NO 23, or an amino acid sequence derived from SEQ ID NO 23 via substitution, deletion, or insertion of any one or two amino acid residues, or an amino acid sequence having a homology (preferably an identity) of 60.0 % or more or 80.0 % or more to the amino acid sequence defined in SEQ ID NO 23,

as a CDR-L2 sequence, the amino acid sequence defined in SEQ ID NO 25, or an amino acid sequence derived from SEQ ID NO 25 via substitution, deletion, or insertion of any one amino acid residue, or an amino acid sequence having a homology (preferably an identity) of 66.6 % or more to the amino acid sequence defined in SEQ ID NO 25, and

as a CDR-L3 sequence, the amino acid sequence defined in SEQ ID NO 27, or an amino acid sequence derived from SEQ ID NO 27 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 27, or

(3) as a CDR-L1 sequence, the amino acid sequence defined in SEQ ID NO 39, or an amino acid sequence derived from SEQ ID NO 39 via substitution, deletion, or insertion of any one or two amino acid residues, or an amino acid sequence having a homology (preferably an identity) of 60.0 % or more or 80.0 % or more to the amino acid sequence defined in SEQ ID NO 39,

as a CDR-L2 sequence, the amino acid sequence defined in SEQ ID NO 41, or an amino acid sequence derived from SEQ ID NO 41 via substitution, deletion, or insertion of any one amino acid residue, or an amino acid sequence having a homology (preferably an identity) of 66.6 % or more to the amino acid sequence defined in SEQ ID NO 41, and

as a CDR-L3 sequence, the amino acid sequence defined in SEQ ID NO 43, or an amino acid sequence derived from SEQ ID NO 43 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 43, or

(4) as a CDR-L1 sequence, the amino acid sequence defined in SEQ ID NO 55, or an amino acid sequence derived from SEQ ID NO 55 via substitution, deletion, or insertion of any one or two amino acid residues, or an amino acid sequence having a homology (preferably an identity) of 66.6 % or more or 83.3 % or more to the amino acid sequence defined in SEQ ID NO 55,

as a CDR-L2 sequence, the amino acid sequence defined in SEQ ID NO 57, or an amino acid sequence derived from SEQ ID NO 57 via substitution, deletion, or insertion of any one amino acid residue, or an amino acid sequence having a homology (preferably an identity) of 66.6 % or more to the amino acid sequence defined in SEQ ID NO 57, and

as a CDR-L3 sequence, the amino acid sequence defined in SEQ ID NO 59, or an amino acid sequence derived from SEQ ID NO 59 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 59, or

(5) as a CDR-L1 sequence, the amino acid sequence defined in SEQ ID NO 71, or an amino acid sequence derived from SEQ ID NO 71 via substitution, deletion, or insertion of any one or two amino acid residues, or an amino acid sequence having a homology (preferably an identity) of 60.0 % or more or 80.0 % or more to the amino acid sequence defined in SEQ ID NO 71,

as a CDR-L2 sequence, the amino acid sequence defined in SEQ ID NO 73, or an amino acid sequence derived from SEQ ID NO 73 via substitution, deletion, or insertion of any one amino acid residue, or an amino acid sequence having a homology (preferably an identity) of 66.6 % or more to the amino acid sequence defined in SEQ ID NO 73, and

as a CDR-L3 sequence, the amino acid sequence defined in SEQ ID NO 75, or an amino acid sequence derived from SEQ ID NO 75 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 75, or

(6) as a CDR-L1 sequence, the amino acid sequence defined in SEQ ID NO 87, or an amino acid sequence derived from SEQ ID NO 87 via substitution, deletion, or insertion of any one or two amino acid residues, or an amino acid sequence having a homology (preferably an identity) of 66.6 % or more or 83.3 % or more to the amino acid sequence defined in SEQ ID NO 87,

as a CDR-L2 sequence, the amino acid sequence defined in SEQ ID NO 89, or an amino acid sequence derived from SEQ ID NO 89 via substitution, deletion, or insertion of any one amino acid residue, or an amino acid sequence having a homology (preferably an identity) of 66.6 % or more to the amino acid sequence defined in SEQ ID NO 89, and

as a CDR-L3 sequence, the amino acid sequence defined in SEQ ID NO 91, or an amino acid sequence derived from SEQ ID NO 91 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 91, or

(7) as a CDR-L1 sequence, the amino acid sequence defined in SEQ ID NO 103, or an amino acid sequence derived from SEQ ID NO 103 via substitution, deletion, or insertion of any one or two amino acid residues, or an amino acid sequence having a homology (preferably an identity) of 66.6 % or more or 83.3 % or more to the amino acid sequence defined in SEQ ID NO 103, as a CDR-L2 sequence, the amino acid sequence defined in SEQ ID NO 105, or an amino acid sequence derived from SEQ ID NO 105 via substitution, deletion, or insertion of any one amino acid residue, or an amino acid sequence having a homology (preferably an identity) of 66.6 % or more to the amino acid sequence defined in SEQ ID NO 105, and as a CDR-L3 sequence, the amino acid sequence defined in SEQ ID NO 107, or an amino acid sequence derived from SEQ ID NO 107 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 107, or

(8) as a CDR-L1 sequence, the amino acid sequence defined in SEQ ID NO 119, or an amino acid sequence derived from SEQ ID NO 119 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 119, as a CDR-L2 sequence, the amino acid sequence defined in SEQ ID NO 121, or an amino acid sequence derived from SEQ ID NO 121 via substitution, deletion, or insertion of any one amino acid residue, or an amino acid sequence having a homology (preferably an identity) of 66.6 % or more to the amino acid sequence defined in SEQ ID NO 121, and as a CDR-L3 sequence, the amino acid sequence defined in SEQ ID NO 123, or an amino acid sequence derived from SEQ ID NO 123 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 123, or

(9) as a CDR-L1 sequence, the amino acid sequence defined in SEQ ID NO 135, or an amino acid sequence derived from SEQ ID NO 135 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 135, as a CDR-L2 sequence, the amino acid sequence defined in SEQ ID NO 137, or an amino acid sequence derived from SEQ ID NO 137 via substitution, deletion, or insertion of any one amino acid residue, or an amino acid sequence having a homology (preferably an identity) of 66.6 % or more to the amino acid sequence defined in SEQ ID NO 137, and as a CDR-L3 sequence, the amino acid sequence defined in SEQ ID NO 139, or an amino acid sequence derived from SEQ ID NO 139 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 139, or

(10) as a CDR-L1 sequence, the amino acid sequence defined in SEQ ID NO 151, or an amino acid sequence derived from SEQ ID NO 151 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 151,

as a CDR-L2 sequence, the amino acid sequence defined in SEQ ID NO 153, or an amino acid sequence derived from SEQ ID NO 153 via substitution, deletion, or insertion of any one amino acid residue, or an amino acid sequence having a homology (preferably an identity) of 66.6 % or more to the amino acid sequence defined in SEQ ID NO 153, and as a CDR-L3 sequence, the amino acid sequence defined in SEQ ID NO 155, or an amino acid sequence derived from SEQ ID NO 155 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 155, or

(11) as a CDR-L1 sequence, the amino acid sequence defined in SEQ ID NO 167, or an amino acid sequence derived from SEQ ID NO 167 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 167, as a CDR-L2 sequence, the amino acid sequence defined in SEQ ID NO 169, or an amino acid sequence derived from SEQ ID NO 169 via substitution, deletion, or insertion of any one amino acid residue, or an amino acid sequence having a homology (preferably an identity) of 66.6 % or more to the amino acid sequence defined in SEQ ID NO 169, and as a CDR-L3 sequence, the amino acid sequence defined in SEQ ID NO 171, or an amino acid sequence derived from SEQ ID NO 171 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 171, or

(12) as a CDR-L1 sequence, the amino acid sequence defined in SEQ ID NO 261, or an amino acid sequence derived from SEQ ID NO 261 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 261, as a CDR-L2 sequence, the amino acid sequence defined in SEQ ID NO 263, or an amino acid sequence derived from SEQ ID NO 263 via substitution, deletion, or insertion of any one amino acid residue, or an amino acid sequence having a homology (preferably an identity) of 66.6 % or more to the amino acid sequence defined in SEQ ID NO 263, and as a CDR-L3 sequence, the amino acid sequence defined in SEQ ID NO 265, or an amino acid sequence derived from SEQ ID NO 265 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 265, or

(13) as a CDR-L1 sequence, the amino acid sequence defined in SEQ ID NO 289, or an amino acid sequence derived from SEQ ID NO 289 via substitution, deletion, or insertion of any one or two amino acid residues, or an amino acid sequence having a homology (preferably an identity) of 60.0 % or more or 80.0 % or more to the amino acid sequence defined in SEQ ID NO 289, as a CDR-L2 sequence, the amino acid sequence defined in SEQ ID NO 291, or an amino acid sequence derived from SEQ ID NO 291 via substitution, deletion, or insertion of any one amino acid residue, or an amino acid sequence having a homology (preferably an identity) of 66.6 % or more to the amino acid sequence defined in SEQ ID NO 291, and as a CDR-L3 sequence, the amino acid sequence defined in SEQ ID NO 293, or an amino acid sequence derived from SEQ ID NO 293 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 293, or

(14) as a CDR-L1 sequence, the amino acid sequence defined in SEQ ID NO 317, or an amino acid sequence derived from SEQ ID NO 317 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 317, as a CDR-L2 sequence, the amino acid sequence defined in SEQ ID NO 319, or an amino acid sequence derived from SEQ ID NO 319 via substitution, deletion, or insertion of any one amino acid residue, or an amino acid sequence having a homology (preferably an identity) of 66.6 % or more to the amino acid sequence defined in SEQ ID NO 319, and as a CDR-L3 sequence, the amino acid sequence defined in SEQ ID NO 321, or an amino acid sequence derived from SEQ ID NO 321 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 321, or

(15) as a CDR grafting region L1 sequence, the amino acid sequence defined in SEQ ID NO 277, or an amino acid sequence defined in SEQ ID NO 277 via substitution, deletion, or insertion of any one, two, three, or four amino acid residues other than isoleucine at position 29, tyrosine at position 31, and histidine at position 33,

as a CDR grafting region L2 sequence, the amino acid sequence defined in SEQ ID NO 279, or an amino acid sequence defined in SEQ ID NO 279 via substitution, deletion, or insertion of any one or two amino acid residue other than threonine at position 50,

as a CDR grafting region L3 sequence, the amino acid sequence defined in SEQ ID NO 281, or an amino acid sequence defined in SEQ ID NO 281 via substitution, deletion, or insertion of any one, two, or three amino acid residues other than histidine at position 88, glutamine at position 89, tryptophan at position 90, serine at position 92, tyrosine at position 93, proline at position 94, and cysteine at position 95, or

(16) as a CDR grafting region L1 sequence, the amino acid sequence defined in SEQ ID NO 305, or an amino acid sequence defined in SEQ ID NO 305 via substitution, deletion, or insertion of any one, two, three, four, five, or six amino acid residues other than leucine at position 31, tyrosine at position 37 and glutamic acid at position 39,

as a CDR grafting region L2 sequence, the amino acid sequence defined in SEQ ID NO 307, or an amino acid sequence defined in SEQ ID NO 307 via substitution, deletion, or insertion of any one or two amino acid residue other than lysine at position 55,
as a CDR grafting region L3 sequence, the amino acid sequence defined in SEQ ID NO 309, or an amino acid sequence defined in SEQ ID NO 309 via substitution, deletion, or insertion of any one, two, or three amino acid residues other than phenylalanine at position 94, or

(17) as a CDR-L1 sequence, the amino acid sequence defined in SEQ ID NO 183, or an amino acid sequence derived from SEQ ID NO 183 via substitution, deletion, or insertion of any one or two amino acid residues, or an amino acid sequence having a homology (preferably an identity) of 66.6 % or more or 83.3 % or more to the amino acid sequence defined in SEQ ID NO 183,

as a CDR-L2 sequence, the amino acid sequence defined in SEQ ID NO 185, or an amino acid sequence derived from SEQ ID NO 185 via substitution, deletion, or insertion of any one amino acid residue, or an amino acid sequence having a homology (preferably an identity) of 66.6 % or more to the amino acid sequence defined in SEQ ID NO 185,
as a CDR-L3 sequence, the amino acid sequence defined in SEQ ID NO 187, or an amino acid sequence derived from SEQ ID NO 187 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 187, or

(18) as a CDR-L1 sequence, the amino acid sequence defined in SEQ ID NO 351, or an amino acid sequence derived from SEQ ID NO 351 via substitution, deletion, or insertion of any one or two amino acid residues, or an amino acid sequence having a homology (preferably an identity) of 66.6 % or more or 83.3 % or more to the amino acid sequence defined in SEQ ID NO 351,

as a CDR-L2 sequence, the amino acid sequence defined in SEQ ID NO 352, or an amino acid sequence derived from SEQ ID NO 352 via substitution, deletion, or insertion of any one amino acid residue, or an amino acid sequence having a homology (preferably an identity) of 66.6 % or more to the amino acid sequence defined in SEQ ID NO 352,
as a CDR-L3 sequence, the amino acid sequence defined in SEQ ID NO 353, or an amino acid sequence derived from SEQ ID NO 353 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 353, or

(19) as a CDR-L1 sequence, the amino acid sequence defined in SEQ ID NO 358, or an amino acid sequence derived from SEQ ID NO 358 via substitution, deletion, or insertion of any one or two amino acid residues, or an amino acid sequence having a homology (preferably an identity) of 66.6 % or more or 83.3 % or more to the amino acid sequence defined in SEQ ID NO 358,

as a CDR-L2 sequence, the amino acid sequence defined in SEQ ID NO 359, or an amino acid sequence derived from SEQ ID NO 359 via substitution, deletion, or insertion of any one amino acid residue, or an amino acid sequence having a homology (preferably an identity) of 66.6 % or more to the amino acid sequence defined in SEQ ID NO 359,
as a CDR-L3 sequence, the amino acid sequence defined in SEQ ID NO 360, or an amino acid sequence derived from SEQ ID NO 360 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 360, or

(20) as a CDR-L1 sequence, the amino acid sequence defined in SEQ ID NO 365, or an amino acid sequence derived from SEQ ID NO 365 via substitution, deletion, or insertion of any one or two amino acid residues, or an amino acid sequence having a homology (preferably an identity) of 66.6 % or more or 83.3 % or more to the amino acid sequence defined in SEQ ID NO 365,

as a CDR-L2 sequence, the amino acid sequence defined in SEQ ID NO 366, or an amino acid sequence derived from SEQ ID NO 366 via substitution, deletion, or insertion of any one amino acid residue, or an amino acid sequence having a homology (preferably an identity) of 66.6 % or more to the amino acid sequence defined in SEQ ID NO 366,
as a CDR-L3 sequence, the amino acid sequence defined in SEQ ID NO 367, or an amino acid sequence derived from SEQ ID NO 367 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 367, or

(21) as a CDR grafting region L1 sequence, the amino acid sequence defined in SEQ ID NO 348, or an amino acid sequence derived from SEQ ID NO 348 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 348, as a CDR grafting region L2 sequence, the amino acid sequence defined in SEQ ID NO 349, or an amino acid sequence derived from SEQ ID NO 349 via substitution, deletion, or insertion of any one or two amino acid residues, or an amino acid sequence having a homology (preferably an identity) of 71.4 % or more or 85.7 % or more to the amino acid sequence defined in SEQ ID NO 349,
as a CDR grafting region L3 sequence, the amino acid sequence defined in SEQ ID NO 350, or an amino acid sequence derived from SEQ ID NO 350 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 350, or
(22) as a CDR grafting region L1 sequence, the amino acid sequence defined in SEQ ID NO 355, or an amino acid sequence derived from SEQ ID NO 355 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 355, as a CDR grafting region L2 sequence, the amino acid sequence defined in SEQ ID NO 356, or an amino acid sequence derived from SEQ ID NO 356 via substitution, deletion, or insertion of any one or two amino acid residues, or an amino acid sequence having a homology (preferably an identity) of 71.4 % or more or 85.7 % or more to the amino acid sequence defined in SEQ ID NO 356,
as a CDR grafting region L3 sequence, the amino acid sequence defined in SEQ ID NO 357, or an amino acid sequence derived from SEQ ID NO 357 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 357, or
(23) as a CDR grafting region L1 sequence, the amino acid sequence defined in SEQ ID NO 362, or an amino acid sequence derived from SEQ ID NO 362 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 362, as a CDR grafting region L2 sequence, the amino acid sequence defined in SEQ ID NO 363, or an amino acid sequence derived from SEQ ID NO 363 via substitution, deletion, or insertion of any one or two amino acid residues, or an amino acid sequence having a homology (preferably an identity) of 71.4 % or more or 85.7 % or more to the amino acid sequence defined in SEQ ID NO 363,
as a CDR grafting region L3 sequence, the amino acid sequence defined in SEQ ID NO 364, or an amino acid sequence derived from SEQ ID NO 364 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 364.

[Aspect 13] The pharmaceutical composition according to any one of Aspects 1 to 11, wherein the anti-gpNMB antibody comprises at least a light chain variable region comprising:

(1) the amino acid sequence defined in SEQ ID NO 15, or an amino acid sequence having a homology of 60 % or more to the amino acid sequence defined in SEQ ID NO 15, or
(2) the amino acid sequence defined in SEQ ID NO 31, or an amino acid sequence having a homology of 60 % or more to the amino acid sequence defined in SEQ ID NO 31, or

(3) the amino acid sequence defined in SEQ ID NO 47, or an amino acid sequence having a homology of 60 % or more to the amino acid sequence defined in SEQ ID NO 47, or

(4) the amino acid sequence defined in SEQ ID NO 63, or an amino acid sequence having a homology of 60 % or more to the amino acid sequence defined in SEQ ID NO 63, or

(5) the amino acid sequence defined in SEQ ID NO 79, or an amino acid sequence having a homology of 60 % or more to the amino acid sequence defined in SEQ ID NO 79, or

(6) the amino acid sequence defined in SEQ ID NO 95, or an amino acid sequence having a homology of 60 % or more to the amino acid sequence defined in SEQ ID NO 95, or

(7) the amino acid sequence defined in SEQ ID NO 111, or an amino acid sequence having a homology of 60 % or more to the amino acid sequence defined in SEQ ID NO 111, or

(8) the amino acid sequence defined in SEQ ID NO 127, or an amino acid sequence having a homology of 60 % or more to the amino acid sequence defined in SEQ ID NO 127, or

(9) the amino acid sequence defined in SEQ ID NO 143, or an amino acid sequence having a homology of 60 % or more to the amino acid sequence defined in SEQ ID NO 143, or

(10) the amino acid sequence defined in SEQ ID NO 159, or an amino acid sequence having a homology of 60 % or more to the amino acid sequence defined in SEQ ID NO 159, or

(11) the amino acid sequence defined in SEQ ID NO 175, or an amino acid sequence having a homology of 60 % or more to the amino acid sequence defined in SEQ ID NO 175, or

(12) the amino acid sequence defined in SEQ ID NO 269, or an amino acid sequence having a homology of 60 % or more to the amino acid sequence defined in SEQ ID NO 269, or

(13) the amino acid sequence defined in SEQ ID NO 297, or an amino acid sequence having a homology of 60 % or more to the amino acid sequence defined in SEQ ID NO 297, or

(14) the amino acid sequence defined in SEQ ID NO 325, or an amino acid sequence having a homology of 60 % or more to the amino acid sequence defined in SEQ ID NO 325, or

(17) the amino acid sequence defined in SEQ ID NO 191, or an amino acid sequence having a homology of 60 % or more to the amino acid sequence defined in SEQ ID NO 191, or

(18) the amino acid sequence defined in SEQ ID NO 354, or an amino acid sequence having a homology of 60 % or more to the amino acid sequence defined in SEQ ID NO 354, or

(19) the amino acid sequence defined in SEQ ID NO 361, or an amino acid sequence having a homology of 60 % or more to the amino acid sequence defined in SEQ ID NO 361, or

(20) the amino acid sequence defined in SEQ ID NO 368, or an amino acid sequence having a homology of 60 % or more to the amino acid sequence defined in SEQ ID NO 368.

[Aspect 14] The pharmaceutical composition according to Aspect 12 or 13, wherein the light chain variable region comprises, as a framework sequence, a framework sequence of a class of human immunoglobulin.

[Aspect 15] The pharmaceutical composition according to any one of Aspects 12 to 14, wherein the anti-gpNMB antibody further comprises a light chain constant region having an amino acid sequence of a light chain constant region in a class of human immunoglobulin.

[Aspect 16] The pharmaceutical composition according to any one of Aspects 1 to 15, wherein the anti-gpNMB antibody is a Fab, scFv, Diabody, Nanobody, VHH, bispecific antibody, or multispecific antibody, or a derivative thereof.

[Aspect 17] A pharmaceutical composition for removing disease-related cells, comprising an anti-gpNMB antibody or its fragment or a derivative thereof that binds to at least one site in a region from V78 to a PKD domain of human gpNMB (glycoprotein nonmetastatic melanoma protein B) having the amino acid sequence of SEQ ID NO 209 competitively with an anti-gpNMB antibody or its fragment or a derivative thereof according to any one of Aspects 1 to 16.

[Aspect 18] A pharmaceutical composition for removing disease-related cells, comprising:

a nucleic acid molecule having a polynucleotide sequence encoding an anti-gpNMB antibody or its fragment or a derivative thereof according to any one of Aspects 1 to 16; and/or
a cloning vector or expression vector carrying at least one of the nucleic acid molecule; and/or
a recombinant cell into which the vector has been introduced.

[Aspect 19] The pharmaceutical composition according to any one of Aspects 1 to 18, wherein the disease-related cells are selected from senescent cells, cancer-related cells, and phagocytes.

[Aspect 20] The pharmaceutical composition according to Aspect 19, wherein the cancer-related cells are selected from cancer cells, cancer stem cells, cancer progenitor cells, and cancer infiltration cells.

[Aspect 21] The pharmaceutical composition according to any one of Aspects 1 to 20, wherein the disease is selected from obesity, diabetes, arteriosclerosis, fibrosis, cancer, metabolic disease, circulatory disease, lysosomal stress-associated disease (e.g., lysosome disease), autoimmune disease (e.g., multiple sclerosis), neuropathic pain, fibromyalgia, and glioblastoma.

[Aspect 22] The pharmaceutical composition according to any one of Aspects 1 to 21, for used in treating or preventing a disease by removing disease-related cells.

[Aspect 23] An anti-gpNMB antibody or its fragment or a derivative thereof that binds specifically to at least one site in a region from V78 to S92 of human gpNMB (glycoprotein nonmetastatic melanoma protein B) having the amino acid sequence of SEQ ID NO 209.

[Aspect 24] The anti-gpNMB antibody or its fragment or a derivative thereof according to Aspect 23, wherein the anti-gpNMB antibody also binds specifically to a region containing amino acid residue K186 of human gpNMB having the amino acid sequence of SEQ ID NO 209.

[Aspect 25] The anti-gpNMB antibody or its fragment or a derivative thereof according to Aspect 23 or 24, wherein the anti-gpNMB antibody also binds specifically to at least one site in a region from V78 to S92 of mouse gpNMB having the amino acid sequence of SEQ ID NO 211.

[Aspect 26] The anti-gpNMB antibody or its fragment or a derivative thereof according to any one of Aspects 23 to 25, which is a monoclonal antibody or its fragment or a derivative thereof.

[Aspect 27] The anti-gpNMB antibody or its fragment or a derivative thereof according to Aspect 26, further comprising at least a heavy chain variable region comprising:

(17) as a CDR-H1 sequence, the amino acid sequence defined in SEQ ID NO 177, or an amino acid sequence derived from SEQ ID NO 177 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 177,

as a CDR-H2 sequence, the amino acid sequence defined in SEQ ID NO 179, or an amino acid sequence derived from SEQ ID NO 179 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 179, and

as a CDR-H3 sequence, the amino acid sequence defined in SEQ ID NO 181, or an amino acid sequence derived from SEQ ID NO 181 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 181, or

(18) as a CDR-H1 sequence, the amino acid sequence defined in SEQ ID NO 330, or an amino acid sequence derived from SEQ ID NO 330 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 330,

as a CDR-H2 sequence, the amino acid sequence defined in SEQ ID NO 331, or an amino acid sequence derived from SEQ ID NO 331 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 331, and

as a CDR-H3 sequence, the amino acid sequence defined in SEQ ID NO 332, or an amino acid sequence derived from SEQ ID NO 332 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 332, or

(19) as a CDR-H1 sequence, the amino acid sequence defined in SEQ ID NO 337, or an amino acid sequence derived from SEQ ID NO 337 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 337,

as a CDR-H2 sequence, the amino acid sequence defined in SEQ ID NO 338, or an amino acid sequence derived from SEQ ID NO 338 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 338, and

as a CDR-H3 sequence, the amino acid sequence defined in SEQ ID NO 339, or an amino acid sequence derived from SEQ ID NO 339 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 339, or

(20) as a CDR-H1 sequence, the amino acid sequence defined in SEQ ID NO 344, or an amino acid sequence derived from SEQ ID NO 344 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 344,

as a CDR-H2 sequence, the amino acid sequence defined in SEQ ID NO 345, or an amino acid sequence derived from SEQ ID NO 345 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 345, and
as a CDR-H3 sequence, the amino acid sequence defined in SEQ ID NO 346, or an amino acid sequence derived from SEQ ID NO 346 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 346, or

(21) as a CDR grafting region H1 sequence, the amino acid sequence defined in SEQ ID NO 327, or an amino acid sequence derived from SEQ ID NO 327 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 327, as a CDR grafting region H2 sequence, the amino acid sequence defined in SEQ ID NO 328, or an amino acid sequence derived from SEQ ID NO 328 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 328,
as a CDR grafting region H3 sequence, the amino acid sequence defined in SEQ ID NO 329, or an amino acid sequence derived from SEQ ID NO 329 via substitution, deletion, or insertion of any one or two amino acid residues, or an amino acid sequence having a homology (preferably an identity) of 66.6 % or more or 83.3 % or more to the amino acid sequence defined in SEQ ID NO 329, or
(22) as a CDR grafting region H1 sequence, the amino acid sequence defined in SEQ ID NO 334, or an amino acid sequence derived from SEQ ID NO 334 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 334, as a CDR grafting region H2 sequence, the amino acid sequence defined in SEQ ID NO 335, or an amino acid sequence derived from SEQ ID NO 335 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 335,
as a CDR grafting region H3 sequence, the amino acid sequence defined in SEQ ID NO 336, or an amino acid sequence derived from SEQ ID NO 336 via substitution, deletion, or insertion of any one or two amino acid residues, or an amino acid sequence having a homology (preferably an identity) of 66.6 % or more or 83.3 % or more to the amino acid sequence defined in SEQ ID NO 336, or
(23) as a CDR grafting region H1 sequence, the amino acid sequence defined in SEQ ID NO 341, or an amino acid sequence derived from SEQ ID NO 341 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 341, as a CDR grafting region H2 sequence, the amino acid sequence defined in SEQ ID NO 342, or an amino acid sequence derived from SEQ ID NO 342 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 342,
as a CDR grafting region H3 sequence, the amino acid sequence defined in SEQ ID NO 343, or an amino acid sequence derived from SEQ ID NO 343 via substitution, deletion, or insertion of any one or two amino acid residues, or an amino acid sequence having a homology (preferably an identity) of 66.6 % or more or 83.3 % or more to the amino acid sequence defined in SEQ ID NO 343.

[Aspect 28] The anti-gpNMB antibody or its fragment or a derivative thereof according to Aspect 26 or 27, wherein the anti-gpNMB antibody comprises at least a heavy chain variable region comprising:

(17) the amino acid sequence defined in SEQ ID NO 189, or an amino acid sequence having a homology of 60 % or more to the amino acid sequence defined in SEQ ID NO 189, or

(18) the amino acid sequence defined in SEQ ID NO 333, or an amino acid sequence having a homology of 60 % or more to the amino acid sequence defined in SEQ ID NO 333, or

(19) the amino acid sequence defined in SEQ ID NO 340, or an amino acid sequence having a homology of 60 % or more to the amino acid sequence defined in SEQ ID NO 340, or

(20) the amino acid sequence defined in SEQ ID NO 347, or an amino acid sequence having a homology of 60 % or more to the amino acid sequence defined in SEQ ID NO 347.

[Aspect 29] The anti-gpNMB antibody or its fragment or a derivative thereof according to any one of Aspects 25 to 28, wherein the heavy chain variable region comprises, as a framework sequence, a framework sequence of a class of human immunoglobulin.

[Aspect 30] The anti-gpNMB antibody or its fragment or a derivative thereof according to any one of Aspects 25 to 29, further comprising a heavy chain constant region having an amino acid sequence of a heavy chain constant region in a class of human immunoglobulin.

[Aspect 31] The anti-gpNMB antibody or its fragment or a derivative thereof according to any one of Aspects 25 to 30, further comprising at least a light chain variable region comprising:

(17) as a CDR-L1 sequence, the amino acid sequence defined in SEQ ID NO 183, or an amino acid sequence derived from SEQ ID NO 183 via substitution, deletion, or insertion of any one or two amino acid residues, or an amino acid sequence having a homology (preferably an identity) of 66.6 % or more or 83.3 % or more to the amino acid sequence defined in SEQ ID NO 183,

as a CDR-L2 sequence, the amino acid sequence defined in SEQ ID NO 185, or an amino acid sequence derived from SEQ ID NO 185 via substitution, deletion, or insertion of any one amino acid residue, or an amino acid sequence having a homology (preferably an identity) of 66.6 % or more to the amino acid sequence defined in SEQ ID NO 185,

as a CDR-L3 sequence, the amino acid sequence defined in SEQ ID NO 187, or an amino acid sequence derived from SEQ ID NO 187 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 187, or

(18) as a CDR-L1 sequence, the amino acid sequence defined in SEQ ID NO 351, or an amino acid sequence derived from SEQ ID NO 351 via substitution, deletion, or insertion of any one or two amino acid residues, or an amino acid sequence having a homology (preferably an identity) of 66.6 % or more or 83.3 % or more to the amino acid sequence defined in SEQ ID NO 351,

as a CDR-L2 sequence, the amino acid sequence defined in SEQ ID NO 352, or an amino acid sequence derived from SEQ ID NO 352 via substitution, deletion, or insertion of any one amino acid residue, or an amino acid sequence having a homology (preferably an identity) of 66.6 % or more to the amino acid sequence defined in SEQ ID NO 352,

as a CDR-L3 sequence, the amino acid sequence defined in SEQ ID NO 353, or an amino acid sequence derived from SEQ ID NO 353 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 353, or

(19) as a CDR-L1 sequence, the amino acid sequence defined in SEQ ID NO 358, or an amino acid sequence derived from SEQ ID NO 358 via substitution, deletion, or insertion of any one or two amino acid residues, or an amino acid sequence having a homology (preferably an identity) of 66.6 % or more or 83.3 % or more to the amino acid sequence defined in SEQ ID NO 358,

as a CDR-L2 sequence, the amino acid sequence defined in SEQ ID NO 359, or an amino acid sequence derived from SEQ ID NO 359 via substitution, deletion, or insertion of any one amino acid residue, or an amino acid sequence having a homology (preferably an identity) of 66.6 % or more to the amino acid sequence defined in SEQ ID NO 359,

as a CDR-L3 sequence, the amino acid sequence defined in SEQ ID NO 360, or an amino acid sequence derived from SEQ ID NO 360 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 360, or

(20) as a CDR-L1 sequence, the amino acid sequence defined in SEQ ID NO 365, or an amino acid sequence derived from SEQ ID NO 365 via substitution, deletion, or insertion of any one or two amino acid residues, or an amino acid sequence having a homology (preferably an identity) of 66.6 % or more or 83.3 % or more to the amino acid sequence defined in SEQ ID NO 365,

as a CDR-L2 sequence, the amino acid sequence defined in SEQ ID NO 366, or an amino acid sequence derived from SEQ ID NO 366 via substitution, deletion, or insertion of any one amino acid residue, or an amino acid sequence having a homology (preferably an identity) of 66.6 % or more to the amino acid sequence defined in SEQ ID NO 366,
as a CDR-L3 sequence, the amino acid sequence defined in SEQ ID NO 367, or an amino acid sequence derived from SEQ ID NO 367 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 367, or

(21) as a CDR grafting region L1 sequence, the amino acid sequence defined in SEQ ID NO 348, or an amino acid sequence derived from SEQ ID NO 348 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 348, as a CDR grafting region L2 sequence, the amino acid sequence defined in SEQ ID NO 349, or an amino acid sequence derived from SEQ ID NO 349 via substitution, deletion, or insertion of any one or two amino acid residues, or an amino acid sequence having a homology (preferably an identity) of 71.4 % or more or 85.7 % or more to the amino acid sequence defined in SEQ ID NO 349,
as a CDR grafting region L3 sequence, the amino acid sequence defined in SEQ ID NO 350, or an amino acid sequence derived from SEQ ID NO 350 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 350, or
(22) as a CDR grafting region L1 sequence, the amino acid sequence defined in SEQ ID NO 355, or an amino acid sequence derived from SEQ ID NO 355 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 355, as a CDR grafting region L2 sequence, the amino acid sequence defined in SEQ ID NO 356, or an amino acid sequence derived from SEQ ID NO 356 via substitution, deletion, or insertion of any one or two amino acid residues, or an amino acid sequence having a homology (preferably an identity) of 71.4 % or more or 85.7 % or more to the amino acid sequence defined in SEQ ID NO 356,
as a CDR grafting region L3 sequence, the amino acid sequence defined in SEQ ID NO 357, or an amino acid sequence derived from SEQ ID NO 357 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 357, or
(23) as a CDR grafting region L1 sequence, the amino acid sequence defined in SEQ ID NO 362, or an amino acid sequence derived from SEQ ID NO 362 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 362, as a CDR grafting region L2 sequence, the amino acid sequence defined in SEQ ID NO 363, or an amino acid sequence derived from SEQ ID NO 363 via substitution, deletion, or insertion of any one or two amino acid residues, or an amino acid sequence having a homology (preferably an identity) of 71.4 % or more or 85.7 % or more to the amino acid sequence defined in SEQ ID NO 363,
as a CDR grafting region L3 sequence, the amino acid sequence defined in SEQ ID NO 364, or an amino acid sequence derived from SEQ ID NO 364 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 364.

[Aspect 32] The anti-gpNMB antibody or its fragment or a derivative thereof according to any one of Aspects 25 to 31, further comprising at least a light chain variable region comprising:

(17) the amino acid sequence defined in SEQ ID NO 191, or an amino acid sequence having a homology of 60 % or more to the amino acid sequence defined in SEQ ID NO 191, or

(18) the amino acid sequence defined in SEQ ID NO 354, or an amino acid sequence having a homology of 60 % or more to the amino acid sequence defined in SEQ ID NO 354, or

(19) the amino acid sequence defined in SEQ ID NO 361, or an amino acid sequence having a homology of 60 % or more to the amino acid sequence defined in SEQ ID NO 361, or

(20) the amino acid sequence defined in SEQ ID NO 368, or an amino acid sequence having a homology of 60 % or more to the amino acid sequence defined in SEQ ID NO 368.

[Aspect 33] The anti-gpNMB antibody or its fragment or a derivative thereof according to Aspect 31 or 32, wherein the light chain variable region comprises, as a framework sequence, a framework sequence of a class of human immunoglobulin.

[Aspect 34] The anti-gpNMB antibody or its fragment or a derivative thereof according to any one of Aspects 25 to 33, further comprising a light chain constant region having an amino acid sequence of a light chain constant region in a class of human immunoglobulin.

[Aspect 35] The anti-gpNMB antibody or its fragment or a derivative thereof according to any one of Aspects 25 to 34, which is a Fab, scFv, Diabody, Nanobody, VHH, bispecific antibody, or multispecific antibody, or a derivative thereof.

## EFFECT OF THE INVENTION

The present invention provides a new means of effectively eliminating various disease-associated cells that are gpNMB positive by utilizing a novel anti-gpNMB antibody that binds to a specific epitope on gpNMB.

## BRIEF EXPLANATION OF FIGURES

[Figure 1] Figure 1 shows an amino acid sequence alignment of human gpNMB (isotype a) and mouse gpNMB. In this figure, the region spanning from valine at amino acid residue 78 (V78) to the PKD domain is shown enclosed by a single dotted line, the region at amino acid residues 256 to 319 is shown with dotted lines as an example of the PKD domain, and the region at amino acid residues 172 to 246 is shown with solid lines as an example of the PMEL-CAF-like domain.

[Figure 2] Figure 2 is a graph showing results of evaluation of the ability of anti-gpNMB antibody clones to remove human gpNMB-expressing cells.

[Figure 3] Figure 3 is a graph showing results of evaluation of the ability of anti-gpNMB antibody clones to remove mouse gpNMB-expressing cells.

[Figure 4] Figure 4 shows the alignment of humanized mutant sequences hGPN06-1_VH1 to VH5 to mouse GPN06-1_VH.

[Figure 5] Figure 5 shows the alignment of humanized mutant sequences hGPN06-1_VL1 to VL5 to mouse GPN06-1_VL.

[Figure 6] Figure 6 shows the alignment of humanized mutant sequences hGPN18-2_VH1 to VH5 to mouse GPN18-2_VH.

[Figure 7] Figure 7 shows the alignment of humanized mutant sequences hGPN18-2_VL1 to VL5 to mouse GPN18-2_VL.

[Figure 8] Figure 8 shows the alignment of humanized anti-gpNMB antibody variant hGPN18-5_VH1 to VH3 to mouse GPN18-5_VH0.

[Figure 9] Figure 9 shows the alignment of humanized anti-gpNMB antibody variant hGPN18-5_VL1 to VL3 to mouse GPN18-5_VL0.

[Figure 10] Figure 10 shows results of evaluation of pain amelioration by anti-gpNMB antibody clones using neuropathic pain model mice.

[Figure 11] Figure 11 shows a schematic diagram of a administration schedule of anti-gpNMB antibody clones to obese model mice.

[Figure 12] Figure 12 is a graph showing results of evaluation of the amount of gpNMB-positive SA-$\beta$-Gal-positive macrophages in epididymal fat of anti-gpNMB antibody clones on obese model mice.

[Figure 13] Figure 13 is a graph showing results of evaluation of TNF-$\alpha$ gene expression in epididymal fat of anti-gpNMB antibody clones using obese model mice.

## EMBODIMENTS

The present invention will now be described based on specific embodiments. These embodiments should not be construed to limit the scope of the present invention. All references, including patent publications, unexamined patent publications, and non-patent publications cited in this specification, can be incorporated by reference in their entirety for all purposes.

[Overview]

Various attempts have been made to develop means to eliminate various cells that induce or accelerate the progression of various diseases (referred to as "disease-associated cells"). Examples include senescent cell-eliminating drugs targeting senescent cells, anticancer drugs targeting specific cancer cells (cancer-targeted drugs), anticancer drugs targeting removal of cancer-infiltrating macrophages, and drugs targeting nonspecific macrophages and microglia.

However, reports on methods to eliminate such disease-involved cells have been extremely limited so far. In particular, the development of a method that can specifically remove highly malignant disease-involved cells, which are the root cause of disease induction, has not been achieved to date. Examples of such highly malignant disease-associated cells include senescent infiltrating cells or senescent resident cells, which significantly affect surrounding cells and tissues to form or progress disease states, or cancer cells and cancer stem cells that are extremely malignant in nature.

As a result of intensive study to develop a means to efficiently remove disease-associated cells, the present inventors focused on gpNMB (glycoprotein nonmetastatic melanoma protein B) as an antigen common to cells involved in various diseases with high malignant potential. Since cells that highly express gpNMB (gpNMB-positive cells) are observed in various diseases, they may play a significant role in the pathogenesis and progression of these diseases. Examples of disease-associated cells that can highly express such gpNMBs include senescent cells, cancer-related cells (e.g., cancer cells, cancer progenitor cells, cancer-infiltrating cells (e.g., cancer-infiltrating immune cells), etc.), phagocytic cells such as tissue-infiltrating phagocytes and chronic or abnormally activated (resident) phagocytes (e.g., macrophages, microglia etc.) such as tissue-infiltrating phagocytes and chronic or abnormally activated (resident) phagocytes.

On the other hand, while gpNMBs have been suggested to be involved in various diseases, it was unclear whether they are protective or aggravating for various diseases and whether they are appropriate therapeutic targets. In other words, soluble gpNMBs secreted from various cells were also expected to play a protective role in various pathological conditions. Therefore, the present inventors hypothesized that eliminating only the disease-associated cells that chronically overexpress gpNMB, without adversely affecting these protective (expected) gpNMB-secreting cells, would have a therapeutic effect on the disease.

To prove the hypothesis, it is necessary first to devise a method to remove gpNMB-overexpressing cells. The present inventors chose an antibody as the most specific means of removing gpNMB-overexpressing cells. In obtaining an anti-gpNMB antibody, the present inventors aimed to obtain an antibody with mouse-human cross-reactivity that could be tested for efficacy in animal models and also used in human clinical trials without modification.

To date, no anti-gpNMB antibodies have been obtained that are cross-reactive in mice and humans. The only anti-gpNMB antibody that has progressed to clinical trials, glembatumumab (Patent Literature 2), was a human antibody specific for human gpNMB for delivery of anticancer drugs to cancer cells. As mentioned earlier, an antibody-drug conjugate (ADC) called glembatumumab vedotin, which is based on glembatumumab, advanced to phase II trials as a cancer drug candidate, but its development was later discontinued.

In addition, if the removal activity of T cells or NK cells is used as a means to remove such disease-associated cells, inflammation may be induced. Therefore, the present inventors aimed to obtain antibodies that can induce removal activity by phagocytic cells such as macrophages, rather than by T cells or NK cells.

Under such various restrictions, the present inventors made full use of various experimental means and methods, and have succeeded in obtaining anti-gpNMB antibodies with such a level of mouse-human cross-reactivity as to show efficacy in vivo. Analysis of the epitopes of the anti-gpNMB antibodies confirmed that they are novel antibodies that recognize epitopes different from those of conventional vaccine peptides that have been reported to eliminate cells (Patent Literature 1, Non-Patent Literature 67).

In addition, the present inventors have selected several antibodies recognizing representative epitopes from the obtained anti-gpNMB antibodies, and constructed a system to evaluate their removal activity by phagocytic cells. Specifically, the present inventors have evaluated the removal effect of gpNMB-expressing cells by macrophage-derived cell lines and selected antibodies with high removal activity. As a result, several antibodies recognizing epitope regions different from those of conventional vaccine epitopes were found to have the ability to remove gpNMB -expressing cells, leading to the present invention.

The anti-gpNMB antibodies of the present invention can effectively remove gpNMB-positive cells involved in various disease progressions without inducing inflammation through the removal action by phagocytic cells. This makes it possible to provide effective treatment for various diseases that have been difficult to treat.

The following description will be made first on the details of the anti-gpNMB antibodies used to eliminate disease-associated cells in the present invention (the anti-gpNMB antibodies of the present invention) and their production method. Then, pharmaceutical compositions (the pharmaceutical compositions of the present invention) and treatment methods (the treatment methods of the present invention) for eliminating disease-associated cells using such antibodies shall be described.

Where some of the anti-gpNMB antibodies of the present invention described below have not been disclosed or suggested in the previous literature and are patentable, these novel anti-gpNMB antibodies themselves shall also be included in the scope of the present invention.

[Anti-gpNMB antibody]

An embodiment of the present invention relates to an anti-gpNMB antibody, preferably an anti-gpNMB antibody that binds specifically to at least one site in a region ranging from V78 through a PMEL-CAF-like (PMEL core amyloid fragment-like) domain to a PKD domain of human gpNMB (glycoprotein nonmetastatic melanoma protein B) (hereinafter also referred to as "the anti-gpNMB antibody of the present invention" or "the antibody of the present invention"). The statement "at least one site in a region from V78 to a PKD domain of gpNMB" herein refers to at least one site in a region with, e.g., the amino acid residues at positions 78 to 319 in human gpNMB having the amino acid sequence defined in SEQ ID NO 209.

*Antibody:

The term "antibody" herein refers to a glycoprotein containing at least two heavy (H) chains and two light (L) chains interconnected through disulfide bonds. Each heavy chain contains a heavy chain variable region (abbreviated as VH) and a heavy chain constant region, and the heavy chain constant region contains three domains, CH1, CH2 and CH3. Each light chain contains a light chain variable region (abbreviated as VL) and a light chain constant region. The light chain constant region contains one domain, CL. The light chain constant region has two chains called a λ chain and a κ chain. The heavy chain constant region has a γ chain, a μ chain, an α chain, a δ chain, and an ε chain, and has isotypes of antibodies called IgG, IgM, IgA, IgD and IgE, respectively, depending on the difference in the heavy chain. The VH and VL regions are further divided into four regions (FR-1, FR-2, FR-3, FR-4) having higher preservability called framework regions (FR) and three regions (CDR-1, CDR-2, CDR-3) called complementarity determining regions (CDR). The VH region comprises three CDRs and four FRs, in detail, a sequence of FR-1, CDR-1 (CDR-H1), FR-2, CDR-2 (CDR-H2), FR-3, CDR-3 (CDR-H3), and FR4 from the amino terminal to the carboxy terminal. The VL region comprises three CDRs and four FRs, in detail, a sequence of FR-1, CDR-1 (CDR-L1), FR-2, CDR-2 (CDR-L2), FR-3, CDR-3 (CDR-L3), and FR4 from the amino terminal to the carboxy terminal. The variable regions of the heavy and light chains contain binding domains that interact with an antigen.

The antibody of the present invention may be a fragment (e.g., an antigen-binding fragment) and/or a derivative of an antibody, as long as it has the activity to bind specifically to at least one site in a region from V78 to a PKD domain of gpNMB. Examples of fragments of antibodies include F (ab') 2, Fab, and Fv. Examples of derivatives of antibodies include an antibody in which an amino acid mutation is artificially incorporated in the constant region, an antibody in which the domain configuration of the constant region is modified, an antibody in the form having two or more Fc fragments per molecule, an antibody composed of only a heavy chain or only a light chain, a sugar chain modified antibody, a bispecific antibody, an antibody conjugate combined with an antibody or a fragment compound of an antibody or a protein other than antibodies, an antibody enzyme, a nanobody, a tandem scFv fragment, a bispecific tandem scFv fragment, a diabody, and a VHH body. In the present invention, the term "antibody" simply includes a fragment and/or a derivative of an antibody unless otherwise specified.

The antibody of the present invention may be either a monoclonal antibody or a polyclonal antibody, but may preferably be a monoclonal antibody. The term monoclonal antibody classically refers to an antibody molecule obtained from a clone derived from a single antibody producing cell, and is a single type of antibody molecule containing a combination of VH and VL each consisting of a specific amino acid sequence. It is possible to obtain from a monoclonal antibody a nucleic acid molecule having a gene sequence encoding the amino acids of each of the proteins constituting the antibody, and it is also possible to produce the antibody using such nucleic acid molecules using genetic engineering methods. It is well known to a person skilled in the art that genetic information on the sequences of, e.g., the H and L chains, their variable regions, and their CDRs can be used to modify the antibody to improve its binding and specificity, etc., and that antibodies from animals such as mice can be modified into human-type antibodies to produce antibodies with a structure suitable for use as therapeutic agents. A human monoclonal antibody can be also produced with a transgenic animal into which a human antibody gene has been introduced to be sensitized with an antigen. Other methods that do not require sensitization of animals include the use of phage libraries expressing antigen-binding regions of human antibodies or portions thereof (human antibody phage display) to obtain antibodies that bind specifically to the corresponding antigens or phage clones consisting of specific amino acid sequences, and those skilled in the art can use such information to produce human antibodies as appropriate (see, e.g., the review by Taketo Tanaka et al., in Keio J. Med., (2011), 60:37-46). Those skilled in the art can also design antibodies to be administered to non-human animals using the amino acid sequence information of CDRs and variable regions as appropriate, using a technique similar to the humanization technique.

The specificity of an antibody herein means that the antibody exhibits a high antigen-antibody reaction to a specific antigen. Those skilled in the art can determine the antigen-antibody reaction by appropriately selecting a binding measurement method in a solid phase or liquid phase system. Examples of such methods include, although not limited to, enzyme-linked immunosorbent assay (ELISA), enzyme immunoassay (EIA), surface plasmon resonance (SPR), fluorescence resonance energy transfer (FRET), and luminescence resonance energy transfer (LRET). Before the binding between an antigen and an antibody is measured, the antibody and/or the antigen may be labeled with, e.g., an enzyme, a fluorescent substance, a luminescent substance, or a radioisotope, and then the antigen-antibody reaction may be detected by a method suitable for the physical and/or chemical properties of the labeled substance.

*Specific binding to at least one site in a region from V78 to a PKD domain of gpNMB:

The antibody of the present invention specifically binds to at least one site in a region from valine at position 78 (V78) to a PKD domain of human gpNMB. The region includes a region spanning from valine at position 78 (V78) to serine at position 92 (S92), a PMEL-CAF-like domain, and the PKD domain, as well as the intervening regions between these regions. The term "gpNMB" (glycoprotein nonmetastatic melanoma protein B; also referred to as Osteoactivin or DC-HIL) herein refers to the single transmembrane glycoprotein mentioned above. For more details, see each of the documents mentioned above (especially Non-Patent Literature 20).

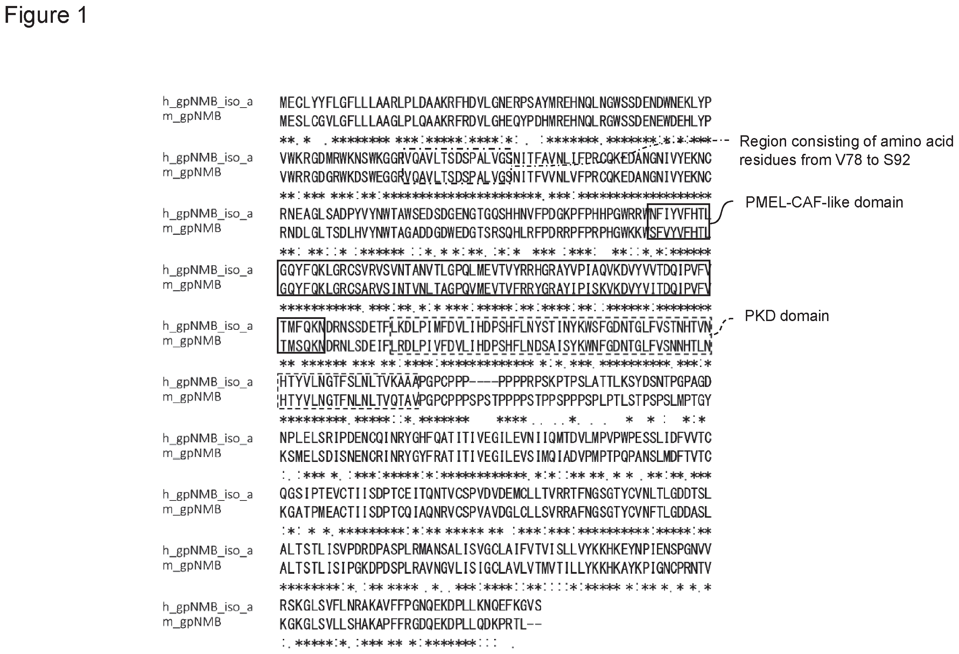

The amino acid sequence of the total length protein of human gpNMB (isotype a) is shown in SEQ ID NO 209, and the nucleic acid sequence of a gene encoding the protein is shown in SEQ ID NO 210. The amino acid sequence of the total length protein of mouse gpNMB is shown in SEQ ID NO 211, and the nucleic acid sequence of a gene encoding the protein is shown in SEQ ID NO 212. The amino acid sequence of the total length protein of rat gpNMB is shown in SEQ ID NO 213, and the nucleic acid sequence of a gene encoding the protein is shown in SEQ ID NO 214. The alignment of the amino acid sequences of human gpNMB (isotype a) and mouse gpNMB is shown in Figure 1.

The region from V78 to S92 of gpNMB is a region including 7 amino acid residues before and after aspartic acid at position 85 (D85) of gpNMB. In Figure 1, the region from V78 to S92 of each of human gpNMB and mouse gpNMB is indicated with a single dotted line.

The PKD (Polycystic Kidney Disease homology) domain of gpNMB is a domain containing a beta sheet consisting of three strands and a beta sheet consisting of four strands folded together to form a beta sandwich structure. It was named the PKD domain because it is a characteristic structure possessed by polycystin-1 (polycystin-1), the gene responsible for polycystic kidney disease (Non-Patent Literature 26). According to an embodiment, in the case of the human gpNMB defined in SEQ ID NO 209 and the mouse gpNMB defined in SEQ ID NO 211, the region containing the amino acid residues at positions 256 to 319 corresponds to the PKD domain. According to an embodiment, the PKD domain may preferably be a region containing the amino acid residues at positions 256 to 316, more preferably a region containing positions 257 to 316, still more preferably a region containing 268 to 316, even more preferably a region containing 270 to 316, particularly preferably a region containing 282 to 316, especially preferably a region containing 287 to 316. According to an embodiment, the PKD domain may preferably be a region containing the amino acid residues at positions 257 to 319, more preferably a region containing positions 257 to 316, still more preferably a region containing positions 257 to 301. According to an embodiment, the PKD domain may preferably be a region containing the amino acid residues at positions 287 to 301. The region at amino acid residues 256 to 319 surrounded by a solid line in Figure 1 is an example of the PDK domain of each of human gpNMB and mouse gpNMB.

The PMEL-CAF-like (PMEL Core Amyloid Fragment-like) domain of gpNMB is a region that is homologous to a CAF region in PMEL of a protein of the same family as described above, which is a region that induces the formation of amyloid -like aggregates in a PMEL protein (Non-Patent Literature 80). The PMEL-CAF-like domain of gpNMB is considered to prevent aggregation formation by having many N-type glycan binding sites in the neighboring PKD region (Non-Patent Literature 90). According to an embodiment, in the case of the human gpNMB defined in SEQ ID NO 209 and the mouse gpNMB defined in SEQ ID NO 211, the region containing the amino acid residues at positions 172 to 246 corresponds to the PMEL-CAF-like domain. The PMEL-CAF-like domain may preferably be a region containing the amino acid residues at positions178 to 228, more preferably a region containing the amino acid residues at positions186 to 218. The region at amino acid residues 172 to 246 surrounded by a solid line in Figure 1 is an example of the PMEL-CAF-like domain of each of human gpNMB and mouse gpNMB.

As mentioned above, the term "region from V78 to a PKD domain of gpNMB" herein refers to a region containing not only the region spanning from V78 to S92, the PMEL-CAF-like domain and the PKD domain defined above, but also a region intervening between the region spanning from V78 to S92 and the PMEL-CAF-like domain and a region intervening between the PMEL-CAF-like domain and the PKD domain. According to an embodiment, in the case of the human gpNMB defined in SEQ ID NO 209 and the mouse gpNMB defined in SEQ ID NO 211, the region containing the amino acid residues at positions 78 to 319 corresponds to a region from V78 to a PKD domain.

The antibody of the present invention may have the ability to specifically bind to at least one site in a region from V78 to a PKD domain of gpNMB of any vertebrate. However, in view of human pharmaceutical uses, the antibody of the present invention may preferably have the ability to specifically bind to at least one site in a region from V78 to a PKD domain of at least the human gpNMB defined in SEQ ID NO 209. From the viewpoint of evaluating the effect on endogenous gpNMB in disease model mice and disease model rats, the antibody of the present invention may more preferably have the ability to specifically bind to at least one site in a region from V78 to the PKD domain of the mouse gpNMB defined in SEQ ID NO 211, in addition to at least one site in a region from a PMEL-CAF-like domain to a PKD domain of human gpNMB. The statement that an antibody "specifically" binds to gpNMB herein refers to the binding ability of the antibody to gpNMB with a binding strength of 10-fold or more, preferably 100-fold or more, more preferably 1000-fold or more, compared to the binding ability of the antibody to proteins other than gpNMB (e.g. albumin). In addition, the "region from V78 to the PKD domain" herein refers to a region including the region spanning from V78 to S92, the PMEL-CAF-like domain, the PKD domain, the region intervening between the region spanning from V78 to S92 and the PMEL-CAF-like domain, and the region intervening between the PMEL-CAF-like domain and the PKD domain.

According to another embodiment, it is preferred that the antibodies of the present invention may preferably bind specifically to at least one site in the region from V78 to S92 of human gpNMB shown in SEQ ID NO 209, i.e., the region containing 7 amino acid residues before and after D85. It may more preferably bind to at least one site in the region containing four amino acid residues before and after D85, even more preferably to at least one site in the region containing two amino acid residues before and after D85, especially preferably to at least D85.

When the antibody of the present invention specifically binds to at least one site in a region from V78 to a PKD domain of human gpNMB, the epitope in the region to which the antibody binds is not particularly limited. However, according to an embodiment, the antibody of the present invention may preferably have the ability to specifically bind to at least one site or more in a region from the PMEL-CAF-like domain to the PKD domain, and may more preferably have the specifically ability to bind to one or more sites selected from a site containing amino acid residue(s) K257 and/or D258 of the PKD domain, a site containing amino acid residue(s) H268 and/or D269 of the PKD domain, a site containing an amino acid residue K282 of the PKD domain, a site containing amino acid residue(s) D287 and/or H301 of the PKD domain, a site containing an amino acid residue K316 of the PKD domain, a site containing an amino acid residue K186 of the PMEL-CAF-like domain, a site containing amino acid residue(s) R214 and/or R215 of the PMEL-CAF-like domain, and a site containing amino acid residue(s) H216 and/or R218 of the PMEL-CAF-like domain of the human gpNMB defined in SEQ ID NO 209. Among them, the antibody of the present invention may preferably have the ability to specifically bind to at least either or both of a site containing amino acid residue(s) D287 and/or H301 of the PKD domain and a site containing amino acid residue(s) R214 and/or R215 of the PMEL-CAF-like domain of the human gpNMB defined in SEQ ID NO 209, and in addition to these sites, it may more preferably have the ability to also specifically bind to one or more sites selected from a site containing amino acid residue(s) K257 and/or D258, a site containing amino acid residue(s) H268 and/or D269, a site containing an amino acid residue K282, a site containing an amino acid residue K316, K186 a region containing an amino acid residue, and a site containing amino acid residue(s) H216 and/or R218 of human gpNMB.

The epitope of human gpNMB to which the antibody of the present invention binds may be a linear epitope, which consists of a contiguous region in the primary structure of human gpNMB (amino acid sequence shown in SEQ ID NO 209), as long as the epitope includes at least one region from V78 to the PKD domain. Alternatively, it may be a steric epitope, which is formed in the steric structure by two or more discrete subregions in the primary structure. Each of the region forming the linear epitope and the subregions forming the steric epitope may either be located in any one, or span two or more, of the region spanning from V78 to S92, the PMEL-CAF-like domain, the PKD domain, the region intervening between the region spanning from V78 to S92 and the PMEL-CAF-like domain, and the region intervening between the PMEL-CAF-like domain and the PKD domain. It may be present only within the region from V78 to the PKD domain, or it may span other regions as well. In addition, in the case of the steric epitope, as long as a portion or the entirety of at least one of the two or more partial regions forming the steric epitope is located in the region from V78 to the PKD domain, the other partial regions may be present outside the region from V78 to the PKD domain.

The binding strength of the antibody of the present invention to the region from V78 to the PKD domain of gpNMB is not particularly limited. However, as measured by antigen ELISA using recombinant gpNMB and/or cell ELISA using gpNMB-expressing cells, the antibody of the present invention may preferably have a 50% effective concentration ($EC_{50}$) to the region from V78 to the PKD domain of gpNMB of typically $1 \times 10^{-7}$M or lower, or $1 \times 10^{-8}$M or lower, or $1 \times 10^{-9}$M or lower. The "50% effective concentration" ($EC_{50}$) of an antibody herein refers to the concentration of the antibody that exhibits 50% of the maximum binding affinity with which that antibody binds to the antigen. Specific conditions for measuring $EC_{50}$ by antigen ELISA using recombinant gpNMBs and/or cell ELISA using gpNMB-expressing cells include those employed in the Examples described below.

The antibody of the present invention may also bind to one or more sites in any region other than the region from V78 to the PKD domain of gpNMB. Examples of amino acid sites to which the antibody of the present invention may bind other than the region from V78 to the PKD domain of gpNMB may preferably include, although not limited to, E385 of the human gpNMB defined in SEQ ID NO 209.

*Amino acid sequence and nucleic acid sequence of each region of each chain of the antibody:

The amino acid sequence of the antibody of the present invention is not particularly limited, as long as it has the ability to specifically bind to at least one site in a region from V78 to a PKD domain of gpNMB. However, the antibody of the present invention may preferably have a specific amino acid sequence as each CDR sequence. The details will be explained below. Incidentally, the "identity" of amino acid sequences herein refers to the ratio of matching amino acid residues, and the "homology" of amino acid sequences herein refers to the ratio of matching or similar amino acid residues. The homology and identity of amino acid sequences can be determined by, e.g., BLAST method (default conditions of NCBI's PBLAST). It is also clear that when the phrase "a homology of 80% or more" is used, for example, it includes "an identity of 80% or more."

The term "similar amino acid residues" herein refers to amino acid residues having side chains with similar chemical characteristics (e.g., charge or hydrophobicity). Similar amino acid residues include, for example, the following combinations.

(1) Amino acid residues with aliphatic side chains: glycine (Gly or G), alanine (Ala or A), valine (Val or V), leucine (Leu or L), and isoleucine (Ile or I) residue.
(2) Amino acid residues with aliphatic hydroxyl side chains: serine (Ser or S) and threonine (Thr or T) residue.
(3) Amino acid residues with amide-containing side chains: asparagine (Asn or N) and glutamine (Gln or Q) residue.
(4) Amino acid residues with aromatic side chains: phenylalanine (Phe or F), tyrosine (Tyr or Y), and tryptophan (Trp or W) residue.
(5) Amino acid residues with basic side chains: lysine (Lys or K), arginine (Arg or R) and histidine (His or H) residue.
(6) Amino acid residues with acidic side chains: aspartic acid (Asp or D) and glutamic acid (Glu or E) residue.
(7) Amino acid residues with sulfur-containing side chains: cysteine (Cys or C), and methionine (Met or M) residue.

In addition, the combination of (1) and methionine (Met or M) and the combination of (4) and histidine (His or H) are also treated as similar amino acid residues.

In some cases, substitutions can be made regardless of similarity if the corresponding variants exist with reference to the germline sequence. Germline-referenced variants include substitutions, deletions, or additions. Substitutions include cases where amino acid residues after substitution are presumed to affect the direction of the side chain such as proline (Pro or P) and glycine (Gly or G).

An embodiment of the antibody of the present invention relates to antibodies an antibody containing a heavy chain variable region having any of the combination of the CDR-H1 to -H3 sequences specified in (1) to (14) below. The term "CDR grafting region" as used herein refers to a region of the amino acid sequence of an antibody having an amino acid sequence identical or overlapping with the amino acid sequence of a CDR as defined herein, and which, like the CDR, comes into contact with the antigen to determine antigen specificity and is important for binding to the antigen.

(1) As a CDR-H1 sequence, the amino acid sequence defined in SEQ ID NO 1, or an amino acid sequence derived from SEQ ID NO 1 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 1,

as a CDR-H2 sequence, the amino acid sequence defined in SEQ ID NO 3, or an amino acid sequence derived from SEQ ID NO 3 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 3, and
as a CDR-H3 sequence, the amino acid sequence defined in SEQ ID NO 5, or an amino acid sequence derived from SEQ ID NO 5 via substitution, deletion, or insertion of any one, two, three, or four amino acid residues, or an amino acid sequence having a homology (preferably an identity) of 66.6 % or more, 75.0 % or more, 83.3 % or more, or 91.6 % or more to the amino acid sequence defined in SEQ ID NO 5.

(2) As a CDR-H1 sequence, the amino acid sequence defined in SEQ ID NO 17, or an amino acid sequence derived from SEQ ID NO 17 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 17,

as a CDR-H2 sequence, the amino acid sequence defined in SEQ ID NO 19, or an amino acid sequence derived from SEQ ID NO 19 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 19, and

as a CDR-H3 sequence, the amino acid sequence defined in SEQ ID NO 21, or an amino acid sequence derived from SEQ ID NO 21 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 21.

(3) As a CDR-H1 sequence, the amino acid sequence defined in SEQ ID NO 33, or an amino acid sequence derived from SEQ ID NO 33 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 33,

as a CDR-H2 sequence, the amino acid sequence defined in SEQ ID NO 35, or an amino acid sequence derived from SEQ ID NO 35 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 35, and

as a CDR-H3 sequence, the amino acid sequence defined in SEQ ID NO 37, or an amino acid sequence derived from SEQ ID NO 37 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 37.

(4) As a CDR-H1 sequence, the amino acid sequence defined in SEQ ID NO 49, or an amino acid sequence derived from SEQ ID NO 49 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 49,

as a CDR-H2 sequence, the amino acid sequence defined in SEQ ID NO 51, or an amino acid sequence derived from SEQ ID NO 51 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 51, and

as a CDR-H3 sequence, the amino acid sequence defined in SEQ ID NO 53, or an amino acid sequence derived from SEQ ID NO 53 via substitution, deletion, or insertion of any one, two, three, four, five, or six amino acid residues, or an amino acid sequence having a homology (preferably an identity) of 62.5 % or more, 68.7 % or more, 75.0 % or more, 81.2 % or more, 87.5 % or more, or 93.7 % or more to the amino acid sequence defined in SEQ ID NO 53.

(5) As a CDR-H1 sequence, the amino acid sequence defined in SEQ ID NO 65, or an amino acid sequence derived from SEQ ID NO 65 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 65,

as a CDR-H2 sequence, the amino acid sequence defined in SEQ ID NO 67, or an amino acid sequence derived from SEQ ID NO 67 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 67, and

as a CDR-H3 sequence, the amino acid sequence defined in SEQ ID NO 69, or an amino acid sequence derived from SEQ ID NO 69 via substitution, deletion, or insertion of any one, two, three, four, or five amino acid residues, or an amino acid sequence having a homology (preferably an identity) of 64.2 % or more, 71.4 % or more, 78.5 % or more, 85.7 % or more, or 92.8 % or more to the amino acid sequence defined in SEQ ID NO 69.

(6) As a CDR-H1 sequence, the amino acid sequence defined in SEQ ID NO 81, or an amino acid sequence derived from SEQ ID NO 81 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 81,

as a CDR-H2 sequence, the amino acid sequence defined in SEQ ID NO 83, or an amino acid sequence derived from SEQ ID NO 83 via substitution, deletion, or insertion of any one or two amino acid residues, or an amino acid sequence having a homology (preferably an identity) of 71.4 % or more or 85.7 % or more to the amino acid sequence defined in SEQ ID NO 83, and
as a CDR-H3 sequence, the amino acid sequence defined in SEQ ID NO 85, or an amino acid sequence derived from SEQ ID NO 85 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 85.

(7) As a CDR-H1 sequence, the amino acid sequence defined in SEQ ID NO 97, or an amino acid sequence derived from SEQ ID NO 97 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 97,

as a CDR-H2 sequence, the amino acid sequence defined in SEQ ID NO 99, or an amino acid sequence derived from SEQ ID NO 99 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 99, and
as a CDR-H3 sequence, the amino acid sequence defined in SEQ ID NO 101, or an amino acid sequence derived from SEQ ID NO 101 via substitution, deletion, or insertion of any one or two amino acid residues, or an amino acid sequence having a homology (preferably an identity) of 60.0 % or more or 80.0 % or more to the amino acid sequence defined in SEQ ID NO 101.

(8) As a CDR-H1 sequence, the amino acid sequence defined in SEQ ID NO 113, or an amino acid sequence derived from SEQ ID NO 113 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 113,

as a CDR-H2 sequence, the amino acid sequence defined in SEQ ID NO 115, or an amino acid sequence derived from SEQ ID NO 115 via substitution, deletion, or insertion of any one or two amino acid residues, or an amino acid sequence having a homology (preferably an identity) of 71.4 % or more or 85.7 % or more to the amino acid sequence defined in SEQ ID NO 115, and
as a CDR-H3 sequence, the amino acid sequence defined in SEQ ID NO 117, or an amino acid sequence derived from SEQ ID NO 117 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 117 .

(9) As a CDR-H1 sequence, the amino acid sequence defined in SEQ ID NO 129, or an amino acid sequence derived from SEQ ID NO 129 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 129,

as a CDR-H2 sequence, the amino acid sequence defined in SEQ ID NO 131, or an amino acid sequence derived from SEQ ID NO 131 via substitution, deletion, or insertion of any one or two amino acid residues, or an amino acid sequence having a homology (preferably an identity) of 71.4 % or more or 85.7 % or more to the amino acid sequence defined in SEQ ID NO 131, and
as a CDR-H3 sequence, the amino acid sequence defined in SEQ ID NO 133, or an amino acid sequence derived from SEQ ID NO 133 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 133.

(10) As a CDR-H1 sequence, the amino acid sequence defined in SEQ ID NO 145, or an amino acid sequence derived from SEQ ID NO 145 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 145,

as a CDR-H2 sequence, the amino acid sequence defined in SEQ ID NO 147, or an amino acid sequence derived from SEQ ID NO 147 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 147, and
as a CDR-H3 sequence, the amino acid sequence defined in SEQ ID NO 149, or an amino acid sequence derived from SEQ ID NO 149 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 149.

(11) As a CDR-H1 sequence, the amino acid sequence defined in SEQ ID NO 161, or an amino acid sequence derived from SEQ ID NO 161 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 161,

as a CDR-H2 sequence, the amino acid sequence defined in SEQ ID NO 163, or an amino acid sequence derived from SEQ ID NO 163 via substitution, deletion, or insertion of any one or two amino acid residues, or an amino acid sequence having a homology (preferably an identity) of 71.4 % or more or 85.7 % or more to the amino acid sequence defined in SEQ ID NO 163, and
as a CDR-H3 sequence, the amino acid sequence defined in SEQ ID NO 165, or an amino acid sequence derived from SEQ ID NO 165 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 165.

(12) As a CDR-H1 sequence, the amino acid sequence defined in SEQ ID NO 255, or an amino acid sequence derived from SEQ ID NO 255 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 255,

as a CDR-H2 sequence, the amino acid sequence defined in SEQ ID NO 257, or an amino acid sequence derived from SEQ ID NO 257 via substitution, deletion, or insertion of any one or two amino acid residues, or an amino acid sequence having a homology (preferably an identity) of 71.4 % or more or 85.7 % or more to the amino acid sequence defined in SEQ ID NO 257, and
as a CDR-H3 sequence, the amino acid sequence defined in SEQ ID NO 259, or an amino acid sequence derived from SEQ ID NO 259 via substitution, deletion, or insertion of any one or two amino acid residues, or an amino acid sequence having a homology (preferably an identity) of 66.6 % or more or 83.3 % or more to the amino acid sequence defined in SEQ ID NO 259.

(13) As a CDR-H1 sequence, the amino acid sequence defined in SEQ ID NO 283, or an amino acid sequence derived from SEQ ID NO 283 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 283,

as a CDR-H2 sequence, the amino acid sequence defined in SEQ ID NO 285, or an amino acid sequence derived from SEQ ID NO 285 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 285,
as a CDR-H3 sequence, the amino acid sequence defined in SEQ ID NO 287, or an amino acid sequence derived from SEQ ID NO 287 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 287.

(14) As a CDR-H1 sequence, the amino acid sequence defined in SEQ ID NO 311, or an amino acid sequence derived from SEQ ID NO 311 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 311,

as a CDR-H2 sequence, the amino acid sequence defined in SEQ ID NO 313, or an amino acid sequence derived from SEQ ID NO 313 via substitution, deletion, or insertion of any one or two amino acid residues, or an amino acid sequence having a homology (preferably an identity) of 71.4 % or more or 85.7 % or more to the amino acid sequence defined in SEQ ID NO 313,

as a CDR-H3 sequence, the amino acid sequence defined in SEQ ID NO 315, or an amino acid sequence derived from SEQ ID NO 315 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 315.

As shown in Example 11 below, the present inventors investigated the importance of each amino acid residue of each CDR grafting region sequence of humanized anti-gpNMB antibody H1L1 (clone hGPN06-1_H1L1) in maintaining its binding ability by alanine scanning. The results showed that in the heavy chain variable region of the humanized anti-gpNMB antibody hGPN06-1_H1L1 (SEQ ID NO 295), the following amino acid residues were identified as important for maintaining binding: in CDR grafting region-H1 (SEQ ID NO 271), tyrosine at position 27, asparagine at position 32, and tryptophan at position 33; in CDR grafting region-H2 (SEQ ID NO 273), aspartic acid at position 55, phenylalanine at position 57, threonine at position 58, asparagine at position 59, tyrosine at position 60, and asparagine at position 61; and in CDR grafting region-H3 (SEQ ID NO 275), arginine at position 98, glycine at position 100, and glycine at position 109. On the other hand, the amino acid residues of each CDR grafting region other than those listed above were identified as having little effect on the maintenance of the antibody's binding ability. Based on these findings, one embodiment of the antibody of the present invention relates to an antibody containing a heavy chain variable region with the CDR grafting regions-H1 to -H3 sequences specified in (15) below (the amino acid positions specified below are indicated with respect to the amino acid sequence of the heavy chain variable region of humanized anti-gpNMB antibody hGPN06-1_H1L1 (SEQ ID NO 295)).

(15) As a CDR grafting region H1 sequence, the amino acid sequence defined in SEQ ID NO 271, or an amino acid sequence derived from SEQ ID NO 271 via substitution, deletion, or insertion of any one, two, three, or four amino acid residues other than tyrosine at position 27, asparagine at position 32, and tryptophan at position 33,

as a CDR grafting region H2 sequence, the amino acid sequence defined in SEQ ID NO 273, or an amino acid sequence derived from SEQ ID NO 273 via substitution, deletion, or insertion of any one, two, three, four, five, or six amino acid residues other than aspartic acid at position 55, phenylalanine at position 57, threonine at position 58, asparagine at position 59, tyrosine at position 60, and asparagine at position 61, and

as a CDR grafting region H3 sequence, the amino acid sequence defined in SEQ ID NO 275, or an amino acid sequence derived from SEQ ID NO 275 via substitution, deletion, or insertion of any one, two, three, four, five, or six amino acid residues other than arginine at position 98, glycine at position 100, and glycine at position 109.

As shown in Example 15 below, the present inventors investigated the importance of each amino acid residue of each CDR grafting region sequence of humanized anti-gpNMB antibody H2L3 (clone hGPN18-2_H2L3) in maintaining its binding ability by alanine scanning. The results showed that in the heavy chain variable region of the humanized anti-gpNMB antibody hGPN18-2_H2L3 (SEQ ID NO 323), the following amino acid residues were identified as important for maintaining binding: in CDR grafting region-H2 (SEQ ID NO 301), aspartic acid at position 52, tryptophan at position 54, aspartic acid at position 58, and proline at position 63; and in CDR grafting region-H3 (SEQ ID NO 303), arginine at position 99, threonine at position 100, tyrosine at position 102, tyrosine at position 105, tyrosine at position 107, and methionine at position 110. On the other hand, the amino acid residues of each CDR grafting region other than those listed above were identified as having little effect on the maintenance of the antibody's binding ability. Based on these findings, one embodiment of the antibody of the present invention relates to an antibody containing a heavy chain variable region with the CDR grafting regions-H1 to -H3 sequences specified in (16) below (the amino acid positions specified below are indicated with respect to the amino acid sequence of the heavy chain variable region of humanized anti-gpNMB antibody hGPN18-2_H2L3 (SEQ ID NO 323)).

(16) As a CDR grafting region H1 sequence, the amino acid sequence defined in SEQ ID NO 299, or an amino acid sequence derived from SEQ ID NO 299 via substitution, deletion, or insertion of any one, two, three, or four amino acid residues,

as a CDR grafting region H2 sequence, the amino acid sequence defined in SEQ ID NO 301, or an amino acid sequence derived from SEQ ID NO 301 via substitution, deletion, or insertion of any one, two, three, four, five, or six amino acid residues other than aspartic acid at position 52, tryptophan at position 54, aspartic acid at position 58, and proline at position 63,

as a CDR grafting region H3 sequence, the amino acid sequence defined in SEQ ID NO 303, or an amino acid sequence derived from SEQ ID NO 303 via substitution, deletion, or insertion of any one, two, three, four, five, or six amino acid residues other than arginine at position 99, threonine at position 100, tyrosine at position 102, tyrosine at position 105, tyrosine at position 107, and methionine at position 110.

An embodiment of the antibody of the present invention is an antibody containing a heavy chain variable region having the combination of CDR-H1 to -H3 sequences selected from (17) to (23) below. Antibodies containing a heavy chain variable region having the combination of CDR-H1 to -H3 sequences selected from (17) to (23) below have neither been disclosed nor suggested in the previous literature, and correspond to novel anti-gpNMB antibodies.

(17) As a CDR-H1 sequence, the amino acid sequence defined in SEQ ID NO 177, or an amino acid sequence derived from SEQ ID NO 177 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 177,

as a CDR-H2 sequence, the amino acid sequence defined in SEQ ID NO 179, or an amino acid sequence derived from SEQ ID NO 179 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 179, and
as a CDR-H3 sequence, the amino acid sequence defined in SEQ ID NO 181, or an amino acid sequence derived from SEQ ID NO 181 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 181.

(18) As a CDR-H1 sequence, the amino acid sequence defined in SEQ ID NO 330, or an amino acid sequence derived from SEQ ID NO 330 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 330,

as a CDR-H2 sequence, the amino acid sequence defined in SEQ ID NO 331, or an amino acid sequence derived from SEQ ID NO 331 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 331, and
as a CDR-H3 sequence, the amino acid sequence defined in SEQ ID NO 332, or an amino acid sequence derived from SEQ ID NO 332 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 332.

(19) As a CDR-H1 sequence, the amino acid sequence defined in SEQ ID NO 337, or an amino acid sequence derived from SEQ ID NO 337 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 337,

as a CDR-H2 sequence, the amino acid sequence defined in SEQ ID NO 338, or an amino acid sequence derived from SEQ ID NO 338 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 338, and
as a CDR-H3 sequence, the amino acid sequence defined in SEQ ID NO 339, or an amino acid sequence derived from SEQ ID NO 339 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 339 .

(20) As a CDR-H1 sequence, the amino acid sequence defined in SEQ ID NO 344, or an amino acid sequence derived from SEQ ID NO 344 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 344,

as a CDR-H2 sequence, the amino acid sequence defined in SEQ ID NO 345, or an amino acid sequence derived from SEQ ID NO 345 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 345, and

as a CDR-H3 sequence, the amino acid sequence defined in SEQ ID NO 346, or an amino acid sequence derived from SEQ ID NO 346 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 346 .

(21) As a CDR grafting region H1 sequence, the amino acid sequence defined in SEQ ID NO 327, or an amino acid sequence derived from SEQ ID NO 327 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 327, as a CDR grafting region H2 sequence, the amino acid sequence defined in SEQ ID NO 328, or an amino acid sequence derived from SEQ ID NO 328 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 328,

as a CDR grafting region H3 sequence, the amino acid sequence defined in SEQ ID NO 329, or an amino acid sequence derived from SEQ ID NO 329 via substitution, deletion, or insertion of any one or two amino acid residues, or an amino acid sequence having a homology (preferably an identity) of 66.6 % or more or 83.3 % or more to the amino acid sequence defined in SEQ ID NO 329.

(22) As a CDR grafting region H1 sequence, the amino acid sequence defined in SEQ ID NO 334, or an amino acid sequence derived from SEQ ID NO 334 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 334, as a CDR grafting region H2 sequence, the amino acid sequence defined in SEQ ID NO 335, or an amino acid sequence derived from SEQ ID NO 335 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 335,

as a CDR grafting region H3 sequence, the amino acid sequence defined in SEQ ID NO 336, or an amino acid sequence derived from SEQ ID NO 336 via substitution, deletion, or insertion of any one or two amino acid residues, or an amino acid sequence having a homology (preferably an identity) of 66.6 % or more or 83.3 % or more to the amino acid sequence defined in SEQ ID NO 336.

(23) As a CDR grafting region H1 sequence, the amino acid sequence defined in SEQ ID NO 341, or an amino acid sequence derived from SEQ ID NO 341 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 341, as a CDR grafting region H2 sequence, the amino acid sequence defined in SEQ ID NO 342, or an amino acid sequence derived from SEQ ID NO 342 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 342,

as a CDR grafting region H3 sequence, the amino acid sequence defined in SEQ ID NO 343, or an amino acid sequence derived from SEQ ID NO 343 via substitution, deletion, or insertion of any one or two amino acid residues, or an amino acid sequence having a homology (preferably an identity) of 66.6 % or more or 83.3 % or more to the amino acid sequence defined in SEQ ID NO 343.

An embodiment of the antibody of the present invention relates to an antibody containing a light chain variable region having a combination of CDR-L1 to -L3 sequences selected from (1) to (14) below.

(1) As a CDR-L1 sequence, the amino acid sequence defined in SEQ ID NO 7, or an amino acid sequence derived from SEQ ID NO 7 via substitution, deletion, or insertion of any one or two amino acid residues, or an amino acid sequence having a homology (preferably an identity) of 66.6 % or more or 83.3 % or more to the amino acid sequence defined in SEQ ID NO 7,

as a CDR-L2 sequence, the amino acid sequence defined in SEQ ID NO 9, or an amino acid sequence derived from SEQ ID NO 9 via substitution, deletion, or insertion of any one amino acid residue, or an amino acid sequence having a homology (preferably an identity) of 66.6 % or more to the amino acid sequence defined in SEQ ID NO 9, and

as a CDR-L3 sequence, the amino acid sequence defined in SEQ ID NO 11, or an amino acid sequence derived from SEQ ID NO 11 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 11.

(2) As a CDR-L1 sequence, the amino acid sequence defined in SEQ ID NO 23, or an amino acid sequence derived from SEQ ID NO 23 via substitution, deletion, or insertion of any one or two amino acid residues, or an amino acid sequence having a homology (preferably an identity) of 60.0 % or more or 80.0 % or more to the amino acid sequence defined in SEQ ID NO 23,

as a CDR-L2 sequence, the amino acid sequence defined in SEQ ID NO 25, or an amino acid sequence derived from SEQ ID NO 25 via substitution, deletion, or insertion of any one amino acid residue, or an amino acid sequence having a homology (preferably an identity) of 66.6 % or more to the amino acid sequence defined in SEQ ID NO 25, and
as a CDR-L3 sequence, the amino acid sequence defined in SEQ ID NO 27, or an amino acid sequence derived from SEQ ID NO 27 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 27.

(3) As a CDR-L1 sequence, the amino acid sequence defined in SEQ ID NO 39, or an amino acid sequence derived from SEQ ID NO 39 via substitution, deletion, or insertion of any one or two amino acid residues, or an amino acid sequence having a homology (preferably an identity) of 60.0 % or more or 80.0 % or more to the amino acid sequence defined in SEQ ID NO 39,

as a CDR-L2 sequence, the amino acid sequence defined in SEQ ID NO 41, or an amino acid sequence derived from SEQ ID NO 41 via substitution, deletion, or insertion of any one amino acid residue, or an amino acid sequence having a homology (preferably an identity) of 66.6 % or more to the amino acid sequence defined in SEQ ID NO 41, and
as a CDR-L3 sequence, the amino acid sequence defined in SEQ ID NO 43, or an amino acid sequence derived from SEQ ID NO 43 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 43.

(4) As a CDR-L1 sequence, the amino acid sequence defined in SEQ ID NO 55, or an amino acid sequence derived from SEQ ID NO 55 via substitution, deletion, or insertion of any one or two amino acid residues, or an amino acid sequence having a homology (preferably an identity) of 66.6 % or more or 83.3 % or more to the amino acid sequence defined in SEQ ID NO 55,
as a CDR-L2 sequence, the amino acid sequence defined in SEQ ID NO 57, or an amino acid sequence derived from SEQ ID NO 57 via substitution, deletion, or insertion of any one amino acid residue, or an amino acid sequence having a homology (preferably an identity) of 66.6 % or more to the amino acid sequence defined in SEQ ID NO 57, and as a CDR-L3 sequence, the amino acid sequence defined in SEQ ID NO 59, or an amino acid sequence derived from SEQ ID NO 59 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 59.

(5) As a CDR-L1 sequence, the amino acid sequence defined in SEQ ID NO 71, or an amino acid sequence derived from SEQ ID NO 71 via substitution, deletion, or insertion of any one or two amino acid residues, or an amino acid sequence having a homology (preferably an identity) of 60.0 % or more or 80.0 % or more to the amino acid sequence defined in SEQ ID NO 71,

as a CDR-L2 sequence, the amino acid sequence defined in SEQ ID NO 73, or an amino acid sequence derived from SEQ ID NO 73 via substitution, deletion, or insertion of any one amino acid residue, or an amino acid sequence having a homology (preferably an identity) of 66.6 % or more to the amino acid sequence defined in SEQ ID NO 73, and
as a CDR-L3 sequence, the amino acid sequence defined in SEQ ID NO 75, or an amino acid sequence derived from SEQ ID NO 75 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 75.

(6) As a CDR-L1 sequence, the amino acid sequence defined in SEQ ID NO 87, or an amino acid sequence derived from SEQ ID NO 87 via substitution, deletion, or insertion of any one or two amino acid residues, or an amino acid sequence having a homology (preferably an identity) of 66.6 % or more or 83.3 % or more to the amino acid sequence defined in SEQ ID NO 87,

as a CDR-L2 sequence, the amino acid sequence defined in SEQ ID NO 89, or an amino acid sequence derived from SEQ ID NO 89 via substitution, deletion, or insertion of any one amino acid residue, or an amino acid sequence having a homology (preferably an identity) of 66.6 % or more to the amino acid sequence defined in SEQ ID NO 89, and as a CDR-L3 sequence, the amino acid sequence defined in SEQ ID NO 91, or an amino acid sequence derived from SEQ ID NO 91 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 91.

(7) As a CDR-L1 sequence, the amino acid sequence defined in SEQ ID NO 103, or an amino acid sequence derived from SEQ ID NO 103 via substitution, deletion, or insertion of any one or two amino acid residues, or an amino acid sequence having a homology (preferably an identity) of 66.6 % or more or 83.3 % or more to the amino acid sequence defined in SEQ ID NO 103,

as a CDR-L2 sequence, the amino acid sequence defined in SEQ ID NO 105, or an amino acid sequence derived from SEQ ID NO 105 via substitution, deletion, or insertion of any one amino acid residue, or an amino acid sequence having a homology (preferably an identity) of 66.6 % or more to the amino acid sequence defined in SEQ ID NO 105, and as a CDR-L3 sequence, the amino acid sequence defined in SEQ ID NO 107, or an amino acid sequence derived from SEQ ID NO 107 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 107.

(8) As a CDR-L1 sequence, the amino acid sequence defined in SEQ ID NO 119, or an amino acid sequence derived from SEQ ID NO 119 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 119,

as a CDR-L2 sequence, the amino acid sequence defined in SEQ ID NO 121, or an amino acid sequence derived from SEQ ID NO 121 via substitution, deletion, or insertion of any one amino acid residue, or an amino acid sequence having a homology (preferably an identity) of 66.6 % or more to the amino acid sequence defined in SEQ ID NO 121, and as a CDR-L3 sequence, the amino acid sequence defined in SEQ ID NO 123, or an amino acid sequence derived from SEQ ID NO 123 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 123.

(9) As a CDR-L1 sequence, the amino acid sequence defined in SEQ ID NO 135, or an amino acid sequence derived from SEQ ID NO 135 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 135,

as a CDR-L2 sequence, the amino acid sequence defined in SEQ ID NO 137, or an amino acid sequence derived from SEQ ID NO 137 via substitution, deletion, or insertion of any one amino acid residue, or an amino acid sequence having a homology (preferably an identity) of 66.6 % or more to the amino acid sequence defined in SEQ ID NO 137, and as a CDR-L3 sequence, the amino acid sequence defined in SEQ ID NO 139, or an amino acid sequence derived from SEQ ID NO 139 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 139.

(10) As a CDR-L1 sequence, the amino acid sequence defined in SEQ ID NO 151, or an amino acid sequence derived from SEQ ID NO 151 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 151,

as a CDR-L2 sequence, the amino acid sequence defined in SEQ ID NO 153, or an amino acid sequence derived from SEQ ID NO 153 via substitution, deletion, or insertion of any one amino acid residue, or an amino acid sequence having a homology (preferably an identity) of 66.6 % or more to the amino acid sequence defined in SEQ ID NO 153, and as a CDR-L3 sequence, the amino acid sequence defined in SEQ ID NO 155, or an amino acid sequence derived from SEQ ID NO 155 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 155.

(11) As a CDR-L1 sequence, the amino acid sequence defined in SEQ ID NO 167, or an amino acid sequence derived from SEQ ID NO 167 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 167,
as a CDR-L2 sequence, the amino acid sequence defined in SEQ ID NO 169, or an amino acid sequence derived from SEQ ID NO 169 via substitution, deletion, or insertion of any one amino acid residue, or an amino acid sequence having a homology (preferably an identity) of 66.6 % or more to the amino acid sequence defined in SEQ ID NO 169, and as a CDR-L3 sequence, the amino acid sequence defined in SEQ ID NO 171, or an amino acid sequence derived from SEQ ID NO 171 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 171.

(12) As a CDR-L1 sequence, the amino acid sequence defined in SEQ ID NO 261, or an amino acid sequence derived from SEQ ID NO 261 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 261,
as a CDR-L2 sequence, the amino acid sequence defined in SEQ ID NO 263, or an amino acid sequence derived from SEQ ID NO 263 via substitution, deletion, or insertion of any one amino acid residue, or an amino acid sequence having a homology (preferably an identity) of 66.6 % or more to the amino acid sequence defined in SEQ ID NO 263, and as a CDR-L3 sequence, the amino acid sequence defined in SEQ ID NO 265, or an amino acid sequence derived from SEQ ID NO 265 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 265.

(13) As a CDR-L1 sequence, the amino acid sequence defined in SEQ ID NO 289, or an amino acid sequence derived from SEQ ID NO 289 via substitution, deletion, or insertion of any one or two amino acid residues, or an amino acid sequence having a homology (preferably an identity) of 60.0 % or more or 80.0 % or more to the amino acid sequence defined in SEQ ID NO 289,
as a CDR-L2 sequence, the amino acid sequence defined in SEQ ID NO 291, or an amino acid sequence derived from SEQ ID NO 291 via substitution, deletion, or insertion of any one amino acid residue, or an amino acid sequence having a homology (preferably an identity) of 66.6 % or more to the amino acid sequence defined in SEQ ID NO 291, and as a CDR-L3 sequence, the amino acid sequence defined in SEQ ID NO 293, or an amino acid sequence derived from SEQ ID NO 293 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 293.

(14) As a CDR-L1 sequence, the amino acid sequence defined in SEQ ID NO 317, or an amino acid sequence derived from SEQ ID NO 317 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 317,
as a CDR-L2 sequence, the amino acid sequence defined in SEQ ID NO 319, or an amino acid sequence derived from SEQ ID NO 319 via substitution, deletion, or insertion of any one amino acid residue, or an amino acid sequence having a homology (preferably an identity) of 66.6 % or more to the amino acid sequence defined in SEQ ID NO 319, and as a CDR-L3 sequence, the amino acid sequence defined in SEQ ID NO 321, or an amino acid sequence derived from SEQ ID NO 321 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 321.

As shown in Example 11 below, the present inventors investigated the importance of each amino acid residue of each CDR grafting region sequence of humanized anti-gpNMB antibody H1L1 (clone hGPN06-1_H1L1) in maintaining its binding ability by alanine scanning. The results showed that in the light chain variable region of the humanized anti-gpNMB antibody hGPN06-1_H1L1 (SEQ ID NO 297), the following amino acid residues were identified as important for maintaining binding: in CDR grafting region-L1 (SEQ ID NO 277), isoleucine at position 29, tyrosine at position 31 and histidine at position 33; in CDR grafting region-L2 (SEQ ID NO 279), threonine at position 50; and in CDR grafting region-L3 (SEQ ID NO 281), histidine at position 88, glutamine at position 89, tryptophan at position 90, serine at position 92, tyrosine at position 93, proline at position 94, and cysteine at position 95. On the other hand, the amino acid residues of each CDR grafting region other than those listed above were identified as having little effect on the maintenance of the antibody's binding ability. Based on these findings, one embodiment of the antibody of the present invention relates to an antibody containing a light chain variable region with the CDR grafting regions-L1 to -L3 sequences specified in (15) below (the amino acid positions specified below are indicated with respect to the amino acid sequence of the light chain variable region of humanized anti-gpNMB antibody hGPN06-1_H1L1 (SEQ ID NO 297)).

(15) As a CDR grafting region L1 sequence, the amino acid sequence defined in SEQ ID NO 277, or an amino acid sequence derived from SEQ ID NO 277 via substitution, deletion, or insertion of any one, two, three, or four amino acid residues other than isoleucine at position 29, tyrosine at position 31, and histidine at position 33,
as a CDR grafting region L2 sequence, the amino acid sequence defined in SEQ ID NO 279, or an amino acid sequence derived from SEQ ID NO 279 via substitution, deletion, or insertion of any one or two amino acid residue other than threonine at position 50,
as a CDR grafting region L3 sequence, the amino acid sequence defined in SEQ ID NO 281, or an amino acid sequence derived from SEQ ID NO 281 via substitution, deletion, or insertion of any one, two, or three amino acid residues other than histidine at position 88, glutamine at position 89, tryptophan at position 90, serine at position 92, tyrosine at position 93, proline at position 94, and cysteine at position 95 .

As shown in Example 15 below, the present inventors investigated the importance of each amino acid residue of each CDR grafting region sequence of humanized anti-gpNMB antibody H2L3 (clone hGPN18-2_H2L3) in maintaining its binding ability by alanine scanning. The results showed that in the light chain variable region of the humanized anti-gpNMB antibody hGPN18-2_H2L3 (SEQ ID NO 325), the following amino acid residues were identified as important for maintaining binding: in CDR grafting region-L1 (SEQ ID NO 305), leucine at position 31 and tyrosine at position 37; in CDR grafting region-L2 (SEQ ID NO 307), lysine at position 55; and in CDR grafting region-L3 (SEQ ID NO 309), phenylalanine at position 94. On the other hand, the amino acid residues of each CDR grafting region other than those listed above were identified as having little effect on the maintenance of the antibody's binding ability. Based on these findings, one embodiment of the antibody of the present invention relates to an antibody containing a light chain variable region with the CDR grafting regions-L1 to -L3 sequences specified in (16) below (the amino acid positions specified below are indicated with respect to the amino acid sequence of the light chain variable region of humanized anti-gpNMB antibody hGPN18-2_H2L3 (SEQ ID NO 325)).

(16) As a CDR grafting region L1 sequence, the amino acid sequence defined in SEQ ID NO 305, or an amino acid sequence derived from SEQ ID NO 305 via substitution, deletion, or insertion of any one, two, three, four, five, or six amino acid residues other than leucine at position 31, tyrosine at position 37 and glutamic acid at position 39,
as a CDR grafting region L2 sequence, the amino acid sequence defined in SEQ ID NO 307, or an amino acid sequence derived from SEQ ID NO 307 via substitution, deletion, or insertion of any one or two amino acid residue other than lysine at position 55,
as a CDR grafting region L3 sequence, the amino acid sequence defined in SEQ ID NO 309, or an amino acid sequence derived from SEQ ID NO 309 via substitution, deletion, or insertion of any one, two, or three amino acid residues other than phenylalanine at position 94.

An embodiment of the antibody of the present invention is an antibody containing a light chain variable region having the combination of CDR-L1 to -L3 sequences selected from (17) to (23) below. Antibodies containing a heavy chain variable region having the combination of CDR-L1 to -L3 sequences selected from (17) to (23) below have neither been disclosed nor suggested in the previous literature, and correspond to novel anti-gpNMB antibodies.

(17) As a CDR-L1 sequence, the amino acid sequence defined in SEQ ID NO 183, or an amino acid sequence derived from SEQ ID NO 183 via substitution, deletion, or insertion of any one or two amino acid residues, or an amino acid sequence having a homology (preferably an identity) of 66.6 % or more or 83.3 % or more to the amino acid sequence defined in SEQ ID NO 183,

as a CDR-L2 sequence, the amino acid sequence defined in SEQ ID NO 185, or an amino acid sequence derived from SEQ ID NO 185 via substitution, deletion, or insertion of any one amino acid residue, or an amino acid sequence having a homology (preferably an identity) of 66.6 % or more to the amino acid sequence defined in SEQ ID NO 185,
as a CDR-L3 sequence, the amino acid sequence defined in SEQ ID NO 187, or an amino acid sequence derived from SEQ ID NO 187 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 187.

(18) As a CDR-L1 sequence, the amino acid sequence defined in SEQ ID NO 351, or an amino acid sequence derived from SEQ ID NO 351 via substitution, deletion, or insertion of any one or two amino acid residues, or an amino acid sequence having a homology (preferably an identity) of 66.6 % or more or 83.3 % or more to the amino acid sequence defined in SEQ ID NO 351,

as a CDR-L2 sequence, the amino acid sequence defined in SEQ ID NO 352, or an amino acid sequence derived from SEQ ID NO 352 via substitution, deletion, or insertion of any one amino acid residue, or an amino acid sequence having a homology (preferably an identity) of 66.6 % or more to the amino acid sequence defined in SEQ ID NO 352,

as a CDR-L3 sequence, the amino acid sequence defined in SEQ ID NO 353, or an amino acid sequence derived from SEQ ID NO 353 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 353.

(19) As a CDR-L1 sequence, the amino acid sequence defined in SEQ ID NO 358, or an amino acid sequence derived from SEQ ID NO 358 via substitution, deletion, or insertion of any one or two amino acid residues, or an amino acid sequence having a homology (preferably an identity) of 66.6 % or more or 83.3 % or more to the amino acid sequence defined in SEQ ID NO 358,

as a CDR-L2 sequence, the amino acid sequence defined in SEQ ID NO 359, or an amino acid sequence derived from SEQ ID NO 359 via substitution, deletion, or insertion of any one amino acid residue, or an amino acid sequence having a homology (preferably an identity) of 66.6 % or more to the amino acid sequence defined in SEQ ID NO 359,

as a CDR-L3 sequence, the amino acid sequence defined in SEQ ID NO 360, or an amino acid sequence derived from SEQ ID NO 360 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 360.

(20) As a CDR-L1 sequence, the amino acid sequence defined in SEQ ID NO 365, or an amino acid sequence derived from SEQ ID NO 365 via substitution, deletion, or insertion of any one or two amino acid residues, or an amino acid sequence having a homology (preferably an identity) of 66.6 % or more or 83.3 % or more to the amino acid sequence defined in SEQ ID NO 365,

as a CDR-L2 sequence, the amino acid sequence defined in SEQ ID NO 366, or an amino acid sequence derived from SEQ ID NO 366 via substitution, deletion, or insertion of any one amino acid residue, or an amino acid sequence having a homology (preferably an identity) of 66.6 % or more to the amino acid sequence defined in SEQ ID NO 366,

as a CDR-L3 sequence, the amino acid sequence defined in SEQ ID NO 367, or an amino acid sequence derived from SEQ ID NO 367 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 367.

(21) As a CDR grafting region L1 sequence, the amino acid sequence defined in SEQ ID NO 348, or an amino acid sequence derived from SEQ ID NO 348 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 348, as a CDR grafting region L2 sequence, the amino acid sequence defined in SEQ ID NO 349, or an amino acid sequence derived from SEQ ID NO 349 via substitution, deletion, or insertion of any one or two amino acid residues, or an amino acid sequence having a homology (preferably an identity) of 71.4 % or more or 85.7 % or more to the amino acid sequence defined in SEQ ID NO 349,

as a CDR grafting region L3 sequence, the amino acid sequence defined in SEQ ID NO 350, or an amino acid sequence derived from SEQ ID NO 350 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 350.

(22) As a CDR grafting region L1 sequence, the amino acid sequence defined in SEQ ID NO 355, or an amino acid sequence derived from SEQ ID NO 355 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 355, as a CDR grafting region L2 sequence, the amino acid sequence defined in SEQ ID NO 356, or an amino acid sequence derived from SEQ ID NO 356 via substitution, deletion, or insertion of any one or two amino acid residues, or an amino acid sequence having a homology (preferably an identity) of 71.4 % or more or 85.7 % or more to the amino acid sequence defined in SEQ ID NO 356,

as a CDR grafting region L3 sequence, the amino acid sequence defined in SEQ ID NO 357, or an amino acid sequence derived from SEQ ID NO 357 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 357.

(23) As a CDR grafting region L1 sequence, the amino acid sequence defined in SEQ ID NO 362, or an amino acid sequence derived from SEQ ID NO 362 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 362, as a CDR grafting region L2 sequence, the amino acid sequence defined in SEQ ID NO 363, or an amino acid sequence derived from SEQ ID NO 363 via substitution, deletion, or insertion of any one or two amino acid residues, or an amino acid sequence having a homology (preferably an identity) of 71.4 % or more or 85.7 % or more to the amino acid sequence defined in SEQ ID NO 363,

as a CDR grafting region L3 sequence, the amino acid sequence defined in SEQ ID NO 364, or an amino acid sequence derived from SEQ ID NO 364 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 364.

An embodiment of the antibody of the present invention relates to an antibody having a heavy chain variable region having any of the combinations of the CDR-H1 to H3 sequences described above and a light chain variable region having any of the combinations of the CDR-L1 to L3 sequences described above.

An embodiment of the antibody of the present invention relates to an antibody having any of the following amino acid sequences (a) to (c) as the sequence of the heavy chain variable region. An antibody containing a heavy chain variable region having the amino acid sequence of SEQ ID NO 189 in (a) below, or a corresponding amino acid sequence of (b) or (c) below, have neither been disclosed nor suggested in the art and correspond to a novel anti-gpNMB antibody.

(a) An amino acid sequence selected from SEQ ID NO 13, SEQ ID NO 29, SEQ ID NO 45, SEQ ID NO 61, SEQ ID NO 77, SEQ ID NO 93, SEQ ID NO 109, SEQ ID NO 125, SEQ ID NO 141, SEQ ID NO 157, SEQ ID NO 173, SEQ ID NO 189, and SEQ ID NO 267.

(b) An amino acid sequence having a homology (preferably identity) of at least 60 % or higher, or 65 % or higher, or 70 % or higher, or 75 % or higher, or 80 % or higher, or 85 % or higher, or 90 % or higher, or 95 % or higher, or 96 % or higher, or 97 % or higher, or 98 % or higher, or 99 % or higher to any of the amino acid sequences specified in (a) above.

(c) An amino acid sequence derived from any of the amino acid sequences specified in (a) above via substitution, deletion, or addition of 1 to 12 (preferably 1 to 11, more preferably 1 to 10, 1 to 9, 1 to 8, 1 to 7, 1 to 6, 1 to 5, 1 to 4, 1 to 3, 1 to 2, or 1) amino acid residues.

An embodiment of the antibody of the present invention relates to an antibody (especially a humanized antibody) having any of the following amino acid sequences (a) to (c) as the sequence of the heavy chain variable region. An antibody (especially a humanized antibody) containing a heavy chain variable region having the amino acid sequence of SEQ ID NO 333, SEQ ID NO 340, or SEQ ID NO 347 in (a) below, or a corresponding amino acid sequence of (b) or (c) below, have neither been disclosed nor suggested in the art and correspond to a novel anti-gpNMB antibody (especially a humanized antibody).

(a) An amino acid sequence selected from SEQ ID NO 295, SEQ ID NO 323, SEQ ID NO 333, SEQ ID NO 340, and SEQ ID NO 347.

(b) An amino acid sequence having a homology (preferably identity) of at least 60 % or higher, or 65 % or higher, or 70 % or higher, or 75 % or higher, or 80 % or higher, or 85 % or higher, or 90 % or higher, or 95 % or higher, or 96 % or higher, or 97 % or higher, or 98 % or higher, or 99 % or higher to any of the amino acid sequences specified in (a) above.

(c) An amino acid sequence derived from any of the amino acid sequences specified in (a) above via substitution, deletion, or addition of 1 to 12 (preferably 1 to 11, more preferably 1 to 10, 1 to 9, 1 to 8, 1 to 7, 1 to 6, 1 to 5, 1 to 4, 1 to 3, 1 to 2, or 1) amino acid residues.

An embodiment of the antibody of the present invention relates to an antibody having any of the following amino acid sequences (a) to (c) as the sequence of the light chain variable region. An antibody containing a heavy chain variable region having the amino acid sequence of SEQ ID NO 191 in (a) below, or a corresponding amino acid sequence of (b) or (c) below, have neither been disclosed nor suggested in the art and correspond to a novel anti-gpNMB antibody.

(a) An amino acid sequence selected from SEQ ID NO 15, SEQ ID NO 31, SEQ ID NO 47, SEQ ID NO 63, SEQ ID NO 79, SEQ ID NO 95, SEQ ID NO 111, SEQ ID NO 127, SEQ ID NO 143, SEQ ID NO 159, SEQ ID NO 175, SEQ ID NO 191, and SEQ ID NO 269.

(b) An amino acid sequence having a homology (preferably identity) of at least 60 % or higher, or 65 % or higher, or 70 % or higher, or 75 % or higher, or 80 % or higher, or 85 % or higher, or 90 % or higher, or 95 % or higher, or 96 % or higher, or 97 % or higher, or 98 % or higher, or 99 % or higher to any of the amino acid sequences specified in (a) above.

(c) An amino acid sequence derived from any of the amino acid sequences specified in (a) above via substitution, deletion, or addition of 1 to 10 (preferably 1 to 9, more preferably 1 to 8, 1 to 7, 1 to 6, 1 to 5, 1 to 4, 1 to 3, 1 to 2, or 1) amino acid residues.

An embodiment of the antibody of the present invention relates to an antibody (especially a humanized antibody) having any of the following amino acid sequences (a) to (c) as the sequence of the light chain variable region. An antibody (especially a humanized antibody) containing a heavy chain variable region having the amino acid sequence of SEQ ID NO 354, SEQ ID NO 361, or SEQ ID NO 368 in (a) below, or a corresponding amino acid sequence of (b) or (c) below, have neither been disclosed nor suggested in the art and correspond to a novel anti-gpNMB antibody (especially a humanized antibody).

(a) An amino acid sequence selected from SEQ ID NO 297, SEQ ID NO 325, SEQ ID NO 354, SEQ ID NO 361, and SEQ ID NO 368.

(b) An amino acid sequence having a homology (preferably identity) of at least 60 % or higher, or 65 % or higher, or 70 % or higher, or 75 % or higher, or 80 % or higher, or 85 % or higher, or 90 % or higher, or 95 % or higher, or 96 % or higher, or 97 % or higher, or 98 % or higher, or 99 % or higher to any of the amino acid sequences specified in (a) above.

(c) An amino acid sequence derived from any of the amino acid sequences specified in (a) above via substitution, deletion, or addition of 1 to 10 (preferably 1 to 9, more preferably 1 to 8, 1 to 7, 1 to 6, 1 to 5, 1 to 4, 1 to 3, 1 to 2, or 1) amino acid residues.

An embodiment of the antibody of the present invention relates to an antibody having any of the heavy chain variable region sequences described above and any of the light chain variable region sequences described above.
The CDR-H1, CDR-H2, CDR-H3, CDR-L1, CDR-L2, and CDR-L3 sequences in the antibody can be identified by the Kabat method (NIH Publication, (1991) No. 91-3242) or the Chothia method (J. Mol. Biol., (1997), 273). These methods are within the technical common knowledge for those skilled in the art, and are also outlined in, e.g., the Dr. Andrew C.R. Martin's Group Internet home page (http://www.bioinf.org.uk/abs/). The CDRs described herein are indicated as Kabat's CDRs.
Framework sequences of each of the heavy chain variable region and the light chain variable region of the immunoglobulin as the antibody of the present invention may preferably be framework sequences in each class of vertebrate immunoglobulin and, more preferably, framework sequences in each class of immunoglobulin of human or a non-human animal such as mouse or rat.
A person skilled in the art would be able to design the anti-gpNMB-specific antibody of the present invention by combining the amino acid sequence of each of the CDRs and/or each of the heavy and light chain variable regions described above with the amino acid sequence of each of the framework regions and/or each of the heavy and light chain constant regions of an antibody derived from human or non-human animals including mouse or rat, as appropriate. In this regard, a humanized anti-gpNMB-specific antibody can be designed by using the amino acid sequence of each of the framework regions and/or each of the heavy and light chain constant regions of a human antibody. The amino acid sequence of each of the framework regions and/or each of the heavy and light chain constant regions of such a humanized antibody can be selected from, e.g., each class of human IgG, IgA, IgM, IgE, and IgD, and their variants. There are four subclasses of IgG: IgG1, IgG2, IgG3, and IgG4. When the anti-gpNMB-specific antibody of the present invention is a humanized antibody containing each framework region and/or each constant region of the heavy and light chains of the human IgG class, the amino acid sequence of each framework region and/or each constant region of the heavy and light chains of such human IgG may be derived from any of the human IgG1 subclass, human IgG2 subclass, human IgG3 subclass, and human IgG4 subclass.

The antibody of the present invention may preferably be an antibody of the IgG class or its variant. The antibody of the present invention may more preferably be of the human IgG class or its variant, of the human IgG4 subclass or its variant, or the human IgG1 subclass or its variant. In one example, the stabilized IgG4 constant region contains a proline at position 241 in the hinge region, according to Kabat's system. This position corresponds to position 228 of the hinge region according to the EU numbering scheme (Proc. Natl. Acad. Sci. USA, (1969), 63[1]:78-85), Sequences of proteins of immunological interest (Washington DC United States Department of Health and Human Services, 2001and NIH Publication, (1991), No. 91-3242). This residue is generally serine in human IgG4, but its stabilization can be induced by substituting the serine with proline. In one example, the N297A mutation can be incorporated into the constant region of IgG1 to suppress its ability to bind to the Fc receptor and/or anchor complement as much as possible.

*Competitive binding:

An embodiment of the present invention relates to an antibody that binds to at least one site in a region from V78 to a PKD domain of gpNMB competitively with the binding of the antibody of the present invention to the same site. Such a competitively binding antibody is also included in the scope of the present invention. The term "competitive binding" herein refers to phenomenon where when at least two monoclonal antibodies coexist with an antigen, the binding of one antibody to the antigen is inhibited by the binding of the other antibody to the antigen. Competitive binding ability between two monoclonal antibodies can generally be measured by using a fixed amount (concentration) of a first monoclonal antibody, adding varying amounts (concentrations) of a second monoclonal antibody thereto, and measuring the amount (concentration) of the second monoclonal antibody added that decreases the binding of the fixed amount of the first monoclonal antibody to the antigen. The degree of inhibition can be expressed in terms of $IC_{50}$ or Ki. A monoclonal antibody that competitively binds to the antibody of the present invention may refer to, e.g., an antibody whose $IC_{50}$ measured in terms of competitive antigen-antibody binding to at least one site in a region from V78 to a PKD domain of gpNMB using 10 nM of the antibody of the invention is usually 1000nM or lower, particularly 100nM or lower, more particularly 10nM or lower. Competitive binding ability can be measured by, e.g., labelling the antibody with an enzyme, fluorescent substance, luminescent substance, radioisotope, etc., and carrying out detection using a measurement method suitable for the physical and/or chemical properties of the labeled substance, or by using a biosensor based on, e.g., surface plasmon resonance (SPR) and bio-layer interferometry (BLI).

*Activity to remove disease-related cells:

The anti-gpNMB antibodies of the present invention have activity to eliminate various disease-associated cells that highly express gpNMB, as will be described later. Therefore, the use of the anti-gpNMB antibodies of the present invention makes it possible to efficiently eliminate various disease-related cells, and thereby prevent or treat various diseases associated with such disease-associated cells, as will be described later. The following are examples of suitable characteristics of the antibodies of the present invention for the removal of such disease-associated cells and the treatment/prevention of disease through such removal of disease-associated cells, although the antibodies of the present invention are not limited to those with these characteristics. The Examples described below confirm that some anti-gpNMB antibodies have signaling activity via gpNMB, resulting in increased phosphorylation of ERK1/2 (extracellular signal-regulated kinase), S6 (ribosomal protein) and Akt (serine-threonine kinase). Thus, according to an embodiment, an anti-gpNMB antibody having signaling activity via gpNMB is provided as an anti-gpNMB antibody of the present invention. According to another embodiment, an anti-gpNMB antibody lacking signaling activity via gpNMB is provided as an anti-gpNMB antibody of the present invention.

The anti-gpNMB antibodies of the present invention may exhibit the activity to remove disease-related cells either in vivo or ex vivo, or both in vivo and ex vivo. The anti-gpNMB antibodies of the present invention may exhibit such activity in vivo and/or ex vivo in any subject, preferably at least in a vertebrate, preferably at least in a mammal, especially preferably either in human or in a non-human animal.

Preferred embodiments of the antibodies of the present invention for the removal of such disease-related cells and the treatment/prevention of diseases through such removal are exemplified below, but the antibodies of the present invention are not limited to those having these features.

*Effector function of the antibodies

According to an embodiment, the antibody of the present invention may preferably have the ability to remove cells involved in pathological conditions. Preferred among them are antibodies with an Fc structure that confers phagocytosis ability by phagocytic cells to remove cells involved in pathological conditions. They may also preferably be subclasses or mutants that do not confer or inhibit the ability to induce inflammation. However, it is assumed that the way the effector function is exerted may differ depending on the pathological conditions, the cells to be removed, and the tissues in which the cells are present. Therefore, the antibodies of the present invention are not necessarily limited to the above, but may preferably be provided with the effector function and strength appropriate for the treatment of each disease (Non-Patent Literature 91).

*Half-life of the antibodies

According to an embodiment, the antibody of the present invention can extend the half-life in blood by using previously reported Fc mutants, a specific example of which is YTE mutant. Longer blood half-life reduces the administration frequency of the antibody (Non-Patent Literature 92).

*Crossing the blood-tissue barrier of the antibodies

According to one aspect, the antibody of the present invention may be fused with an anti-transferrin antibody or other antibody to enhance their migration from the blood into isolated tissues such as the blood brain barrier (BBB) (Non-Patent Literature 93).

*Bispecific and multispecific antibodies

The present inventors have created anti-gpNMB antibodies that have specificity and binding affinity sufficient to produce bispecific antibodies and multivalent antibodies with high therapeutic efficacy and can eliminate gpNMB-positive cells. Thus, according to an embodiment, the antibody of the present invention can be used to eliminate disease-related cells by engaging and attracting T cells, NK cells, macrophages, etc. to various gpNMB-positive disease-related cells (e.g., dysfunctional macrophages, senescent cells, cancer cells, cancer-infiltrating cells, etc.) (Non-Patent Literature 94).

*CAR cells

The present inventors have created small molecule antibodies, such as single chain variable region fragments (single chain Fv: scFv), of anti-gpNMB antibodies that have specificity and binding affinity sufficient to produce bispecific antibodies and multivalent antibodies with high therapeutic efficacy and can eliminate gpNMB-positive cells. Thus, according to an embodiment, the antibody of the present invention can be used to create CAR cells to deliver T cells, NK cells, macrophages, etc. to various gpNMB-positive disease-involved cells (dysfunctional macrophages, senescent cells, cancer cells, cancer-infiltrating cells, etc.) and eliminate disease-related cells (Non-Patent Literature 95). In addition, pre-treatment with the anti-gpNMB antibody of the present invention in advance will provide higher therapeutic efficacy of CAR cell therapy.

[Production method of the anti-gpNMB antibody]

The antibody according to the present invention can be obtained using techniques known to those skilled in the art. The antibody according to the present invention may be either a polyclonal antibody or a monoclonal antibody (Nature, (1983), 305(5934): 537-40). A polyclonal antibody according to the present invention can be produced, e.g., by preparing a human gpNMB protein having the amino acid sequence defined in SEQ ID NO 209 or a gpNMB partial peptide having a part of the amino acid sequence (e.g., a peptide containing a PKD domain with the amino acid residues 256 to 319) as an antigen, or preparing an antigen-expressing polynucleotide which encodes the amino acid sequence of such a human gpNMB protein or gpNMB partial peptide, administering the antigen or antigen-expressing polynucleotide to a mammal intramuscularly or subcutaneously to express the antigen in the animal body and thereby sensitize the animal, and recovering the antibody produced in the animal body from, e.g., serum of the animal. When a peptide is used as an antigen, it may be bound to a carrier protein such as BSA or KLH or a polylysine.

A monoclonal antibody according to the present invention can be produced, e.g., by administering the human gpNMB protein or the gpNMB partial peptide or the antigen-expressing polynucleotide encoding the human gpNMB protein or gpNMB partial peptide mentioned above to a mammal to thereby sensitize the animal, collecting immune cells from the mammal and fusing them with myeloma cells to produce hybridomas, cloning each hybridoma, and recovering the antibody produced by a culture of the cloned hybridoma. Such methods for obtaining monoclonal antibodies (Nature, (1992), 356[6365]:152-4) and cell fusion techniques (Nature, (1975), 256[5517]:495-7) have already been reported and generalized, and the administration of immunostimulants can further enhance the acquisition efficiency (Cancer Gene Ther., (2007), 14[11]:904-17). Monoclonal antibodies obtained by this method include, although not limited to, the following. Of the following antibodies, the anti-gpNMB monoclonal antibody (GPN18-5) containing the heavy chain variable region having the amino acid sequence of SEQ ID NO 189 and the light chain variable region having the amino acid sequence of SEQ ID NO 191 has neither been disclosed nor suggested in the prior art documents and therefore is a novel anti-gpNMB antibody (especially a humanized antibody).

*An antibody containing a heavy chain variable region the amino acid sequence defined in SEQ ID NO 13 and a light chain variable region the amino acid sequence defined in SEQ ID NO 15 (GPN05-1).
* An antibody containing a heavy chain variable region the amino acid sequence defined in SEQ ID NO 29 and a light chain variable region the amino acid sequence defined in SEQ ID NO 31 (GPN06-1).
* An antibody containing a heavy chain variable region the amino acid sequence defined in SEQ ID NO 45 and a light chain variable region the amino acid sequence defined in SEQ ID NO 47 (GPN07-1).
* An antibody containing a heavy chain variable region the amino acid sequence defined in SEQ ID NO 61 and a light chain variable region the amino acid sequence defined in SEQ ID NO 63 (GPN11-10).
* An antibody containing a heavy chain variable region the amino acid sequence defined in SEQ ID NO 77 and a light chain variable region the amino acid sequence defined in SEQ ID NO 79 (GPN15-2).
* An antibody containing a heavy chain variable region the amino acid sequence defined in SEQ ID NO 93 and a light chain variable region the amino acid sequence defined in SEQ ID NO 95 (GPN15-3).
* An antibody containing a heavy chain variable region the amino acid sequence defined in SEQ ID NO 109 and a light chain variable region the amino acid sequence defined in SEQ ID NO 111 (GPN18-4).
* An antibody containing a heavy chain variable region the amino acid sequence defined in SEQ ID NO 125 and a light chain variable region the amino acid sequence defined in SEQ ID NO 127 (GPN18-1).
* An antibody containing a heavy chain variable region the amino acid sequence defined in SEQ ID NO 141 and a light chain variable region the amino acid sequence defined in SEQ ID NO 143 (GPN18-2).
* An antibody containing a heavy chain variable region the amino acid sequence defined in SEQ ID NO 157 and a light chain variable region the amino acid sequence defined in SEQ ID NO 159 (GPN18-6).
* An antibody containing a heavy chain variable region the amino acid sequence defined in SEQ ID NO 173 and a light chain variable region the amino acid sequence defined in SEQ ID NO 175 (GPN18-7).
* An antibody containing a heavy chain variable region the amino acid sequence defined in SEQ ID NO 189 and a light chain variable region the amino acid sequence defined in SEQ ID NO 191 (GPN18-5).
* An antibody containing a heavy chain variable region the amino acid sequence defined in SEQ ID NO 267 and a light chain variable region the amino acid sequence defined in SEQ ID NO 269 (GPN09-1).

Another production method includes producing a gene encoding the heavy chain and/or the light chain of the antibody, or more specifically, a nucleic acid molecule encoding the heavy chain and/or the light chain of immunoglobulin of the antibody, from a hybridoma which produces the antibody to be obtained, or from a phage clone obtained by human antibody phage display. Here, the nucleic acid molecule may be introduced into various vectors or plasmids to create a vector or plasmid containing the nucleic acid molecule. Host cells are then transformed with the nucleic acid molecule, vector, or plasmid described above. The host cells may be selected from eukaryotic cells, such as mammalian, insect, yeast, or plant cells, or from bacterial cells. The transformed host cells are then cultured under appropriate conditions for producing the anti-gpNMB-specific antibody of the present invention. If necessary, the anti-gpNMB-specific antibody of the present invention produced may be isolated from the host cells. The various methods used in these procedures are all well known to those skilled in the art.

A person skilled in the art can also produce antibody chimeric proteins, small molecule antibodies, scaffolded antibodies, etc., using known techniques, by genetic modification of genes encoding heavy and/or light chains of immunoglobulins to introduce desired traits or by using structural information of variable regions or CDR regions of heavy and/or light chains of immunoglobulins. A person skilled in the art can also make any modifications to the structure of the constant regions or to the glycosylation sites of the antibody using techniques well known to those skilled in the art in order to improve the performance of the antibody or to avoid any side effects.

[Humanized antibody]

According to an embodiment, the anti-gpNMB antibodies of the present invention may be a humanized antibody. A humanized product of the anti-gpNMB antibodies of the present invention may herein be referred to as "the anti-gpNMB humanized antibody of the present invention" or simply as "the humanized antibody of the present invention." Accordingly, "the anti-gpNMB antibodies of the present invention" naturally include "the anti-gpNMB humanized antibodies of the present invention."

Humanization of the non-humanized anti-gpNMB antibody of the present invention, i.e., production of the anti-gpNMB humanized antibody of the present invention, can be carried out according to, e.g., the following procedure.

Specifically, for the amino acid sequences of the non-humanized (e.g., mouse) anti-gpNMB antibody, CDR regions are identified using IMGT (Lefranc, M.-P., Pommie, C., Ruiz, M., Giudicelli, V., Foulquier, E., Truong, L., Thouvenin-Contet, V. and Lefranc, G. "IMGT unique numbering for immunoglobulin and T cell receptor variable domains and Ig superfamily V-like domains". Dev. Comp. Immunol., 27, 55-77 (2003) PMID: 12477501 LIGM:268) and Kabat numbering. The combined IMGT/Kabat method is used to ensure that the CDR loop structure is optimized. Next, the human antibody frames are selected to maintain the CDR loop structure by the CDR grafting method to obtain antibody variants. A humanized antibody can be obtained by selecting an antibody variant that has the desired binding affinity (e.g., binding affinity for at least one region from the PMEL-CAF-like region to the PKD region of gpNMB) from among the antibody variants obtained.

Specifically, a human germline gene is selected that most closely approximates the amino acid sequences of the heavy and light chain variable regions of non-humanized (e.g., mouse) anti-gpNMB antibodies obtained by the various methods described above. On the other hand, BLAST search is used to select about 200 human IgG sequences that are homologous to the heavy and light chain variable regions as candidates. Then, four framework sequences are selected, for example, in terms of framework homology, retention of key amino acids in the framework, and the loop structure. CDR grafting is performed on these sequences to obtain four humanized variants. In addition, CDR grafting is performed on the human germline sequence as a framework to obtain one humanized variant. The resulting five humanized variants of heavy and light chains are combined to express 25 humanized antibody variants, which are purified and evaluated for their binding activity. Variants with a given (e.g., three-fold or one-third) binding affinity are selected via activity comparison using SPR with human chimeric antibodies with mouse heavy and light chains of the parent antibody, whereby humanized antibodies can be obtained.

As another example, anti-gpNMB antibodies that recognize at least one site within a region from the PMEL-CAF-like region to the PKD region, which is also recognized by the anti-gpNMB antibodies disclosed herein, can be obtained directly from human antibody-producing mice or human antibody phagemid libraries.

Examples of humanized anti-gpNMB monoclonal antibodies obtained by such methods include, although are not limited to, the following. Of the following antibodies, the humanized anti-gpNMB monoclonal antibodies containing the heavy chain variable region having the amino acid sequence of SEQ ID NO 333, SEQ ID NO 340, or SEQ ID NO 347 and the light chain variable region having the amino acid sequence of SEQ ID NO 354, SEQ ID NO 361, or SEQ ID NO 368 (hGPN18-5_H1-H3/L1-L3) have neither been disclosed nor suggested in the prior art documents and therefore are novel anti-gpNMB antibodies (especially a humanized antibodies).

*An antibody containing a heavy chain variable region the amino acid sequence defined in SEQ ID NO 295 and a light chain variable region the amino acid sequence defined in SEQ ID NO 297 (hGPN06-1_H1L1).

* An antibody containing a heavy chain variable region the amino acid sequence defined in SEQ ID NO 323 and a light chain variable region the amino acid sequence defined in SEQ ID NO 325 (hGPN18-2_H2L3).

* An antibody containing a heavy chain variable region the amino acid sequence defined in SEQ ID NO 333 and a light chain variable region the amino acid sequence defined in SEQ ID NO 354 (hGPN18-5_H1L1).

* An antibody containing a heavy chain variable region the amino acid sequence defined in SEQ ID NO 333 and a light chain variable region the amino acid sequence defined in SEQ ID NO 361 (hGPN18-5_H1L2).

* An antibody containing a heavy chain variable region the amino acid sequence defined in SEQ ID NO 333 and a light chain variable region the amino acid sequence defined in SEQ ID NO 368 (hGPN18-5_H1L3).

* An antibody containing a heavy chain variable region the amino acid sequence defined in SEQ ID NO 340 and a light chain variable region the amino acid sequence defined in SEQ ID NO 354 (hGPN18-5_H2L1).

* An antibody containing a heavy chain variable region the amino acid sequence defined in SEQ ID NO 340 and a light chain variable region the amino acid sequence defined in SEQ ID NO 361 (hGPN18-5_H2L2).

* An antibody containing a heavy chain variable region the amino acid sequence defined in SEQ ID NO 340 and a light chain variable region the amino acid sequence defined in SEQ ID NO 368 (hGPN18-5_H2L3).

* An antibody containing a heavy chain variable region the amino acid sequence defined in SEQ ID NO 347 and a light chain variable region the amino acid sequence defined in SEQ ID NO 354 (hGPN18-5_H3L1).

* An antibody containing a heavy chain variable region the amino acid sequence defined in SEQ ID NO 347 and a light chain variable region the amino acid sequence defined in SEQ ID NO 361 (hGPN18-5_H3L2).

* An antibody containing a heavy chain variable region the amino acid sequence defined in SEQ ID NO 347 and a light chain variable region the amino acid sequence defined in SEQ ID NO 368 (hGPN18-5_H3L3).

[Pharmaceutical composition]

An embodiment of the present invention relates to a pharmaceutical composition for removing disease-related cells that contains, as an active ingredient, an anti-gpNMB-specific antibody of the present invention (hereinafter collectively referred to as "the pharmaceutical compositions of the present invention"). By using the anti-gpNMB antibody of the present invention as an active ingredient, the pharmaceutical composition of the present invention is capable of eliminating various disease-related cells. Therefore, the pharmaceutical compositions of the present invention can be used for the treatment and prevention of various diseases.

Disease-related cells that can be removed using the anti-gpNMB antibody of the present invention include, although are not limited to, senescent cells, cancer-related cells, and phagocytes.

Senescent cells, as mentioned above, are cells in which a sustained arrest of cell proliferation has been induced due to DNA damage, activation of oncogenes, or stress. Recently, senescent cells have been shown to acquire a pro-inflammatory phenotype called SASP (senescence-associated secretory phenotype), which has been implicated in many diseases (Non-Patent Literature 1). While cellular senescence is thought to be a mechanism that prevents damaged cells from undergoing malignant transformation and becoming cancerous, there are also numerous reports of its involvement in various aging-related pathologies (e.g., tissue degeneration, chronic inflammation, cancer progression, etc.). Senescent cells with particularly high malignant potential include senescent infiltration cells or senescent resident cells, which form or progress pathological conditions by significantly affecting surrounding cells and tissues.

Examples of cancer-related cells include cancer cells, cancer stem cells, cancer progenitor cells, and cancer infiltration cells. Examples of cancer infiltration cells include cancer infiltration macrophages, cancer infiltration microglia, myeloid-derived suppressor cells (MDSC), and cancer infiltration dendritic cells.

Although not being limited to these, examples of phagocytes include macrophages, microglia, dendritic cells, monocytes, Kupffer cells, Langerhans cells, and osteoclasts. Examples of macrophages include cancer infiltration macrophages, senescent macrophages, fat-laden macrophages, sphingomyelin-laden macrophages, foam cells, alveolar macrophages, peritoneal macrophages, splenic macrophages, thymic macrophages. Examples of microglia include cancer infiltration microglia, senescent microglia, fat-laden microglia, and sphingomyelin-laden microglia. Subtypes of dendritic cells include type 1 conventional dendritic cells (cDC1), type 2 conventional dendritic cells (cDC2), monocyte-derived dendritic cells (MoDC), and plasmacyotid dendritic cells (pDC).

Examples of diseases associated with disease-related cells to be removed by the pharmaceutical compositions of the present invention include, although are not limited to, obesity-metabolic disease (e.g., diabetes and fibrosis), circulatory disease (e.g., cardiomyopathy and arterysclerosis), lysosomal stress-associated disease (e.g., lysosome disease), autoimmune disease (e.g., multiple sclerosis), and cancer (e.g., triple negative breast cancer (TNBC), melanoma, non-small cell lung cancer, and glioblastoma).

The pharmaceutical composition of the present invention may be formulated in the form of a pharmaceutical composition containing, in addition to the anti-gpNMB-specific antibody of the present invention as the active ingredient, a pharmaceutically acceptable carrier and/or other additive. Formulation using a pharmaceutically acceptable carrier and/or other additive may be carried out using, e.g., the methods described in University of the Sciences in Philadelphia, "Remington: The Science and Practice of Pharmacy, 20th EDITION", Lippincott Williams & Wilkins, (2000).

One form of such a therapeutic or prophylactic agent is a liquid or lyophilized form prepared by dissolving, suspending, or emulsifying the ingredients in a sterile aqueous or oily solution. Examples of such solvents or dissolving solutions as aqueous media include distilled water for injection and saline. In addition, osmotics (e.g., D-glucose, D-sorbitol, D-mannitol, sodium chloride, etc.) may be added, as well as suitable dissolution aids, such as alcohols (e.g., ethanol), poly alcohols (e.g., propylene glycol, polyethylene glycol), nonionic surfactants (e.g., polysorbate 80, polyoxyethylene hardened castor oil 50), etc., may be used in combination. Oil-based liquids may also be used as solvents or dissolving solutions. Examples of such oily liquids include sesame oil and soybean oil. Benzyl benzoate, benzyl alcohol, etc., may also be used as a dissolution aid. In such formulations, additives may be used as appropriate. Examples of additives include: buffers (e.g., phosphate buffer and acetate buffer), soothing agents (e.g., benzalkonium chloride and procaine hydrochloride), stabilizers (e.g., human serum albumin and polyethylene glycol), preservatives (e.g., ascorbic acid, erythorbic acid, and their salts), colorants (e.g., copper chlorophyll, β-carotene, red #2, and blue #1), antiseptics (e.g., paraoxybenzoate, phenol, benzethonium chloride, and benzalkonium chloride), thickeners (e.g., hydroxy propyl cellulose, carboxyl methyl cellulose, and their salts), stabilizing agents (e.g., human serum albumin, mannitol, and sorbitol), and deodorants (e.g., menthol and citrus flavoring).

Other forms of therapeutic or prophylactic agents include solid dosage forms such as dispersions, tablets, granules, capsules, pills, suppositories, and lozenges. Solid dosage forms administered in the form of oral preparations may contain additives such as excipients (e.g., crystalline cellulose, lactose, and starch), lubricants (e.g., magnesium stearate and talc), binding agents (e.g., hydroxypropyl cellulose, hydroxypropyl methylcellulose, and macrogol), and disintegrants (e.g., starch and calcium carboxymethylcellulose). In addition, additives such as preservatives (e.g., benzyl alcohol, chlorobutanol, methyl paraoxibenzoate, propyl paraoxibenzoate, etc.), antioxidants, colorants, and sweetening agents can be used as needed. Another form of the therapeutic or prophylactic agent is intended for mucosal application. This type of formulation may contain adhesives, adhesion enhancers, viscosity agents, and viscosity enhancers (e.g., mucin, canten, gelatin, pectin, carrageenan, sodium alginate, locust bean gum, xanthan gum, tragacanth gum, gum arabic, chitosan, pullulan, waxy starch, sucralfate, cellulose, and their derivatives) are contained as additives mainly to provide absorption and retention on the mucosa. gum arabic, chitosan, pullulan, waxy starches, sucralfate, cellulose, and their derivatives). However, the form of the therapeutic or prophylactic agent to be provided to the living body and the solvent and additives used therein are not limited to these, and can be selected by a person skilled in the art as appropriate.

The pharmaceutical composition of the present invention may contain, in addition to the anti-gpNMB-specific antibody of the present invention as the active ingredient, another known drug (active ingredient). Examples of such other known drugs (active ingredients) include, although not limited to, various therapeutic drugs for central neurological diseases, therapeutic drugs for neurodegenerative diseases, nerve function recovery drugs, and therapeutic drugs for Alzheimer's disease. Specific examples include anti-Tau antibodies, anti-amyloid β antibodies, anti-CD33 antibodies, anti-semaphorin 4D antibodies, anti-TNFα antibodies, anti-sortilin antibodies, anti-galactose-specific-lectin (galectin) 3 antibodies, and anti-TREM2 (Triggering receptor expressed on myeloid cells 2) antibodies. They may be used singly or in combination of any two or more.

The pharmaceutical composition of the present invention may also be combined with another known drug in the form of a kit. Examples of other drugs (active ingredients) that can be combined with the anti-gpNMB-specific antibody of the present invention include the known drugs (active ingredients) described above for combination drugs. The dose of the drugs other than the anti-gpNMB-specific antibody used in the form of the combination drug or kit can be determined based on doses normally used for treatment, but can be increased or decreased depending on the circumstances.

The pharmaceutical compositions of the present invention may be administered parenterally for the purpose of improving symptoms. For parenteral administration, it can be administered as, e.g., an intranasal formulation, and liquid, turbid, or solid formulations can be selected. Another parenteral dosage form may be an injectable form, which may be administered by subcutaneous, intravenous, intravenous, intramuscular, intracerebroventricular, or intraperitoneal injection. Other parenteral formulations include those for transmucosal administration other than suppositories, sublingual, transdermal, and other than nasal administration. In addition, the antibody can be locally administered intravascularly as contained in or applied to a stent or intravascular occlusive agent. In some cases, it is also possible to administer the antibody in the form of DNA or RNA encoding the antibody, or in the form of cells or commensal bacteria producing the antibody.

The dosage of the pharmaceutical composition of the present invention varies depending on the age, sex, weight, symptoms, therapeutic effect, method of administration, treatment time, or type of active ingredient contained in the pharmaceutical composition, etc. of the patient, but may usually be from 0.1 mg to 1 g, preferably from 0.5 mg to 300 mg, of the active ingredient per adult per dose. It may be administered once every week to every four weeks or once every month to every six months. However, the dosage and frequency of administration may vary depending on various conditions, and a lower dosage and/or frequency of administration than the aforementioned dosage and/or frequency may be sufficient, or a higher dosage and/or frequency of administration than the aforementioned dosage and/or frequency may be necessary. In addition, the therapeutic or prophylactic agent according to the present invention can be effective in a short period of administration or can be administered for a long period of time by reducing side effects.

The pharmaceutical composition of the present invention may be administered to any subject, examples of which include humans and non-human animals (e.g., dogs, cats, cows, horses, sheep, pigs, etc.). Preferred among these are humans.

[Treatment and prevention of diseases]

The pharmaceutical composition of the present invention makes it possible to treat or prevent various diseases by removing disease-related cells involved in various diseases through the action of the anti-gpNMB antibody of the present invention. The following are examples of treatment/prevention of some specific diseases. However, diseases that can be treated/prevented by the pharmaceutical composition of the present invention are not limited to these diseases, but can be applied to the treatment/prevention of various diseases as exemplified above.

* Treatment and prevention of obesity, diabetes, and arteriosclerosis:

The anti-gpNMB antibody of the present invention can be used to treat obesity, diabetes and atherosclerosis. Specifically, administration of the antibody of the present invention to wild-type mice or ApoE-KO mice fed a high-fat diet can reduce gpNMB-positive fat-laden macrophages and improve atherosclerosis. It can also improve insulin resistance, suppress weight gain, and reduce accumulated fat. It can also improve fatty liver.

* Treatment and prevention of fibrosis:

It is thought that gpNMB-positive macrophages in fibrosis pathological tissues induce increased collagen production or collagen-producing cells. Administration of the anti-gpNMB antibody of the present invention, which has activity to remove gpNMB-positive cells, to fibrosis model mice lead to removal of gpNMB-positive macrophages in tissues and improvement in the pathophysiology of fibrosis in various organs. Specifically, administration of an anti-gpNMB antibody to fibrosis models, such as bleomycin model, silica model, UUO renal fibrosis model, and liver fibrosis model, can inhibit fibrosis in organs. In addition, administration of the antibody of the present invention to DMD model animals can eliminate gpNMB-positive cells in skeletal muscle tissue, inhibit muscle fibrosis, and improve muscle quality.

* Treatment and prevention of cancer:

Administration of the antibody of the present invention eliminates immunosuppressive macrophages, microglia, dendritic cells, and myeloid-derived suppressor cells (MDSCs) that infiltrate cancer cells, thereby inhibiting cancer cell growth and improving the survival period and survival rate. In addition, pre-treatment administration of the present antibody or its combination with various conventional cancer therapies, such as anticancer agents, targeted cancer therapies, immune checkpoint inhibitors, and CAR cell therapy, can enhance the therapeutic effect of these therapies.
For example, administration of the antibody of the present invention to a mouse model of melanoma transplantation can eliminate macrophages infiltrating cancer cells, inhibit cancer cell proliferation, and improve the survival period and survival rate. Furthermore, the survival period and survival rate can be further improved by using the antibody of the present invention in combination with anticancer agents or immune checkpoint inhibitors.

* Treatment and prevention of glioblastoma:

Administration of the antibody of the present invention to a syngeneic mouse model of glioblastoma implanted in the brain can eliminate immunosuppressive microglia and macrophages that infiltrate the glioblastoma, inhibit its growth, and improve the survival period and survival rate. and survival rate. Furthermore, the combination of the antibody of the present invention with anti-cancer agents such as temozolomide and immune checkpoint inhibitors can further improve the survival period and survival rate.

* Treatment and prevention of age-related diseases:

The accumulation of senescent cells induces various diseases and accelerates the progression of these conditions. The anti-gpNMB antibody of the present invention makes it possible to specifically remove senescent cells, which are the main cause of these diseases, and to treat and inhibit the progression of various diseases, whereby the anti-gpNMB antibody of the present invention can be used as a senescent cell-removing drug with fewer side effects. For example, specifically, administration of the present antibody to a high-fat diet-loaded mouse model, which suffers from accelerated aging and accumulation of senescent cells, improves chronic inflammatory conditions and suppresses insulin resistance, fat accumulation, organ fibrosis and atherosclerosis. In the treatment of various other chronic inflammatory diseases, the therapeutic effect of the original drug can be enhanced by preadministration or concomitant use of the present antibody.

*Treatment of lysosomal stress diseases:

A certain therapeutic effect is expected by removing dysfunctional senescent cells in lysosomal stress diseases, which are caused by the accumulation of sugars, glycolipids, and proteins in lysosomes due to deficiency or reduced activity of various lysosomal enzymes and other factors, resulting in stress and reduced cell functions, which in turn causes pathological conditions. Specifically, administration of the present antibody to mice with reduced or deficient lysosomal enzyme activity is expected to remove senescent cells, relieve inflammation, and improve the disease condition.

[Others]

As mentioned above, the anti-gpNMB-specific antibody of the present invention has the activity to eliminate various disease-related cells when administered to humans and other subjects. Based on such activity, the anti-gpNMB-specific antibody of the present invention can be used for the treatment or prevention of various diseases. Thus, in addition to the pharmaceutical composition of the present invention described above, several other embodiments of the present invention can be mentioned, for example, as follows.
Specifically, an embodiment of the present invention relates to a method for eliminating various disease-related cells in a subject, comprising administering an effective amount of the anti-gpNMB-specific antibody of the present invention to the subject in need thereof. Another embodiment of the present invention relates to a method for treating or preventing various diseases by removing various disease-related cells in a subject, comprising administering an effective amount of the anti-gpNMB-specific antibody of the present invention to the subject in need thereof. The details of the method using such antibodies are described above.

Another embodiment of the present invention relates to an anti-gpNMB-specific antibody of the present invention for use in eliminating various disease-related cells in the subject. Another embodiment of the present invention relates to an anti-gpNMB-specific antibody of the present invention for use in treating or preventing various diseases by removing various disease-related cells in a subject. The details of the antibody of the present invention for use in these purposes are described above.

Another embodiment of the present invention relates to the use of an anti-gpNMB-specific antibody of the present invention in the manufacture of a medicament for eliminating various disease-related cells in the subject. Another embodiment of the present invention relates to the use of an anti-gpNMB-specific antibody of the present invention in the manufacture of a medicament for treating or preventing various diseases by removing various disease-related cells in a subject. The details of the antibody of the present invention for use in these purposes are described above.

In addition to the above, the anti-gpNMB-specific antibody of the present invention can be used for various other purposes. For example, the anti-gpNMB-specific antibody of the present invention can be used as an Immuno-PET probe for the imaging diagnosis of where and how many gpNMB-positive disease-related cells are present in the body. In addition, the anti-gpNMB-specific antibody of the present invention can be used alone or two or more in combination to measure specific gpNMB fragments for selecting the appropriate patient population for antibody treatment and determining therapeutic efficacy.

## EXAMPLES

The present invention will now be described in further detail by way of Examples. These examples are shown merely for convenience of the description, and should not be construed as limitations to the present invention in any sense.

[Common procedures]

*Antigen ELISA (Enzyme-Linked Immunosorbent Assay):

Recombinant soluble human or mouse gpNMB was diluted by PBS to 2 $\mu$g/mL, added to a 96-well plate (Nunc, MaxiSorp) at 50 $\mu$L/well, and allowed to stand overnight at 4°C. The 96-well plate was blocked with 3% BSA/PBS and used for ELISA as recombinant soluble gpNMB-fixed 96-well plate.

Hybridoma culture supernatant containing an anti-gpNMB antibody or 3% BSA/PBS solution was added at 30 $\mu$L/well to the above recombinant soluble gpNMB-fixed 96-well plate and allowed to react for 1 hour at room temperature. After washing with washing buffer (TBS-T: Tris Buffered Saline, 0.025% Tween™ 20), anti-mouse IgG antibody-ALP conjugate (SBA, 1050 -04), diluted 1000-fold in 3% BSA/PBS, was added at 30 $\mu$L/well and allowed to react for 1 hour at room temperature. Substrate (PNPP (p-nitrophenyl phosphate), 1 mg/mL) solution was added at 100 $\mu$L/well and allowed to react for 1 hour at room temperature, and absorbance at 405-550 nm was calculated. The absorbance values obtained were evaluated as binding activity.

*Cell-based ELISA:

The gpNMB-expressing cells were seeded into a polylysine-coated 96-well plate and allowed to adhere, and the culture supernatant was removed. The cells were then fixed with a 10% formalin-containing solution for 10 minutes at room temperature, and the supernatant was removed. The cells were then blocked with 3% BSA/PBS solution, and stored under refrigeration.

After the blocking solution was removed from the cell-fixed plate obtained above, and antibody-containing culture medium or solution was added at 30 $\mu$L/well, and incubated at room temperature for about 1 hour to 1 hour 30 minutes. The cells were washed 2 to 4 times with washing buffer (TBS-T), and a 1000-fold dilution of anti-mouse IgG-HRP labeled secondary antibody solution was added to the plate, and incubated at room temperature for 1 hour. The cells were washed 2 to 4 times with washing buffer (TBS-T), and substrate TMB solution was then added at 50 $\mu$L/well, and incubated at room temperature for 30 minutes. The reaction was then stopped by adding 1N sulfuric acid at 50 $\mu$L/well, and the absorbance at 450-650 nm was calculated. The absorbance value obtained was evaluated as binding activity.

*Antibody-dependent cellular phagocytosis (ADCP) assay:

AZAMI-GREEN fused mouse gpNMB-expressing cells or AZAMI-GREEN fused human gpNMB-expressing cells were seeded into 96-well plates (IWAKI, EZ-VIEW), suspended in medium containing the anti-gpNMB antibody in a total of 80 $\mu$L/well, and reacted for 2 hours at 37°C and 5% $CO_2$. 20 $\mu$L of phagocytic cells were added and reacted at 37°C, 5% $CO_2$ for 0 to 12 days. The fluorescence intensity of AZAMI-GREEN was measured under a fluorescence microscope, and the decrease in fluorescence was evaluated as the cell removal effect.

[Example 1] Production and screening of mouse or rat monoclonal antibodies (antigen ELISA and cell-based ELISA):

Mouse or rat monoclonal antibodies can be produced by the hybridoma method described in, e.g., Koehler et al., Nature, (1975), 256:495-497. Anti-gpNMB antibodies were produced by inducing immunity in mice or rats using nucleic acids, proteins, or peptides containing the full length or part of the amino acid sequence (SEQ ID NO 209) or the nucleic acid sequence (SEQ ID NO 210) of human gpNMB (isotype a), or the amino acid sequence (SEQ ID NO 211) or the nucleic acid sequence (SEQ ID NO 212) of mouse gpNMB, and collecting lymphocyte cells from the mice or rats with elevated antibody titer. All animal experiments were performed according to institutional regulations. Hybridoma generation by fusion of lymphocyte cells from mice or rats with mouse myeloma cell lines (P3U1 (P3X63Ag8U.1) or SP2/0 (SP2/0-Ag14)) was performed using standard hybridoma techniques. Hybridomas were selected using so-called HAT medium, which contains hypoxanthine, aminopterin, and thymidine. An amino acid sequence alignment of human gpNMB (isotype a) (SEQ ID NO 209) and mouse gpNMB (SEQ ID NO 211) is shown in Figure 1, in which the region spanning from valine at amino acid residue 78 (V78) to the PKD domain is shown enclosed by a single dotted line, the region at amino acid residues 256 to 319 is shown with dotted lines as an example of the PKD domain, and the region at amino acid residues 172 to 246 is shown with solid lines as an example of the PMEL-CAF-like domain.

The resulting hybridoma cultures were subjected to antigen ELISA with fixed recombinant human soluble gpNMB (SEQ ID NO 221) or mouse soluble gpNMB (SEQ ID NO 223) or cell-based ELISA with cells expressing proteins containing human gpNMB (SEQ ID NO 209) to evaluate binding ability, and the wells containing positive hybridomas were selected. Hybridomas in these wells were single-cloned by limiting dilution. The single-cloned positive hybridomas were cultured, and monoclonal antibodies were purified from the culture media using Protein A column (Ab-Capture, ProteNova) to obtain multiple anti-gpNMB antibodies.

[Example 2] Sequencing of the antibodies:

Each anti-gpNMB antibody clone obtained in Example 1 was subjected to the SMARTer(™) RACE method to determine the gene sequences of its light and heavy chains. RNA derived from the hybridoma producing the antibody was subjected to the SMARTer(™) RACE method to thereby obtain gene fragments of the heavy and light chains of the antibody, including the start and end codons, and their nucleotide sequences were determined. Specifically, the 1st strand cDNA was synthesized with the SMARTer(™) RACE 5'/3' Kit (634859, Clontech) using total RNA from the hybridoma as a template, and the cDNA was amplified by PCR reaction. Using the cDNA as a template, PCR reactions were performed using primers for the universal sequence provided in the kit and primers specific for each of the heavy and light chains of the antibody. The resulting PCR products were used as 5'RACE PCR products for TA cloning.

When sequencing a rat monoclonal antibody gene, PCR reactions were performed on the variable regions of the heavy and light chains of the rat antibody, and the resulting PCR fragments were bound to the mouse constant regions to produce a mouse chimeric antibody, which was then subjected to sequencing.

For TA cloning, the 5' RACE PCR products were electrophoresed, and cDNA fragments having desired molecular weights were purified using the QIAEX II Gel Extraction Kit (20021, Qiagen). The purified cDNA fragments were subjected to reaction using TaKaRa-Taq (R001A, Takara) at 72°C for 5 minutes to add adenine at the 3' end. The cDNA fragments were then cloned into the pMD20-T vector (hereafter referred to as MD20 vector) using the Mighty TA-cloning Kit (6028, Takara) according to the attached protocol. The MD20 vector in which the target cDNA fragments were cloned was transformed into E. coli TOP10, and cultured on agar medium containing 100 μg/mL ampicillin. Insertion of the target cDNA fragments into the MD20 vector was confirmed by colony PCR. The nucleotide sequences of the cloned cDNA fragments were determined. Similarly, the nucleotide sequences of the 3' RACE PCR products were determined, whereby the full-length sequence of the gene for each antibody was determined.

Of the anti-gpNMB antibody clones obtained in Example 1, GPN05-1, GPN06-1, GPN07-1, GPN09-1, GPN11-10, GPN15-2, GPN15-3, GPN18-1, GPN18-2, GPN18-4, GPN18-5, GPN18-6, GPN18-7, and 1-5E are shown in Tables 1-1 to 1-5, along with the Sequence Identification Nos. of the amino acid sequences and nucleotide sequences of their heavy chain and light chain variable regions (hereinafter also referred to as VH and VL, respectively) and the complementarity determining regions included therein (hereinafter also referred to as CDR-H1 to H3 and CDR-L1 to L3, respectively), as determined by the methods explained above.

[Table 1-1]

| Antibody clone | Description | Amino acid sequence | | Nucleic acid sequence | |
|---|---|---|---|---|---|
| GPN05-1 | CDR-H1 | SEQ ID NO | 1 | SEQ ID NO | 2 |
| | CDR-H2 | SEQ ID NO | 3 | SEQ ID NO | 4 |
| | CDR-H3 | SEQ ID NO | 5 | SEQ ID NO | 6 |
| | CDR-L1 | SEQ ID NO | 7 | SEQ ID NO | 8 |
| | CDR-L2 | SEQ ID NO | 9 | SEQ ID NO | 10 |
| | CDR-L3 | SEQ ID NO | 11 | SEQ ID NO | 12 |
| | Heavy chain variable region (VH) | SEQ ID NO | 13 | SEQ ID NO | 14 |
| | Light chain variable region (VL) | SEQ ID NO | 15 | SEQ ID NO | 16 |
| GPN06-1 | CDR-H1 | SEQ ID NO | 17 | SEQ ID NO | 18 |
| | CDR-H2 | SEQ ID NO | 19 | SEQ ID NO | 20 |
| | CDR-H3 | SEQ ID NO | 21 | SEQ ID NO | 22 |
| | CDR-L1 | SEQ ID NO | 23 | SEQ ID NO | 24 |
| | CDR-L2 | SEQ ID NO | 25 | SEQ ID NO | 26 |
| | CDR-L3 | SEQ ID NO | 27 | SEQ ID NO | 28 |
| | Heavy chain variable region (VH) | SEQ ID NO | 29 | SEQ ID NO | 30 |
| | Light chain variable region (VL) | SEQ ID NO | 31 | SEQ ID NO | 32 |
| GPN07-1 | CDR-H1 | SEQ ID NO | 33 | SEQ ID NO | 34 |
| | CDR-H2 | SEQ ID NO | 35 | SEQ ID NO | 36 |
| | CDR-H3 | SEQ ID NO | 37 | SEQ ID NO | 38 |
| | CDR-L1 | SEQ ID NO | 39 | SEQ ID NO | 40 |
| | CDR-L2 | SEQ ID NO | 41 | SEQ ID NO | 42 |
| | CDR-L3 | SEQ ID NO | 43 | SEQ ID NO | 44 |
| | Heavy chain variable region (VH) | SEQ ID NO | 45 | SEQ ID NO | 46 |
| | Light chain variable region (VL) | SEQ ID NO | 47 | SEQ ID NO | 48 |

[Table 1-2]

| Antibody clone | Description | Amino acid sequence | | Nucleic acid sequence | |
|---|---|---|---|---|---|
| GPN11-10 | CDR-H1 | SEQ ID NO | 49 | SEQ ID NO | 50 |
| | CDR-H2 | SEQ ID NO | 51 | SEQ ID NO | 52 |
| | CDR-H3 | SEQ ID NO | 53 | SEQ ID NO | 54 |
| | CDR-L1 | SEQ ID NO | 55 | SEQ ID NO | 56 |
| | CDR-L2 | SEQ ID NO | 57 | SEQ ID NO | 58 |
| | CDR-L3 | SEQ ID NO | 59 | SEQ ID NO | 60 |
| | Heavy chain variable region (VH) | SEQ ID NO | 61 | SEQ ID NO | 62 |
| | Light chain variable region (VL) | SEQ ID NO | 63 | SEQ ID NO | 64 |

(continued)

| Antibody clone | Description | Amino acid sequence | | Nucleic acid sequence | |
|---|---|---|---|---|---|
| GPN15-2 | CDR-H1 | SEQ ID NO | 65 | SEQ ID NO | 66 |
| | CDR-H2 | SEQ ID NO | 67 | SEQ ID NO | 68 |
| | CDR-H3 | SEQ ID NO | 69 | SEQ ID NO | 70 |
| | CDR-L1 | SEQ ID NO | 71 | SEQ ID NO | 72 |
| | CDR-L2 | SEQ ID NO | 73 | SEQ ID NO | 74 |
| | CDR-L3 | SEQ ID NO | 75 | SEQ ID NO | 76 |
| | Heavy chain variable region (VH) | SEQ ID NO | 77 | SEQ ID NO | 78 |
| | Light chain variable region (VL) | SEQ ID NO | 79 | SEQ ID NO | 80 |
| GPN15-3 | CDR-H1 | SEQ ID NO | 81 | SEQ ID NO | 82 |
| | CDR-H2 | SEQ ID NO | 83 | SEQ ID NO | 84 |
| | CDR-H3 | SEQ ID NO | 85 | SEQ ID NO | 86 |
| | CDR-L1 | SEQ ID NO | 87 | SEQ ID NO | 88 |
| | CDR-L2 | SEQ ID NO | 89 | SEQ ID NO | 90 |
| | CDR-L3 | SEQ ID NO | 91 | SEQ ID NO | 92 |
| | Heavy chain variable region (VH) | SEQ ID NO | 93 | SEQ ID NO | 94 |
| | Light chain variable region (VL) | SEQ ID NO | 95 | SEQ ID NO | 96 |

[Table 1-3]

| Antibody clone | Description | Amino acid sequence | | Nucleic acid sequence | |
|---|---|---|---|---|---|
| GPN18-4 | CDR-H1 | SEQ ID NO | 97 | SEQ ID NO | 98 |
| | CDR-H2 | SEQ ID NO | 99 | SEQ ID NO | 100 |
| | CDR-H3 | SEQ ID NO | 101 | SEQ ID NO | 102 |
| | CDR-L1 | SEQ ID NO | 103 | SEQ ID NO | 104 |
| | CDR-L2 | SEQ ID NO | 105 | SEQ ID NO | 106 |
| | CDR-L3 | SEQ ID NO | 107 | SEQ ID NO | 108 |
| | Heavy chain variable region (VH) | SEQ ID NO | 109 | SEQ ID NO | 110 |
| | Light chain variable region (VL) | SEQ ID NO | 111 | SEQ ID NO | 112 |
| GPN18-1 | CDR-H1 | SEQ ID NO | 113 | SEQ ID NO | 114 |
| | CDR-H2 | SEQ ID NO | 115 | SEQ ID NO | 116 |
| | CDR-H3 | SEQ ID NO | 117 | SEQ ID NO | 118 |
| | CDR-L1 | SEQ ID NO | 119 | SEQ ID NO | 120 |
| | CDR-L2 | SEQ ID NO | 121 | SEQ ID NO | 122 |
| | CDR-L3 | SEQ ID NO | 123 | SEQ ID NO | 124 |
| | Heavy chain variable region (VH) | SEQ ID NO | 125 | SEQ ID NO | 126 |
| | Light chain variable region (VL) | SEQ ID NO | 127 | SEQ ID NO | 128 |

(continued)

| Antibody clone | Description | Amino acid sequence | | Nucleic acid sequence | |
|---|---|---|---|---|---|
| GPN18-2 | CDR-H1 | SEQ ID NO | 129 | SEQ ID NO | 130 |
| | CDR-H2 | SEQ ID NO | 131 | SEQ ID NO | 132 |
| | CDR-H3 | SEQ ID NO | 133 | SEQ ID NO | 134 |
| | CDR-L1 | SEQ ID NO | 135 | SEQ ID NO | 136 |
| | CDR-L2 | SEQ ID NO | 137 | SEQ ID NO | 138 |
| | CDR-L3 | SEQ ID NO | 139 | SEQ ID NO | 140 |
| | Heavy chain variable region (VH) | SEQ ID NO | 141 | SEQ ID NO | 142 |
| | Light chain variable region (VL) | SEQ ID NO | 143 | SEQ ID NO | 144 |

[Table 1-4]

| Antibody clone | Description | Amino acid sequence | | Nucleic acid sequence | |
|---|---|---|---|---|---|
| GPN18-6 | CDR-H1 | SEQ ID NO | 145 | SEQ ID NO | 146 |
| | CDR-H2 | SEQ ID NO | 147 | SEQ ID NO | 148 |
| | CDR-H3 | SEQ ID NO | 149 | SEQ ID NO | 150 |
| | CDR-L1 | SEQ ID NO | 151 | SEQ ID NO | 152 |
| | CDR-L2 | SEQ ID NO | 153 | SEQ ID NO | 154 |
| | CDR-L3 | SEQ ID NO | 155 | SEQ ID NO | 156 |
| | Heavy chain variable region (VH) | SEQ ID NO | 157 | SEQ ID NO | 158 |
| | Light chain variable region (VL) | SEQ ID NO | 159 | SEQ ID NO | 160 |
| GPN18-7 | CDR-H1 | SEQ ID NO | 161 | SEQ ID NO | 162 |
| | CDR-H2 | SEQ ID NO | 163 | SEQ ID NO | 164 |
| | CDR-H3 | SEQ ID NO | 165 | SEQ ID NO | 166 |
| | CDR-L1 | SEQ ID NO | 167 | SEQ ID NO | 168 |
| | CDR-L2 | SEQ ID NO | 169 | SEQ ID NO | 170 |
| | CDR-L3 | SEQ ID NO | 171 | SEQ ID NO | 172 |
| | Heavy chain variable region (VH) | SEQ ID NO | 173 | SEQ ID NO | 174 |
| | Light chain variable region (VL) | SEQ ID NO | 175 | SEQ ID NO | 176 |
| GPN18-5 | CDR-H1 | SEQ ID NO | 177 | SEQ ID NO | 178 |
| | CDR-H2 | SEQ ID NO | 179 | SEQ ID NO | 180 |
| | CDR-H3 | SEQ ID NO | 181 | SEQ ID NO | 182 |
| | CDR-L1 | SEQ ID NO | 183 | SEQ ID NO | 184 |
| | CDR-L2 | SEQ ID NO | 185 | SEQ ID NO | 186 |
| | CDR-L3 | SEQ ID NO | 187 | SEQ ID NO | 188 |
| | Heavy chain variable region (VH) | SEQ ID NO | 189 | SEQ ID NO | 190 |
| | Light chain variable region (VL) | SEQ ID NO | 191 | SEQ ID NO | 192 |

[Table 1-5]

| Antibody clone | Description | Amino acid sequence | | Nucleic acid sequence | |
|---|---|---|---|---|---|
| 1-5E | CDR-H1 | SEQ ID NO | 193 | SEQ ID NO | 194 |
| | CDR-H2 | SEQ ID NO | 195 | SEQ ID NO | 196 |
| | CDR-H3 | SEQ ID NO | 197 | SEQ ID NO | 198 |
| | CDR-L1 | SEQ ID NO | 199 | SEQ ID NO | 200 |
| | CDR-L2 | SEQ ID NO | 201 | SEQ ID NO | 202 |
| | CDR-L3 | SEQ ID NO | 203 | SEQ ID NO | 204 |
| | Heavy chain variable region (VH) | SEQ ID NO | 205 | SEQ ID NO | 206 |
| | Light chain variable region (VL) | SEQ ID NO | 207 | SEQ ID NO | 208 |
| GPN09-1 | CDR-H1 | SEQ ID NO | 255 | SEQ ID NO | 256 |
| | CDR-H2 | SEQ ID NO | 257 | SEQ ID NO | 258 |
| | CDR-H3 | SEQ ID NO | 259 | SEQ ID NO | 260 |
| | CDR-L1 | SEQ ID NO | 261 | SEQ ID NO | 262 |
| | CDR-L2 | SEQ ID NO | 263 | SEQ ID NO | 264 |
| | CDR-L3 | SEQ ID NO | 265 | SEQ ID NO | 266 |
| | Heavy chain variable region (VH) | SEQ ID NO | 267 | SEQ ID NO | 268 |
| | Light chain variable region (VL) | SEQ ID NO | 269 | SEQ ID NO | 270 |

[Example 3] Analysis of the binding sites of the anti-gpNMB antibodies (antigen ELISA):

To analyze the binding sites of each anti-gpNMB antibody obtained in Example 1 to the human gpNMB protein, the following experiment was performed.

First, based on the amino acid sequence of the human gpNMB extracellular domain (SEQ ID NO 215, in which the amino acid residues at positions 1 to 21 constitute a signal peptide, while the amino acid residues at positions 22 and onward constitute an isolated peptide of the human gpNMB extracellular domain), an N-terminal deletion variant (hgpNMB _d07_76-498) and C-terminal deletion variants (hgpNMB _d03_1-251, hgpNMB_d04_1-321, hgpNMB_d05_1-375, hgpNMB_d06_1-418, hgpNMB_d13_1-412, hgpNMB_d14_1-406, hgpNMB_d15_1-400, hgpNMB_d16_1-394, hgpNMB_d17_1-388, hgpNMB_d18_1-382, and hgpNMB_d19_1-234) of the human gpNMB extracellular domain were produced as shown in Table 2 below. Each purified gpNMB protein was obtained by constructing a transient animal cell expression vector into which each gpNMB gene had been inserted, and having it expressed in a medium using the Expi293 Expression System (A14635, Thermo Fisher Scientific). The expressed protein was then purified based on affinity with Ni-NTA agarose (143-09763, FUJIFILM Wako) using a histidine tag added to the C-terminus to thereby obtain the purified protein.

[Table 2]

| Table 2: N-terminal or C-terminal deletion variants of human gpNMB extracellular domain | |
|---|---|
| Variant No. | Description |
| hgpNMB_d03_1-251 | C-terminal deletion variant consisting of 1 to 251 of hgpNMB |
| hgpNMB_d04_1-321 | C-terminal deletion variant consisting of 1 to 321 of hgpNMB |
| hgpNMB_d05_1-375 | C-terminal deletion variant consisting of 1 to 375 of hgpNMB |
| hgpNMB_d06_1-418 | C-terminal deletion variant consisting of 1 to 418 of hgpNMB |
| hgpNMB_d07_76-498 | N-terminal deletion variant consisting of 76 to 498 of hgpNMB |
| hgpNMB_d13_1-412 | C-terminal deletion variant consisting of 1 to 412 of hgpNMB |
| hgpNMB_d14_1-406 | C-terminal deletion variant consisting of 1 to 406 of hgpNMB |
| hgpNMB_d15_1-400 | C-terminal deletion variant consisting of 1 to 400 of hgpNMB |

(continued)

| Table 2: N-terminal or C-terminal deletion variants of human gpNMB extracellular domain | |
|---|---|
| Variant No. | Description |
| hgpNMB_d16_1-394 | C-terminal deletion variant consisting of 1 to 394 of hgpNMB |
| hgpNMB_d17_1-388 | C-terminal deletion variant consisting of 1 to 388 of hgpNMB |
| hgpNMB_d18_1-382 | C-terminal deletion variant consisting of 1 to 382 of hgpNMB |
| hgpNMB_d19_1-234 | C-terminal deletion variant consisting of 1 to 234 of hgpNMB |

Next, 17 nmol/L of each of purified recombinant human soluble gpNMB (SEQ ID NO 221), the N-terminal deletion variant (hgpNMB_d07_76-498) and the C-terminal deletion variants (hgpNMB_d03_1-251, hgpNMB_d04_1-321, hgpNMB_d05_1-375, hgpNMB_d06_1-418, hgpNMB_d13_1-412, hgpNMB_d14_1-406, hgpNMB_d15_1-400, hgpNMB_d16_1-394, hgpNMB_d17_1-388, hgpNMB_d18_1-382, and hgpNMB_d19_1-234) of the human gpNMB extracellular domain shown in Table 2 above, and purified recombinant mouse soluble gpNMB (SEQ ID NO 223) was added to a 96-well plate for ELISA at 100 μL/well, and allowed to stand at room temperature for 90 minutes. The plate was then blocked with 3% BSA/PBS to thereby obtain an antigen ELISA plate.

After the blocking solution was removed from the antigen ELISA plate, a diluent of the anti-gpNMB antibody (3% BSA/PBS solution) was added at 50 μL/well, and allowed to react for 1 hour at room temperature. Then, after washing with washing buffer, an antibody that specifically reacts with mouse IgG, anti-mouse IgG antibody-ALP conjugate (SBA, 1050-04), was added at 50 μL/well and allowed to react for 1 hour at room temperature. Substrate (PNPP) was added at 100 μL/well and allowed to react for 1 hour at room temperature, and the absorbance at 405 to 550 nm was measured and evaluated as binding activity.

[Example 4] Determination of the epitopes of the anti-gpNMB antibodies (antigen ELISA):

To determine the epitope of each anti-gpNMB antibody obtained in Example 1 in the human gpNMB protein, the following experiment was performed.

First, a C-terminal deletion variant (hgpNMB_d19_1-234) and point mutants (hgpNMB_d22_K245A, hgpNMB_d23_D252A, hgpNMB_d24_E253A, hgpNMB_d25_D264A, hgpNMB_d26_H272A, hgpNMB_d28_H297A, hgpNMB_d30_H376A, hgpNMBd31_D247A_R248A, hgpNMB_d34_D287A, hgpNMB_d35_H301A, hgpNMB_d36_R331A_K334A, hgpNMB_d37_K344A_D347A, hgpNMB_d38_D356A, hgpNMB_d39_E360A, hgpNMB_d40_E367A, and hgpNMB_d41_R373A) of human gpNMB extracellular domain as shown in Table 3 below were produced. Each purified gpNMB protein was obtained by constructing a transient animal cell expression vector into which each gpNMB gene had been inserted, and having it expressed in a medium using the Expi293 Expression System (A14635, Thermo Fisher Scientific). The expressed protein was then purified based on affinity with Ni-NTA agarose (143-09763, FUJIFILM Wako) using a histidine tag added to the C-terminus to thereby obtain the purified protein. The C-terminal deletion variant and the point mutants of human gpNMB extracellular domain are summarized in Table 3.

[Table 3]

| Table 3: C-terminal deletion variants and point mutants of human gpNMB extracellular domain | |
|---|---|
| Variant No. | Description |
| hgpNMB_d19_1-234 | C-terminal deletion variant consisting of 1 to 234 of hgpNMB |
| hgpNMB_d22_K245A | K245A point mutant of hgpNMB |
| hgpNMB_d23_D252A | D252A point mutant of hgpNMB |
| hgpNMB_d24_E253A | E253A point mutant of hgpNMB |
| hgpNMB_d25_D264A | D264A point mutant of hgpNMB |
| hgpNMB_d26_H272A | H272A point mutant of hgpNMB |
| hgpNMB_d28_H297A | H297A point mutant of hgpNMB |
| hgpNMB_d30_H376A | H376A point mutant of hgpNMB |
| hgpNMBd31_D247A_R248A | D247A and R248A point mutant of hgpNMB |
| hgpNMB_d34_D287A | D287A point mutant of hgpNMB |

(continued)

| Table 3: C-terminal deletion variants and point mutants of human gpNMB extracellular domain | |
|---|---|
| Variant No. | Description |
| hgpNMB_d35_H301A | H301A point mutant of hgpNMB |
| hgpNMB_d36_R331A_K334A | R331A and K334A point mutant of hgpNMB |
| hgpNMB_d37_K344A_D347A | K344A and D347A point mutant of hgpNMB |
| hgpNMB_d38_D356A | D356A point mutant of hgpNMB |
| hgpNMB_d39_E360A | E360A point mutant of hgpNMB |
| hgpNMB_d40_E367A | E367A point mutant of hgpNMB |
| hgpNMB_d41_R373A | R373A point mutant of hgpNMB |

These alanine point mutants of human gpNMB extracellular domain were subjected to antigen ELISA analysis using the same procedure explained in Example 4 to evaluate the binding ability of each anti-gpNMB antibody. Specifically, each of the C-terminal deletion variant and the alanine point mutants of human gpNMB extracellular domain shown in Table 2 above was added to a 96-well plate for ELISA at a concentration of 17 nmol/L at 100 μL/well and allowed to stand at room temperature for 90 minutes. The antigen ELISA plate was then blocked with 3% BSA/PBS to obtain an antigen ELISA plate.

After the blocking solution was removed from the antigen ELISA plate, a diluent of the anti-gpNMB antibody (3% BSA/PBS solution) was added at 50 μL/well and allowed to react for 1 hour at room temperature. Then, after washing with washing buffer, an antibody that specifically reacts with mouse IgG, i.e., an anti-mouse IgG antibody-ALP conjugate (SBA, 1050-04), was added at 50 μL/well and allowed to react for 1 hour at room temperature. Substrate (PNPP) was added at 100 μL/well and reacted at room temperature for 1 hour, and the absorbance at 405 to 550 nm was calculated and evaluated as binding activity. Compared to the mutant that showed the maximum absorbance at 2 nM concentration, mutants that showed a decrease in absorbance of 50% or more are marked as +, and those that showed an absorbance of 50% or more are marked as -. The results are shown in Table 4.

[Table 4]

| Table 4: Results of ELISA binding analysis of each anti-gpNMB antibody clone to human gpNMB extracellular domain variants | | | | | | | | |
|---|---|---|---|---|---|---|---|---|
| | GPN 05-1 | GPN 06-1 | GPN 07-1 | GPN 09-1 | GPN 11-10 | GPN 15-2 | GPN 15-3 | GPN 18-4 |
| 22-234_N terminal | - | - | - | - | - | - | - | - |
| K245A | - | - | - | - | - | - | - | - |
| D252A | - | - | - | - | - | - | - | - |
| E253A | - | - | - | - | - | - | - | - |
| K257A,D258A | - | - | - | + | + | - | - | - |
| D264A | - | - | - | - | - | - | - | - |
| H268A,D269A | - | - | + | - | - | - | - | - |
| H272A | - | - | - | - | - | - | - | - |
| K282A | - | + | + | - | - | - | - | - |
| H297A | - | - | - | - | - | - | - | - |
| H376A | - | - | - | - | - | - | - | - |
| D247A,R248A | - | - | - | - | - | - | - | - |
| **D287A** | + | + | + | + | + | + | + | + |
| **H301A** | + | + | + | + | + | + | + | + |
| K316A | - | + | + | - | - | - | - | - |

(continued)

| Table 4: Results of ELISA binding analysis of each anti-gpNMB antibody clone to human gpNMB extracellular domain variants | | | | | | | | |
|---|---|---|---|---|---|---|---|---|
| | GPN 05-1 | GPN 06-1 | GPN 07-1 | GPN 09-1 | GPN 11-10 | GPN 15-2 | GPN 15-3 | GPN 18-4 |
| R331A,K334A | - | - | - | - | - | - | - | - |
| K344A,D347A | - | - | - | - | - | - | - | - |
| D356A | - | - | - | - | - | - | - | - |
| E360A | - | - | - | - | - | - | - | - |
| E367A | - | - | - | - | - | - | - | - |
| R373A | - | - | - | - | - | - | - | - |
| E385A | - | - | - | + | + | - | - | - |

In addition, the anti-gpNMB antibodies that were bound to a C-terminal deletion mutant of the human gpNMB extracellular domain (hgpNMB_d19_1-234) were subjected to analysis for binding ability to alanine point mutants of N-terminal human gpNMB extracellular domain using antigen ELISA in a similar manner.

First, a C-terminal deletion variant (hgpNMB _d19_1-234) and point mutants (hgpNMB_d19_1-234, hgpNMB_d43_1-234_R77A, hgpNMB_d44_1-234_D85A, hgpNMB_d45_1-234_R104A, hgpNMB_d47_1-234_E117A,K118A, hgpNMB_d48_1-234_R121A,E123A, hgpNMB_d49_1-234_D129A, hgpNMB_d50_1-234_E140A,D141A, hgpNMB_d51_1-234_D143A,E145A, hgpNMB_d52_1-234_H152A,H153A, hgpNMB_d53_1-234_D158A,K160A, hgpNMB_d54_1-234_H164A,H165A, hgpNMB_d55_1-234_R169A, hgpNMB_d58_1-234_R189A, hgpNMB_d59_1-234_R193A, hgpNMB_d61_1-234_R214A,R215A, and hgpNMB_d64_1-234_D109A) of human gpNMB extracellular domain shown in Table 5 below were generated. Each purified gpNMB protein was obtained by constructing a transient animal cell expression vector into which each gpNMB gene had been inserted, and having it expressed in a medium using the Expi293 Expression System (A14635, Thermo Fisher Scientific). The expressed protein was then purified based on affinity with Ni-NTA agarose (143-09763, FUJIFILM Wako) using a histidine tag added to the C-terminus to thereby obtain the purified protein. The C-terminal deletion variant and the point mutants of human gpNMB extracellular domain are summarized in Table 5.

[Table 5]

| Table 5: C-terminal deletion variant and point mutants of human gpNMB extracellular domain | |
|---|---|
| Variant No. | Description |
| hgpNMB_d19_1-234 | C-terminal deletion variant consisting of 1 to 234 of hgpNMB |
| hgpNMB_d43_1-234_R77A | R77A point mutant of 1 to 234 of hgpNMB |
| hgpNMB_d44_1-234_D85A | D85A point mutant of 1 to 234 of hgpNMB |
| hgpNMB_d45_1-234_R104A | R104A point mutant of 1 to 234 of hgpNMB |
| hgpNMB_d47_1-234_E117A,K118A | E117A and K118A point mutant of 1 to 234 of hgpNMB |
| hgpNMB_d48_1-234_R121A,E123A | R121A and E123A point mutant of 1 to 234 of hgpNMB |
| hgpNMB_d49_1-234_D129A | D129A point mutant of 1 to 234 of hgpNMB |
| hgpNMB_d50_1-234_E140A,D141A | E140A and D141A point mutant of 1 to 234 of hgpNMB |
| hgpNMB_d51_1-234_D143A,E145A | D143A and E145A point mutant of 1 to 234 of hgpNMB |
| hgpNMB_d52_1-234_H152A,H153A | H152A and H153A point mutant of 1 to 234 of hgpNMB |
| hgpNMB_d53_1-234_D158A,K160A | D158A and K160A point mutant of 1 to 234 of hgpNMB |
| hgpNMB_d54_1-234_H164A,H165A | H164A and H165A point mutant of 1 to 234 of hgpNMB |
| hgpNMB_d55_1-234_R169A | R169A point mutant of 1 to 234 of hgpNMB |
| hgpNMB_d58_1-234_R189A | R189A point mutant of 1 to 234 of hgpNMB |
| hgpNMB_d59_1-234_R193A | R193A point mutant of 1 to 234 of hgpNMB |

(continued)

| Table 5: C-terminal deletion variant and point mutants of human gpNMB extracellular domain | |
|---|---|
| Variant No. | Description |
| hgpNMB_d61_1-234_R214A,R215A | R214A and R215A point mutant of 1 to 234 of hgpNMB |
| hgpNMB_d64_1-234_D109A | D109A point mutant of 1 to 234 of hgpNMB |

These alanine point mutants of human gpNMB extracellular domain were subjected to antigen ELISA analysis using the same procedure explained in Example 4 to evaluate the binding ability of each anti-gpNMB antibody. Specifically, each of the C-terminal deletion variant and the alanine point mutants of human gpNMB extracellular domain shown in Table 5 above was added to a 96-well plate for ELISA at a concentration of 17 nmol/L at 100 μL/well and allowed to stand at room temperature for 90 minutes. The antigen ELISA plate was then blocked with 3% BSA/PBS to obtain an antigen ELISA plate.

After the blocking solution was removed from the antigen ELISA plate, a diluent of the anti-gpNMB antibody (3% BSA/PBS solution) was added at 50 μL/well and allowed to react for 1 hour at room temperature. Then, after washing with washing buffer, an antibody that specifically reacts with mouse IgG, i.e., an anti-mouse IgG antibody-ALP conjugate (SBA, 1050-04), was added at 50 μL/well and allowed to react for 1 hour at room temperature. Substrate (PNPP) was added at 100 μL/well and reacted at room temperature for 1 hour, and the absorbance at 405 to 550 nm was calculated and evaluated as binding activity. The results are shown in Table 6 (As mentioned above, in the amino acid sequence shown in SEQ ID NO 215, the amino acid residues at positions 1 to 21 constitute a signal peptide, while the amino acid residues at positions 22 and onward constitute an isolated peptide of the human gpNMB extracellular domain. Accordingly, each mutant protein shown in Table 6 starts at amino acid residue at position 22.). The binding activity to the C-terminal deletion mutant (hgpNMB_d19_22-234) is set as 100%, mutants that showed an absorbance of 50% or less are marked as +, and those that showed an absorbance of 50% or more are marked as -.

[Table 6]

| Table 6: Results of ELISA binding analysis of each anti-gpNMB antibody clone to human gpNMB extracellular domain variants | | | | | |
|---|---|---|---|---|---|
| gpNMB variant protein | GPN 18-1 | GPN 18-2 | GPN 18-5 | GPN 18-6 | GPN 18-7 |
| 22-234_N terminal | - | - | - | - | - |
| 22-234_R77A | - | - | - | - | - |
| 22-234_D85A | - | - | + | - | - |
| 22-234_R104A | - | - | - | - | - |
| 22-234_D109A | - | - | - | - | - |
| 22-234_E117A,K118A | - | - | - | - | - |
| 22-234_R121A,E123A | - | - | - | - | - |
| 22-234_D129A | - | - | - | - | - |
| 22-234_E140A,D141A | - | - | - | - | - |
| 22-234_D143A,E145A | - | - | - | - | - |
| 22-234_H152A,H153A | - | - | - | - | - |
| 22-234_D158A,K160A | - | - | - | - | - |
| 22-234_H164A,H165A | - | - | - | - | - |
| 22-234_R169A | - | - | - | - | - |
| 22-234_K186A | - | - | - | + | - |
| 22-234_R189A | - | - | - | - | - |
| 22-234_R193A | - | - | - | - | - |
| 22-234_R214A,R215A | + | + | - | + | + |
| 22-234_H216A, R218A | + | + | - | - | + |

The epitope of each anti-gpNMB antibody was determined by considering the stability of the purified gpNMB mutant and the mutation sites that affected the binding.

The results showed that antibody clones GPN18-1, GPN18-2, GPN18-6, and GPN18-7 interact with amino acids present in the PMEL-CAF-like region (R214 and/or R215). On the other hand, antibody clone GPN18-5 was found to further recognize the N-terminal portion. Antibody clone 1-5E is an anti-gpNMB antibody obtained by immunization with the vaccine peptide identified in a previous report (Non-Patent Literature 36), and the epitope recognized by this antibody is "RRGDGRWKD" at amino acid residues 63 to 71 at the N-terminus.

[Example 5] Production of recombinant anti-gpNMB antibodies:

Plasmids were prepared by adding a gene sequence encoding a signal sequence to the 5' end of each of a gene sequence encoding the H chain and a gene sequence encoding the L chain of the anti-gpNMB antibody obtained in Example 2, and inserting each of the gene sequences into the animal cell expression vector pcDNA3.4. The prepared plasmids were then transiently expressed using the ExpiCHO Expression System (A29133, Thermo Fisher Scientific) such that the antibodies were secreted into the medium. The culture supernatant was then collected and purified using a Protein A column (Ab-Capture, Protenova) and a gel filtration column (Superdex 200 Increase, Cytiva).

[Example 6] Interaction analysis of the anti-gpNMB antibodies by surface plasmon resonance method - 1

To compare the binding properties (binding and dissociation kinetics) of each anti-gpNMB antibody clone obtained in Example 1 to gpNMB, surface plasmon resonance (SPR) measurement was performed. Specifically, the following conditions were used.

The BIACORE T200 system was used as the measurement system. An anti-His tag monoclonal antibody was fixed at around 5000 RU in all flow cells of the sensor chip CM5 (BR-1005-30, Cytiva) with the Amine Coupling Kit (BR-1000-50, GE) and His Capture Kit (28 -9950-56, Cytiva). The running buffer used was HBS-EP+ (BR-1006-69, Cytiva).

Recombinant human gpNMB-FLAG-His (SEQ ID NO 221) or recombinant mouse gpNMB-FLAG-His (SEQ ID NO 223) was captured in the measurement system and used as the ligand. Each anti-gpNMB antibody clone was used at a concentration of 100 nmol/L as an analyte. Shiga toxin 2 (Shiga toxin 2) antibody (11E10) or control mouse IgG1 (leinco: Pro# M1411) was used as an analyte negative control.

The temperature of the measurement system was set at 25°C. As the ligand, recombinant human gpNMB-FLAG-His was reacted with the anti-His monoclonal antibody in the flow cell (2) so as to achieve 100 RU or lower, and recombinant mouse gpNMB-FLAG-His was reacted with the anti-His monoclonal antibody in the flow cell (4) so as to achieve 100 RU or lower. The flow rate was set to 20 μL/min, and 10 nmol/L of purified mouse IgG2a, κ, Isotype Ctrl, Clone: MG2a-53 (401502, BioLegend, hereinafter ctrl IgG2a) was reacted for 1 min, and HBS-EP+ was flowed for at least 10 minutes. The analyte was diluted with HBS-EP+ (100 nmol/L) and reacted with all flow cells for 600 seconds each to obtain a binding curve, and then reacted with HBS-EP+ for 600 seconds to obtain a dissociation curve.

After the reaction, regeneration buffer 1 (0.2% SDS), regeneration buffer 2 (100 mmol/L Tris-HCl (pH 8.5), 1 mol/L NaCl, 15 mmol/L MgCl$_2$), and regeneration buffer 3 (10 mmol/L glycine -HCl (pH 1.5)) were reacted for 1 minute each to remove and wash gpNMB-FLAG-His in the measurement system. Biacore T200 Evaluation software (ver. 2.0) was used to analyze the data with the 1:1 Binding Model, and the binding rate value (Ass: 1/Ms), binding stability value (Diss: 1/s) and equilibrium dissociation value (Diss/Ass: M) were quantified as ordinal numbers. The results are shown in Table 7.

[Table 7]

| Table 7: Results of SPR interaction analysis of each anti-gpNMB antibody clone against human gpNMB and mouse gpNMB - 1 | | | | | | | |
|---|---|---|---|---|---|---|---|
| Antibody clone | | Human gpNMB | | | Mouse gpNMB | | |
| Sample | Recognized domain | Ass. (1/Ms) | Diss (1/s) | Diss/Ass (M) | Ass (1/Ms) | Diss (1/s) | Diss/Ass (M) |
| GPN05-1 | PKD | 4.8.E+04 | 1.6.E-04 | 3.3.E-09 | 6.1.E+04 | 1.3.E-03 | 2.0.E-08 |
| GPN06-1 | PKD | 3.4.E+05 | 4.9.E-04 | 1.4.E-09 | 1.5.E+05 | 1.8.E-03 | 1.2.E-08 |
| GPN09-1 | PKD | - | - | - | 1.6.E+05 | 4.2.E-04 | 2.6.E-09 |
| GPN11-10 | PKD | 8.1.E+04 | 1.0.E-03 | 1.3.E-08 | 7.5.E+04 | 3.7.E-04 | 4.9.E-09 |
| GPN15-2 | PKD | 1.7.E+05 | 6.0.E-07 | 3.5.E-12 | 1.4.E+05 | 2.8.E-04 | 2.0.E-09 |
| GPN15-3 | PKD | 1.3.E+05 | 3.7.E-04 | 2.9.E-09 | 1.3.E+05 | 3.6.E-04 | 2.9.E-09 |

(continued)

| Table 7: Results of SPR interaction analysis of each anti-gpNMB antibody clone against human gpNMB and mouse gpNMB - 1 | | | | | | | |
|---|---|---|---|---|---|---|---|
| Antibody clone | | Human gpNMB | | | Mouse gpNMB | | |
| Sample | Recognized domain | Ass. (1/Ms) | Diss (1/s) | Diss/Ass (M) | Ass (1/Ms) | Diss (1/s) | Diss/Ass (M) |
| GPN18-4 | PKD | 4.7.E+04 | 1.5.E-05 | 3.2.E-10 | 3.0.E+04 | 1.2.E-04 | 3.8.E-09 |
| GPN18-1 | PMEL -CAF-like | 3.8.E+04 | 5.4.E-04 | 1.4.E-08 | 3.8.E+04 | 9.1.E-04 | 2.4.E-08 |
| GPN18-2 | PMEL -CAF-like | 3.1.E+04 | 6.8.E-05 | 2.2.E-09 | 2.4.E+04 | 7.9.E-05 | 3.3.E-09 |
| GPN18-6 | PMEL -CAF-like | 2.7.E+04 | 6.5.E-04 | 2.4.E-08 | 3.2.E+04 | 9.8.E-04 | 3.1.E-08 |
| GPN18-7 | PMEL -CAF-like | 2.6.E+04 | 5.9.E-04 | 2.3.E-08 | 2.0.E+04 | 9.9.E-04 | 4.9.E-08 |
| GPN18-5 | N-frg. | 1.5.E+05 | 3.0.E-04 | 2.0.E-09 | 9.1.E+04 | 7.0.E-04 | 7.7.E-09 |
| 1-5E | N-terminal vaccine | - | - | - | 6.7.E+05 | 6.1.E-03 | 9.1.E-09 |

[Example 7(1)] Evaluation of the ability to remove human gpNMB-expressing cells (cell removal inducing activity) by addition of anti-gpNMB antibody:

The ability of anti-gpNMB antibody clones obtained in Example 1 (GPN15-2, GPN15-3, GPN18-2, and GPN18-5) to remove human gpNMB-expressing cells were evaluated. Specifically, AZAMI-GREEN fused human gpNMB-expressing cells were seeded into 96-well plates (IWAKI, EZ-VIEW) at $7 \times 10^5$ cells/well, suspended in medium containing the anti-gpNMB antibody in a total of 80 $\mu$L/well, and reacted for 2 hours at 37°C and 5% $CO_2$. 20 $\mu$L of phagocytic cells were added at $8 \times 10^3$ cells/well and reacted at 37°C, 5% $CO_2$ for 6 to 7 days. The fluorescence intensity (a.u. = arbitrary unit) of AZAMI-GREEN was measured under a fluorescence microscope, and the decrease in fluorescence was evaluated as the cell removal effect. The results were compared with the non-Azami-Green fused human gpNMB-expressing cell group (Non) and the control mouse IgG-added group (NC IgG).

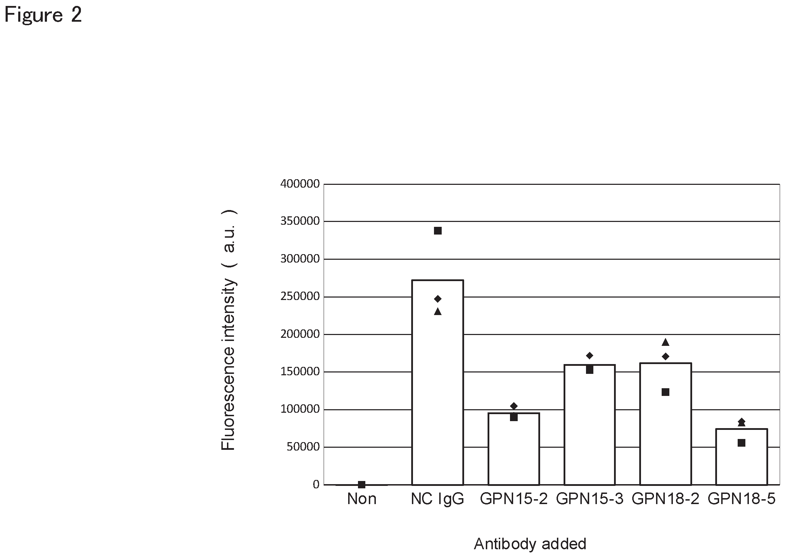

The results obtained are shown in the graph of Figure 2. These results indicate that antibodies GPN15-2 and GPN15-3, which recognize the PKD region, antibody GPN18-2, which recognizes the PMEL-CAF-like region, and antibody GPN18-5, which recognizes the region from V78 to S92, all exhibited activity to induce removal of human gpNMB-expressing cells.

[Example 7(2)] Evaluation of the ability to remove mouse gpNMB-expressing cells (cell removal inducing activity) by addition of anti-gpNMB antibody:

The ability of two anti-gpNMB antibody clones obtained in Example 1 (GPN18-5 and 1-5E), which recognize the N-terminal region, to remove mouse gpNMB-expressing cells were evaluated. Specifically, AZAMI-GREEN fused mouse gpNMB-expressing cells were seeded into 96-well plates (IWAKI, EZ-VIEW) at $7 \times 10^5$ cells/well, suspended in medium containing the anti-gpNMB antibody in a total of 80 $\mu$L/well, and reacted for 2 hours at 37°C and 5% $CO_2$. 20 $\mu$L of phagocytic cells were added at $8 \times 10^3$ cells/well and reacted at 37°C, 5% $CO_2$ for 6 to 7 days. The fluorescence intensity (a.u. = arbitrary unit) of AZAMI-GREEN was measured under a fluorescence microscope, and the decrease in fluorescence was evaluated as the cell removal effect. The results were compared with the control mouse IgG-added group (NC IgG).

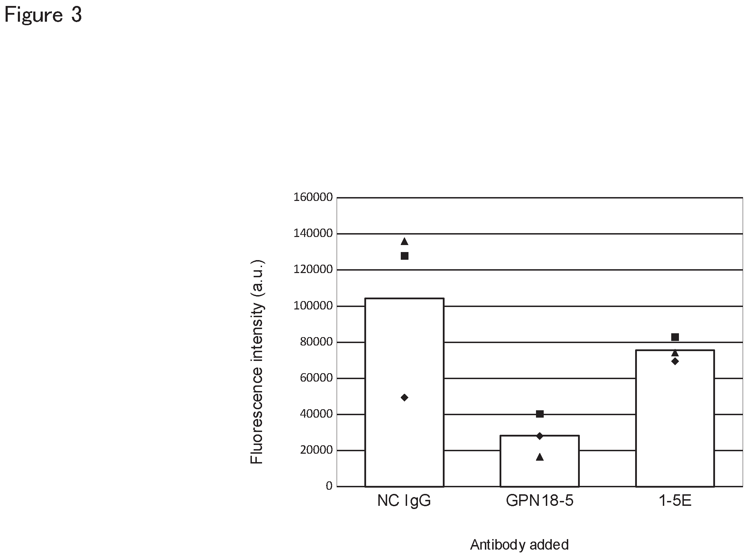

The results obtained are shown in the graph of Figure 3. These results indicate that of the two anti-gpNMB antibodies that recognize the N-terminal region, antibody GPN18-5, which recognizes the region from V78 to S92, exhibited stronger activity to induce removal of mouse gpNMB-expressing cells compared to antibody 1-5E, which is a vaccine peptide antibody.

[Example 8] Humanization design of mouse anti-gpNMB antibody: GPN06-1

The amino acid sequence of the anti-gpNMB antibody GPN06-1 was analyzed for identifying its CDR regions, in view of IMGT (Lefranc, M.-P., Pommié, C., Ruiz, M., Giudicelli, V., Foulquier, E., Truong, L., Thouvenin-Contet, V. and Lefranc, G. "IMGT unique numbering for immunoglobulin and T cell receptor variable domains and Ig superfamily V-like domains". Dev. Comp. Immunol., 27, 55-77 (2003) PMID: 12477501 LIGM:268.) and Kabat numbering. Combined IMGT/Kabat method was used to keep the CDR loop structure optimal.

<Heavy chain humanization design>

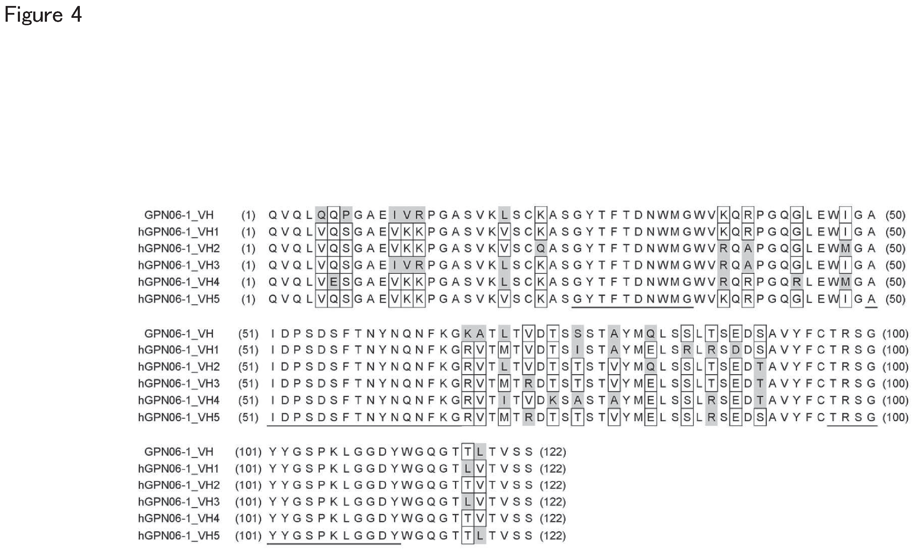

The human germline gene that most closely approximated the amino acid sequence of the mouse heavy chain variable region (GPN06-1_VH) (SEQ ID NO 29) was IGHV1-46*01. BLAST search was used to select 200 candidate human IgG sequences with high homology to GPN06-1_VH. Then, four framework sequences were finally selected in terms of framework homology, retention of key amino acids in the framework, and loop structure. These were subjected to CDR grafting to thereby obtain humanized mutant sequences hGPN06-1_VH1, hGPN06-1_VH2, hGPN06-1_VH3, and hGPN06-1_VH4. In addition, a sequence designed via CDR-grafting of IGHV1-46*01 as a framework was designated as humanized mutant sequence hGPN06-1_VH5. The alignment of these humanized mutant sequences hGPN06-1_VH1 to VH5 to mouse GPN06-1_VH is shown in Figure 4, in which the identified CDR regions are underlined. Table 8 shows the identity and homology of these humanized mutant sequences hGPN06-1_VH1 to VH5 to the mouse sequence GPN06-1_VH. The order of identity and homology of the humanized mutant sequences hGPN06-1_VH1 to VH5 to mouse GPN06-1_VH was VH3 = VH5 > VH2 > VH1 > VH4.

[Table 8]

| Table 8: Identity and homology of humanized mutation sequence VH1 to VH5 to mouse sequence VH0 | | |
|---|---|---|
| | Amino acid sequence identity | Amino acid sequence homology |
| hGPN06-1_VH1 | 86.9% | 92.6% |
| hGPN06-1_VH2 | 86.9% | 94.3% |
| hGPN06-1_VH3 | 88.5% | 93.4% |
| hGPN06-1_VH4 | 85.2% | 92.6% |
| hGPN06-1_VH5 | 88.5% | 93.4% |

<Light chain humanized design>

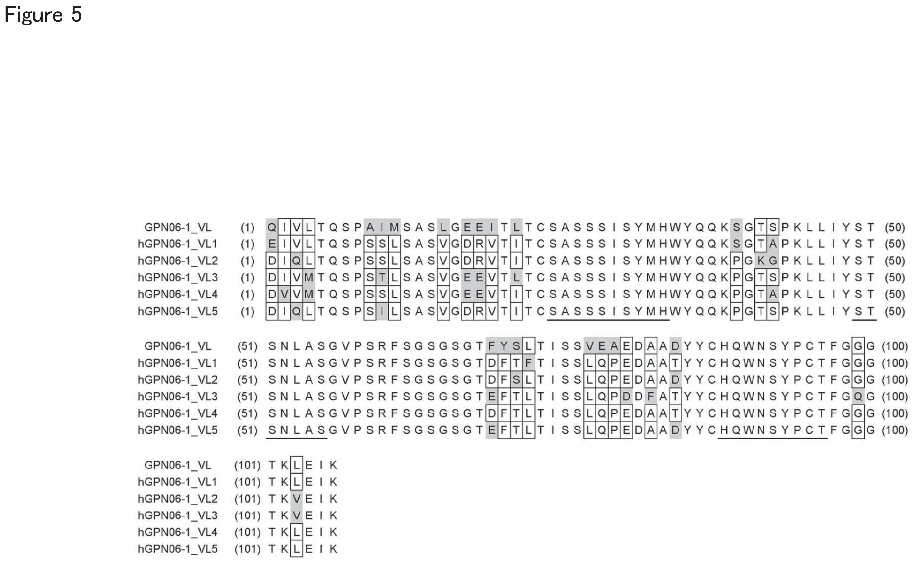

The human germline gene that most closely approximated the amino acid sequence of the mouse light chain variable region (GPN06-1_VL) (SEQ ID NO 31) was IGKV1-9*01. BLAST search was used to select 200 candidate human IgG sequences with high homology to GPN06-1_VL. Then, four framework sequences were finally selected in terms of framework homology, retention of key amino acids in the framework, and loop structure. These were subjected to CDR grafting to thereby obtain humanized mutant sequences hGPN06-1_VL1, hGPN06-1_VL2, hGPN06-1_VL3, and hGPN06-1_VL4. In addition, a sequence designed via CDR-grafting of IGHV1-9*01 as a framework was designated as humanized mutant sequence hGPN06-1_VL5. The alignment of these humanized mutant sequences hGPN06-1_VL1 to VL5 to mouse GPN06-1_VL is shown in Figure 5, in which the identified CDR regions are underlined. Table 9 shows the identity and homology of these humanized mutant sequences hGPN06-1_VL1 to VL5 to the mouse sequence GPN06-1_VL. The order of identity and homology of the humanized mutant sequences hGPN06-1_VL1 to VL5 to mouse GPN06-1_VL was VL5 > VL4 > VL1 > VL3 > VL2.

[Table 9]

| Table 9: Identity and homology of humanized mutation sequence VL1 to VL5 to mouse sequence VL0 | | |
|---|---|---|
| | Amino acid sequence identity | Amino acid sequence homology |
| hGPN06-1VL1 | 83.0% | 92.5% |
| hGPN06-1_VL2 | 82.1% | 90.6% |
| hGPN06-1_VL3 | 82.1% | 91.5% |
| hGPN06-1_VL4 | 83.0% | 93.4% |
| hGPN06-1_VL5 | 84.9% | 93.4% |

[Example 9] Selection of humanized antibodies

The five humanized heavy chain sequences and the five humanized light chain sequences derived from the anti-gpNMB antibody GPN06-1 designed in Example 8 were combined to prepare twenty-five humanized antibodies. These humanized antibodies were evaluated for binding by antigen ELISA to select humanized antibodies whose binding activity was similar to that of human chimeric antibodies.

Specifically, a gene sequence encoding a signal sequence was added to the 5' end of each of the gene sequences encoding the H chains and the L chains of the humanized anti-gpNMB antibodies designed in Example 8, and the resulting sequence was inserted into the animal cell expression vector pcDNA3.4 to form a plasmid, which was transiently expressed using the ExpiCHO Expression System (A29133, Thermo Fisher Scientific) to secrete antibodies into the medium. The culture supernatant was then collected and purified using a Protein A column (Ab-Capture, Protenova) and a gel filtration column (Superdex 200 Increase, Cytiva).

Antigen ELISA was performed by adding each of serially-diluted solutions of the purified humanized antibody to a plate fixed with recombinant human soluble gpNMB (SEQ ID NO 221). The results are shown in Table 10. The results showed that hGPN06-1_H1L1, hGPN06-1_H1L2, hGPN06-1_H2L1, hGPN06-1_H2L2, hGPN06-1_H3L1, hGPN06-1_H3L2, hGPN06-1_H4L1, hGPN06-1_H5L2, hGPN06-1_H5L4, and hGPN06-1_H5L5 had similar levels of binding ability to human chimeric antibody (hGPN06-1_H0L0) (i.e., $EC_{50}$ values were within 3-fold compared to chimeric antibody hGPN06-1_H0L0).

[Table 10]

| Table 10: Evaluation results of the binding ability of each anti-gpNMB antibody clone to human gpNMB by antigen ELISA | | |
|---|---|---|
| Antibody name | $EC_{50}$ (M) | Relative binding activity |
| hGPN06-1_H0L0 | 3.83E-10 | 1 |
| **hGPN06-1_H1L1** | **5.75E-10** | **0.92** |
| hGPN06-1_H1L2 | **5.18E-10** | 0.88 |
| hGPN06-1_H1L3 | 1.58E-09 | 0.83 |
| hGPN06-1_H1L4 | 1.64E-09 | 0.86 |
| hGPN06-1_H1L5 | 2.32E-09 | 0.81 |
| hGPN06-1_H2L1 | **5.57E-10** | 0.91 |
| hGPN06-1_H2L2 | **4.44E-10** | 0.89 |
| hGPN06-1_H2L3 | 3.50E-09 | 0.68 |
| hGPN06-1_H2L4 | 1.38E-09 | 0.82 |
| hGPN06-1_H2L5 | 1.15E-09 | 0.79 |
| hGPN06-1_H3L1 | **6.74E-10** | 0.85 |
| hGPN06-1_H3L2 | **7.99E-10** | 0.85 |
| hGPN06-1_H3L3 | 1.48E-09 | 0.7 |
| hGPN06-1_H3L4 | 1.45E-09 | 0.69 |
| hGPN06-1_H3L5 | 1.32E-08 | 0.68 |
| hGPN06-1_H4L1 | **4.31E-10** | 0.87 |
| hGPN06-1_H4L2 | 5.04E-09 | 0.71 |
| hGPN06-1_H4L3 | 1.42E-08 | 0.37 |
| hGPN06-1_H4L4 | 1.69E-08 | 0.49 |
| hGPN06-1_H4L5 | 1.90E-09 | 0.57 |
| hGPN06-1_H5L1 | 1.76E-09 | 0.65 |
| hGPN06-1_H5L2 | **5.94E-10** | 0.84 |
| hGPN06-1_H5L3 | 1.87E-09 | 0.7 |
| hGPN06-1_H5L4 | **8.60E-10** | 0.77 |
| hGPN06-1_H5L5 | **5.00E-10** | 0.78 |

[Example 101 Identification of amino acids in the CDR regions in humanized antibodies critical for binding by alanine substitution 2

Among the humanized GPN06 antibody variants obtained in Example 9, hGPN06-1_H1L1, hGPN06-1_H1L2, hGPN06-1_H1L4,hGPN06-1_H2L1, hGPN06-1_H2L2, hGPN06-1_H3L1, hGPN06-1_H3L2, hGPN06-1_H4L1, hGPN06-1_H5L2 were compared in terms of the binding properties (binding and dissociation kinetics) to human soluble gpNMB (SEQ ID NO 221) by means of surface plasmon resonance (SPR). Specifically, the same measurement and analysis conditions as in Example 6 were used, except for the conditions described below.

The humanized GPN06 antibody variants hGPN06-1_H1L1, hGPN06-1_H1L2, hGPN06-1_H1L4, hGPN06-1_H2L1, hGPN06-1_H2L2, hGPN06-1_H3L1, hGPN06-1_H3L2, hGPN06-1_H4L1, hGPN06-1_H5L2 were used as analytes. Each analyte at each concentration was reacted for 600 seconds to obtain a binding curve, and HBS-EP+ was reacted for 1200 seconds to obtain a dissociation curve. After the reaction finished, regeneration buffer 1 (0.2% SDS), regeneration buffer 2 (100 mmol/L Tris-HCl (pH 8.5), 1 mol/L NaCl, 15 mmol/L MgCl2) and regeneration buffer 3 (10 mmol/L glycine -HCl (pH 1.5)) were reacted for 1 minute each to remove and wash gpNMB-FLAG-His in the measurement system. The dissociation rate constant (ka, 1/Ms), binding rate constant (kd, 1/s), and dissociation constant (kd, M) were calculated using BIACORE T200 Evaluation software (ver 2.0) with a Model of 1:1 Binding. The results are shown in Table 11.

[Table 11]

Table 11: Results of SPR kinetics analysis of each anti-gpNMB antibody clone against human gpNMB

| Clone | ka (1/Ms) | kd (1/s) | KD (M) |
|---|---|---|---|
| hGPN06-1_H0L0 | 7.89E+05 | 2.09E-04 | 2.65E-10 |
| hGPN06-1_H1L1 | 9.16E+05 | 5.67E-04 | 6.20E-10 |
| hGPN06-1_H1L2 | 3.24E+06 | 8.08E-04 | 2.49E-10 |
| hGPN06-1_H1L4 | 4.98E+05 | 2.13E-04 | 4.28E-10 |
| hGPN06-1_H2L1 | 4.79E+05 | 3.30E-04 | 6.89E-10 |
| hGPN06-1_H2L2 | 5.06E+05 | 3.54E-04 | 7.00E-10 |
| hGPN06-1_H3L1 | 3.51E+06 | 1.54E-03 | 4.38E-10 |
| hGPN06-1_H3L2 | 9.83E+05 | 6.36E-04 | 6.46E-10 |
| hGPN06-1_H4L1 | 7.29E+05 | 4.15E-04 | 5.70E-10 |
| hGPN06-1_H5L2 | 6.02E+05 | 3.54E-04 | 5.87E-10 |

[Example 11] Identification of amino acids in the CDR regions in humanized antibodies critical for binding by alanine substitution

Each amino acid in the CDR regions of humanized anti-gpNMB antibody hGPN06-1_H1L1 produced in Example 9 was substituted with alanine to produce sixty-five CDR substitution mutants of humanized anti-gpNMB antibody hGPN06-1_H1L1. The binding of these alanine-substituted antibodies to recombinant human soluble gpNMB (SEQ ID NO 221) was evaluated by antigen ELISA to determine which amino acids in the CDR grafting regions are critical for binding. The alanine-substitution mutants of the humanized anti-gpNMB antibody hGPN06-1_H1L1 and their binding activity results are shown in Tables 12, 13-1 and 13-2 below.

The results show that alanine-substitution of the following amino acids in each CDR region caused reduced binding activity and are therefore critical for maintaining the binding ability: in the light chain variable region of the humanized anti-gpNMB antibody hGPN06-1_H1L1 (SEQ ID NO 297), in CDR grafting region-L1 (SEQ ID NO 277), isoleucine at position 29, tyrosine at position 31, and histidine at position 33; in CDR grafting region-L2 (SEQ ID NO 279), threonine at position 50; and in CDR grafting region-L3 (SEQ ID NO 281), histidine at position 88, glutamine at position 89, tryptophan at position 90, serine at position 92, tyrosine at position 93, proline at position 94, and cysteine at position 95; and in the heavy chain variable region of the humanized anti-gpNMB antibody hGPN06-1_H1L1 (SEQ ID NO 295), in CDR grafting region-H1 (SEQ ID NO 271), tyrosine at position 27, asparagine at position 32 and tryptophan at position 33; in CDR grafting region-H2 (SEQ ID NO 273), aspartic acid at position 55, phenylalanine at position 57, threonine at position 58, asparagine at position 59, tyrosine at position 60, and asparagine at position 61; and in CDR grafting region-H3 (SEQ ID NO 275), arginine at position 98, glycine at position 100, and glycine at position 109.

[Table 12]

| Table 12: Evaluation results of binding activity of alanine substitutions in the light chain CDR regions | | | | |
|---|---|---|---|---|
| | Alanine substitution position | Amino acid before substitution | $EC_{50}$ (M) | Relative binding activity |
| hGPN06-1 _H1L1 | - | - | 1.98E-09 | 1.00 |
| CDR grafting region-L 1 | 24 | S | 1.99E-09 | 0.98 |
| | 25 | A | No data | No data |
| | 26 | S | 1.09E-09 | 0.75 |
| | 27 | S | 1.56E-09 | 0.99 |
| | 28 | S | 1.27E-09 | 1.18 |
| | 29 | I | 2.30E-08 | 0.27 |
| | 30 | S | 8.63E-09 | 0.58 |
| | **31** | **Y** | **1.49E-08** | **0.36** |
| | 32 | M | 6.40E-09 | 0.65 |
| | **33** | **H** | **4.29E-08** | **0.28** |
| CDR grafting region-L2 | 49 | S | 7.00E-10 | 0.79 |
| | **50** | **T** | **3.19E-09** | **0.30** |
| | 51 | S | 9.27E-10 | 0.82 |
| | 52 | N | 8.50E-10 | 0.74 |
| | 53 | L | 5.64E-10 | 0.98 |
| | 54 | A | No data | No data |
| | 55 | S | 6.03E-10 | 1.00 |
| CDR grafting region-L3 | **88** | **H** | **1.47E-07** | **0.08** |
| | **89** | **Q** | **2.75E-08** | **0.06** |
| | **90** | **W** | **1.22E-07** | **0.13** |
| | 91 | N | 1.84E-09 | 0.58 |
| | **92** | **S** | **1.86E-09** | **0.19** |
| | **93** | **Y** | **2.53E-08** | **0.00** |
| | **94** | **P** | **2.64E-08** | **0.05** |
| | **95** | **C** | **3.15E-09** | **0.45** |
| | 96 | T | 1.03E-09 | 0.83 |

[Table 13-1]

| Table 13-1: Evaluation results of binding activity of alanine substitutions in the heavy chain CDR regions | | | | |
|---|---|---|---|---|
| | Alanine substitution position | Amino acid before substitution | $EC_{50}$ (M) | Relative binding activity |
| hGPN06-1 H1L1 | - | - | 1.98E-09 | 1.00 |
| CDR grafting region-H1 | 26 | G | 1.21E-09 | 1.08 |
| | 27 | **Y** | **6.99E-08** | **0.02** |
| | 28 | T | 1.98E-09 | 1.49 |
| | 29 | F | 4.80E-09 | 0.54 |
| | 30 | T | 2.02E-09 | 1.23 |

(continued)

| Table 13-1: Evaluation results of binding activity of alanine substitutions in the heavy chain CDR regions | | | | |
|---|---|---|---|---|
| | Alanine substitution position | Amino acid before substitution | EC$_{50}$ (M) | Relative binding activity |
| | 31 | D | 5.15E-10 | 1.26 |
| | **32** | **N** | **7.83E-09** | **0.35** |
| | **33** | **W** | **2.81E-08** | **0.06** |
| | 34 | M | 3.55E-09 | 1.06 |
| | 35 | G | 3.55E-09 | 1.40 |
| CDR grafting region-H2 | 51 | I | 9.93E-10 | 0.89 |
| | 52 | D | 2.79E-09 | 0.51 |
| | 53 | P | 8.01E-10 | 0.85 |
| | 54 | S | 6.52E-10 | 0.92 |
| | 55 | **D** | **3.15E-08** | **0.00** |
| | 56 | S | 4.29E-10 | 1.14 |
| | 57 | **F** | **3.25E-08** | **0.09** |
| | **58** | **T** | **2.80E-08** | **0.04** |
| | **59** | N | **2.94E-08** | **0.04** |
| | **60** | Y | **1.28E-07** | **0.04** |
| | **61** | N | **2.61E-08** | **0.04** |
| | 62 | Q | 4.06E-10 | 1.14 |
| | 63 | N | 4.39E-10 | 1.07 |
| | 64 | F | 4.48E-10 | 1.11 |
| | 65 | K | 4.06E-10 | 1.10 |
| | 66 | G | 3.73E-10 | 1.09 |

[Table 13-2]

| Table 13-2: Evaluation results of binding activity of alanine substitutions in the heavy chain CDR regions (continued) | | | | |
|---|---|---|---|---|
| | Alanine substitution position | Amino acid before substitution | EC$_{50}$ (M) | Relative binding activity |
| hGPN06-1 H1L1 | - | - | 1.98E-09 | 1.00 |
| CDR grafting region-H3 | 97 | T | 3.60E-10 | 0.98 |
| | **98** | **R** | **5.32E-08** | **0.05** |
| | 99 | S | 5.87E-10 | 0.92 |
| | **100** | G | **6.58E-08** | **0.06** |
| | 101 | Y | 5.02E-10 | 1.04 |
| | 102 | Y | 2.21E-09 | 0.68 |
| | 103 | G | 7.62E-10 | 1.14 |
| | 104 | S | 7.12E-10 | 0.88 |
| | 105 | P | 1.18E-09 | 1.09 |
| | 106 | K | 4.41E-10 | 1.00 |

(continued)

| Table 13-2: Evaluation results of binding activity of alanine substitutions in the heavy chain CDR regions (continued) | | | | |
|---|---|---|---|---|
| | Alanine substitution position | Amino acid before substitution | EC$_{50}$ (M) | Relative binding activity |
| | 107 | L | 1.05E-09 | 0.71 |
| | 108 | G | 4.76E-10 | 0.96 |
| | **109** | **G** | **4.89E-09** | **0.34** |
| | 110 | D | 9.56E-10 | 0.79 |
| | 111 | Y | 5.84E-10 | 0.99 |

[Example 12] Humanization design of mouse anti-gpNMB antibody: GPN18-2

The amino acid sequence of the anti-gpNMB antibody GPN18-2 was analyzed for identifying its CDR regions, in view of IMGT (Lefranc, M.-P., Pommié, C., Ruiz, M., Giudicelli, V., Foulquier, E., Truong, L., Thouvenin-Contet, V. and Lefranc, G. "IMGT unique numbering for immunoglobulin and T cell receptor variable domains and Ig superfamily V-like domains". Dev. Comp. Immunol., 27, 55-77 (2003) PMID: 12477501 LIGM:268.) and Kabat numbering. Combined IMGT/Kabat method was used to keep the CDR loop structure optimal.

<Heavy chain humanization design>

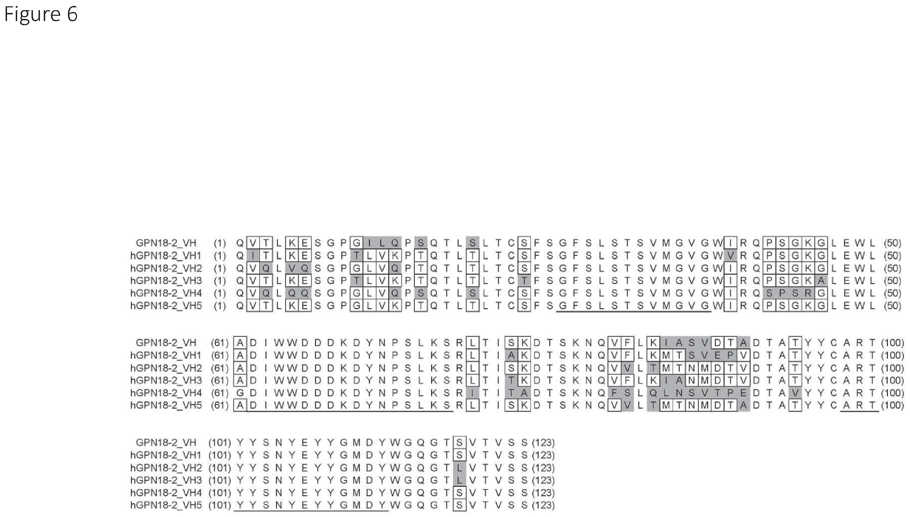

The human germline gene that most closely approximated the amino acid sequence of the mouse heavy chain variable region (GPN18-2_VH) (SEQ ID NO 141) was IGHV2-05*09. BLAST search was used to select 200 candidate human IgG sequences with high homology to GPN18-2_VH. Then, four framework sequences were finally selected in terms of framework homology, retention of key amino acids in the framework, and loop structure. These were subjected to CDR grafting to thereby obtain humanized mutant sequences hGPN18-2_VH1, hGPN18-2_VH2, hGPN18-2_VH3, and hGPN18-2_VH4. In addition, a sequence designed via CDR-grafting of IGHV2-05*09 as a framework was designated as humanized mutant sequence hGPN18-2_VH5. The alignment of these humanized mutant sequences hGPN18-2_VH1 to VH5 to mouse GPN06-1_VH is shown in Figure 6, in which the identified CDR regions are underlined. Table 14 shows the identity and homology of these humanized mutant sequences hGPN18-2 _VH1 to VH5 to the mouse sequence GPN18-2_VH. The order of identity and homology of the humanized mutant sequences hGPN18-2_VH1 to VH5 to mouse GPN18-2_VH was VH5 > VH3 > VH1 > VH2 > VH4.

[Table 14]

| Table 14: Identity and homology of humanized mutation sequence hGPN18-2_VH1 to VH5 to mouse sequence hGPN18-2VH0 | | |
|---|---|---|
| | Amino acid sequence identity | Amino acid sequence homology |
| hGPN18-2_VH1 | 88.6% | 96.7% |
| hGPN18-2_VH2 | 87.8% | 94.3% |
| hGPN18-2_VH3 | 89.4% | 96.7% |
| hGPN18-2_VH4 | 82.1% | 89.4% |
| hGPN18-2_VH5 | 91.1% | 97.6% |

<Light chain humanized design>

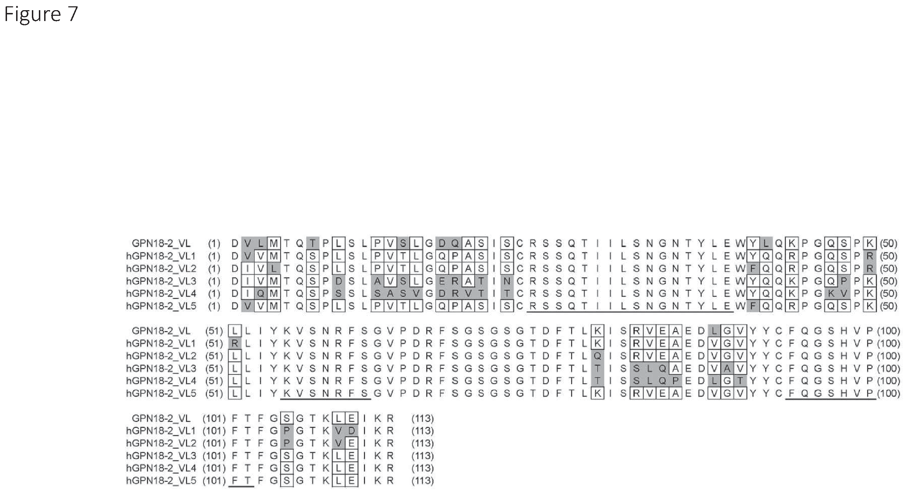

The human germline gene that most closely approximated the amino acid sequence of the mouse light chain variable region (GPN18-2_VL) (SEQ ID NO 31) was IGKV2-30*01. BLAST search was used to select 200 candidate human IgG sequences with high homology to GPN18-2_VL. Then, four framework sequences were finally selected in terms of framework homology, retention of key amino acids in the framework, and loop structure. These were subjected to CDR grafting to thereby obtain humanized mutant sequences hGPN18-2_VL1, hGPN18-2_VL2, hGPN18-2_VL3, and hGPN18-2_VL4. In addition, a sequence designed via CDR-grafting of IGKV2-30*01 as a framework was designated as humanized mutant sequence GPN18-2_VL5. The alignment of these humanized mutant sequences GPN18-2_VL1 to VL5 to mouse GPN18-2_VL is shown in Figure 7, in which the identified CDR regions are underlined. Table 15 shows the identity and homology of these humanized mutant sequences hGPN18-2_VL1 to VL5 to the mouse sequence GPN18-2_VL. The order of identity and homology of the humanized mutant sequences hGPN18-2_VL1 to VL5 to mouse GPN18-2_VL was VL5 > VL1 > VL2 > VL3 > VL4.

[Table 15]

| Table 16: Identity and homology of humanized mutation sequence hGPN18-2_VL1 to VL5 to GPN18-2_VL | | |
|---|---|---|
| | Amino acid sequence identity | Amino acid sequence homology |
| hGPN18-2_VL1 | 88.4% | 95.5% |
| hGPN18-2_VL2 | 86.6% | 96.4% |
| hGPN18-2_VL3 | 84.8% | 93.8% |
| hGPN18-2_VL4 | 82.1% | 90.2% |
| hGPN18-2_VL5 | 92.0% | 97.3% |

[Example 13] Selection of humanized antibodies

The five humanized heavy chain sequences and the five humanized light chain sequences derived from the anti-gpNMB antibody GPN18-2 designed in Example 12 were combined to prepare twenty-five humanized antibodies. These humanized antibodies were evaluated for binding by antigen ELISA to select humanized antibodies whose binding activity was similar to that of human chimeric antibody hGPN18-2_H0L0.

Specifically, a gene sequence encoding a signal sequence was added to the 5' end of each of the gene sequences encoding the H chains and the L chains of the humanized anti-gpNMB antibodies designed in Example 12, and the resulting sequence was inserted into the animal cell expression vector pcDNA3.4 to form a plasmid, which was transiently expressed using the ExpiCHO Expression System (A29133, Thermo Fisher Scientific) to secrete antibodies into the medium. The culture supernatant was then collected and purified using a Protein A column (Ab-Capture, Protenova) and a gel filtration column (Superdex 200 Increase, Cytiva).

Antigen ELISA was performed by adding each of serially-diluted solutions of the purified humanized antibody to a plate fixed with recombinant human soluble gpNMB (SEQ ID NO 221). The results are shown in Table 16. The results showed that hGPN18-2_H1L3, hGPN18-2_H1L4, hGPN18-2_H2L3, hGPN18-2_H2L4, hGPN18-2_H2L5, hGPN18-2_H3L3, hGPN18-2_H3L4, hGPN18-2_H5L3, and hGPN18-2_H5L4 had similar levels of binding ability to human chimeric antibody (hGPN18-2_H0L0) (i.e., $EC_{50}$ values were within 3-fold compared to human chimeric antibody hGPN18-2_H0L0).

[Table 16]

| Table 16: Evaluation results of the binding ability of each GPN18-22 humanized antibody to human gpNMB by antigen ELISA | | |
|---|---|---|
| Antibody name | $EC_{50}$ (M) | Relative binding activity |
| hGPN18-2_H0L0 | 1.21E-09 | 1 |
| hGPN18-2_H1L1 | 4.95E-09 | 0.59 |
| hGPN18-2_H1L2 | 1.62E-08 | 0.25 |
| hGPN18-2_H1L3 | **1.93E-09** | **0.77** |
| hGPN18-2_H1L4 | **3.07E-09** | **0.68** |
| hGPN18-2_H1L5 | 9.55E-09 | 0.36 |
| hGPN18-2_H2L1 | 9.12E-09 | 0.41 |
| hGPN18-2_H2L2 | 6.91E-09 | 0.46 |
| **hGPN18-2_H2L3** | **1.84E-09** | **0.84** |
| hGPN18-2_H2L4 | **2.00E-09** | **0.81** |
| hGPN18-2_H2L5 | **3.35E-09** | **0.61** |

(continued)

| Table 16: Evaluation results of the binding ability of each GPN18-22 humanized antibody to human gpNMB by antigen ELISA | | |
|---|---|---|
| Antibody name | EC$_{50}$ (M) | Relative binding activity |
| hGPN18-2_H3L1 | 5.50E-09 | 0.55 |
| hGPN18-2_H3L2 | 2.35E-08 | 0.23 |
| hGPN18-2_H3L3 | **2.11E-09** | **0.79** |
| hGPN18-2_H3L4 | **2.59E-09** | **0.73** |
| hGPN18-2_H3L5 | 1.01E-08 | 0.35 |
| hGPN18-2_H4L1 | 4.54E-08 | 0.05 |
| hGPN18-2_H4L2 | 2.99E-08 | 0.20 |
| hGPN18-2_H4L3 | 6.09E-09 | 0.46 |
| hGPN18-2_H4L4 | 5.54E-09 | 0.50 |
| hGPN18-2_H4L5 | 2.54E-08 | 0.15 |
| hGPN18-2_H5L1 | 9.25E-09 | 0.46 |
| hGPN18-2_H5L2 | 1.76E-08 | 0.23 |
| hGPN18-2_H5L3 | **2.41E-09** | **0.79** |
| hGPN18-2_H5L4 | **3.10E-09** | **0.72** |
| hGPN18-2_H5L5 | 1.10E-08 | 0.35 |

[Example 14] Identification of amino acids in the CDR regions in humanized antibodies critical for binding by alanine substitution

Twenty-five humanized GPN 18-2 antibody variants obtained in Example 13 were compared in terms of the binding properties (binding and dissociation kinetics) to human gpNMB by means of surface plasmon resonance (SPR). Specifically, the same measurement and analysis conditions as in Example 6 were used, except for the conditions described below.

The temperature of the measurement system was set at 36°C. The humanized GPN18-2 antibodies produce in Example 20 were used as analytes. Each analyte was diluted with HBS-EP+ (100nmol/L) and was reacted for 300 seconds in each flow cell to obtain a binding curve, and HBS-EP+ was reacted for 300 seconds to obtain a dissociation curve. After the reaction finished, regeneration buffer 1 (0.2% SDS), regeneration buffer 2 (100 mmol/L Tris-HCl (pH 8.5), 1 mol/L NaCl, 15 mmol/L MgCl2) and regeneration buffer 3 (10 mmol/L glycine -HCl (pH 1.5)) were reacted for 1 minute each to remove and wash gpNMB-FLAG-His in the measurement system. BIACORE T200 Evaluation software (ver. 2.0) was used to analyze the data with the 1:1 Binding model, and the binding rate value (Ass: 1/Ms), binding stability value (Diss: 1/s) and equilibrium dissociation value (Diss/Ass: M) were quantified as ordinal numbers. The results are shown in Table 17.

[Table 17]

| Table 17: Kinetic analysis of each anti-gpNMB antibody against human gpNMB by SPR method | | | |
|---|---|---|---|
| Antibody name | Ass (1/Ms) | Diss (1/s) | Diss/Ass (M) |
| hGPN18-2_H0L0 | 1.41E+05 | 4.71E-07 | 3.46E-12 |
| hGPN18-2_H1L1 | 8.79E+04 | 6.01E-07 | 6.84E-12 |
| hGPN18-2_H1L2 | 3.19E+04 | 3.50E-08 | 1.10E-12 |
| hGPN18-2_H1L3 | 9.15E+04 | 8.15E-08 | 8.91E-13 |
| hGPN18-2_H1L4 | 7.67E+04 | 5.59E-07 | 7.30E-12 |
| hGPN18-2_H1L5 | 4.63E+04 | 4.37E-08 | 9.43E-13 |
| hGPN18-2_H2L1 | 2.84E+04 | 3.92E-06 | 1.38E-10 |

(continued)

| Table 17: Kinetic analysis of each anti-gpNMB antibody against human gpNMB by SPR method | | | |
|---|---|---|---|
| Antibody name | Ass (1/Ms) | Diss (1/s) | Diss/Ass (M) |
| hGPN18-2_H2L2 | 8.02E+04 | 5.20E-07 | 6.48E-12 |
| hGPN18-2_H2L3 | 9.62E+04 | 7.49E-07 | 7.78E-12 |
| hGPN18-2_H2L4 | 8.90E+04 | 1.47E-07 | 1.65E-12 |
| hGPN18-2_H2L5 | 5.65E+04 | 8.61E-08 | 1.52E-12 |
| hGPN18-2_H3L1 | 5.04E+04 | 2.53E-08 | 5.01E-13 |
| hGPN18-2_H3L2 | 6.31E+04 | 1.69E-04 | 2.68E-09 |
| hGPN18-2_H3L3 | 3.04E+04 | 1.48E-08 | 4.87E-13 |
| hGPN18-2_H3L4 | 7.79E+04 | 3.70E-10 | 4.74E-15 |
| hGPN18-2_H3L5 | 4.45E+04 | 2.49E-08 | 5.60E-13 |
| hGPN18-2_H4L1 | 1.66E+04 | 3.51E-04 | 2.11E-08 |
| hGPN18-2_H4L2 | 9.08E+04 | 9.93E-08 | 1.09E-12 |
| hGPN18-2_H4L3 | 1.03E+05 | 8.11E-07 | 7.91E-12 |
| hGPN18-2_H4L4 | 9.14E+04 | 2.06E-07 | 2.26E-12 |
| hGPN18-2_H4L5 | 9.63E+04 | 2.68E-05 | 2.78E-10 |
| hGPN18-2_H5L1 | not measured | not measured | not measured |
| hGPN18-2_H5L2 | 2.91E+04 | 2.16E-08 | 7.40E-13 |
| hGPN18-2_H5L3 | 7.45E+04 | 1.68E-07 | 2.25E-12 |
| hGPN18-2_H5L4 | 7.20E+04 | 5.19E-09 | 7.21E-14 |
| hGPN18-2_H5L5 | 2.68E+04 | 3.40E-07 | 1.27E-11 |

[Example 15] Identification of amino acids in the CDR regions in GPN18-2 humanized antibodies critical for binding by alanine substitution

Each amino acid in the CDR regions of humanized anti-gpNMB antibody hGPN18-2_H2L3 produced in Example 13 was substituted with alanine to produce seventy-four CDR substitution mutants of humanized anti-gpNMB antibody hGPN18-2_H2L3. The binding of these alanine-substituted antibodies to recombinant human soluble gpNMB (SEQ ID NO 221) was evaluated by antigen ELISA to determine which amino acids in the CDR grafting regions are critical for binding. The alanine-substitution mutants of the humanized anti-gpNMB antibody hGPN18-2_H2L3 and their binding activity results are shown in Tables 18, 19-1, and 19-2 below.

The results show that alanine-substitution of the following amino acids in each CDR region caused reduced binding activity and are therefore critical for maintaining the binding ability: in the light chain variable region of the humanized anti-gpNMB antibody hGPN18-2_H2L3 (SEQ ID NO 325), in CDR grafting region-L1 (SEQ ID NO 305), leucine at position 31, tyrosine at position 37, and glutamic acid at position 39; in CDR grafting region-L2 (SEQ ID NO 307), lysine at position 55; and in CDR grafting region-L3 (SEQ ID NO 309), phenylalanine at position 94; and in the heavy chain variable region of the humanized anti-gpNMB antibody hGPN18-2_H2L3 (SEQ ID NO 323), in CDR grafting region-H2 (SEQ ID NO 301), aspartic acid at position 52, tryptophan at position 54, aspartic acid at position 58, and proline at position 63; and in CDR grafting region-H3 (SEQ ID NO 303), arginine at position 99, threonine at position 100, tyrosine at position 102, tyrosine at position 105, tyrosine at position 107, and methionine at position 110.

[Table 18]

| Table 18: Evaluation results of binding activity of alanine substitutions in the light chain CDR regions | | | | |
|---|---|---|---|---|
| | Alanine substitution position | Amino acid before substitution | EC$_{50}$ (M) | Relative binding activity |
| hGPN18-2 H2L3 | - | - | 4.39E-09 | 1.00 |

**EP 4 772 193 A1**

(continued)

| Table 18: Evaluation results of binding activity of alanine substitutions in the light chain CDR regions | | | | |
|---|---|---|---|---|
| | Alanine substitution position | Amino acid before substitution | EC$_{50}$ (M) | Relative binding activity |
| CDR grafting region-L 1 | 24 | R | 4.73E-09 | 1.04 |
| | 25 | S | 3.76E-09 | 1.01 |
| | 26 | S | 3.14E-09 | 1.03 |
| | 27 | Q | 4.00E-09 | 1.04 |
| | 28 | T | 2.91E-09 | 1,05 |
| | 29 | I | 6.04E-09 | 0.75 |
| | 30 | I | 4.10E-09 | 1.00 |
| | **31** | **L** | **2.07E-08** | **0.41** |
| | 32 | S | 8.67E-09 | 0.92 |
| | 33 | N | 3.66E-09 | 0.82 |
| | 34 | G | 3.94E-09 | 1.00 |
| | 35 | N | 3.08E-09 | 1.11 |
| | 36 | T | 4.74E-09 | 1.06 |
| | **37** | **Y** | **2.71E-08** | **0.37** |
| | 38 | L | 4.15E-09 | 1.06 |
| | **39** | **E** | **8.48E-08** | **0.10** |
| CDR grafting region-L2 | **55** | **K** | **4.83E-08** | **0.31** |
| | 56 | V | 2.99E-09 | 1.07 |
| | 57 | S | 3.14E-09 | 1.05 |
| | 58 | N | 2.86E-09 | 1.04 |
| | 59 | R | 2.36E-09 | 1.11 |
| | 60 | F | 2.28E-09 | 1.12 |
| | 61 | S | 3.63E-09 | 0.94 |
| CDR grafting region-L3 | **94** | **F** | **1.80E-08** | **0.07** |
| | 95 | Q | 5.49E-09 | 0.79 |
| | 96 | G | 1.08E-08 | 0.91 |
| | 97 | S | 3.29E-09 | 1.04 |
| | 98 | H | 5.69E-09 | 0.80 |
| | 99 | V | 9.93E-09 | 0.81 |
| | 100 | P | 4.22E-09 | 0.84 |
| | 101 | F | 7.66E-09 | 0.69 |
| | 102 | T | 4.73E-09 | 0.81 |

[Table 19-1]

| Table 19-1: Evaluation results of binding activity of alanine substitutions in the heavy chain CDR regions | | | | |
|---|---|---|---|---|
| | Alanine substitution position | Amino acid before substitution | EC$_{50}$ (M) | Relative binding activity |
| hGPN18-2 H2L3 | - | - | 4.39E-09 | 1.00 |

75

(continued)

| Table 19-1: Evaluation results of binding activity of alanine substitutions in the heavy chain CDR regions | | | | |
|---|---|---|---|---|
| | Alanine substitution position | Amino acid before substitution | $EC_{50}$ (M) | Relative binding activity |
| CDR grafting region-H 1 | 26 | G | 3.92E-09 | 0.84 |
| | 27 | F | 5.47E-09 | 0.69 |
| | 28 | S | 3.22E-09 | 0.88 |
| | 29 | L | 4.41E-09 | 0.82 |
| | 30 | S | 6.60E-09 | 0.97 |
| | 31 | T | 3.12E-09 | 0.97 |
| | 32 | S | 4.31E-09 | 0.78 |
| | 33 | V | 8.88E-09 | 0.55 |
| | 34 | M | 3.58E-09 | 0.91 |
| | 35 | G | 5.16E-09 | 0.68 |
| | 36 | V | 3.43E-09 | 0.92 |
| | 37 | G | 3.32E-09 | 0.87 |
| CDR grafting region-H2 | **52** | **D** | **1.00E-07** | **0.05** |
| | 53 | I | 6.94E-09 | 0.60 |
| | **54** | **W** | **3.80E-08** | **0.20** |
| | 55 | W | 8.36E-09 | 0.66 |
| | 56 | D | 8.98E-09 | 0.63 |
| | 57 | D | 5.61E-09 | 0.86 |
| | **58** | **D** | **1.02E-08** | **0.50** |
| | 59 | K | 4.42E-09 | 0.78 |
| | 60 | D | 3.13E-09 | 0.98 |

[Table 19-2]

| Table 19-2: Evaluation results of binding activity of alanine substitutions in the heavy chain CDR regions (continued) | | | | |
|---|---|---|---|---|
| | Alanine substitution position | Amino acid before substitution | $EC_{50}$ (M) | Relative binding activity |
| hGPN18-2 H2L3 | - | - | 4.39E-09 | 1.00 |
| CDR grafting region-H2 (continued) | 61 | Y | 7.55E-09 | 0.70 |
| | 62 | N | 4.58E-09 | 0.97 |
| | **63** | **P** | **1.20E-08** | **0.43** |
| | 64 | S | 3.59E-09 | 1.09 |
| | 65 | L | 5.04E-09 | 0.94 |
| | 66 | K | 4.96E-09 | 0.96 |
| | 67 | S | 4.25E-09 | 1.01 |
| | **99** | **R** | **1.06E-08** | **0.37** |
| | **100** | **T** | **8.44E-09** | **0.47** |
| | 101 | Y | 7.68E-09 | 0.61 |

(continued)

| Table 19-2: Evaluation results of binding activity of alanine substitutions in the heavy chain CDR regions (continued) | | | | |
|---|---|---|---|---|
| | Alanine substitution position | Amino acid before substitution | EC$_{50}$ (M) | Relative binding activity |
| CDR grafting region-H3 | **102** | **Y** | **4.91E-08** | **0.07** |
| | 103 | S | 4.04E-09 | 0.94 |
| | 104 | N | 5.62E-09 | 0.80 |
| | **105** | **Y** | **1.56E-08** | **0.47** |
| | 106 | E | 1.08E-08 | 0.64 |
| | **107** | **Y** | **6.09E-08** | **0.06** |
| | 108 | Y | 4.70E-09 | 0.91 |
| | 109 | G | 1.05E-08 | 0.61 |
| | **110** | **M** | **2.34E-08** | **0.41** |
| | 111 | D | 1.13E-08 | 0.62 |
| | 112 | Y | 3.73E-09 | 1.05 |

[Example 16] Humanization design of mouse anti-gpNMB antibody: GPN18-5:

Based on the amino acid sequence of anti-gpNMB antibody GPN18-5, the CDR region was identified using Kabat's numbering as a reference.

<Heavy chain humanization design>

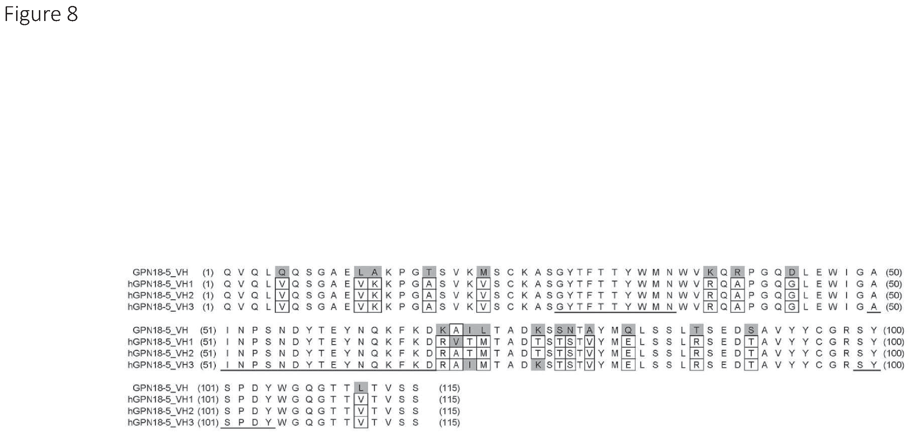

The human germline gene that most closely resembles the amino acid sequence of the mouse heavy chain variable region (GPN18-5_VH) (SEQ ID NO 189) was IGHV1-46*01. The sequence of IGHV1-46*01 was used as the framework and CDR sequences were graphed to produce humanized mutant sequence hGPN18-5_VH1. The humanized mutant sequence hGPN18-5_VH1 was then mutated so as to be closer to the mouse heavy chain variable sequence GPN18-5_VH to produce humanized mutant sequences hGPN18-5_VH2 and hGPN18-5_VH3. The alignment of hGPN18-5_VH1 to VH3 to mouse GPN18-5_VH is shown in Figure 8. The underlined areas are the identified CDR regions. Table 20 below shows the identity and homology of these humanized mutant sequences hGPN18-5_VH1 to VH3 to the mouse sequence GPN18-5_VH.

[Table 20]

| Table 20: Identity and homology of humanized mutation sequences hGPN18-5_VH1 to VH3 to mouse sequence GPN18-5 _VH0 | | |
|---|---|---|
| | Amino acid sequence identity | Amino acid sequence homology |
| hGPN18-5_VH1 | 82% | 96% |
| hGPN18-5_VH2 | 83% | 96% |
| hGPN18-5 VH3 | 85% | 96% |

<Light chain humanization design>

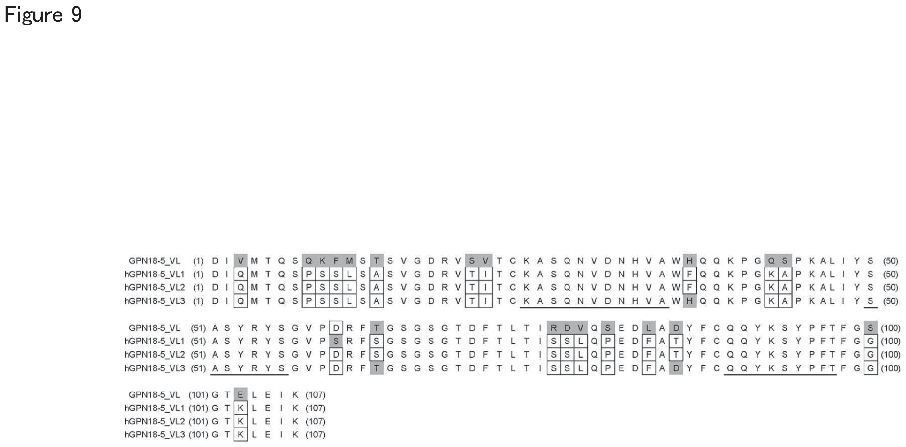

The human germline gene that most closely resembles the amino acid sequence of the mouse light chain variable region (GPN18-5_VL) (SEQ ID NO 191) was IGKV1-16*01. The sequence of IGKV1-16*01 was used as the framework and CDR sequences were graphed to produce humanized mutant sequence hGPN18-5_VL1. The humanized mutant sequence hGPN18-5_VL1 was then mutated so as to be closer to the mouse light chain variable sequence GPN18-5_VL to produce humanized mutant sequences hGPN18-5_VL2 and hGPN18-5_VL3. The alignment of hGPN18-5_VL1 to VL3 to mouse GPN18-5_VL is shown in Figure 9. The underlined areas are the identified CDR regions. Table 21 below shows the identity and homology of these humanized mutant sequences hGPN18-5_VL1 to VL3 to the mouse sequence GPN18-5 _VL.

[Table 21]

| Table 21: Identity and homology of humanized mutation sequences hGPN18-5_VL1 to VL3 to mouse sequence GPN18-5_VL0 | | |
|---|---|---|
| | Amino acid sequence identity | Amino acid sequence homology |
| hGPN18-5 _VL1 | 80% | 97% |
| hGPN18-5_VL2 | 81% | 97% |
| hGPN18-5_VL3 | 84% | 98% |

[Example 17] Selection of GPN18-5 humanized antibody variants by surface plasmon resonance (SPR) - 1:

The three humanized heavy chain variable region sequences and the three humanized light chain variable region sequences of anti-gpNMB antibody GPN18-5 designed in Example 16 were combined to create antibody variants with nine different humanized combinations of heavy chain variable regions and humanized light chain variable regions. The binding of these humanized antibody variants to human gpNMB was evaluated by surface plasmon resonance (SPR). The humanized chimeric antibody HGPN18-5_VH0+VL0 was used as a comparison control. Specifically, Biacore 8K was used as the SPR measurement system. Each antibody variant was captured on a protein A sensor chip as a ligand, and human gpNMB (Human Osteoactivin, His-tag, AcroBioSystems) was used as the sample (analyte) to flow over the sensor chip surface. The antibody concentration was set at 800 nM. The rate constant for dissociation rate (kd) and the binding constant (ka) were obtained using an evaluation software. The equilibrium dissociation constant (kd) was calculated from the ratio of kd to ka. The results are shown in Table 22 below. From these results, three humanized antibody variants GPN18-5-VH1+VL3, GPN18-5-VH2+VL3, and GPN18-5 -VH3 + VL3 were selected.

[Table 22]

| Table 22: SPR analysis results of binding of GPN 18-5 humanized antibody variant to human gpNMB | | | |
|---|---|---|---|
| antibody variant | ka (1/Ms) | kd (1/s) | KD (M) |
| hGPN18-5_VH0+VL0 | 8.44E+03 | 2.00E-03 | 2.37E-07 |
| hGPN18-5_VH1+VL1 | 6.64E+03 | 4.32E-03 | 6.51E-07 |
| hGPN18-5_VH1+VL2 | 9.38E+03 | 3.96E-03 | 4.23E-07 |
| **hGPN18-5_VH1+VL3** | **7.15E+03** | **1.53E-03** | **2.14E-07** |
| hGPN18-5_VH2+VL1 | 6.87E+03 | 4.54E-03 | 6.61E-07 |
| hGPN18-5_VH2+VL2 | 5.28E+03 | 4.03E-03 | 7.63E-07 |
| **hGPN18-5_VH2+VL3** | **7.78E+03** | **1.48E-03** | **1.90E-07** |
| hGPN18-5 VH3+VL1 | 6.81E+03 | 4.84E-03 | 7.11E-07 |
| hGPN18-5 VH3+VL2 | 7.95E+03 | 4.36E-03 | 5.48E-07 |
| **hGPN18-5_VH3+VL3** | **5.72E+03** | **2.01E-03** | **3.52E-07** |

[Example 18] Selection of GPN18-5 humanized antibody variants by surface plasmon resonance (SPR) - 2:

Concentration-dependent binding properties of three anti-gpNMB antibody variants: GPN18-5_H1L1, GPN18-5_H3L1, and GPN18-5_H3L3, to human and mouse gpNMB were evaluated by SPR to assess human/mouse cross-reactivity. The BIACORE T200 system was used as the measurement system. Anti-His monoclonal antibodies were fixed in all flow cells of the sensor chip CM3 (BR-1005-36, Cytiva) at around 5000 RU using the Amine Coupling Kit (BR-1000-50, GE) and His Capture Kit (28 -9950-56, Cytiva). The running buffer used was HBS-EP+ (BR-1006-69, Cytiva). Each anti-gpNMB antibody clone at concentrations of 100 nmol/L or lower were used as the analyte. The measurement conditions used were single-cycle kinetics method. The temperature of the measurement system was set at 36°C. Recombinant human gpNMB-FLAG-His was used as the ligand and captured by the anti-His monoclonal antibody in flow cell (2) such that the final concentration was 100 RU or lower. Recombinant mouse gpNMB-FLAG-His was reacted with the anti-His monoclonal antibody in flow cell (4) such that the final concentration was 100 RU or lower. In the analysis, the SPR change due to anti-gpNMB antibody binding to the recombinant human gpNMB-FLAG-His was calculated by subtracting the response value of the reference flow cell (1) from the response value of the flow cell (2). Likewise, the SPR change due to anti-gpNMB antibody binding to the recombinant human gpNMB-FLAG-His was obtained by subtracting the response value of the reference flow cell (3) from the response value of the flow cell (4). Measurements were taken twice, and the dissociation rate constant (ka, 1/Ms), binding rate constant (kd, 1/s) and dissociation constant (kd, M) of each clone were averaged. The results are shown in Table 23.

[Table 23]

Table 23: SPR analysis results of binding affinity of GPN18-5 humanized antibody variants to human and mouse gpNMBs.

| Clone | Human gpNMB | | | Mouse gpNMB | | |
|---|---|---|---|---|---|---|
| | ka (1/Ms) | kd (1/s) | KD (M) | ka (1/Ms) | kd (1/s) | KD (M) |
| hGPN18-5_H0L0 | 2.37E+05 | 7.27E-04 | 3.08E-09 | 1.42E+05 | 7.50E-04 | 5.31E-09 |
| hGPN18-5_H1L1 | 3.08E+05 | 3.07E-03 | 1.62E-08 | 7.38E+04 | 1.43E-03 | 2.19E-08 |
| hGPN18-5_H3L1 | 1.42E+05 | 1.38E-03 | 1.13E-08 | 5.29E+04 | 8.62E-04 | 1.78E-08 |
| hGPN18-5_H3L3 | 1.76E+05 | 1.21E-03 | 6.98E-09 | 1.11E+05 | 7.11E-04 | 6.39E-09 |

[Example 191 Evaluation of the pain-improving effect of anti-gpNMB antibodies on neuropathic pain

A mouse partial sciatic nerve ligation model (PSNL model) was created as a pathological model of neuropathic pain, and the pain-improving effect of each anti-gpNMB antibody was evaluated by the von Frey test.

*Creation of mouse PSNL model

C57BL/6J mice were used to create a neuropathic pain model according to the method of Selzer et al. (Selzer et al., Pain, 1990, 43[2]:205-218). Under general anesthesia with isoflurane inhalation, mice were incised through the skin of the upper left thigh and near the muscle head of the biceps femoris muscle to expose the sciatic nerve, ligated tightly with 7-0 nylon thread so that the proximal sciatic nerve bundle was 1/3 to 1/2 of the lateral width, and the muscle and skin at the incision was sutured. For the sham operation (Sham) group, nerve ligation was not performed, and other manipulations were performed in the same manner.

*The von Frey test

The 50% hindlimb escape threshold (PWT: paw withdrawal threshold) was calculated by the up-down method according to the method of Chaplan et al. (Chaplan et al., J. Neurosci. Methods, 1994, 53[1]:55-63). Specifically, von Frey filaments (North Coast Medical) were placed on the hind paws of mice on a test stand and observed for escape behavior. 0.16 g filaments were used as a starting point, and when an escape response was observed, a filament of one size smaller was used for the next stimulation. When no response was observed, a filament of one size larger was used for the next stimulation. The two responses before and after the change from positive to negative or negative to positive in response to stimulation were considered as the first responses, and then the same up-down stimulation was repeated four times (six times in total), and the 50% PWT was obtained from the results obtained based on the following formula. However, if the filament did not react up to 2.0 g filament, 2.0 g was used as 50% PWT.

$$50\%\mathrm{PWT(g)} = (10^{\wedge}(\mathrm{Xf}+\mathrm{k}\times\mathrm{d}))/10000$$

Xf: Logarithm value of filament used in the last stimulus
k: Constant obtained by the response pattern
d: Average logarithm value of the differences (g) in the stimuli between each adjacent filaments

The von Frey test was performed on Day 7 with the model creation date as Day 0. Using 50% PWT as the index, the mice were divided into: Sham group (n=4), Control group (n=5), GPN06-1 group (n=5), GPN18-2 group (n=5), GPN18-5 group (n=5), and Pregabalin group (n=5). On Day8, Day15, and Day22, the Sham group and the Control group received mouse control IgG1 (30mg/kg), the GPN06-1 group received GPN06-1 (30mg/kg), the GPN18-2 group received GPN18-2 (30mg/kg), and the GPN18-5 group received GPN18-5 (30mg/kg) via subcutaneous administration. The Pregabalin group received pregabalin (20 mg/kg, Tokyo Kasei Kogyo) via subcutaneous administration 30 minutes before the von Frey test on Day 14, Day 21 and Day 23. The von Frey test was performed on Day 23 and the 50% PWT was calculated.

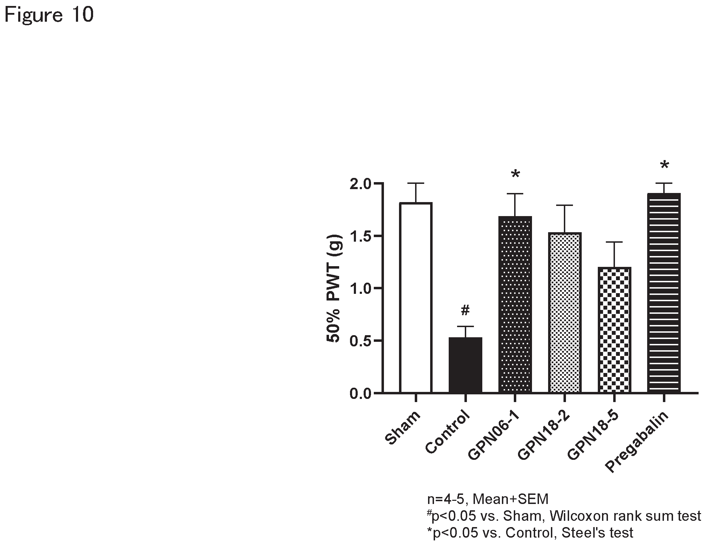

A graph of the results is shown in Figure 10. Administration of GPN06-1, GPN18-2, or GPN18-5 significantly increased 50% PWT. Thus, it was confirmed that the anti-gpNMB antibodies GPN06-1, GPN18-2, and GPN18-5 exhibit pain-improving activity against neuropathic pain.

[Example 20] Evaluation of gpNMB-positive SA-β-Gal-positive macrophages and inflammatory markers in adipose tissue of obese model mice by administration of anti-gpNMB antibodies

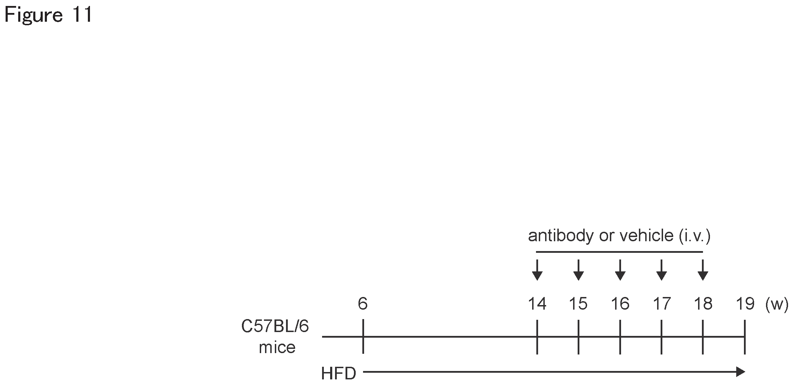

C57BL/6 mice purchased from Jackson Laboratory Japan (Kanagawa, Japan). Figure 11 shows the method of producing DIO (Diet Induced Obesity) mice and the timing of antibody administration. GPN18-5 and GPN18-2 were administered by intravenous infusion to DIO mice bred on a high-fat diet (HFD, CLEA Japan) from 6 weeks of age for 8 weeks.

One week after the first administration, autopsies were performed to analyze the amount of gpNMB-positive SA-β-Gal-positive macrophages in epididymal fat. Stromal vascular fraction (SVF) was isolated from the excised epididymal fat using the Adipose Tissue Dissociation Kit (Miltenyi Biotec). The SVFs were stained with SA-β-Gal stain (a stain commonly used to detect senescent cells) using Cellular Senescence Detection Kit - SPiDER-βGal (Dojin Chemical Laboratory), and after Fc blocking, CD11b and GPN15-3 antibodies were used for staining. The stained SVF was subjected to detection of gpNMB positive SA-β-Gal positive macrophages using FACS Canto II (BD Biosciences). The antibodies used are shown in Table 24 below.

[Table 24]

| Abbreviation | Product name or product information | Manufacturer |
|---|---|---|
| Fc blocking | TruStain FcX™ (Anti-mouse CD16/32) Antibody | BioLegend |
| CD11b antibody | Pacific Blue™ anti-mouse/human CD11b Antibody | BioLegend |
| GPN15-3 antibody | Antibody prepared from GPN15-3 via APC labeling using Allophycocyanin Laveling Kit (Dojindo Laboratories) followed by purification using gel filtration | - |

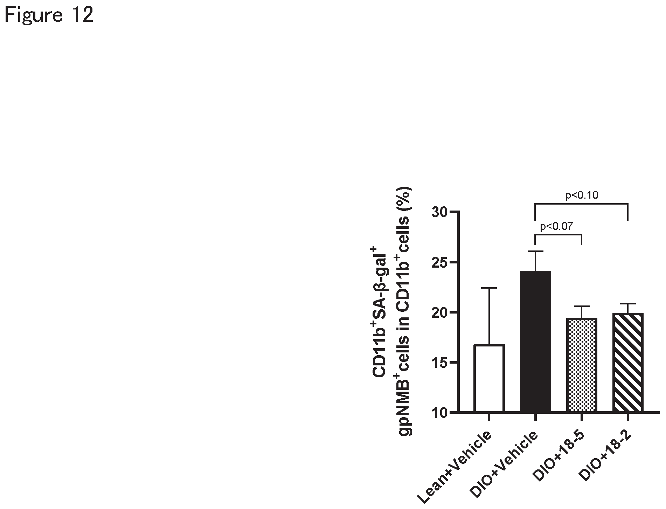

Results are shown in Figure 12. The results showed that HFD loading increased the percentage of gpNMB-positive SA-β-Gal-positive macrophages in the SVF of epididymal fat. The percentage of gpNMB-positive SA-β-Gal-positive macrophages was decreased by administration of GPN18-5 or GPN18-2, which are anti-gpNMB antibodies of the present invention.

One week after the fifth administration, autopsies were performed to analyze gene expression in epididymal fat. The total RNA was purified from epididymal fat removed from each mouse using the RNeasy Lipid Tissue Mini Kit (QIAGEN). The purified total RNA was reverse transcribed using SuperScript VILO MasterMix (Thermo Fisher Scientific Inc.) to prepare cDNA. Gene expression analysis was performed on this cDNA by real-time PCR using Fast SYBR™ Green Master Mix (Thermo Fisher Scientific Inc.) and StepOnePlus (Applied Biosystems). The gene expression levels of TNF-$\alpha$ were compared by calculating relative expression levels using the 18S rRNA expression level as an internal standard.

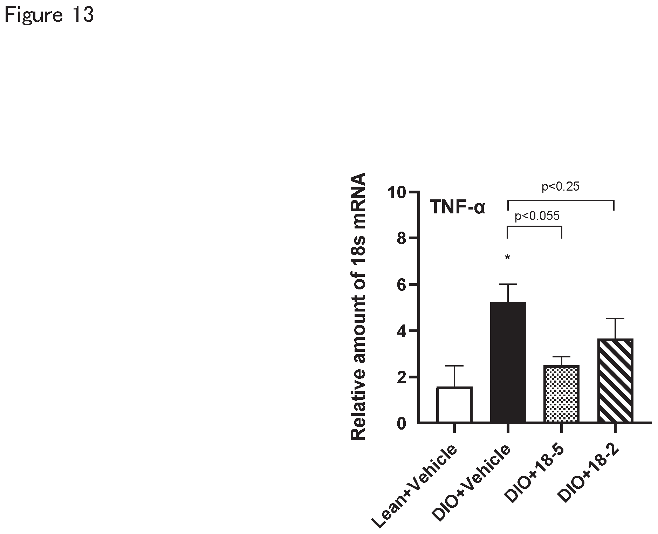

The results are shown in Figure 13. The results showed a clear increase in TNF-$\alpha$ gene expression in epididymal fat under HFD loading conditions compared to NC. The TNF-$\alpha$ gene expression was decreased with administration of GPN 18-5 or GPN 18-2, anti-gpNMB antibodies of the present invention. These results suggest that administration of the anti-gpNMB antibodies of the present invention eliminated gpNMB-positive SA-β-Gal-positive macrophages and reduced inflammatory markers.

Statistical analysis of the study data was performed using GraphPad Prism (Version 8.4.3). Differences between the control group and the pathological condition groups (Lean+Vehicle vs DIO+Vehicle) were tested using a two-tailed Student's T test. Differences between the pathological condition groups (DIO+Vehicle vs DIO+18-5 vs DIO+18-2) were tested using one-way ANOVA followed by Dunnett multiple comparison test. Figure "*" indicates $P < 0.05$.

INDUSTRIAL APPLICABILITY

The present invention provides, e.g., a pharmaceutical containing an anti-gpNMB antibody or its fragment or a derivative thereof as a new means of eliminating disease-related cells. Thus, the present invention is extremely useful in the field of medicine, especially in the field of treatment and prevention of various diseases that are known to be associated with disease-related cells.

**Claims**

1. A pharmaceutical composition for removing disease-related cells, comprising an anti-gpNMB antibody or its fragment or a derivative thereof that binds specifically to at least one site in a region from V78 to a PKD domain of human gpNMB (glycoprotein nonmetastatic melanoma protein B) having the amino acid sequence of SEQ ID NO 209.

2. The pharmaceutical composition according to claim 1, wherein the anti-gpNMB antibody specifically binds to a region containing amino acid residue(s) D287 and/or H301 of human gpNMB having the amino acid sequence of SEQ ID NO 209.

3. The pharmaceutical composition according to claim 1 or 2, wherein the anti-gpNMB antibody specifically binds to a region containing amino acid residue(s) R214 and/or R215 of human gpNMB having the amino acid sequence of SEQ ID NO 209.

4. The pharmaceutical composition according to claim 2 or 3, wherein the anti-gpNMB antibody specifically binds to one or more regions selected from a region containing amino acid residue(s) K257 and/or D258, a region containing amino acid residue(s) H268 and/or D269, a region containing amino acid residue K282, a region containing amino acid residue K316, a region containing amino acid residue K186, and a region containing amino acid residue(s) H216 and/or R218 of human gpNMB having the amino acid sequence of SEQ ID NO 209.

5. The pharmaceutical composition according to claim 1, wherein the anti-gpNMB antibody binds specifically to at least one site in a region from V78 to S92 of human gpNMB having the amino acid sequence of SEQ ID NO 209.

6. The pharmaceutical composition according to any one of claims 1 to 5, wherein the anti-gpNMB antibody specifically binds to at least one site in a region from V78 to a PKD domain of mouse gpNMB having the amino acid sequence of SEQ ID NO 211.

7. The pharmaceutical composition according to any one of claims 1 to 6, wherein the anti-gpNMB antibody is a monoclonal antibody or its fragment or a derivative thereof.

8. The pharmaceutical composition according to any one of claims 1 to 7, wherein the anti-gpNMB antibody comprises at least a heavy chain variable region comprising:

(1) as a CDR-H1 sequence, the amino acid sequence defined in SEQ ID NO 1, or an amino acid sequence derived from SEQ ID NO 1 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 1,

as a CDR-H2 sequence, the amino acid sequence defined in SEQ ID NO 3, or an amino acid sequence derived from SEQ ID NO 3 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 3, and
as a CDR-H3 sequence, the amino acid sequence defined in SEQ ID NO 5, or an amino acid sequence derived from SEQ ID NO 5 via substitution, deletion, or insertion of any one, two, three, or four amino acid residues, or an amino acid sequence having a homology (preferably an identity) of 66.6 % or more, 75.0 % or more, 83.3 % or more, or 91.6 % or more to the amino acid sequence defined in SEQ ID NO 5, or

(2) as a CDR-H1 sequence, the amino acid sequence defined in SEQ ID NO 17, or an amino acid sequence derived from SEQ ID NO 17 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 17,

as a CDR-H2 sequence, the amino acid sequence defined in SEQ ID NO 19, or an amino acid sequence derived from SEQ ID NO 19 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 19, and
as a CDR-H3 sequence, the amino acid sequence defined in SEQ ID NO 21, or an amino acid sequence derived from SEQ ID NO 21 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 21, or

(3) as a CDR-H1 sequence, the amino acid sequence defined in SEQ ID NO 33, or an amino acid sequence derived from SEQ ID NO 33 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 33,

as a CDR-H2 sequence, the amino acid sequence defined in SEQ ID NO 35, or an amino acid sequence derived from SEQ ID NO 35 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 35, and
as a CDR-H3 sequence, the amino acid sequence defined in SEQ ID NO 37, or an amino acid sequence derived from SEQ ID NO 37 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 37, or

(4) as a CDR-H1 sequence, the amino acid sequence defined in SEQ ID NO 49, or an amino acid sequence derived from SEQ ID NO 49 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 49,

as a CDR-H2 sequence, the amino acid sequence defined in SEQ ID NO 51, or an amino acid sequence derived from SEQ ID NO 51 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 51, and
as a CDR-H3 sequence, the amino acid sequence defined in SEQ ID NO 53, or an amino acid sequence derived from SEQ ID NO 53 via substitution, deletion, or insertion of any one, two, three, four, five, or six amino acid residues, or an amino acid sequence having a homology (preferably an identity) of 62.5 % or more, 68.7 % or more, 75.0 % or more, 81.2 % or more, 87.5 % or more, or 93.7 % or more to the amino acid sequence defined in SEQ ID NO 53, or

(5) as a CDR-H1 sequence, the amino acid sequence defined in SEQ ID NO 65, or an amino acid sequence derived from SEQ ID NO 65 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 65,

as a CDR-H2 sequence, the amino acid sequence defined in SEQ ID NO 67, or an amino acid sequence derived from SEQ ID NO 67 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 67, and
as a CDR-H3 sequence, the amino acid sequence defined in SEQ ID NO 69, or an amino acid sequence derived from SEQ ID NO 69 via substitution, deletion, or insertion of any one, two, three, four, or five amino acid residues, or an amino acid sequence having a homology (preferably an identity) of 64.2 % or more, 71.4 % or more, 78.5 % or more, 85.7 % or more, or 92.8 % or more to the amino acid sequence defined in SEQ ID NO 69, or

(6) as a CDR-H1 sequence, the amino acid sequence defined in SEQ ID NO 81, or an amino acid sequence derived from SEQ ID NO 81 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 81,

as a CDR-H2 sequence, the amino acid sequence defined in SEQ ID NO 83, or an amino acid sequence derived from SEQ ID NO 83 via substitution, deletion, or insertion of any one or two amino acid residues, or an amino acid sequence having a homology (preferably an identity) of 71.4 % or more or 85.7 % or more to the amino acid sequence defined in SEQ ID NO 83, and

as a CDR-H3 sequence, the amino acid sequence defined in SEQ ID NO 85, or an amino acid sequence derived from SEQ ID NO 85 via substitution, deletion, or insertion of any one, two, three, or four amino acid residues, or an amino acid sequence having a homology (preferably an identity) 63.6 % or more, 72.7 % or more, 81.8 % or more, or 90.9 % or more to the amino acid sequence defined in SEQ ID NO 85, or

(7) as a CDR-H1 sequence, the amino acid sequence defined in SEQ ID NO 97, or an amino acid sequence derived from SEQ ID NO 97 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 97,

as a CDR-H2 sequence, the amino acid sequence defined in SEQ ID NO 99, or an amino acid sequence derived from SEQ ID NO 99 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 99, and
as a CDR-H3 sequence, the amino acid sequence defined in SEQ ID NO 101, or an amino acid sequence derived from SEQ ID NO 101 via substitution, deletion, or insertion of any one or two amino acid residues, or an amino acid sequence having a homology (preferably an identity) of 60.0 % or more or 80.0 % or more to the amino acid sequence defined in SEQ ID NO 101, or

(8) as a CDR-H1 sequence, the amino acid sequence defined in SEQ ID NO 113, or an amino acid sequence derived from SEQ ID NO 113 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 113,

as a CDR-H2 sequence, the amino acid sequence defined in SEQ ID NO 115, or an amino acid sequence derived from SEQ ID NO 115 via substitution, deletion, or insertion of any one or two amino acid residues, or an amino acid sequence having a homology (preferably an identity) of 71.4 % or more or 85.7 % or more to the amino acid sequence defined in SEQ ID NO 115, and
as a CDR-H3 sequence, the amino acid sequence defined in SEQ ID NO 117, or an amino acid sequence derived from SEQ ID NO 117 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 117, or

(9) as a CDR-H1 sequence, the amino acid sequence defined in SEQ ID NO 129, or an amino acid sequence derived from SEQ ID NO 129 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 129,

as a CDR-H2 sequence, the amino acid sequence defined in SEQ ID NO 131, or an amino acid sequence derived from SEQ ID NO 131 via substitution, deletion, or insertion of any one or two amino acid residues, or an amino acid sequence having a homology (preferably an identity) of 71.4 % or more or 85.7 % or more to the amino acid sequence defined in SEQ ID NO 131, and
as a CDR-H3 sequence, the amino acid sequence defined in SEQ ID NO 133, or an amino acid sequence derived from SEQ ID NO 133 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 133, or

(10) as a CDR-H1 sequence, the amino acid sequence defined in SEQ ID NO 145, or an amino acid sequence derived from SEQ ID NO 145 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 145,

as a CDR-H2 sequence, the amino acid sequence defined in SEQ ID NO 147, or an amino acid sequence derived from SEQ ID NO 147 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 147, and

as a CDR-H3 sequence, the amino acid sequence defined in SEQ ID NO 149, or an amino acid sequence derived from SEQ ID NO 149 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 149, or

(11) as a CDR-H1 sequence, the amino acid sequence defined in SEQ ID NO 161, or an amino acid sequence derived from SEQ ID NO 161 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 161,

as a CDR-H2 sequence, the amino acid sequence defined in SEQ ID NO 163, or an amino acid sequence derived from SEQ ID NO 163 via substitution, deletion, or insertion of any one or two amino acid residues, or an amino acid sequence having a homology (preferably an identity) of 71.4 % or more or 85.7 % or more to the amino acid sequence defined in SEQ ID NO 163, and
as a CDR-H3 sequence, the amino acid sequence defined in SEQ ID NO 165, or an amino acid sequence derived from SEQ ID NO 165 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 165, or

(12) as a CDR-H1 sequence, the amino acid sequence defined in SEQ ID NO 255, or an amino acid sequence derived from SEQ ID NO 255 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 255,

as a CDR-H2 sequence, the amino acid sequence defined in SEQ ID NO 257, or an amino acid sequence derived from SEQ ID NO 257 via substitution, deletion, or insertion of any one or two amino acid residues, or an amino acid sequence having a homology (preferably an identity) of 71.4 % or more or 85.7 % or more to the amino acid sequence defined in SEQ ID NO 257, and
as a CDR-H3 sequence, the amino acid sequence defined in SEQ ID NO 259, or an amino acid sequence derived from SEQ ID NO 259 via substitution, deletion, or insertion of any one or two amino acid residues, or an amino acid sequence having a homology (preferably an identity) of 66.6 % or more or 83.3 % or more to the amino acid sequence defined in SEQ ID NO 259, or

(13) as a CDR-H1 sequence, the amino acid sequence defined in SEQ ID NO 283, or an amino acid sequence derived from SEQ ID NO 283 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 283,

as a CDR-H2 sequence, the amino acid sequence defined in SEQ ID NO 285, or an amino acid sequence derived from SEQ ID NO 285 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 285,
as a CDR-H3 sequence, the amino acid sequence defined in SEQ ID NO 287, or an amino acid sequence derived from SEQ ID NO 287 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 287, or

(14) as a CDR-H1 sequence, the amino acid sequence defined in SEQ ID NO 311, or an amino acid sequence derived from SEQ ID NO 311 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 311,

as a CDR-H2 sequence, the amino acid sequence defined in SEQ ID NO 313, or an amino acid sequence derived from SEQ ID NO 313 via substitution, deletion, or insertion of any one or two amino acid residues, or an amino acid sequence having a homology (preferably an identity) of 71.4 % or more or 85.7 % or more to the amino acid sequence defined in SEQ ID NO 313,
as a CDR-H3 sequence, the amino acid sequence defined in SEQ ID NO 315, or an amino acid sequence derived from SEQ ID NO 315 via substitution, deletion, or insertion of any one, two, three, four, five, or six amino acid residues, or an amino acid sequence having a homology (preferably an identity) of 60.0 % or more, 66.6 % or more, 73.3 % or more, 80.0 % or more, 86.6 % or more, or 93.3 % or more to the amino acid sequence defined in SEQ ID NO 315, or

(15) as a CDR grafting region H1 sequence, the amino acid sequence defined in SEQ ID NO 271, or an amino acid sequence derived from SEQ ID NO 271 via substitution, deletion, or insertion of any one, two, three, or four amino acid residues other than tyrosine at position 27, asparagine at position 32, and tryptophan at position 33,

as a CDR grafting region H2 sequence, the amino acid sequence defined in SEQ ID NO 273, or an amino acid sequence derived from SEQ ID NO 273 via substitution, deletion, or insertion of any one, two, three, four, five, or six amino acid residues other than aspartic acid at position 55, phenylalanine at position 57, threonine at position 58, asparagine at position 59, tyrosine at position 60, and asparagine at position 61, as a CDR grafting region H3 sequence, the amino acid sequence defined in SEQ ID NO 275, or an amino acid sequence defined in SEQ ID NO 275 via substitution, deletion, or insertion of any one, two, three, four, five, or six amino acid residues other than arginine at position 98, glycine at position 100, and glycine at position 109, or

(16) as a CDR grafting region H1 sequence, the amino acid sequence defined in SEQ ID NO 299, or an amino acid sequence derived from SEQ ID NO 299 via substitution, deletion, or insertion of any one, two, three, or four amino acid residues,

as a CDR grafting region H2 sequence, the amino acid sequence defined in SEQ ID NO 301, or an amino acid sequence derived from SEQ ID NO 301 via substitution, deletion, or insertion of any one, two, three, four, five, or six amino acid residues other than aspartic acid at position 52, tryptophan at position 54, aspartic acid at position 58, and proline at position 63, as a CDR grafting region H3 sequence, the amino acid sequence defined in SEQ ID NO 303, or an amino acid sequence derived from SEQ ID NO 303 via substitution, deletion, or insertion of any one, two, three, four, five, or six amino acid residues other than arginine at position 99, threonine at position 100, tyrosine at position 102, tyrosine at position 105, tyrosine at position 107, and methionine at position 110, or

(17) as a CDR-H1 sequence, the amino acid sequence defined in SEQ ID NO 177, or an amino acid sequence derived from SEQ ID NO 177 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 177,

as a CDR-H2 sequence, the amino acid sequence defined in SEQ ID NO 179, or an amino acid sequence derived from SEQ ID NO 179 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 179, and as a CDR-H3 sequence, the amino acid sequence defined in SEQ ID NO 181, or an amino acid sequence derived from SEQ ID NO 181 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 181, or

(18) as a CDR-H1 sequence, the amino acid sequence defined in SEQ ID NO 330, or an amino acid sequence derived from SEQ ID NO 330 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 330,

as a CDR-H2 sequence, the amino acid sequence defined in SEQ ID NO 331, or an amino acid sequence derived from SEQ ID NO 331 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 331, and as a CDR-H3 sequence, the amino acid sequence defined in SEQ ID NO 332, or an amino acid sequence derived from SEQ ID NO 332 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 332, or

(19) as a CDR-H1 sequence, the amino acid sequence defined in SEQ ID NO 337, or an amino acid sequence derived from SEQ ID NO 337 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 337,

as a CDR-H2 sequence, the amino acid sequence defined in SEQ ID NO 338, or an amino acid sequence derived from SEQ ID NO 338 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 338, and
as a CDR-H3 sequence, the amino acid sequence defined in SEQ ID NO 339, or an amino acid sequence derived from SEQ ID NO 339 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 339, or

(20) as a CDR-H1 sequence, the amino acid sequence defined in SEQ ID NO 344, or an amino acid sequence derived from SEQ ID NO 344 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 344,

as a CDR-H2 sequence, the amino acid sequence defined in SEQ ID NO 345, or an amino acid sequence derived from SEQ ID NO 345 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 345, and
as a CDR-H3 sequence, the amino acid sequence defined in SEQ ID NO 346, or an amino acid sequence derived from SEQ ID NO 346 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 346, or

(21) as a CDR grafting region H1 sequence, the amino acid sequence defined in SEQ ID NO 327, or an amino acid sequence derived from SEQ ID NO 327 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 327, as a CDR grafting region H2 sequence, the amino acid sequence defined in SEQ ID NO 328, or an amino acid sequence derived from SEQ ID NO 328 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 328,
as a CDR grafting region H3 sequence, the amino acid sequence defined in SEQ ID NO 329, or an amino acid sequence derived from SEQ ID NO 329 via substitution, deletion, or insertion of any one or two amino acid residues, or an amino acid sequence having a homology (preferably an identity) of 66.6 % or more or 83.3 % or more to the amino acid sequence defined in SEQ ID NO 329, or
(22) as a CDR grafting region H1 sequence, the amino acid sequence defined in SEQ ID NO 334, or an amino acid sequence derived from SEQ ID NO 334 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 334, as a CDR grafting region H2 sequence, the amino acid sequence defined in SEQ ID NO 335, or an amino acid sequence derived from SEQ ID NO 335 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 335,
as a CDR grafting region H3 sequence, the amino acid sequence defined in SEQ ID NO 336, or an amino acid sequence derived from SEQ ID NO 336 via substitution, deletion, or insertion of any one or two amino acid residues, or an amino acid sequence having a homology (preferably an identity) of 66.6 % or more or 83.3 % or more to the amino acid sequence defined in SEQ ID NO 336, or
(23) as a CDR grafting region H1 sequence, the amino acid sequence defined in SEQ ID NO 341, or an amino acid sequence derived from SEQ ID NO 341 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 341, as a CDR grafting region H2 sequence, the amino acid sequence defined in SEQ ID NO 342, or an amino acid sequence derived from SEQ ID NO 342 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 342,

as a CDR grafting region H3 sequence, the amino acid sequence defined in SEQ ID NO 343, or an amino acid sequence derived from SEQ ID NO 343 via substitution, deletion, or insertion of any one or two amino acid residues, or an amino acid sequence having a homology (preferably an identity) of 66.6 % or more or 83.3 % or more to the amino acid sequence defined in SEQ ID NO 343.

**9.** The pharmaceutical composition according to any one of claims 1 to 7, wherein the anti-gpNMB antibody comprises at least a heavy chain variable region comprising:

(1) the amino acid sequence defined in SEQ ID NO 13, or an amino acid sequence having a homology of 60 % or more to the amino acid sequence defined in SEQ ID NO 13, or

(2) the amino acid sequence defined in SEQ ID NO 29, or an amino acid sequence having a homology of 60 % or more to the amino acid sequence defined in SEQ ID NO 29, or

(3) the amino acid sequence defined in SEQ ID NO 45, or an amino acid sequence having a homology of 60 % or more to the amino acid sequence defined in SEQ ID NO 45, or

(4) the amino acid sequence defined in SEQ ID NO 61, or an amino acid sequence having a homology of 60 % or more to the amino acid sequence defined in SEQ ID NO 61, or

(5) the amino acid sequence defined in SEQ ID NO 77, or an amino acid sequence having a homology of 60 % or more to the amino acid sequence defined in SEQ ID NO 77, or

(6) the amino acid sequence defined in SEQ ID NO 93, or an amino acid sequence having a homology of 60 % or more to the amino acid sequence defined in SEQ ID NO 93, or

(7) the amino acid sequence defined in SEQ ID NO 109, or an amino acid sequence having a homology of 60 % or more to the amino acid sequence defined in SEQ ID NO 109, or

(8) the amino acid sequence defined in SEQ ID NO 125, or an amino acid sequence having a homology of 60 % or more to the amino acid sequence defined in SEQ ID NO 125, or

(9) the amino acid sequence defined in SEQ ID NO 141, or an amino acid sequence having a homology of 60 % or more to the amino acid sequence defined in SEQ ID NO 141, or

(10) the amino acid sequence defined in SEQ ID NO 157, or an amino acid sequence having a homology of 60 % or more to the amino acid sequence defined in SEQ ID NO 157, or

(11) the amino acid sequence defined in SEQ ID NO 173, or an amino acid sequence having a homology of 60 % or more to the amino acid sequence defined in SEQ ID NO 173, or

(12) the amino acid sequence defined in SEQ ID NO 267, or an amino acid sequence having a homology of 60 % or more to the amino acid sequence defined in SEQ ID NO 267, or

(13) the amino acid sequence defined in SEQ ID NO 295, or an amino acid sequence having a homology of 60 % or more to the amino acid sequence defined in SEQ ID NO 295, or

(14) the amino acid sequence defined in SEQ ID NO 323, or an amino acid sequence having a homology of 60 % or more to the amino acid sequence defined in SEQ ID NO 323, or

(17) the amino acid sequence defined in SEQ ID NO 189, or an amino acid sequence having a homology of 60 % or more to the amino acid sequence defined in SEQ ID NO 189, or

(18) the amino acid sequence defined in SEQ ID NO 333, or an amino acid sequence having a homology of 60 % or more to the amino acid sequence defined in SEQ ID NO 333, or

(19) the amino acid sequence defined in SEQ ID NO 340, or an amino acid sequence having a homology of 60 % or more to the amino acid sequence defined in SEQ ID NO 340, or

(20) the amino acid sequence defined in SEQ ID NO 347, or an amino acid sequence having a homology of 60 % or more to the amino acid sequence defined in SEQ ID NO 347.

**10.** The pharmaceutical composition according to claim 8 or 9, wherein the heavy chain variable region comprises, as a framework sequence, a framework sequence of a class of human immunoglobulin.

**11.** The pharmaceutical composition according to any one of claims 8 to 10, wherein the anti-gpNMB antibody further comprises a heavy chain constant region having an amino acid sequence of a heavy chain constant region in a class of human immunoglobulin.

12. The pharmaceutical composition according to any one of claims 1 to 11, wherein the anti-gpNMB antibody comprises at least a light chain variable region comprising:

(1) as a CDR-L1 sequence, the amino acid sequence defined in SEQ ID NO 7, or an amino acid sequence derived from SEQ ID NO 7 via substitution, deletion, or insertion of any one or two amino acid residues, or an amino acid sequence having a homology (preferably an identity) of 66.6 % or more or 83.3 % or more to the amino acid sequence defined in SEQ ID NO 7,

as a CDR-L2 sequence, the amino acid sequence defined in SEQ ID NO 9, or an amino acid sequence derived from SEQ ID NO 9 via substitution, deletion, or insertion of any one amino acid residue, or an amino acid sequence having a homology (preferably an identity) of 66.6 % or more to the amino acid sequence defined in SEQ ID NO 9, and
as a CDR-L3 sequence, the amino acid sequence defined in SEQ ID NO 11, or an amino acid sequence derived from SEQ ID NO 11 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 11, or

(2) as a CDR-L1 sequence, the amino acid sequence defined in SEQ ID NO 23, or an amino acid sequence derived from SEQ ID NO 23 via substitution, deletion, or insertion of any one or two amino acid residues, or an amino acid sequence having a homology (preferably an identity) of 60.0 % or more or 80.0 % or more to the amino acid sequence defined in SEQ ID NO 23,

as a CDR-L2 sequence, the amino acid sequence defined in SEQ ID NO 25, or an amino acid sequence derived from SEQ ID NO 25 via substitution, deletion, or insertion of any one amino acid residue, or an amino acid sequence having a homology (preferably an identity) of 66.6 % or more to the amino acid sequence defined in SEQ ID NO 25, and
as a CDR-L3 sequence, the amino acid sequence defined in SEQ ID NO 27, or an amino acid sequence derived from SEQ ID NO 27 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 27, or

(3) as a CDR-L1 sequence, the amino acid sequence defined in SEQ ID NO 39, or an amino acid sequence derived from SEQ ID NO 39 via substitution, deletion, or insertion of any one or two amino acid residues, or an amino acid sequence having a homology (preferably an identity) of 60.0 % or more or 80.0 % or more to the amino acid sequence defined in SEQ ID NO 39,

as a CDR-L2 sequence, the amino acid sequence defined in SEQ ID NO 41, or an amino acid sequence derived from SEQ ID NO 41 via substitution, deletion, or insertion of any one amino acid residue, or an amino acid sequence having a homology (preferably an identity) of 66.6 % or more to the amino acid sequence defined in SEQ ID NO 41, and
as a CDR-L3 sequence, the amino acid sequence defined in SEQ ID NO 43, or an amino acid sequence derived from SEQ ID NO 43 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 43, or

(4) as a CDR-L1 sequence, the amino acid sequence defined in SEQ ID NO 55, or an amino acid sequence derived from SEQ ID NO 55 via substitution, deletion, or insertion of any one or two amino acid residues, or an amino acid sequence having a homology (preferably an identity) of 66.6 % or more or 83.3 % or more to the amino acid sequence defined in SEQ ID NO 55,
as a CDR-L2 sequence, the amino acid sequence defined in SEQ ID NO 57, or an amino acid sequence derived from SEQ ID NO 57 via substitution, deletion, or insertion of any one amino acid residue, or an amino acid sequence having a homology (preferably an identity) of 66.6 % or more to the amino acid sequence defined in SEQ ID NO 57, and as a CDR-L3 sequence, the amino acid sequence defined in SEQ ID NO 59, or an amino acid sequence derived from SEQ ID NO 59 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 59, or

(5) as a CDR-L1 sequence, the amino acid sequence defined in SEQ ID NO 71, or an amino acid sequence derived from SEQ ID NO 71 via substitution, deletion, or insertion of any one or two amino acid residues, or an amino acid sequence having a homology (preferably an identity) of 60.0 % or more or 80.0 % or more to the amino acid sequence defined in SEQ ID NO 71,

as a CDR-L2 sequence, the amino acid sequence defined in SEQ ID NO 73, or an amino acid sequence derived from SEQ ID NO 73 via substitution, deletion, or insertion of any one amino acid residue, or an amino acid sequence having a homology (preferably an identity) of 66.6 % or more to the amino acid sequence defined in SEQ ID NO 73, and as a CDR-L3 sequence, the amino acid sequence defined in SEQ ID NO 75, or an amino acid sequence derived from SEQ ID NO 75 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 75, or

(6) as a CDR-L1 sequence, the amino acid sequence defined in SEQ ID NO 87, or an amino acid sequence derived from SEQ ID NO 87 via substitution, deletion, or insertion of any one or two amino acid residues, or an amino acid sequence having a homology (preferably an identity) of 66.6 % or more or 83.3 % or more to the amino acid sequence defined in SEQ ID NO 87,

as a CDR-L2 sequence, the amino acid sequence defined in SEQ ID NO 89, or an amino acid sequence derived from SEQ ID NO 89 via substitution, deletion, or insertion of any one amino acid residue, or an amino acid sequence having a homology (preferably an identity) of 66.6 % or more to the amino acid sequence defined in SEQ ID NO 89, and as a CDR-L3 sequence, the amino acid sequence defined in SEQ ID NO 91, or an amino acid sequence derived from SEQ ID NO 91 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 91, or

(7) as a CDR-L1 sequence, the amino acid sequence defined in SEQ ID NO 103, or an amino acid sequence derived from SEQ ID NO 103 via substitution, deletion, or insertion of any one or two amino acid residues, or an amino acid sequence having a homology (preferably an identity) of 66.6 % or more or 83.3 % or more to the amino acid sequence defined in SEQ ID NO 103,

as a CDR-L2 sequence, the amino acid sequence defined in SEQ ID NO 105, or an amino acid sequence derived from SEQ ID NO 105 via substitution, deletion, or insertion of any one amino acid residue, or an amino acid sequence having a homology (preferably an identity) of 66.6 % or more to the amino acid sequence defined in SEQ ID NO 105, and as a CDR-L3 sequence, the amino acid sequence defined in SEQ ID NO 107, or an amino acid sequence derived from SEQ ID NO 107 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 107, or

(8) as a CDR-L1 sequence, the amino acid sequence defined in SEQ ID NO 119, or an amino acid sequence derived from SEQ ID NO 119 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 119,

as a CDR-L2 sequence, the amino acid sequence defined in SEQ ID NO 121, or an amino acid sequence derived from SEQ ID NO 121 via substitution, deletion, or insertion of any one amino acid residue, or an amino acid sequence having a homology (preferably an identity) of 66.6 % or more to the amino acid sequence defined in SEQ ID NO 121, and as a CDR-L3 sequence, the amino acid sequence defined in SEQ ID NO 123, or an amino acid sequence derived from SEQ ID NO 123 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 123, or

(9) as a CDR-L1 sequence, the amino acid sequence defined in SEQ ID NO 135, or an amino acid sequence derived from SEQ ID NO 135 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 135,

as a CDR-L2 sequence, the amino acid sequence defined in SEQ ID NO 137, or an amino acid sequence derived from SEQ ID NO 137 via substitution, deletion, or insertion of any one amino acid residue, or an amino acid sequence having a homology (preferably an identity) of 66.6 % or more to the amino acid sequence defined in SEQ ID NO 137, and as a CDR-L3 sequence, the amino acid sequence defined in SEQ ID NO 139, or an amino acid sequence derived from SEQ ID NO 139 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 139, or

(10) as a CDR-L1 sequence, the amino acid sequence defined in SEQ ID NO 151, or an amino acid sequence derived from SEQ ID NO 151 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 151,
as a CDR-L2 sequence, the amino acid sequence defined in SEQ ID NO 153, or an amino acid sequence derived from SEQ ID NO 153 via substitution, deletion, or insertion of any one amino acid residue, or an amino acid sequence having a homology (preferably an identity) of 66.6 % or more to the amino acid sequence defined in SEQ ID NO 153, and as a CDR-L3 sequence, the amino acid sequence defined in SEQ ID NO 155, or an amino acid sequence derived from SEQ ID NO 155 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 155, or

(11) as a CDR-L1 sequence, the amino acid sequence defined in SEQ ID NO 167, or an amino acid sequence derived from SEQ ID NO 167 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 167,
as a CDR-L2 sequence, the amino acid sequence defined in SEQ ID NO 169, or an amino acid sequence derived from SEQ ID NO 169 via substitution, deletion, or insertion of any one amino acid residue, or an amino acid sequence having a homology (preferably an identity) of 66.6 % or more to the amino acid sequence defined in SEQ ID NO 169, and as a CDR-L3 sequence, the amino acid sequence defined in SEQ ID NO 171, or an amino acid sequence derived from SEQ ID NO 171 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 171, or

(12) as a CDR-L1 sequence, the amino acid sequence defined in SEQ ID NO 261, or an amino acid sequence derived from SEQ ID NO 261 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 261,
as a CDR-L2 sequence, the amino acid sequence defined in SEQ ID NO 263, or an amino acid sequence derived from SEQ ID NO 263 via substitution, deletion, or insertion of any one amino acid residue, or an amino acid sequence having a homology (preferably an identity) of 66.6 % or more to the amino acid sequence defined in SEQ ID NO 263, and as a CDR-L3 sequence, the amino acid sequence defined in SEQ ID NO 265, or an amino acid sequence derived from SEQ ID NO 265 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 265, or

(13) as a CDR-L1 sequence, the amino acid sequence defined in SEQ ID NO 289, or an amino acid sequence derived from SEQ ID NO 289 via substitution, deletion, or insertion of any one or two amino acid residues, or an amino acid sequence having a homology (preferably an identity) of 60.0 % or more or 80.0 % or more to the amino acid sequence defined in SEQ ID NO 289,
as a CDR-L2 sequence, the amino acid sequence defined in SEQ ID NO 291, or an amino acid sequence derived from SEQ ID NO 291 via substitution, deletion, or insertion of any one amino acid residue, or an amino acid sequence having a homology (preferably an identity) of 66.6 % or more to the amino acid sequence defined in SEQ ID NO 291, and as a CDR-L3 sequence, the amino acid sequence defined in SEQ ID NO 293, or an amino acid sequence derived from SEQ ID NO 293 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 293, or

(14) as a CDR-L1 sequence, the amino acid sequence defined in SEQ ID NO 317, or an amino acid sequence derived from SEQ ID NO 317 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 317,
as a CDR-L2 sequence, the amino acid sequence defined in SEQ ID NO 319, or an amino acid sequence derived from SEQ ID NO 319 via substitution, deletion, or insertion of any one amino acid residue, or an amino acid sequence having a homology (preferably an identity) of 66.6 % or more to the amino acid sequence defined in SEQ ID NO 319, and as a CDR-L3 sequence, the amino acid sequence defined in SEQ ID NO 321, or an amino acid sequence derived from SEQ ID NO 321 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 321, or

(15) as a CDR grafting region L1 sequence, the amino acid sequence defined in SEQ ID NO 277, or an amino acid sequence defined in SEQ ID NO 277 via substitution, deletion, or insertion of any one, two, three, or four amino acid residues other than isoleucine at position 29, tyrosine at position 31, and histidine at position 33,

as a CDR grafting region L2 sequence, the amino acid sequence defined in SEQ ID NO 279, or an amino acid sequence defined in SEQ ID NO 279 via substitution, deletion, or insertion of any one or two amino acid residue other than threonine at position 50,
as a CDR grafting region L3 sequence, the amino acid sequence defined in SEQ ID NO 281, or an amino acid sequence defined in SEQ ID NO 281 via substitution, deletion, or insertion of any one, two, or three amino acid residues other than histidine at position 88, glutamine at position 89, tryptophan at position 90, serine at position 92, tyrosine at position 93, proline at position 94, and cysteine at position 95, or

(16) as a CDR grafting region L1 sequence, the amino acid sequence defined in SEQ ID NO 305, or an amino acid sequence defined in SEQ ID NO 305 via substitution, deletion, or insertion of any one, two, three, four, five, or six amino acid residues other than leucine at position 31, tyrosine at position 37 and glutamic acid at position 39,

as a CDR grafting region L2 sequence, the amino acid sequence defined in SEQ ID NO 307, or an amino acid sequence defined in SEQ ID NO 307 via substitution, deletion, or insertion of any one or two amino acid residue other than lysine at position 55,
as a CDR grafting region L3 sequence, the amino acid sequence defined in SEQ ID NO 309, or an amino acid sequence defined in SEQ ID NO 309 via substitution, deletion, or insertion of any one, two, or three amino acid residues other than phenylalanine at position 94, or

(17) as a CDR-L1 sequence, the amino acid sequence defined in SEQ ID NO 183, or an amino acid sequence derived from SEQ ID NO 183 via substitution, deletion, or insertion of any one or two amino acid residues, or an amino acid sequence having a homology (preferably an identity) of 66.6 % or more or 83.3 % or more to the amino acid sequence defined in SEQ ID NO 183,

as a CDR-L2 sequence, the amino acid sequence defined in SEQ ID NO 185, or an amino acid sequence derived from SEQ ID NO 185 via substitution, deletion, or insertion of any one amino acid residue, or an amino acid sequence having a homology (preferably an identity) of 66.6 % or more to the amino acid sequence defined in SEQ ID NO 185,
as a CDR-L3 sequence, the amino acid sequence defined in SEQ ID NO 187, or an amino acid sequence derived from SEQ ID NO 187 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 187, or

(18) as a CDR-L1 sequence, the amino acid sequence defined in SEQ ID NO 351, or an amino acid sequence derived from SEQ ID NO 351 via substitution, deletion, or insertion of any one or two amino acid residues, or an amino acid sequence having a homology (preferably an identity) of 66.6 % or more or 83.3 % or more to the amino acid sequence defined in SEQ ID NO 351,

as a CDR-L2 sequence, the amino acid sequence defined in SEQ ID NO 352, or an amino acid sequence derived from SEQ ID NO 352 via substitution, deletion, or insertion of any one amino acid residue, or an amino acid sequence having a homology (preferably an identity) of 66.6 % or more to the amino acid sequence defined in SEQ ID NO 352,
as a CDR-L3 sequence, the amino acid sequence defined in SEQ ID NO 353, or an amino acid sequence derived from SEQ ID NO 353 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 353, or

(19) as a CDR-L1 sequence, the amino acid sequence defined in SEQ ID NO 358, or an amino acid sequence derived from SEQ ID NO 358 via substitution, deletion, or insertion of any one or two amino acid residues, or an amino acid sequence having a homology (preferably an identity) of 66.6 % or more or 83.3 % or more to the amino acid sequence defined in SEQ ID NO 358,

as a CDR-L2 sequence, the amino acid sequence defined in SEQ ID NO 359, or an amino acid sequence derived from SEQ ID NO 359 via substitution, deletion, or insertion of any one amino acid residue, or an amino acid sequence having a homology (preferably an identity) of 66.6 % or more to the amino acid sequence defined in SEQ ID NO 359,
as a CDR-L3 sequence, the amino acid sequence defined in SEQ ID NO 360, or an amino acid sequence derived from SEQ ID NO 360 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 360, or

(20) as a CDR-L1 sequence, the amino acid sequence defined in SEQ ID NO 365, or an amino acid sequence derived from SEQ ID NO 365 via substitution, deletion, or insertion of any one or two amino acid residues, or an amino acid sequence having a homology (preferably an identity) of 66.6 % or more or 83.3 % or more to the amino acid sequence defined in SEQ ID NO 365,

as a CDR-L2 sequence, the amino acid sequence defined in SEQ ID NO 366, or an amino acid sequence derived from SEQ ID NO 366 via substitution, deletion, or insertion of any one amino acid residue, or an amino acid sequence having a homology (preferably an identity) of 66.6 % or more to the amino acid sequence defined in SEQ ID NO 366,

as a CDR-L3 sequence, the amino acid sequence defined in SEQ ID NO 367, or an amino acid sequence derived from SEQ ID NO 367 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 367, or

(21) as a CDR grafting region L1 sequence, the amino acid sequence defined in SEQ ID NO 348, or an amino acid sequence derived from SEQ ID NO 348 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 348, as a CDR grafting region L2 sequence, the amino acid sequence defined in SEQ ID NO 349, or an amino acid sequence derived from SEQ ID NO 349 via substitution, deletion, or insertion of any one or two amino acid residues, or an amino acid sequence having a homology (preferably an identity) of 71.4 % or more or 85.7 % or more to the amino acid sequence defined in SEQ ID NO 349,

as a CDR grafting region L3 sequence, the amino acid sequence defined in SEQ ID NO 350, or an amino acid sequence derived from SEQ ID NO 350 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 350, or

(22) as a CDR grafting region L1 sequence, the amino acid sequence defined in SEQ ID NO 355, or an amino acid sequence derived from SEQ ID NO 355 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 355, as a CDR grafting region L2 sequence, the amino acid sequence defined in SEQ ID NO 356, or an amino acid sequence derived from SEQ ID NO 356 via substitution, deletion, or insertion of any one or two amino acid residues, or an amino acid sequence having a homology (preferably an identity) of 71.4 % or more or 85.7 % or more to the amino acid sequence defined in SEQ ID NO 356,

as a CDR grafting region L3 sequence, the amino acid sequence defined in SEQ ID NO 357, or an amino acid sequence derived from SEQ ID NO 357 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 357, or

(23) as a CDR grafting region L1 sequence, the amino acid sequence defined in SEQ ID NO 362, or an amino acid sequence derived from SEQ ID NO 362 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 362, as a CDR grafting region L2 sequence, the amino acid sequence defined in SEQ ID NO 363, or an amino acid sequence derived from SEQ ID NO 363 via substitution, deletion, or insertion of any one or two amino acid residues, or an amino acid sequence having a homology (preferably an identity) of 71.4 % or more or 85.7 % or more to the amino acid sequence defined in SEQ ID NO 363,

as a CDR grafting region L3 sequence, the amino acid sequence defined in SEQ ID NO 364, or an amino acid sequence derived from SEQ ID NO 364 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 364.

13. The pharmaceutical composition according to any one of claims 1 to 11, wherein the anti-gpNMB antibody comprises at least a light chain variable region comprising:

(1) the amino acid sequence defined in SEQ ID NO 15, or an amino acid sequence having a homology of 60 % or more to the amino acid sequence defined in SEQ ID NO 15, or

(2) the amino acid sequence defined in SEQ ID NO 31, or an amino acid sequence having a homology of 60 % or more to the amino acid sequence defined in SEQ ID NO 31, or

(3) the amino acid sequence defined in SEQ ID NO 47, or an amino acid sequence having a homology of 60 % or more to the amino acid sequence defined in SEQ ID NO 47, or

(4) the amino acid sequence defined in SEQ ID NO 63, or an amino acid sequence having a homology of 60 % or more to the amino acid sequence defined in SEQ ID NO 63, or

(5) the amino acid sequence defined in SEQ ID NO 79, or an amino acid sequence having a homology of 60 % or more to the amino acid sequence defined in SEQ ID NO 79, or

(6) the amino acid sequence defined in SEQ ID NO 95, or an amino acid sequence having a homology of 60 % or more to the amino acid sequence defined in SEQ ID NO 95, or

(7) the amino acid sequence defined in SEQ ID NO 111, or an amino acid sequence having a homology of 60 % or more to the amino acid sequence defined in SEQ ID NO 111, or

(8) the amino acid sequence defined in SEQ ID NO 127, or an amino acid sequence having a homology of 60 % or more to the amino acid sequence defined in SEQ ID NO 127, or

(9) the amino acid sequence defined in SEQ ID NO 143, or an amino acid sequence having a homology of 60 % or more to the amino acid sequence defined in SEQ ID NO 143, or

(10) the amino acid sequence defined in SEQ ID NO 159, or an amino acid sequence having a homology of 60 % or more to the amino acid sequence defined in SEQ ID NO 159, or

(11) the amino acid sequence defined in SEQ ID NO 175, or an amino acid sequence having a homology of 60 % or more to the amino acid sequence defined in SEQ ID NO 175, or

(12) the amino acid sequence defined in SEQ ID NO 269, or an amino acid sequence having a homology of 60 % or more to the amino acid sequence defined in SEQ ID NO 269, or

(13) the amino acid sequence defined in SEQ ID NO 297, or an amino acid sequence having a homology of 60 % or more to the amino acid sequence defined in SEQ ID NO 297, or

(14) the amino acid sequence defined in SEQ ID NO 325, or an amino acid sequence having a homology of 60 % or more to the amino acid sequence defined in SEQ ID NO 325, or

(17) the amino acid sequence defined in SEQ ID NO 191, or an amino acid sequence having a homology of 60 % or more to the amino acid sequence defined in SEQ ID NO 191, or

(18) the amino acid sequence defined in SEQ ID NO 354, or an amino acid sequence having a homology of 60 % or more to the amino acid sequence defined in SEQ ID NO 354, or

(19) the amino acid sequence defined in SEQ ID NO 361, or an amino acid sequence having a homology of 60 % or more to the amino acid sequence defined in SEQ ID NO 361, or

(20) the amino acid sequence defined in SEQ ID NO 368, or an amino acid sequence having a homology of 60 % or more to the amino acid sequence defined in SEQ ID NO 368.

**14.** The pharmaceutical composition according to claim 12 or 13, wherein the light chain variable region comprises, as a framework sequence, a framework sequence of a class of human immunoglobulin.

**15.** The pharmaceutical composition according to any one of claims 12 to 14, wherein the anti-gpNMB antibody further comprises a light chain constant region having an amino acid sequence of a light chain constant region in a class of human immunoglobulin.

**16.** The pharmaceutical composition according to any one of claims 1 to 15, wherein the anti-gpNMB antibody is a Fab, scFv, Diabody, Nanobody, VHH, bispecific antibody, or multispecific antibody, or a derivative thereof.

**17.** A pharmaceutical composition for removing disease-related cells, comprising an anti-gpNMB antibody or its fragment or a derivative thereof that binds to at least one site in a region from V78 to a PKD domain of human gpNMB (glycoprotein nonmetastatic melanoma protein B) having the amino acid sequence of SEQ ID NO 209 competitively with an anti-gpNMB antibody or its fragment or a derivative thereof according to any one of claims 1 to 16.

**18.** A pharmaceutical composition for removing disease-related cells, comprising:

a nucleic acid molecule having a polynucleotide sequence encoding an anti-gpNMB antibody or its fragment or a derivative thereof according to any one of claims 1 to 16; and/or
a cloning vector or expression vector carrying at least one of the nucleic acid molecule; and/or
a recombinant cell into which the vector has been introduced.

**19.** The pharmaceutical composition according to any one of claims 1 to 18, wherein the disease-related cells are selected from senescent cells, cancer-related cells, and phagocytes.

20. The pharmaceutical composition according to claim 19, wherein the cancer-related cells are selected from cancer cells, cancer stem cells, cancer progenitor cells, and cancer infiltration cells.

21. The pharmaceutical composition according to any one of claims 1 to 20, wherein the disease is selected from obesity, diabetes, arteriosclerosis, fibrosis, cancer, metabolic disease, circulatory disease, lysosomal stress-associated disease (e.g., lysosome disease), autoimmune disease (e.g., multiple sclerosis), neuropathic pain, fibromyalgia, and glioblastoma.

22. The pharmaceutical composition according to any one of claims 1 to 21, for used in treating or preventing a disease by removing disease-related cells.

23. An anti-gpNMB antibody or its fragment or a derivative thereof that binds specifically to at least one site in a region from V78 to S92 of human gpNMB (glycoprotein nonmetastatic melanoma protein B) having the amino acid sequence of SEQ ID NO 209.

24. The anti-gpNMB antibody or its fragment or a derivative thereof according to claim 23, wherein the anti-gpNMB antibody also binds specifically to a region containing amino acid residue K186 of human gpNMB having the amino acid sequence of SEQ ID NO 209.

25. The anti-gpNMB antibody or its fragment or a derivative thereof according to claim 23 or 24, wherein the anti-gpNMB antibody also binds specifically to at least one site in a region from V78 to S92 of mouse gpNMB having the amino acid sequence of SEQ ID NO 211.

26. The anti-gpNMB antibody or its fragment or a derivative thereof according to any one of claims 23 to 25, which is a monoclonal antibody or its fragment or a derivative thereof.

27. The anti-gpNMB antibody or its fragment or a derivative thereof according to claim 26, further comprising at least a heavy chain variable region comprising:

(17) as a CDR-H1 sequence, the amino acid sequence defined in SEQ ID NO 177, or an amino acid sequence derived from SEQ ID NO 177 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 177,

as a CDR-H2 sequence, the amino acid sequence defined in SEQ ID NO 179, or an amino acid sequence derived from SEQ ID NO 179 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 179, and
as a CDR-H3 sequence, the amino acid sequence defined in SEQ ID NO 181, or an amino acid sequence derived from SEQ ID NO 181 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 181, or

(18) as a CDR-H1 sequence, the amino acid sequence defined in SEQ ID NO 330, or an amino acid sequence derived from SEQ ID NO 330 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 330,

as a CDR-H2 sequence, the amino acid sequence defined in SEQ ID NO 331, or an amino acid sequence derived from SEQ ID NO 331 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 331, and
as a CDR-H3 sequence, the amino acid sequence defined in SEQ ID NO 332, or an amino acid sequence derived from SEQ ID NO 332 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 332, or

(19) as a CDR-H1 sequence, the amino acid sequence defined in SEQ ID NO 337, or an amino acid sequence derived from SEQ ID NO 337 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 337,

as a CDR-H2 sequence, the amino acid sequence defined in SEQ ID NO 338, or an amino acid sequence derived from SEQ ID NO 338 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 338, and
as a CDR-H3 sequence, the amino acid sequence defined in SEQ ID NO 339, or an amino acid sequence derived from SEQ ID NO 339 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 339, or

(20) as a CDR-H1 sequence, the amino acid sequence defined in SEQ ID NO 344, or an amino acid sequence derived from SEQ ID NO 344 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 344,

as a CDR-H2 sequence, the amino acid sequence defined in SEQ ID NO 345, or an amino acid sequence derived from SEQ ID NO 345 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 345, and
as a CDR-H3 sequence, the amino acid sequence defined in SEQ ID NO 346, or an amino acid sequence derived from SEQ ID NO 346 via substitution, deletion, or insertion of any one, two, or three amino acid residues, or an amino acid sequence having a homology (preferably an identity) of 62.5 % or more, 75.0 % or more, or 87.5 % or more to the amino acid sequence defined in SEQ ID NO 346, or

(21) as a CDR grafting region H1 sequence, the amino acid sequence defined in SEQ ID NO 327, or an amino acid sequence derived from SEQ ID NO 327 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 327, as a CDR grafting region H2 sequence, the amino acid sequence defined in SEQ ID NO 328, or an amino acid sequence derived from SEQ ID NO 328 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 328,
as a CDR grafting region H3 sequence, the amino acid sequence defined in SEQ ID NO 329, or an amino acid sequence derived from SEQ ID NO 329 via substitution, deletion, or insertion of any one or two amino acid residues, or an amino acid sequence having a homology (preferably an identity) of 66.6 % or more or 83.3 % or more to the amino acid sequence defined in SEQ ID NO 329, or
(22) as a CDR grafting region H1 sequence, the amino acid sequence defined in SEQ ID NO 334, or an amino acid sequence derived from SEQ ID NO 334 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 334, as a CDR grafting region H2 sequence, the amino acid sequence defined in SEQ ID NO 335, or an amino acid sequence derived from SEQ ID NO 335 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 335,
as a CDR grafting region H3 sequence, the amino acid sequence defined in SEQ ID NO 336, or an amino acid sequence derived from SEQ ID NO 336 via substitution, deletion, or insertion of any one or two amino acid residues, or an amino acid sequence having a homology (preferably an identity) of 66.6 % or more or 83.3 % or more to the amino acid sequence defined in SEQ ID NO 336, or
(23) as a CDR grafting region H1 sequence, the amino acid sequence defined in SEQ ID NO 341, or an amino acid sequence derived from SEQ ID NO 341 via substitution, deletion, or insertion of any one, two, three, or four amino acid residues, or an amino acid sequence having a homology (preferably an identity) of 60.0 % or more, 70.0 % or more, 80.0 % or more, or 90.0 % or more to the amino acid sequence defined in SEQ ID NO 341, as a CDR grafting region H2 sequence, the amino acid sequence defined in SEQ ID NO 342, or an amino acid sequence derived from SEQ ID NO 342 via substitution, deletion, or insertion of any one, two, three, four, five, or six amino acid residues, or an amino acid sequence having a homology (preferably an identity) of 64.7 % or more, 70.5 % or more, 76.4 % or more, 82.3 % or more, 88.2 % or more, or 94.1 % or more to the amino acid sequence defined in SEQ ID NO 342,

as a CDR grafting region H3 sequence, the amino acid sequence defined in SEQ ID NO 343, or an amino acid sequence derived from SEQ ID NO 343 via substitution, deletion, or insertion of any one or two amino acid residues, or an amino acid sequence having a homology (preferably an identity) of 66.6 % or more or 83.3 % or more to the amino acid sequence defined in SEQ ID NO 343.

28. The anti-gpNMB antibody or its fragment or a derivative thereof according to claim 26 or 27, wherein the anti-gpNMB antibody comprises at least a heavy chain variable region comprising:

(17) the amino acid sequence defined in SEQ ID NO 189, or an amino acid sequence having a homology of 60 % or more to the amino acid sequence defined in SEQ ID NO 189, or

(18) the amino acid sequence defined in SEQ ID NO 333, or an amino acid sequence having a homology of 60 % or more to the amino acid sequence defined in SEQ ID NO 333, or

(19) the amino acid sequence defined in SEQ ID NO 340, or an amino acid sequence having a homology of 60 % or more to the amino acid sequence defined in SEQ ID NO 340, or

(20) the amino acid sequence defined in SEQ ID NO 347, or an amino acid sequence having a homology of 60 % or more to the amino acid sequence defined in SEQ ID NO 347.

29. The anti-gpNMB antibody or its fragment or a derivative thereof according to any one of claims 25 to 28, wherein the heavy chain variable region comprises, as a framework sequence, a framework sequence of a class of human immunoglobulin.

30. The anti-gpNMB antibody or its fragment or a derivative thereof according to any one of claims 25 to 29, further comprising a heavy chain constant region having an amino acid sequence of a heavy chain constant region in a class of human immunoglobulin.

31. The anti-gpNMB antibody or its fragment or a derivative thereof according to any one of claims 25 to 30, further comprising at least a light chain variable region comprising:

(17) as a CDR-L1 sequence, the amino acid sequence defined in SEQ ID NO 183, or an amino acid sequence derived from SEQ ID NO 183 via substitution, deletion, or insertion of any one or two amino acid residues, or an amino acid sequence having a homology (preferably an identity) of 66.6 % or more or 83.3 % or more to the amino acid sequence defined in SEQ ID NO 183,

as a CDR-L2 sequence, the amino acid sequence defined in SEQ ID NO 185, or an amino acid sequence derived from SEQ ID NO 185 via substitution, deletion, or insertion of any one amino acid residue, or an amino acid sequence having a homology (preferably an identity) of 66.6 % or more to the amino acid sequence defined in SEQ ID NO 185,

as a CDR-L3 sequence, the amino acid sequence defined in SEQ ID NO 187, or an amino acid sequence derived from SEQ ID NO 187 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 187, or

(18) as a CDR-L1 sequence, the amino acid sequence defined in SEQ ID NO 351, or an amino acid sequence derived from SEQ ID NO 351 via substitution, deletion, or insertion of any one or two amino acid residues, or an amino acid sequence having a homology (preferably an identity) of 66.6 % or more or 83.3 % or more to the amino acid sequence defined in SEQ ID NO 351,

as a CDR-L2 sequence, the amino acid sequence defined in SEQ ID NO 352, or an amino acid sequence derived from SEQ ID NO 352 via substitution, deletion, or insertion of any one amino acid residue, or an amino acid sequence having a homology (preferably an identity) of 66.6 % or more to the amino acid sequence defined in SEQ ID NO 352,

as a CDR-L3 sequence, the amino acid sequence defined in SEQ ID NO 353, or an amino acid sequence derived from SEQ ID NO 353 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 353, or

(19) as a CDR-L1 sequence, the amino acid sequence defined in SEQ ID NO 358, or an amino acid sequence derived from SEQ ID NO 358 via substitution, deletion, or insertion of any one or two amino acid residues, or an amino acid sequence having a homology (preferably an identity) of 66.6 % or more or 83.3 % or more to the amino acid sequence defined in SEQ ID NO 358,

as a CDR-L2 sequence, the amino acid sequence defined in SEQ ID NO 359, or an amino acid sequence derived from SEQ ID NO 359 via substitution, deletion, or insertion of any one amino acid residue, or an amino acid sequence having a homology (preferably an identity) of 66.6 % or more to the amino acid sequence defined in SEQ ID NO 359,
as a CDR-L3 sequence, the amino acid sequence defined in SEQ ID NO 360, or an amino acid sequence derived from SEQ ID NO 360 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 360, or

(20) as a CDR-L1 sequence, the amino acid sequence defined in SEQ ID NO 365, or an amino acid sequence derived from SEQ ID NO 365 via substitution, deletion, or insertion of any one or two amino acid residues, or an amino acid sequence having a homology (preferably an identity) of 66.6 % or more or 83.3 % or more to the amino acid sequence defined in SEQ ID NO 365,

as a CDR-L2 sequence, the amino acid sequence defined in SEQ ID NO 366, or an amino acid sequence derived from SEQ ID NO 366 via substitution, deletion, or insertion of any one amino acid residue, or an amino acid sequence having a homology (preferably an identity) of 66.6 % or more to the amino acid sequence defined in SEQ ID NO 366,
as a CDR-L3 sequence, the amino acid sequence defined in SEQ ID NO 367, or an amino acid sequence derived from SEQ ID NO 367 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 367, or

(21) as a CDR grafting region L1 sequence, the amino acid sequence defined in SEQ ID NO 348, or an amino acid sequence derived from SEQ ID NO 348 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 348, as a CDR grafting region L2 sequence, the amino acid sequence defined in SEQ ID NO 349, or an amino acid sequence derived from SEQ ID NO 349 via substitution, deletion, or insertion of any one or two amino acid residues, or an amino acid sequence having a homology (preferably an identity) of 71.4 % or more or 85.7 % or more to the amino acid sequence defined in SEQ ID NO 349,
as a CDR grafting region L3 sequence, the amino acid sequence defined in SEQ ID NO 350, or an amino acid sequence derived from SEQ ID NO 350 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 350, or
(22) as a CDR grafting region L1 sequence, the amino acid sequence defined in SEQ ID NO 355, or an amino acid sequence derived from SEQ ID NO 355 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 355, as a CDR grafting region L2 sequence, the amino acid sequence defined in SEQ ID NO 356, or an amino acid sequence derived from SEQ ID NO 356 via substitution, deletion, or insertion of any one or two amino acid residues, or an amino acid sequence having a homology (preferably an identity) of 71.4 % or more or 85.7 % or more to the amino acid sequence defined in SEQ ID NO 356,
as a CDR grafting region L3 sequence, the amino acid sequence defined in SEQ ID NO 357, or an amino acid sequence derived from SEQ ID NO 357 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 357, or
(23) as a CDR grafting region L1 sequence, the amino acid sequence defined in SEQ ID NO 362, or an amino acid sequence derived from SEQ ID NO 362 via substitution, deletion, or insertion of any one, two, three, or four amino acid residues, or an amino acid sequence having a homology (preferably an identity) of 63.6 % or more, 72.7 % or more, 81.8 % or more, or 90.9 % or more to the amino acid sequence defined in SEQ ID NO 362, as a CDR grafting region L2 sequence, the amino acid sequence defined in SEQ ID NO 363, or an amino acid sequence derived from SEQ ID NO 363 via substitution, deletion, or insertion of any one or two amino acid residues, or an amino acid sequence having a homology (preferably an identity) of 71.4 % or more or 85.7 % or more to the amino acid sequence defined in SEQ ID NO 363,

as a CDR grafting region L3 sequence, the amino acid sequence defined in SEQ ID NO 364, or an amino acid sequence derived from SEQ ID NO 364 via substitution, deletion, or insertion of any one, two, or three amino acid residues, or an amino acid sequence having a homology (preferably an identity) of 66.6 % or more, 77.7 % or more, or 88.8 % or more to the amino acid sequence defined in SEQ ID NO 364.

32. The anti-gpNMB antibody or its fragment or a derivative thereof according to any one of claims 25 to 31, further comprising at least a light chain variable region comprising:

(17) the amino acid sequence defined in SEQ ID NO 191, or an amino acid sequence having a homology of 60 % or more to the amino acid sequence defined in SEQ ID NO 191, or

(18) the amino acid sequence defined in SEQ ID NO 354, or an amino acid sequence having a homology of 60 % or more to the amino acid sequence defined in SEQ ID NO 354, or

(19) the amino acid sequence defined in SEQ ID NO 361, or an amino acid sequence having a homology of 60 % or more to the amino acid sequence defined in SEQ ID NO 361, or

(20) the amino acid sequence defined in SEQ ID NO 368, or an amino acid sequence having a homology of 60 % or more to the amino acid sequence defined in SEQ ID NO 368.

33. The anti-gpNMB antibody or its fragment or a derivative thereof according to claim 31 or 32, wherein the light chain variable region comprises, as a framework sequence, a framework sequence of a class of human immunoglobulin.

34. The anti-gpNMB antibody or its fragment or a derivative thereof according to any one of claims 25 to 33, further comprising a light chain constant region having an amino acid sequence of a light chain constant region in a class of human immunoglobulin.

35. The anti-gpNMB antibody or its fragment or a derivative thereof according to any one of claims 25 to 34, which is a Fab, scFv, Diabody, Nanobody, VHH, bispecific antibody, or multispecific antibody, or a derivative thereof.

# Figure 1

```
h_gpNMB_iso_a    MECLYYFLGFLLLAARLPLDAAKRFHDVLGNERPSAYMREHNQLNGWSSDENDWNEKLYP
m_gpNMB          MESLCGVLGFLLLAAGLPLQAAKRFRDVLGHEQYPDHMREHNQLRGWSSDENEWDEHLYP
                 **.* *  .******** ***:****:****:*: ,  :******,.*******.*:*.***

h_gpNMB_iso_a    VWKRGDMRWKNSWKGGRVQAVLTSDSPALVGSNITFAVNLLFPRCQKEDANGNIVYEKNC
m_gpNMB          VWRRGDGRWKDSWEGGRVQAVLTSDSPALVGSNITFVVNLVFPRCQKEDANGNIVYEKNC
                 **:*** ***:**:*******************. ***:*****************

h_gpNMB_iso_a    RNEAGLSADPYVYNWTAWSEDSDGENGTGQSHHNVFPDGKPFPHHPGWRRWNFIYVFHTL
m_gpNMB          RNDLGLTSDLHVYNWTAGADDGDWEDGTSRSQHLRFPDRRPFPRPHGWKKWSFVYVFHTL
                 **: **:* :****** :*. *.*:*. :*:* *** :***.  **:*.*:*********

h_gpNMB_iso_a    GQYFQKLGRCSVRVSVNTANVTLGPQLMEVTVYRRHGRAYVPIAQVKDVYVVTDQIPVFV
m_gpNMB          GQYFQKLGRCSARVSINTVNLTAGPQVMEVTVFRRYGRAYIPISKVKDVYVITDQIPVFV
                 ***********.***.**.*:*.***:*****:**:****:**:.******:********

h_gpNMB_iso_a    TMFQKNDRNSSDETFLKDLPIMFDVLIHDPSHFLNYSTINYKWSFGDNTGLFVSTNHTVN
m_gpNMB          TMSQKNDRNLSDEIFLRDLPIVFDVLIHDPSHFLNDSAISYKWNFGDNTGLFVSNNHTLN
                 ** ****** *** **:****:************** **:***:.*********** ***:*

h_gpNMB_iso_a    HTYVLNGTFSLNLTVKAAAPGPCPPP----PPPPRPSKPTPSLATTLKSYDSNTPGPAGD
m_gpNMB          HTYVLNGTFNLNLTVQTAVPGPCPPPSPSTPPPPSTPPSPPPSPLPTLSTPSPSLMPTGY
                 ********* *****:*. ******    ****,..,*.. *  * *: *:*

h_gpNMB_iso_a    NPLELSRIPDENCQINRYGHFQATITIVEGILEVNIIQMTDVLMPVPWPESSLIDFVVTC
m_gpNMB          KSMELSDISNENCRINRYGYFRATITIVEGILEVSIMQIADVPMPTPQPANSLMDFTVTC
                 :. :*** *.:***:*****:*:************.*:*:.** **.* * .**:**.***

h_gpNMB_iso_a    QGSIPTEVCTIISDPTCEITQNTVCSPVDVDEMCLLTVRRTFNGSGTYCVNLTLGDDTSL
m_gpNMB          KGATPMEACTIISDPTCQIAQNRVCSPVAVDGLCLLSVRRAFNGSGTYCVNFTLGDDASL
                 :*: * *.***********:** ***** :*:***.:***:**********:*****:**

h_gpNMB_iso_a    ALTSTLISVPDRDPASPLRMANSALISVGCLAIFVTVISLLVYKKHKEYNPIENSPGNVV
m_gpNMB          ALTSTLISIPGKDPDSPLRAVNGVLISIGCLAVLVTMVTILLYKKHKAYKPIGNCPRNTV
                 ********:*.:** **** .*.:*:**.*:***:*.*:***** :* :*:.* .*

h_gpNMB_iso_a    RSKGLSVFLNRAKAVFFPGNQEKDPLLKNQEFKGVS
m_gpNMB          KGKGLSVLLSHAKAPFFRGDQEKDPLLQDKPRTL--
                 :.*****:*.:*** ** *:*******::: .
```

Region consisting of amino acid residues from V78 to S92

PMEL-CAF-like domain

PKD domain

Figure 2

Fluorescence intensity ( a.u. )

Antibody added

Non | NC IgG | GPN15-2 | GPN15-3 | GPN18-2 | GPN18-5

Figure 3

EP 4 772 193 A1

Figure 4

GPN06-1_VH    (1)  QVQLQQPGAEIVRPGASVKLSCKASGYTFTDNWMGWVKQRPGQGLEWIGA  (50)
hGPN06-1_VH1  (1)  QVQLVQSGAEVKKPGASVKVSCKASGYTFTDNWMGWVKQRPGQGLEWIGA  (50)
hGPN06-1_VH2  (1)  QVQLVQSGAEVKKPGASVKVSCQASGYTFTDNWMGWVRQAPGQGLEWMGA  (50)
hGPN06-1_VH3  (1)  QVQLVQSGAEIVRPGASVKLSCKASGYTFTDNWMGWVRQRPGQGLEWIGA  (50)
hGPN06-1_VH4  (1)  QVQLVESGAEVKKPGASVKLSCKASGYTFTDNWMGWVRQRPGQRLEWMGA  (50)
hGPN06-1_VH5  (1)  QVQLVQSGAEVKKPGASVKVSCKASGYTFTDNWMGWVKQRPGQGLEWIGA  (50)

GPN06-1_VH    (51) IDPSDSFTNYNQNFKGKATLTVDTSSSTAYMQLSSLTSEDSAVYFCTRSG  (100)
hGPN06-1_VH1  (51) IDPSDSFTNYNQNFKGRVTMTVDTSISTAYMELSSLRSDDSAVYFCTRSG  (100)
hGPN06-1_VH2  (51) IDPSDSFTNYNQNFKGRVTLTVDTSTSTVYMQLSSLTSEDTAVYFCTRSG  (100)
hGPN06-1_VH3  (51) IDPSDSFTNYNQNFKGRVTMTRDTSTSTVYMELSSLTSEDTAVYFCTRSG  (100)
hGPN06-1_VH4  (51) IDPSDSFTNYNQNFKGRVTITVDKSASTAYMELSSLRSEDTAVYFCTRSG  (100)
hGPN06-1_VH5  (51) IDPSDSFTNYNQNFKGRVTMTRDTSTSTVYMELSSLRSEDSAVYFCTRSG  (100)

GPN06-1_VH    (101) YYGSPKLGGDYWGQGTTLTVSS  (122)
hGPN06-1_VH1  (101) YYGSPKLGGDYWGQGTLVTVSS  (122)
hGPN06-1_VH2  (101) YYGSPKLGGDYWGQGTLVTVSS  (122)
hGPN06-1_VH3  (101) YYGSPKLGGDYWGQGTLVTVSS  (122)
hGPN06-1_VH4  (101) YYGSPKLGGDYWGQGTLVTVSS  (122)
hGPN06-1_VH5  (101) YYGSPKLGGDYWGQGTTLTVSS  (122)

# Figure 5

```
GPN06-1_VL    (1)  Q I V L T Q S P A I M S A S L G E E I T L T C S A S S S I S Y M H W Y Q Q K S G T S P K L L I Y S T  (50)
hGPN06-1_VL1  (1)  E I V L T Q S P S S L S A S V G D R V T I T C S A S S S I S Y M H W Y Q Q K S G T A P K L L I Y S T  (50)
hGPN06-1_VL2  (1)  D I Q L T Q S P S S L S A S V G D R V T I T C S A S S S I S Y M H W Y Q Q K P G K G P K L L I Y S T  (50)
hGPN06-1_VL3  (1)  D I V M T Q S P S T L S A S V G E E V T L T C S A S S S I S Y M H W Y Q Q K P G T S P K L L I Y S T  (50)
hGPN06-1_VL4  (1)  D V V M T Q S P S S L S A S V G E E V T I T C S A S S S I S Y M H W Y Q Q K P G T A P K L L I Y S T  (50)
hGPN06-1_VL5  (1)  D I Q L T Q S P S I L S A S V G D R V T I T C S A S S S I S Y M H W Y Q Q K P G T S P K L L I Y S T  (50)

GPN06-1_VL    (51) S N L A S G V P S R F S G S G S G T F Y S L T I S S V E A E D A A D Y Y C H Q W N S Y P C T F G G G  (100)
hGPN06-1_VL1  (51) S N L A S G V P S R F S G S G S G T D F T F T I S S L Q P E D A A T Y Y C H Q W N S Y P C T F G G G  (100)
hGPN06-1_VL2  (51) S N L A S G V P S R F S G S G S G T D F S L T I S S L Q P E D A A D Y Y C H Q W N S Y P C T F G G G  (100)
hGPN06-1_VL3  (51) S N L A S G V P S R F S G S G S G T E F T L T I S S L Q P D D F A T Y Y C H Q W N S Y P C T F G Q G  (100)
hGPN06-1_VL4  (51) S N L A S G V P S R F S G S G S G T D F T L T I S S L Q P E D A A T Y Y C H Q W N S Y P C T F G G G  (100)
hGPN06-1_VL5  (51) S N L A S G V P S R F S G S G S G T E F T L T I S S L Q P E D A A D Y Y C H Q W N S Y P C T F G G G  (100)

GPN06-1_VL    (101) T K L E I K
hGPN06-1_VL1  (101) T K L E I K
hGPN06-1_VL2  (101) T K V E I K
hGPN06-1_VL3  (101) T K V E I K
hGPN06-1_VL4  (101) T K L E I K
hGPN06-1_VL5  (101) T K L E I K
```

# Figure 6

```
GPN18-2_VH   (1)  Q V T L K E S G P G I L Q P S Q T L S L T C S F S G F S L S T S V M G V G W I R Q P S G K G L E W L (50)
hGPN18-2_VH1 (1)  Q I T L K E S G P T L V K P T Q T L T L T C S F S G F S L S T S V M G V G W V R Q P S G K G L E W L (50)
hGPN18-2_VH2 (1)  Q V Q L V Q S G P G L V Q P T Q T L T L T C S F S G F S L S T S V M G V G W I R Q P S G K G L E W L (50)
hGPN18-2_VH3 (1)  Q V T L K E S G P T L V K P T Q T L T L T C T F S G F S L S T S V M G V G W I R Q P S G K A L E W L (50)
hGPN18-2_VH4 (1)  Q V Q L Q Q S G P G L V Q P S Q T L S L T C S F S G F S L S T S V M G V G W I R Q S P S R G L E W L (50)
hGPN18-2_VH5 (1)  Q V T L K E S G P G L V K P T Q T L T L T C S F S G F S L S T S V M G V G W I R Q P S G K G L E W L (50)

GPN18-2_VH   (61) A D I W W D D D K D Y N P S L K S R L T I S K D T S K N Q V F L K I A S V D T A D T A T Y Y C A R T (100)
hGPN18-2_VH1 (61) A D I W W D D D K D Y N P S L K S R L T I A K D T S K N Q V F L K M T S V E P V D T A T Y Y C A R T (100)
hGPN18-2_VH2 (61) A D I W W D D D K D Y N P S L K S R L T I S K D T S K N Q V V L T M T N M D T V D T A T Y Y C A R T (100)
hGPN18-2_VH3 (61) A D I W W D D D K D Y N P S L K S R L T I T K D T S K N Q V F L K I A N M D T V D T A T Y Y C A R T (100)
hGPN18-2_VH4 (61) G D I W W D D D K D Y N P S L K S R I T I T A D T S K N Q F S L Q L N S V T P E D T A V Y Y C A R T (100)
hGPN18-2_VH5 (61) A D I W W D D D K D Y N P S L K S R L T I S K D T S K N Q V V L T M T N M D T A D T A T Y Y C A R T (100)

GPN18-2_VH   (101) Y Y S N Y E Y Y G M D Y W G Q G T S V T V S S (123)
hGPN18-2_VH1 (101) Y Y S N Y E Y Y G M D Y W G Q G T S V T V S S (123)
hGPN18-2_VH2 (101) Y Y S N Y E Y Y G M D Y W G Q G T L V T V S S (123)
hGPN18-2_VH3 (101) Y Y S N Y E Y Y G M D Y W G Q G T L V T V S S (123)
hGPN18-2_VH4 (101) Y Y S N Y E Y Y G M D Y W G Q G T S V T V S S (123)
hGPN18-2_VH5 (101) Y Y S N Y E Y Y G M D Y W G Q G T S V T V S S (123)
```

EP 4 772 193 A1

Figure 7

# Figure 8

```
GPN18-5_VH    (1)  Q V Q L Q Q S G A E L A K P G T S V K M S C K A S G Y T F T T Y W M N W V K Q R P G Q D L E W I G A (50)
hGPN18-5_VH1  (1)  Q V Q L V Q S G A E V K K P G A S V K V S C K A S G Y T F T T Y W M N W V R Q A P G Q G L E W I G A (50)
hGPN18-5_VH2  (1)  Q V Q L V Q S G A E V K K P G A S V K V S C K A S G Y T F T T Y W M N W V R Q A P G Q G L E W I G A (50)
hGPN18-5_VH3  (1)  Q V Q L V Q S G A E V K K P G A S V K V S C K A S G Y T F T T Y W M N W V R Q A P G Q G L E W I G A (50)

GPN18-5_VH   (51)  I N P S N D Y T E Y N Q K F K D K A I L T A D K S S N A Y M Q L S S L T S E D S A V Y Y C G R S Y (100)
hGPN18-5_VH1 (51)  I N P S N D Y T E Y N Q K F K D R V T M T A D T S T S T V Y M E L S S L R S E D T A V Y Y C G R S Y (100)
hGPN18-5_VH2 (51)  I N P S N D Y T E Y N Q K F K D R A T M T A D T S T S T V Y M E L S S L R S E D T A V Y Y C G R S Y (100)
hGPN18-5_VH3 (51)  I N P S N D Y T E Y N Q K F K D R A I M T A D K S T S T V Y M E L S S L R S E D T A V Y Y C G R S Y (100)

GPN18-5_VH  (101)  S P D Y W G Q G T T L T V S S    (115)
hGPN18-5_VH1 (101) S P D Y W G Q G T T V T V S S    (115)
hGPN18-5_VH2 (101) S P D Y W G Q G T T V T V S S    (115)
hGPN18-5_VH3 (101) S P D Y W G Q G T T V T V S S    (115)
```

Figure 9

```
GPN18-5_VL   (1)  D I V M T Q S Q K F M S T S V G D R V S V T C K A S Q N V D H N V A W H Q Q K P G Q S P K A L I Y S  (50)
hGPN18-5_VL1 (1)  D I Q M T Q S P S S L S A S V G D R V T I T C K A S Q N V D H N V A W F Q Q K P G K A P K A L I Y S  (50)
hGPN18-5_VL2 (1)  D I Q M T Q S P S S L S A S V G D R V T I T C K A S Q N V D H N V A W F Q Q K P G K A P K A L I Y S  (50)
hGPN18-5_VL3 (1)  D I Q M T Q S P S S L S A S V G D R V T I T C K A S Q N V D H N V A W H Q Q K P G K A P K A L I Y S  (50)

GPN18-5_VL   (51) A S Y R Y S G V P D R F T G S G S G T D F T L T I R D V Q S E D L A D Y F C Q Q Y K S Y P F T F G S  (100)
hGPN18-5_VL1 (51) A S Y R Y S G V P S R F S G S G S G T D F T L T I S S L Q P E D F A T Y Y F C Q Q Y K S Y P F T F G G  (100)
hGPN18-5_VL2 (51) A S Y R Y S G V P D R F S G S G S G T D F T L T I S S L Q P E D F A T Y Y F C Q Q Y K S Y P F T F G G  (100)
hGPN18-5_VL3 (51) A S Y R Y S G V P D R F T G S G S G T D F T L T I S S L Q P E D F A D Y F C Q Q Y K S Y P F T F G G  (100)

GPN18-5_VL   (101) G T E L E I K  (107)
hGPN18-5_VL1 (101) G T K L E I K  (107)
hGPN18-5_VL2 (101) G T K L E I K  (107)
hGPN18-5_VL3 (101) G T K L E I K  (107)
```

Figure 10

n=4-5, Mean+SEM
#p<0.05 vs. Sham, Wilcoxon rank sum test
*p<0.05 vs. Control, Steel's test

Figure 11

antibody or vehicle (i.v.)

6        14   15   16   17   18   19   (w)

C57BL/6
mice

HFD

Figure 12

Figure 13

## INTERNATIONAL SEARCH REPORT

| International application No. |
| --- |
| **PCT/JP2024/030861** |

**A. CLASSIFICATION OF SUBJECT MATTER**

*A61K 39/395*(2006.01)i; *A61P 3/00*(2006.01)i; *A61P 3/04*(2006.01)i; *A61P 3/10*(2006.01)i; *A61P 9/00*(2006.01)i; *A61P 9/10*(2006.01)i; *A61P 21/00*(2006.01)i; *A61P 25/02*(2006.01)i; *A61P 25/04*(2006.01)i; *A61P 29/00*(2006.01)i; *A61P 35/00*(2006.01)i; *A61P 37/06*(2006.01)i; *A61P 43/00*(2006.01)i; *C07K 16/28*(2006.01)i; *C12N 15/13*(2006.01)i; *C12N 15/63*(2006.01)i; *C12N 15/85*(2006.01)i; *C12P 21/08*(2006.01)i

FI: A61K39/395 D; C07K16/28; A61K39/395 N; A61P43/00 105; A61P3/04; A61P3/10; A61P9/10 101; A61P35/00; A61P3/00; A61P9/00; A61P37/06; A61P25/04; A61P25/02; A61P21/00; A61P29/00; C12N15/85 Z; C12N15/63 Z; C12P21/08; C12N15/13 ZNA

According to International Patent Classification (IPC) or to both national classification and IPC

**B. FIELDS SEARCHED**

Minimum documentation searched (classification system followed by classification symbols)

A61K39/395; A61P3/00; A61P3/04; A61P3/10; A61P9/00; A61P9/10; A61P21/00; A61P25/02; A61P25/04; A61P29/00; A61P35/00; A61P37/06; A61P43/00; C07K16/28; C12N15/13; C12N15/63; C12N15/85; C12P21/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); UniProt/GeneSeq

**C. DOCUMENTS CONSIDERED TO BE RELEVANT**

| Category* | Citation of document, with indication, where appropriate, of the relevant passages | Relevant to claim No. |
| --- | --- | --- |
| X | JONATHAN, M. et al. Functional TFEB activation characterizes multiple models of renal cystic disease and loss of polycystin-1. Am J Physiol Renal Physiol. 16 February 2023, vol. 324, pp. F404-F422, ISSN 1931-857X<br>MATERIALS AND METHODS, Antibodies, fig. 1 | 23, 25 |
| A | Invitrogen. Catalog # PA5-42585. GPNMB Polyclonal Antibody<br>Immunogen, Peptide sequence | 23, 25 |
| X | WO 2021/020047 A1 (NIIGATA UNIVERSITY) 04 February 2021 (2021-02-04)<br>claims, paragraphs [0007], [0045], examples, etc. | 1-35 |
| Y | | 1-35 |

☑ Further documents are listed in the continuation of Box C.  ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **07 November 2024** | **19 November 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
|---|
| **PCT/JP2024/030861** |

**C.    DOCUMENTS CONSIDERED TO BE RELEVANT**

| Category* | Citation of document, with indication, where appropriate, of the relevant passages | Relevant to claim No. |
|---|---|---|
| X | SUDA, M. et al. Senolytic vaccination improves normal and pathological age-related phenotypes and increases lifespan in progeroid mice. Nat Aging. 2021, vol. 1, pp. 1117-1126, ISSN 2662-8465<br>Abstract, Methods, Vaccination, Discussion, etc. | 1-35 |
| Y | | 1-35 |
| X | JP 2008-521411 A (CURAGEN CORPORATION) 26 June 2008 (2008-06-26)<br>claims, examples, etc. | 1-35 |
| X | ROSE, A. et al. Functional requirement for RGD and PKD domains of GPNMB in breast cancer metastasis. Cancer Res. 2008, vol. 68, no. 9, supply, p. 3681, ISSN 0008-5472<br>abstract | 1, 2, 4-22 |

Form PCT/ISA/210 (second sheet) (July 2022)

**INTERNATIONAL SEARCH REPORT**

International application No.

**PCT/JP2024/030861**

| **Box No. I** | **Nucleotide and/or amino acid sequence(s) (Continuation of item 1.c of the first sheet)** |
|---|---|

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT
### Information on patent family members

International application No.

**PCT/JP2024/030861**

| Patent document cited in search report | | | Publication date (day/month/year) | Patent family member(s) | | | Publication date (day/month/year) |
|---|---|---|---|---|---|---|---|
| WO | 2021/020047 | A1 | 04 February 2021 | US | 2022/0267374 | A1 | |
| | | | | claims, paragraphs [0009], [0078], examples | | | |
| | | | | EP | 3991743 | A1 | |
| JP | 2008-521411 | A | 26 June 2008 | US | 2013/0022597 | A1 | |
| | | | | claims, examples | | | |
| | | | | WO | 2006/071441 | A2 | |
| | | | | EP | 1827492 | A2 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021020047 A
- JP 6334496 A
- WO 2007053718 A


**Non-patent literature cited in the description**

- **ZHANG et al.** *J. Clin. Invest*, 2022, vol. 132 (15), e158450
- **KURT et al.** *J. Clin. Invest.*, 2023, vol. 133 (13), e170762
- **WEI et al.** *Front. Immunol.*, 2022, vol. 13, 1035276
- **GENG et al.** *Front. Cell Dev. Biol.*, 2023, vol. 11, 1158539
- **PINTO et al.** *Trends Immunol*, 2022, vol. 43 (11), 932-946
- **KLAIHMON et al.** *Int. J. Mol. Sci*, 2023, vol. 24 (13), 10508
- **KAIUCHI-KIYOTA et al.** *Leukemia*, 2022, vol. 36, 1006-1014
- **GATTO et al.** *Immunotherapy*, 2021, vol. 13 (11), 879-883
- **UNVER et al.** *Clin. Exp. Med.*, 2023
- **HUANG et al.** *Am. J. Cancer Res.*, 2021, vol. 11 (6), 2430-2455
- **MALAYAPERUMAL et al.** *Clin. Pract*, 2023, vol. 13, 838-852
- **ZHAO et al.** *Front. Immunol*, 2023, vol. 14, 1157537
- **JAHCHAN et al.** *Front. Immunol.*, 2019, vol. 10, 1611
- **BINNEWIES et al.** *Cell Reports*, 2021, vol. 37, 109844
- **HASTON et al.** *Cancer Cell*, 2023, vol. 41, 1242-1260
- **CHUNG et al.** *Clin. Cancer Res.*, 2020, vol. 26 (6), 1449-1459
- **SZULZEWSKY et al.** *PLoS ONE*, 2015, vol. 10 (2), e0116644
- **NAMBIAR et al.** *eBioMedicine*, 2023, vol. 90, 104481
- **LEASK et al.** *J. Cell Commun. and Signaling*, 2019, vol. 13, 441-442
- **HICKSON et al.** *EBioMedicine*, 2019, vol. 47, 446-456
- **SHIMIZU et al.** *Am. J. Pathol.*, 2022, vol. 192, 31-42
- **OGRODNIK et al.** *Nat. Commun*, 2017, vol. 8, 15691
- **WATANABE et al.** *Front. Immunol.*, 2022, vol. 13, 859502
- **COULIS et al.** *Sci. Adv.*, 2023, vol. 9, eadd9984
- **SUGIHARA et al.** *Sci. Rep*, 2020, vol. 10, 16385
- **YANG et al.** *Part. Fibre Toxicol*, 2023, vol. 20, 29
- **FABRE et al.** *Sci. Immunol.*, 2023, vol. 8, eadd8945
- **KATAYAMA et al.** *Sci. Rep*, 2015, vol. 5, 16920
- **SILVA et al.** *Exp. Dermatol.*, 2018, vol. 27, 630-635
- **DAI et al.** *Transl. Res*, 2023, vol. 255, 128-139
- **AN et al.** *J. Zheijang Univ-Sci. G.*, 2013, vol. 14 (11), 973-982
- **REYNOLDS et al.** *Front. Immunol.*, 2023, vol. 14, 1219598
- **CERVANTES et al.** *Front. Cardiovasc. Med.*, 2023, vol. 10, 1213177
- **HE et al.** *J. Trans. Med.*, 2023, vol. 21, 448
- **HUO et al.** *Front. Endocrinol*, 2023, vol. 14, 1110337
- **GONG et al.** *Nat. Metab.*, 2019, vol. 1 (5), 570-583
- **QUIN et al.** *Mol. Cell Biochem.*, 2023, vol. 478, 697-706
- **HAERDTNER et al.** *Atherosclerosis*, 2023, vol. 371, 1-13
- **VAN EIJK et al.** *Int. J. Mol. Sci.*, 2021, vol. 22, 4039
- **CHEN et al.** *Front. Immunol*, 2023, vol. 14, 1207100
- **LI et al.** *J. Leukoc. Biol.*, 2023, vol. 113, 139-148
- **JARVE et al.** *FASEB J*, 2017, vol. 31, 556-568
- **ESKES et al.** *Mol. Genet. Metab.*, 2023, vol. 139, 107631
- **SHIKANO et al.** *J. Biol. Chem.*, 2001, vol. 276 (11), 8125-8134
- **RIPOLL et al.** *J. Immunol.*, 2007, vol. 178, 6557-6566

- **TSE et al.** *Clin. Cancer Res.*, 2006, vol. 12 (4), 1373-1382
- **MARIC et al.** *OncoTargets Ther.*, 2013, vol. 6, 839-852
- **XIE et al.** *Cancer Sci.*, 2019, vol. 110 (7), 2237-2246
- **HEE et al.** *Scientific Reports*, 2017, vol. 7, 44064
- **BYCROFT et al.** *EMBO J.*, 1999, vol. 18 (2), 297-305
- **LI et al.** *FASEB J.*, 2010, vol. 24 (12), 4767-4781
- **ROBINET et al.** *Scientific Reports*, 2021, vol. 11, 10249
- **ANDERSON et al.** *Nat. Genet.*, 2002, vol. 30, 81-85
- **YANG et al.** *Am. J. Hum. Genet.*, 2018, vol. 102 (2), 219-232
- **MURTHY et al.** *Neurogenetics*, 2017, vol. 18, 121-133
- **DIAZ-ORTIZ et al.** *Science*, 2022, vol. 377, 833
- **GRENDZA et al.** *Neurobiol. Dis.*, 2021, vol. 159, 105494
- **ROSE et al.** *PLoS One*, 2010, vol. 5 (8), e12093
- **WANG et al.** *Int. J. Mol. Sci.*, 2021, vol. 22, 10843
- **NAKANO et al.** *Neuroscience*, 2014, vol. 277, 123-131
- **SAADE et al.** *Front. Immunol*, 2021, vol. 12, 674739
- **NEAL et al.** *J. Neuroinflammation*, 2018, vol. 15, 73
- **WANG et al.** *Cancer Sci.*, 2021, vol. 112, 4187-4197
- **PRABATA et al.** *J. Biol. Chem.*, 2021, vol. 297 (5), 101232
- **MURATA et al.** *J. Neurochem.*, 2015, vol. 132, 583-594
- **ZHU et al.** *Neurosci. Lett.*, 2022, vol. 767, 136300
- **SUDA et al.** *Nat. Aging*, 2021, vol. 1, 1117-1126
- **HANSSON et al.** *EMBO Mol. Med*, 2023, vol. 15, e16359
- **OH et al.** *Biomedicines*, 2023, vol. 11, 1250
- **TAGHIZADEH et al.** *Sci. Rep*, 2022, vol. 12, 7985
- **HUETTENRAUCH et al.** *Acta Neuropathol. Commun.*, 2018, vol. 6, 108
- **AICHHOLZER et al.** *Alzheimers Res. Ther.*, 2021, vol. 13, 94
- **VAHDAT et al.** *npj Breast Cancer*, 2021, vol. 7, 57
- **LI et al.** *Cancers*, 2023, vol. 15, 1589
- **ZHANG et al.** *Monoclon. Antibodies Immunodiagn. Immunother*, 2013, vol. 32 (4), 265-269
- **KAWAHARA et al.** *Glia*, 2016, vol. 64 (11), 1938-1961
- **CHUNG et al.** *J. Immunol.*, 2019, vol. 183 (5), 5190-5198
- **HUANG et al.** *Sci. Rep.*, 2021, vol. 11, 12171
- **HAN et al.** *Cancer Sci*, 2021, vol. 112, 1911-1923
- **SAKANO et al.** *Cancer Sci.*, 2022, vol. 113 (5), 1625-1638
- **KUAN et al.** *Clin. Cancer Res.*, 2006, vol. 12 (7), 1970-1982
- **WU et al.** *PNAS*, 2023, vol. 120 (9), e2210836120
- **FENG.** *Oncol. Lett*, 2020, vol. 20 (3), 2356-2368
- **OKITA et al.** *Oncotarget*, 2018, vol. 9 (99), 37289-37290
- **LAZARATOS et al.** *Oncogene*, 2022, vol. 41, 4573-4590
- **TOMIHARI et al.** *Cancer Res.*, 2010, vol. 70 (14), 5778-5787
- **GABRIEL et al.** *Diabetes*, 2014, vol. 63, 3310-3323
- **CHRYSTAL et al.** *Molecules*, 2021, vol. 26, 3529
- **ALEXANDER et al.** *Pigment Cell Melanoma Res.*, 2013, vol. 26 (4), 470-486
- **STROHL et al.** *Current Opinion in Biotechnology*, 2009, vol. 20, 685-691
- **LEE et al.** *Nat Commun.*, 06 November 2019, vol. 10 (1), 5031
- **KOUHI et al.** *Int. J. Mol. Sci.*, 2019, vol. 20, 3108
- **C. SPIESS et al.** *Molecular Immunolog*, 2015, vol. 67, 95-106
- **CHOCARRO et al.** *Biomedicines*, 2022, vol. 10, 3035
- **HENDRICKX et al.** *Front Immunol.*, 2017, vol. 8, 1810
- **HOU et al.** *J Mol Neurosci*, 2015, vol. 55, 533-540
- **TSOU et al.** *The FASEB Journal.*, 2020, vol. 34, 8810-8823
- **TAKETO TANAKA et al.** *Keio J. Med.*, 2011, vol. 60, 37-46
- *Proc. Natl. Acad. Sci. USA*, 1969, vol. 63 (1), 78-85
- Washington DC United States Department of Health and Human Services. NIH Publication, 1991
- *Nature*, 1983, vol. 305 (5934), 537-40
- *Nature*, 1992, vol. 356 (6365), 152-4
- *Nature*, 1975, vol. 256 (5517), 495-7
- *Cancer Gene Ther.*, 2007, vol. 14 (11), 904-17

- **LEFRANC, M.-P.** ; **POMMIE, C** ; **RUIZ, M** ; **GIUDI-CELLI, V** ; **FOULQUIER, E** ; **TRUONG, L** ; **THOU-VENIN-CONTET, V** ; **LEFRANC, G.** IMGT unique numbering for immunoglobulin and T cell receptor variable domains and Ig superfamily V-like domains. *Dev. Comp. Immunol.*, 2003, vol. 27, 55-77
- Remington: The Science and Practice of Pharmacy, 20th EDITION. Lippincott Williams & Wilkins, 2000
- **KOEHLER et al.** *Nature*, 1975, vol. 256, 495-497

- **LEFRANC, M.-P** ; **POMMIÉ, C.** ; **RUIZ, M** ; **GIUDI-CELLI, V** ; **FOULQUIER, E** ; **TRUONG, L** ; **THOU-VENIN-CONTET, V** ; **LEFRANC, G**. IMGT unique numbering for immunoglobulin and T cell receptor variable domains and Ig superfamily V-like domains. *Dev. Comp. Immunol.*, 2003, vol. 27, 55-77
- **LEFRANC, M.-P.** ; **POMMIÉ, C** ; **RUIZ, M** ; **GIUDI-CELLI, V** ; **FOULQUIER, E** ; **TRUONG, L.** ; **THOU-VENIN-CONTET, V.** ; **LEFRANC, G**. IMGT unique numbering for immunoglobulin and T cell receptor variable domains and Ig superfamily V-like domains. *Dev. Comp. Immunol*, 2003, vol. 27, 55-77
- **SELZER et al.** *Pain*, 1990, vol. 43 (2), 205-218
- **CHAPLAN et al.** *J. Neurosci. Methods*, 1994, vol. 53 (1), 55-63